# EUROPÄISCHE PATENTSCHRIFT

(11) **EP 2 693 878 B1**
(45) Veröffentlichungstag und Bekanntmachung des Hinweises auf die Patenterteilung: **27.06.2018**
(21) Anmeldenummer: 12709074.4
(22) Anmeldetag: 12.03.2012
(51) Int. Cl.: A01N 37/38, A01N 37/48, A01N 41/02, A01N 41/10, A01N 43/20, C07C 255/57, C07C 323/12, A01N 37/34, C07C 255/41

(54) **SUBSTITUIERTE (3R,4R)-4-CYAN-3,4-DIPHENYLBUTANOATE, VERFAHREN ZU DEREN HERSTELLUNG UND DEREN VERWENDUNG ALS HERBIZIDE UND PFLANZENWACHSTUMSREGULATOREN**
SUBSTITUTED (3R,4R)-4-CYAN-3,4-DIPHENYLBUTANOATES, METHOD FOR THE PRODUCTION THEREOF AND USE THEREOF AS HERBICIDES AND PLANT GROWTH REGULATORS
(3R,4R)-4-CYAN-3,4-DIPHÉNYLBUTANOATES SUBSTITUÉS, PROCÉDÉ DE PRÉPARATION DESDITS COMPOSÉS ET LEUR UTILISATION EN TANT QU'HERBICIDES ET RÉGULATEURS DE LA CROISSANCE DES PLANTES

(30) Priorität: 18.03.2011 EP 11158828; 18.03.2011 US 201161454143 P
(43) Veröffentlichungstag der Anmeldung: 12.02.2014
(73) Patentinhaber: Bayer Intellectual Property GmbH, 40789 Monheim am Rhein (DE)
(72) Erfinder: JAKOBI, Harald, 60598 Frankfurt (DE); MOSRIN, Marc, 65933 Frankfurt (DE); GATZWEILER, Elmar, 61231 Bad Nauheim (DE); HÄUSER-HAHN, Isolde, 51375 Leverkusen (DE); HEINEMANN, Ines, 65719 Hofheim (DE); ROSINGER, Christopher Hugh, 65719 Hofheim (DE); ANGERMANN, Alfred, 65830 Kriftel (DE); HOFFMANN, Michael Gerhard, 65439 Flörsheim (DE); SCHNATTERER, Stefan, 65795 Hattersheim (DE); ZEIß, Hans-Joachim, 65843 Sulzbach (DE)
(74) Vertreter: BIP Patents
(86) Internationale Anmeldenummer: PCT/EP2012/054292
(87) Internationale Veröffentlichungsnummer: WO 2012/126765

(56) Entgegenhaltungen:
- EP-A1- 0 266 725
- EP-A1- 0 270 830
- EP-A2- 0 005 341

## Beschreibung

Die Erfindung betrifft das technische Gebiet der Herbizide und Pflanzenwachstumsregulatoren, beispielsweise der Herbizide zur Bekämpfung von Unkräutern und Ungräsern in Nutzpflanzenkulturen oder der Pflanzenwachstumsregulatoren, welche zur Beeinflussung des Wachstums von Kulturpflanzen eingesetzt werden können.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Aus den veröffentlichten Patentanmeldungen EP-A-5341 (US 4,224,052), EP-A-266725, EP-A-270830, JP-04/297454, JP-04/297455, JP-05/058979, WO 2011/003775 A2 und WO 2011/003776 A2 sind herbizide Cyanobutyrate bekannt.

In den genannten Veröffentlichungen werden hinsichtlich der herbiziden Eigenschaften und der Kulturpflanzenverträglichkeit die Gemische der diastereomeren threo- und erythro-Formen oder die erythro- bzw. threo-Racemate der Cyanobutyrate genauer beschrieben.

EP-A-5341 beschreibt herbizide Ester und Amide von 4-Cyano-3,4-diphenyl-butansäuren, die an den Phenylresten gegebenfalls substitutiert sind. Gemäß EP-A-5341 eignen sich die threo-Isomere allgemein zur nicht-selektiven Bekämpfung von Schadpflanzen, während die erythro-threo-Isomerengemische für die selektive Bekämpfung von Schadpflanzen in einigen Nutzpflanzenkulturen in Betracht kommen. In EP-A-5341 wird außerdem erwähnt, dass die 2 Enantiomere, die zur threo-Form gehören, unterschiedlich wirksam sind, was am Beispiel der unterschiedlichen Wirkungen der Enantiomeren des Enantiomerenpaares der in den Phenylresten unsubstituierten 4-Cyano-3,4-diphenyl-butansäure untersucht worden ist.

Aus EP-A-266725 sind einige erythro-threo-Isomerengemische bekannt, die selektiv zur Unkrautkontrolle in Reiskulturen eingesetzt werden können.

EP-A-270830 beschreibt, dass threo-Isomeren und erythro-threo-Isomerengemische als Pflanzenregulatoren verwendet werden können und die Ausbildung eines Fruchtstands bei verschiedenen Schadgräsern verhindern können.

Aus WO 2011/003775 sind spezielle Ester von 4-Cyano-3,4-diphenyl-butansäuren bekannt, die als wirksame Herbizide, vorzugsweise auch in Nutzpflanzenkulturen eingesetzt werden können.

Aus WO 2011/003776 sind 4-Cyano-3,4-diphenyl-butansäuren und -säureester bekannt, die an den Phenylresten spezifisch substituiert sind und als wirksame Herbizide, vorzugsweise auch in Nutzpflanzenkulturen eingesetzt werden können. Abgesehen von EP-A-5341 beschränken sich die in der zitierten Literatur beschriebenen Untersuchungen auf die Anwendung von racemischen Gemischen.

Deren herbizide Wirkung, insbesondere bei niedrigen Aufwandmengen, bzw. deren Verträglichkeit gegenüber Kulturpflanzen bleiben jedoch verbesserungswürdig.

Aus den genannten Gründen besteht weiterhin ein Bedarf nach alternativen wirkungsstarken Herbiziden für die selektive Anwendung in Pflanzenkulturen oder die Anwendung auf Nichtkulturland. Auch ist es wünschenswert, alternative chemische Wirkstoffe bereitzustellen, die gegebenenfalls mit Vorteilen als Herbizide oder Pflanzenwachstumsregulatoren eingesetzt werden können.

Überraschenderweise wurde nun gefunden, dass bestimmte angereicherte optisch active threo-Verbindungen aus der Gruppe der genannten Cyanobutyrate besondere herbizide Wirkungen bei zugleich vorteilhaften Selektivitäten gegenüber einigen Nutzpflanzen aufweisen.

Eine Aufgabe der vorliegenden Erfindung ist die Bereitstellung von Verbindungen mit herbizider Wirkung, die bereits bei relativ niedrigen Aufwandmengen gegen wirtschaftlich wichtige Schadpflanzen hochwirksam sind und vorzugsweise bei guter Wirksamkeit gegen Schadpflanzen selektiv in Kulturpflanzen eingesetzt werden können.

Gegenstand der vorliegenden Erfindung sind Verbindungen der Formel (I) und deren Salze, jeweils in der optisch aktiven (3R, 4R)-threo-Form, 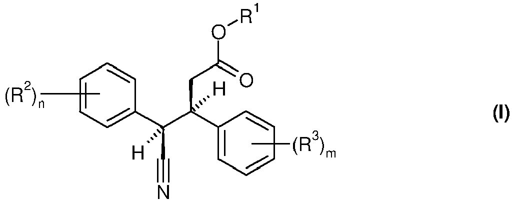 worin
- R¹: Wasserstoff oder einen hydrolysierbaren Rest bedeutet, vorzugsweise R¹ Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C-Atome, vorzugsweise 1 bis 24 C-Atome, insbesondere 1 bis 20 C-Atome aufweist, bedeutet oder
- R¹: einen Rest der Formel SiR^{a}R^{b}R^{c}, -NR^{a}R^{b} oder -N=CR^{c}R^{d} bedeutet, wobei in den letztgenannten 3 Formeln jeder der Reste R^{a}, R^{b}, R^{c} und R^{d} unabhängig voneinander Wasserstoff oder einen gegebenenfalls substituierten Kohlenwasserstoffrest bedeutet, dabei aber SiH₃ für SiR^{a}R^{b}R^{c} ausgenommen ist, oder R^{a} und R^{b} zusammen mit dem N-Atom einen 3- bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C-Atom einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten,
wobei jeder der Reste R^{a}, R^{b}, R^{c} und R^{d} inklusive Substituenten bis 30 C-Atome, vorzugsweise bis 24 C-Atome, insbesondere bis 20 C-Atome aufweist,
oder
- R¹: einen Rest der Formel -C(=O)-R^{e} oder -P(=O)(R^{f})₂ bedeutet, wobei R^{e} und die R^{f} unabhängig voneinander jeweils Wasserstoff, OH, (C₁-C₈)Alkyl, (C₁-C₄)Haloalkyl, (C₂-C₈)Alkenyl, (C₂-C₈)Alkinyl, (C₁-C₆)Alkoxy, (C₁-C₆)Alkoxy-(C₁-C₈)alkyl, (C₁-C₄)Haloalkoxy, (C₁-C₄)Haloalkoxy-(C₁-C₈)alkyl, (C₃-C₈)Alkenyloxy, (C₃-C₈)Alkenyloxy-(C₁-C₈)alkyl, (C₃-C₈)Alkinyloxy, (C₃-C₈)Alkinyloxy-(C₁-C₈)alkyl, -NR*R**, wobei R* und R** weiter unten definiert sind, Tri-[(C₁-C₄)alkyl]-silyl, Tri-[(C₁-C₄)alkyl]-silyl-(C₁-C₈)alkyl, (C₃-C₆)Cycloalkyl, (C₃-C₆)Cycloalkyl-(C₁-C₈)alkyl, (C₅-C₆)Cycloalkenyl, (C₅-C₆)Cycloalkenyl-(C₁-C₈)alkyl, (C₅-C₆)Cycloalkinyl, (C₅-C₆)Cycloalkinyl-(C₁-C₈)alkyl, Phenyl, Phenyl-(C₁-C₈)alkyl, Phenoxy, Phenoxy-(C₁-C₈)alkyl, Phenylamino, Phenylamino-(C₁-C₈)alkyl, einen Rest Het¹, Het¹-(C₁-C₆)alkyl oder Het¹-O-(C₁-C₆)alkyl, wobei der heterocyclische Rest Het¹ weiter unten definiert ist, bedeuten,
wobei jeder der letztgenannten 15 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
- (R²)ₙ: n Substituenten R² bedeutet,
wobei R² (wenn n = 1) oder jeder der Substituenten R² (wenn n größer als 1) unabhängig voneinander Halogen, Cyano, Nitro, (C₁-C₈)Alkyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, Tri-[(C₁-C₄)alkyl]-silyl oder Tri-[(C₁-C₄)alkyl]-silyl-(C₁-C₄)alkyl bedeutet
oder wobei jeweils zwei am Ring ortho-ständige Gruppen R² gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht, wobei die Gruppe -Z¹-A*-Z² zusammen mit den an die Gruppe gebundenen C-Atomen des Phenylrings einen ankondensierten 5 oder 6 Ring bilden, und
- (R³)ₘ: m Substituenten R³ bedeutet,
wobei R³ (wenn m = 1) oder jeder der Substituenten R³ (wenn m größer als 1) unabhängig voneinander Halogen, Cyano, Nitro, (C₁-C₈)Alkyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, NR*R**, wobei R* und R** weiter unten definiert sind, Tri-[(C₁-C₄)alkyl]-silyl oder Tri-[(C₁-C₄)alkyl]-silyl-(C₁-C₄)alkyl bedeutet
oder wobei jeweils zwei am Ring ortho-ständige Gruppen R³ gemeinsam eine Gruppe der Formel -Z³-A**-Z⁴ bedeuten, in welcher
- A**: für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
- Z³: für eine direkte Bindung, O oder S steht und
- Z⁴: für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden,
wobei in den obengenannten und den nachfolgenden Resten
- Het¹: jeweils unabhängig voneinander einen gesättigten, teilweise ungesättigten oder heteroaromatischen monocyclischen Heterocyclylrest mit 3 bis 9 Ringatomen oder einen 9- oder 10-gliedrigen bicyclischen Heterocyclus, jeweils enthaltend 1, 2, 3 oder 4 Heteroatome, ausgewählt aus der Gruppe bestehend aus O, N und S, bedeutet,
- R*, R**: jeweils unabhängig voneinander (unabhängig auch von anderen Resten NR*R**) H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste R^{bb} substituiert ist, bedeuten oder
- R* und R**: zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
- R^{A}: Halogen, Cyano, Hydroxy oder (C₁-C₆)Alkoxy bedeutet,
- R^{B}: Halogen, Cyano, Hydroxy, Oxo, Nitro, (C₁-C₈)Alkyl, (C₁-C₆)Haloalkyl, Cyano-(C₁-C₆)alkyl, Hydroxy-(C₁-C₆)alkyl, Nitro-(C₁-C₆)alkyl, (C₂-C₈)Alkenyl, (C₂-C₈)Haloalkenyl, (C₂-C₈)Alkinyl, (C₂-C₈)Haloalkinyl, (C₁-C₈)Alkoxy, (C₂-C₈)Alkenyloxy, (C₂-C₈)Alkinyloxy, (C₁-C₈)Haloalkoxy, (C₁-C₆)Alkoxy-(C₁-C₆)alkyl, (C₁-C₆)Alkoxy-(C₁-C₄)alkoxy, (C₁-C₆)Haloalkoxy-(C₁-C₄)alkyl, (C₁-C₆)Haloalkoxy-(C₁-C₄)alkoxy, (C₁-C₈)Alkylthio, (C₂-C₆)Alkenylthio, (C₂-C₆)Alkinylthio, (C₁-C₈)Alkylsulfinyl, (C₁-C₆)Haloalkylsulfinyl, (C₁-C₈)Alkylsulfonyl, (C₁-C₆)Haloalkylsulfonyl, einen Rest der Formel R^{aa}-C(=O)-, R^{aa}-C(=O)-(C₁-C₆)alkyl, wobei die R^{aa} weiter unten definiert sind, -NR*R**, wobei R* und R** weiter unten definiert sind, Tri-[(C₁-C₄)alkyl]-silyl, Tri-[(C₁-C₄)alkyl]-silyl-(C₁-C₆)alkyl, (C₃-C₈)Cycloalkyl, (C₃-C₈)Cycloalkoxy, (C₃-C₈)Cycloalkyl-(C₁-C₈)alkyl, (C₃-C₈)Cycloalkyl-(C₁-C₈)alkoxy, Phenyl, Phenyl-(C₁-C₈)alkyl, Phenoxy, Phenoxy-(C₁-C₈)alkyl, Phenylamino, Phenylamino-(C₁-C₈)alkyl oder einen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, wobei jeder der letztgenannten 11 Reste im cyclischen Teil gegebenenfalls durch einen oder mehrere gleiche oder verschiedene Reste R^{bb} substituiert ist, bedeutet,
- R^{aa}: jeweils unabhängig voneinander Wasserstoff, OH, (C₁-C₈)Alkyl, (C₁-C₆)Haloalkyl, (C₂-C₈)Alkenyl, (C₂-C₈)Alkinyl, (C₁-C₈)Alkoxy, (C₁-C₆)Alkoxy-(C₁-C₆)alkyl, (C₁-C₆)Alkoxy-(C₁-C₆)alkyloxy, (C₁-C₆)Haloalkoxy, (C₁-C₆)Haloalkoxy-(C₁-C₆)alkyl, (C₁-C₆)Haloalkoxy-(C₁-C₆)alkoxy, (C₃-C₈)Alkenyloxy, (C₃-C₈)Alkenyloxy-(C₁-C₆)alkyl, (C₃-C₈)Alkenyloxy-(C₁-C₆)alkoxy, (C₃-C₈)Alkinyloxy, (C₃-C₈)Alkinyloxy-(C₁-C₆)alkyl, (C₃-C₈)Alkinyloxy-(C₁-C₆)alkoxy, -NR*R*, wobei R* und R** wie oben definiert sind, Tri-[(C₁-C₄)alkyl]-silyl, Tri-[(C₁-C₄)alkyl]-silyl-(C₁-C₆)alkyl, Tri-[(C₁-C₄)alkyl]-silyl-(C₁-C₆)alkoxy, (C₃-C₆)Cycloalkyl, (C₃-C₈)Cycloalkoxy, (C₃-C₈)Cycloalkyl-(C₁-C₈)alkyl, (C₃-C₈)Cycloalkyl-(C₁-C₈)alkoxy, (C₅-C₈)Cycloalkenyl, (C₅-C₈)Cycloalkenyl-(C₁-C₆)alkyl, (C₅-C₈)Cycloalkenyloxy, (C₅-C₈)Cycloalkinyl, (C₅-C₈)Cycloalkinyl-(C₁-C₆)alkyl, (C₅-C₈)Cycloalkinyl-(C₁-C₆)alkoxy, Phenyl, Phenyl-(C₁-C₈)alkyl, Phenyl-(C₁-C₈)alkoxy, Phenoxy, Phenoxy-(C₁-C₈)alkyl, Phenoxy-(C₁-C₈)alkoxy, Phenylamino, Phenylamino-(C₁-C₈)alkyl, Phenylamino-(C₁-C₈)alkoxy oder einen gegebenenfalls über eine Alkylengruppe oder eine Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 20 Reste im cyclischen Teil gegebenenfalls durch einen oder mehrere gleiche oder verschiedene Reste R^{bb} substituiert ist, bedeutet und
- R^{bb}: jeweils unabhängig voneinander Halogen, (C₁-C₄)Alkyl, (C₁-C₄)Haloalkyl, (C₁-C₄)Alkoxy oder (C₁-C₄)Haloalkoxyoder im Fall gesättigter oder teilungesättigter cyclischer Basisgruppen auch Oxo bedeutet und
- n, m: jeweils unabhängig voneinander 0, 1, 2, 3, 4 oder 5, vorzugsweise 0, 1, 2, oder 3 bedeuten, mit der Maßgabe, dass nicht beide gleichzeitig 0 bedeuten,
wobei die stereochemische Konfiguration am C-Atom in Position 3 des Butansäurederivats eine stereochemische Reinheit von 60 bis 100 % (*R*), vorzugsweise 70 bis 100 % (*R*), mehr bevorzugt 80 bis 100 % (*R*), insbesondere 90 bis 100 % (*R*), bezogen auf das vorliegende Gemisch der threo-Enantiomeren aufweist und
die stereochemische Konfiguration am C-Atom in Position 4 des Butansäurederivats eine stereochemische Reinheit von 60 bis 100 % (*R*), vorzugsweise 70 bis 100 % (*R*), mehr bevorzugt 80 bis 100 % (*R*), insbesondere 90 bis 100 % (*R*), bezogen auf das vorliegende Gemisch der threo-Enantiomeren aufweist.

In der Formel (I) bedeutet die Formel "(R²)ₙ" n Reste R², die als Substituenten am betreffenden Phenylring gebunden sind, wobei die Reste im Falle n größer 1 gleich oder verschieden sein können und die jeweils näher genannte Bedeutung haben. Im Fall n = 0 ist der betreffende Phenylring nicht durch Substituenten R² substituiert, d. h. alle Ring-C-Atome des Phenylrings in den Positionen 2 bis 6 sind mit einem Wasserstoffatom verbunden. Entsprechendes gilt für die Substitution des anderen Phenylrings gemäß der Formel (R³)ₘ.

Die Verbindungen der Formel (I) können im Falle R¹ = H oder im Falle geeigneter saurer Substituenten durch Umsetzung mit Basen Salze bilden, wobei der saure Wasserstoff durch ein für die Landwirtschaft geeignetes Kation ersetzt wird.

Die Verbindungen der Formel (I) können durch Anlagerung einer geeigneten anorganischen oder organischen Säure, an eine basische Gruppe, wie z.B. Amino oder Alkylamino, Salze bilden. Geeignete vorhandene saure Gruppen, wie z.B. Carbonsäuregruppen, können innere Salze mit ihrerseits protonierbaren Gruppen, wie Aminogruppen bilden.

Die Verbindungen der Formel (I) können vorzugsweise in Form landwirtschaftlich einsetzbarer Salze vorliegen, wobei es ansonsten auf die Art des Salzes in der Regel nicht ankommt. Im Allgemeinen kommen dabei die Salze derjenigen Kationen oder die Säureadditionssalze derjenigen Säuren in Betracht, deren Kationen, beziehungsweise Anionen, die herbizide Wirkung der Verbindungen (I) nicht negativ beeinträchtigen.

Es kommen als Kationen insbesondere Ionen der Alkalimetalle, vorzugsweise Lithium, Natrium oder Kalium, der Erdalkalimetalle, vorzugsweise Calcium oder Magnesium, und der Übergangsmetalle, vorzugsweise Mangan, Kupfer, Zink oder Eisen in Betracht. Ebenso kann als Kation Ammonium oder substituiertes Ammonium verwendet werden, wobei hier ein bis vier Wasserstoffatome durch (C₁-C₄)Alkyl, Hydroxy-(C₁-C₄)alkyl, (C₁-C₄)Alkoxy-(C₁-C₄)alkyl, Hydroxy-(C₁-C₄)alkoxy-(C₁-C₄)alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium. Des Weiteren kommen Phosphoniumionen, Sulfoniumionen, vorzugsweise Tri(C₁-C₄)alkyl-sulfonium, insbesondere Trimethylsulfonium, oder Sulfoxoniumionen, vorzugsweise Tri(C₁-C₄)alkyl-sulfoxonium, insbesondere Trimethylsulfoxonium, in Betracht. Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von (C₁-C₄)Alkansäuren, vorzugsweise Formiat, Acetat, Propionat, Butyrat oder Trifluoracetat.

In der Formel (I) und allen nachfolgenden Formeln werden Bezeichnungen für chemische Reste verwendet, die Sammelbegriffe für die Aufzählung individueller Gruppenmitglieder darstellen oder individuelle chemische Reste spezifisch bezeichnen. In der Regel werden Bezeichnungen verwendet, die dem Fachmann geläufig sind und/oder insbesondere die nachstehend erläuterten Bedeutungen haben.

Ein hydrolysierbarer Rest (siehe Definition von R¹) bedeutet einen unter den Anwendungsbedingungen hydrolysierbaren Rest, beispielsweise einen schon in der Spritzbrühe oder insbesondere unter den physiologischen Bedingungen in Pflanzen hydrolysierbaren Rest, wobei eine Verbindung der Formel (I) mit der Carbonsäureestergruppe -CO-OR¹ (R¹ ungleich Wasserstoff) zur Verbindung der Formel (I) mit der Carbonsäuregruppe -CO-OH (d. h. Verbindung (I) mit R¹ = H) hydrolysiert wird. In der Definition der hydrolysierbaren Reste sind auch ausdrücklich die Reste mit R¹ = Kohlenwasserstoffrest oder Heterocyclylrest, wobei die beiden letztgenannten Reste unsubstituiert oder substituiert sind, umfasst, auch wenn sie teilweise vergleichsweise langsam hydrolysierbar sind.

Ein Kohlenwasserstoffrest ist ein aliphatischer, cycloaliphatischer oder aromatischer monocyclischer oder, im Falle eines gegebenenfalls substituierten Kohlenwasserstoffrestes, auch ein bicyclischer oder polycyclischer organischer Rest auf Basis der Elemente Kohlenstoff und Wasserstoff, beispielsweise umfassend die Reste Alkyl, Alkenyl, Alkinyl, Cycloalkyl, Cycloalkenyl, Aryl, Phenyl, Naphthyl, Indanyl, Indenyl, etc.; Entsprechendes gilt für Kohlenwasserstoffreste in zusammengesetzte Bedeutungen wie Kohlenwasserstoffoxyresten oder anderen über Heteroatomgruppen gebundene Kohlenwasserstoffresten.

Wenn nicht näher definiert, weisen die Kohlenwasserstoffreste vorzugsweise 1 bis 20 C-Atome, weiter bevorzugt 1 bis 16 C-Atome, insbesondere 1 bis 12 C-Atome auf.

Die Kohlenwasserstoffreste, auch in den speziellen Resten Alkyl, Alkoxy, Haloalkyl, Haloalkoxy, Alkylamino und Alkylthio, sowie die entsprechenden ungesättigten und/oder substituierten Reste können im Kohlenstoffgerüst jeweils geradkettig oder verzweigt sein.

Der Ausdruck "(C₁-C₄)Alkyl" bedeutet eine Kurzschreibweise für Alkyl mit einem bis 4 Kohlenstoffatomen, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl" umfassen entsprechend auch gradkettige oder verzweigte Alkylreste mit einer größeren Zahl von C-Atomen, d. h. gemäß Beispiel auch die Alkylreste mit 5 und 6 C-Atomen. Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl group, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl. Alkenyl schließt insbesondere auch geradkettige oder verweigte Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl;

Alkinyl bedeutet z.B. Propargyl, But-2-in-1-yl, But-3-in-1-yl, 1-Methyl-but-3-in-1-yl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Ein 3- bis 9-gliedriger carbocyclischer Ring bedeutet (C₃-C₉)Cycloalkyl oder (C₅-C₉)Cycloalkenyl.
(C₃-C₉)Cycloalkyl bedeutet ein carbocyclisches, gesättigtes Ringsystem mit vorzugsweise 3-9 C-Atomen, z.B. Cyclopropyl, Cyclobutyl, Cyclopentyl, Cyclohexyl, Cycloheptyl, Cyclooctyl oder Cyclononyl. Im Falle von substituiertem Cycloalkyl werden cyclische Systeme mit Substituenten umfasst, wobei die Substituenten auch mit einer Doppelbindung am Cycloalkylrest, z. B. eine Alkylidengruppe wie Methyliden, gebunden sein können.
(C₅-C₉)Cycloalkenyl bedeutet ein carbocyclisches, nicht aromatisches, partiell ungesättigtes Ringsystem mit 5-9 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Halogen bedeutet beispielsweise Fluor, Chlor, Brom oder Iod. Haloalkyl, -alkenyl und -alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl, Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Im Falle gegebenenfalls substituiertes Aryl sind auch mehrcyclische Systeme, wie Tetrahydronaphthyl, Indenyl, Indanyl, Fluorenyl, Biphenylyl, umfasst, wobei die Bindungsstelle am aromatischen System ist.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P, B, Si, Se) der gesättigt, ungesättigt oder heteroaromatisch ist und dabei unsubstituiert oder substituiert sein kann, wobei die Bindungsstelle an einem Ringatom lokalisiert ist. Wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält.

Ist der Heterocyclylrest oder der heterocyclische Ring gegebenenfalls substituiert, kann er mit anderen carbocyclischen oder heterocyclischen Ringen annelliert sein. Bevorzugt sind benzokondensierte (benzoannellierte) heterocyclische bzw. heteroaromatische Ringe.

Im Falle von gegebenenfalls substituiertem Heterocyclyl werden auch mehrcyclische Systeme umfasst, wie beispielsweise 8-Aza-bicyclo[3.2.1]octanyl oder 1-Azabicyclo[2.2.1]heptyl.

Im Falle von gegebenenfalls substituiertem Heterocyclyl werden auch spirocyclische Systeme umfasst, wie beispielsweise 1-Oxa-5-aza-spiro[2.3]hexyl.

Vorzugsweise ist er ein Rest eines heteroaromatischen Rings mit einem Heteroatom aus der Gruppe N, O und S, beispielsweise der Rest eines Fünf- oder Sechsrings, wie Pyridyl, Pyrrolyl, Thienyl oder Furyl;
weiterhin bevorzugt ist er ein Rest eines entsprechenden heteroaromatischen Rings mit 2, 3 oder 4 Heteroatomen, z. B. Pyrimidinyl, Pyridazinyl, Pyrazinyl, Triazinyl, Tetrazinyl, Thiazolyl, Thiadiazolyl, Oxazolyl, Isoxazolyl, Pyrazolyl, Imidazolyl oder Triazolyl oder Tetrazolyl.

Bevorzugt ist er dabei ein Rest eines heteroaromatischen Fünf- oder Sechsrings mit 1 bis 4 Heteroatomen, wie z. B. 1,2,3-Triazolyl, 1,2,4-Triazolyl, Tetrazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Tetrazolyl, 1,2,3-Triazinyl, 1,2,4-Triazinyl, 1,3,5-Triazinyl, 1,2,3,4-Tetrazinyl, 1,2,3,5-Tetrazinyl, 1,2,4,5-Tetrazinyl, Thiazolyl, Isothiazolyl, Oxazolyl, Isoxazolyl, Pyrazolyl, Imidazolyl.

Weiter bevorzugt sind dabei heteroaromatische Reste von Fünfring-Heterocyclen mit 3 N-Atomen, wie 1,2,3-Triazol-1-yl, 1,2,3-Triazol-4-yl, 1,2,3-Triazol-5-yl, 1,2,5-Triazol-1-yl, 1,2,5-Triazol-3-yl, 1,3,4-Triazol-1-yl, 1,3,4-Triazol-2-yl, 1,2,4-Triazol-3-yl, 1,2,4-Triazol-5-yl;
weiter bevorzugt sind auch heteroaromatische Reste von Sechsring-Heterocyclen mit 3 N-Atomen, wie 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl;
weiter bevorzugt sind auch heteroaromatische Reste von Fünfring-Heterocyclen mit zwei N-Atomen und einem O-Atom, wie 1,2,4-Oxadiazol-3-yl; 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl,
weiter bevorzugt sind auch heteroaromatische Reste von Fünfring-Heterocyclen mit zwei N-Atomen und einem S-Atom, wie 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl;
weiter bevorzugt sind auch heteroaromatische Reste von Fünfring-Heterocyclen mit vier N-Atomen, wie 1,2,3,4-Tetrazol-1-yl, 1,2,3,4-Tetrazol-5-yl, 1,2,3,5-Tetrazol-1-yl, 1,2,3,5-Tetrazol-4-yl, 2*H*-1,2,3,4-Tetrazol-5-yl, 1*H*-1,2,3,4-Tetrazol-5-yl,
weiter bevorzugt sind auch heteroaromatische Reste von Sechsring-Heterocyclen, wie 1,2,4,5-Tetrazin-3-yl;
weiter bevorzugt sind auch heteroaromatische Reste von Fünfring-Heterocyclen mit drei N-Atomen und einem O- oder S-Atom, wie 1,2,3,4-Oxatriazol-5-yl; 1,2,3,5-Oxatriazol-4-yl; 1,2,3,4-Thiatriazol-5-yl;1,2,3,5-Thiatriazol-4-yl;
weiter bevorzugt sind auch heteroaromatische Reste von Sechsring-Heterocyclen, wie beispielsweise 1,2,4,6-Thiatriazin-1-yl; 1,2,4,6-Thiatriazin-3-yl; 1,2,4,6-Thiatriazin-5-yl.

Weiterhin bevorzugt ist der heterocyclische Rest oder Ring ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl,

Weiterhin bevorzugt ist er auch ein partiell oder vollständig hydrierter heterocyclischer Rest mit 2 Heteroatomen aus der Gruppe N, O und S, beispielsweise Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidyl, Piperidyl, insbesondere Oxiranyl, 2-Oxetanyl, 3-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl.

Bevorzugte heterocyclische Reste sind auch benzokondensierte oder benzoannellierte heteroaromatische Ringe, beispielsweise Benzofuryl, Benzisofuryl, Benzothiophenyl, Benzisothiophenyl, Isobenzothiophenyl, Indolyl, Isoindolyl, Indazolyl, Benzimidazolyl, Benztriazolyl, Benzoxazolyl, 1,2-Benzisoxazolyl, 2,1-Benzisoxazolyl, Benzothiazolyl, 1,2-Benzisothiazolyl, 2,1-Benzoisothiazolyl, 1,2,3-Benzoxadiazolyl, 2,1,3-Benzoxadiazolyl, 1,2,3-Benzothiadiazolyl, 2,1,3-Benzothiadiazolyl, Chinolyl (Chinolinyl), Isochinolyl (Isochinolinyl), Chinnolinyl, Phtalazinyl, Chinazolinyl, Chinoxalinyl, Naphthyridinyl, Benzotriazinyl, Purinyl, Pteridinyl, Indolizinyl, Benzo-1,3-dioxylyl, 4H-Benzo-1,3-dioxinyl, und 4H-Benzo-1,4-dioxinyl, und wo es möglich ist, N-Oxide und Salze davon.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Alkylsulfonyl und, im Falle cyclischer Reste, auch Alkyl, Haloalkyl, Alkylthio-alkyl, Alkoxy-alkyl, gegebenfalls substituiertes Mono- und Dialkylaminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfasst, die mit einer Doppelbindung am Ring gebunden sind, z. B. mit einer Alkylidengruppe wie Methyliden oder Ethyliden substituiert sind.

Der Ausdruck "Reste aus der Gruppe (gefolgt von der Gruppe = Liste der Substituenten)", wo immer verwendet, soll gleichbedeutend sein mit "Reste ausgewählt aus der Gruppe (...)" oder "Reste ausgewählt aus der Gruppe bestehend aus (...)".

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Mit "Basisrest" wird der jeweilige Grundkörper eines Restes bezeichnet, an dem Substituenten einer Substituentenebene gebunden sind.

Bevorzugte Substituenten für die Substituentenebenen sind beispielsweise Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkylaminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.

Zwei Substituenten auch gemeinsam eine gesättigte oder ungesättigte Kohlenwasserstoff-Brücke oder eine entsprechende Brücke, in denen C-Atome, CH-Gruppen oder CH₂-Gruppen durch Heteroatome ersetzt sind, bilden und damit einen ankondensierten oder annellierten Cyclus bilden. Bevorzugt werden dabei benzokondensierte Systeme auf Basis der Grundkörper gebildet.

Gegebenenfalls substituiertes Phenyl bedeutet vorzugsweise Phenyl oder Phenyl, das unsubstituiert oder durch einen oder mehrere Reste aus der Gruppe Halogen, Cyano, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkoxy-(C₁-C₄)alkoxy, (C₁-C₄)Alkylthio und Nitro substituiert ist, insbesondere Phenyl, das gegebenenfalls durch einen oder mehrere Reste aus der Gruppe Halogen, (C₁-C₄)Alkyl, (C₁-C₄)Haloalkyl und (C₁-C₄)Alkoxy substituiert ist.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren oder Phosphinsäuren.

Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch einen oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.

Weiter bevorzugt bedeutet Acyl einen Alkanoylrest mit 1 bis 6 C-Atomen, insbesondere 1 bis 4 C-Atomen. (C₁-C₄)Alkanoyl bedeutet dabei den Rest einer Alkansäure mit 1 bis 4 C-Atomen nach Abtrennen der OH-Gruppe der Säuregruppe, d.h. Formyl, Acetyl, n-Propionyl, i-Propionyl oder n-, i-, sec. oder tert.-Butanoyl.

Die "yl-Position" eines Restes bezeichnet das C-Atom mit der freien Bindung.

Erfindungsgemäße bzw. erfindungsgemäß verwendete Verbindungen der Formel (I) und/oder deren Salze werden abgekürzt auch als "Verbindungen (I)" bezeichnet.

Gegenstand der Erfindung sind auch alle Stereoisomeren, die von Formel (I) umfasst sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfasst und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Gegenstand der Erfindung sind auch alle Tautomere der Verbindungen der Formel (I), die durch Verschiebung eines Wasserstoffatoms entstehen können (z. B. Keto-Enol-Tautomere). Die Tautomere sind ebenfalls von der Verbindung der Formel (I) umfasst, auch wenn die Formel (I) nur eines der jeweiligen im Gleichgewicht stehenden bzw. ineinander umwandelbaren Tautomere formal richtig beschreibt.

Die Verbindungen der Formel (I) umfassen auch alle physikalischen Formen, in denen diese in Reinsubstanz oder gegebenenfalls in Mischung mit anderen Stoffen auftreten können, insbesondere auch polymorphe Kristallformen der Verbindungen der Formel (I) oder deren Salze oder Lösungsmitteladditionsverbindungen (z. B. Hydrate).

Vor allem aus den Gründen der höheren herbiziden Wirkung, besseren Selektivität und/oder besseren Herstellbarkeit sind erfindungsgemäße Verbindungen der genannten Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Unabhängig von den jeweils anderen Resten aus der Gruppe R¹, (R²)ₙ und (R³)ₘ und der den allgemeinen Resten entsprechenden Unterbedeutungen, vorzugsweise in Kombination mit bevorzugten Bedeutungen von einem oder mehreren dieser Reste, sind erfindungsgemäße Verbindungen bzw. erfindungsgemäße Verwendungen von Verbindungen mit nachfolgend aufgeführten bevorzugten Bedeutungen der betreffenden Reste von besonderem Interesse.

Bevorzugt sind die erfindungsgemäßen Verbindungen der Formel (I) oder deren Salze, in welcher
- R¹: Wasserstoff, Alkyl, Alkenyl oder Alkinyl, wobei jeder der letztgenannten 3 Reste unsubstituiert oder substituiert ist und inklusive Substituenten bis zu 30 C-Atome, vorzugsweise bis 24 C-Atome, insbesondere bis 20 C-Atome aufweist, oder
Cycloalkyl, Cycloalkenyl, Cycloalkinyl oder Aryl, wobei jeder der letztgenannten 4 Reste unsubstituiert oder substituiert ist und inklusive Substituenten bis zu 30 C-Atome, vorzugsweise bis 24 C-Atome, insbesondere bis 20 C-Atome aufweist, oder
einen Heterocyclylrest mit 3 bis 9 Ringatomen, der 1 bis 4 Heteroatome aus der Gruppe N, O und S enthält und der unsubstituiert oder substituiert ist und der inklusive Substituenten 1 bis 30 C-Atome, vorzugsweise 1 bis 24 C-Atome, insbesondere 1 bis 20 C-Atome aufweist, bedeutet.

Weiter bevorzugt sind dabei auch Verbindungen (I) oder deren Salze, worin
- R¹: Wasserstoff bedeutet.

Weiter bevorzugt sind dabei auch Verbindungen (I) oder deren Salze, worin
- R¹: H, (C₁-C₁₈)Alkyl, (C₂-C₁₈)Alkenyl oder (C₂-C₁₈)Alkinyl, wobei jeder der letztgenannten 3 Reste unsubstituiert oder substituiert ist und inklusive Substituenten bis zu 30 C-Atome, vorzugsweise bis 24 C-Atome, insbesondere bis 20 C-Atome aufweist, oder (C₃-C₉)Cycloalkyl, (C₅-C₉)Cycloalkenyl, (C₅-C₉)Cycloalkinyl oder Phenyl, wobei jeder der letztgenannten 4 Reste unsubstituiert oder substituiert ist und inklusive Substituenten bis zu 30 C-Atome, vorzugsweise bis 24 C-Atome, insbesondere bis 20 C-Atome aufweist, bedeutet.

Weiter bevorzugt sind dabei auch Verbindungen (I) oder deren Salze, worin
- R¹: Wasserstoff, (C₁-C₁₈)Alkyl, (C₂-C₁₈)Alkenyl oder (C₂-C₁₈)Alkinyl, wobei jeder der letztgenannten 3 Reste unsubstituiert oder durch einen oder mehrere Reste aus der Gruppe, die aus den Resten [Untergruppen (a)-(d)]
(a) Halogen, Cyano, Thio, Nitro, Hydroxy, Carboxy, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, (C₁-C₈)Haloalkylthio, (C₂-C₈)Haloalkenylthio, (C₂-C₈)Haloalkinylthio, (C₁-C₈)Alkylsulfinyl, (C₂-C₈)Alkenylsulfinyl, (C₂-C₈)Alkinylsulfinyl, (C₁-C₈)Haloalkylsulfinyl, (C₂-C₈)Haloalkenylsulfinyl, (C₂-C₈)Haloalkinylsulfinyl, (C₁-C₈)Alkylsulfonyl, (C₂-C₈)Alkenylsulfonyl, (C₂-C₈)Alkinylsulfonyl, (C₁-C₈)Haloalkylsulfonyl, (C₂-C₈)Haloalkenylsulfonyl, (C₂-C₈)Haloalkinylsulfonyl, Reste der Formel -NR^{*}R^{**}, wobei R^{*} und R^{**} weiter unten definiert sind, und
   (C₃-C₈)Cycloalkyl, (C₅-C₈)Cycloalkenyl, (C₅-C₈)Cycloalkinyl, (C₃-C₈)Cycloalkyl-(C₁-C₆)alkoxy, (C₃-C₈)Cycloalkyl-(C₁-C₆)alkyl-S(O)ₚ-, (C₅-C₈)Cycloalkenyl-(C₁-C₆)alkoxy, (C₅-C₈)Cycloalkenyl-(C₁-C₆)alkyl-S(O)ₚ-, (C₅-C₈)Cycloalkinyl-(C₁-C₆)alkoxy, (C₅-C₈)Cycloalkinyl-(C₁-C₆)alkyl-S(O)ₚ-, (C₃-C₈)Cycloalkoxy, (C₃-C₈)Cycloalkyl-S(O)ₚ-, (C₅-C₈)Cycloalkenyloxy, (C₅-C₈)Cycloalkenyl-S(O)ₚ-, (C₅-C₈)Cycloalkinyloxy, (C₅-C₈)Cycloalkinyl-S(O)ₚ-, (C₃-C₈)Cycloalkoxy-(C₁-C₆)alkoxy, (C₃-C₈)Cycloalkoxy-(C₁-C₆)alkyl-S(O)ₚ-, Phenyl, Phenyl-(C₁-C₆)alkoxy, Phenoxy, Phenyl-S(O)ₚ-, Phenyl-(C₁-C₆)alkyl-S(O)ₚ-, Phenoxy-(C₁-C₆)alkoxy, Phenoxy-(C₁-C₆)alkyl-S(O)ₚ-, einen Rest Het¹, Het¹-S(O)ₚ-, Het¹-(C₁-C₆)alkoxy, Het¹-O-, Het¹-O-(C₁-C₆)alkoxy, wobei der heterocyclische Rest Het¹ weiter unten definiert ist,
   wobei jeder der letztgenannten 29 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist und p jeweils unabhängig voneinander 0, 1 oder 2 bedeutet,
   und
   vorzugsweise die Reste (a) Halogen, Cyano, Nitro, Hydroxy, Carboxy, (C₁-C₈)Alkoxy, (C₁-C₈)Haloalkoxy, (C₁-C₄)Alkoxy-(C₁-C₄)alkoxy, (C₁-C₆)Alkylthio, (C₁-C₆)Haloalkylthio, (C₁-C₆)Alkylsulfinyl, (C₁-C₆)Haloalkylsulfinyl, (C₁-C₆)Alkylsulfonyl, (C₁-C₈)Haloalkylsulfonyl, (C₃-C₈)Cycloalkyl, (C₅-C₈)Cycloalkenyl, (C₅-C₈)Cycloalkinyl, (C₃-C₈)Cycloalkyl-(C₁-C₆)alkoxy, (C₅-C₈)Cycloalkenyl-(C₁-C₆)alkoxy, (C₅-C₈)Cycloalkinyl-(C₁-C₆)alkoxy, (C₃-C₈)Cycloalkoxy, (C₃-C₈)Cycloalkylthio, (C₃-C₈)Cycloalkylsulfinyl, (C₃-C₈)Cycloalkylsulfonyl, (C₃-C₈)Cycloalkoxy-(C₁-C₆)alkoxy, Phenyl, Phenoxy, Phenylthio, Phenylsulfinyl, Phenylsulfonyl, Phenyl-(C₁-C₆)alkoxy, Phenyl-(C₁-C₆)alkylthio, Phenyl-(C₁-C₆)alkylsulfinyl, Phenyl-(C₁-C₆)alkylsulfonyl, Phenoxy-(C₁-C₆)alkoxy, Phenoxy-(C₁-C₆)alkylthio, Phenoxy-(C₁-C₆)alkylsulfinyl und Phenoxy-(C₁-C₆)alkylsulfonyl,
   wobei jeder der genannten Reste mit cyclischen Teilen im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
(b) Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-NR*R**, -C(=O)-O-NR*R**, -O-C(=O)-NR*R**, -N(R*)-C(=O)-R^{C}, -N(R*)-C(=O)-NR*R**, -N(R*)-C(=O)-O-R^{C}, -P(=O)(R^{C})(R^{D}), -P(=O)(OR^{C})(R^{D}), -P(=O)(OR^{C})(OR^{D}) oder -O-P(=O)(OR^{C})(OR^{D}), vorzugsweise einen Rest der Formel -C(=O)-R^{C}, -C(=O)-O-R^{C}, -O-C(=O)-R^{C} und -O-C(=O)-O-R^{C}, insbesondere einen Rest der Formel -C(=O)-O-R^{C}, -O-C(=O)-R^{C} und -O-C(=O)-O-R^{C}, wobei R*, R**, R^{C} und R° wie weiter unten definiert sind, vorzugsweise die Reste (b1) [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy und [(C₂-C₈)Alkinyloxy]-carbonyloxy, wobei jeder der letztgenannten 23 Reste unsubstituiert oder durch einen oder mehrere Reste aus der Gruppe Halogen, NO₂, (C₁-C₄)Alkoxy und gegebenenfalls durch Halogen, CN, NO₂, (C₁-C₄)Alkyl, (C₁-C₄)Alkoxy und (C₁-C₄)Alkylthio substituiertes Phenyl substituiert ist, und
   vorzugsweise die Reste (b2) (C₃-C₈)Cycloalkylcarbonyl, (C₃-C₈)Cycloalkyl-[(C₁-C₆)alkyl]-carbonyl, (C₃-C₈)Cycloalkyl-[(C₁-C₆)alkoxy]-carbonyl, (C₃-C₈)Cycloalkoxycarbonyl, (C₃-C₈)Cycloalkoxy-[(C₁-C₆)alkyl]-carbonyl, (C₃-C₈)Cycloalkoxy-[(C₁-C₆)alkoxy]-carbonyl, (C₃-C₈)Cycloalkylcarbonyloxy, (C₃-C₈)Cycloalkyl-[(C₁-C₆)alkyl]-carbonyloxy, (C₅-C₈)Cycloalkenyl-[(C₁-C₆)alkyl]-carbonyloxy, (C₅-C₈)Cycloalkinyl-[(C₁-C₆)alkyl]-carbonyloxy, (C₃-C₈)Cycloalkyl-[(C₁-C₆)alkoxy]-carbonyloxy, (C₅-C₈)Cycloalkenyl-[(C₁-C₆)alkoxy]-carbonyloxy, (C₅-C₈)Cycloalkinyl-[(C₁-C₆)alkoxy]-carbonyloxy, (C₃-C₈)Cycloalkoxycarbonyloxy, (C₃-C₈)Cycloalkoxy-[(C₁-C₆)alkyl]-carbonyloxy, (C₃-C₈)Cycloalkoxy-[(C₁-C₆)alkoxy]-carbonyloxy, (C₃-C₈)Cycloalkylcarbonylamino, (C₃-C₆)Cycloalkyl-[(C₁-C₆)alkyl]-carbonylamino, (C₅-C₈)Cycloalkenyl-[(C₁-C₆)alkyl]-carbonylamino, (C₅-C₈)Cycloalkinyl-[(C₁-C₆)alkyl]-carbonylamino, (C₃-C₈)Cycloalkyl-[(C₁-C₆)alkoxy]-carbonylamino, (C₃-C₈)Cycloalkoxycarbonylamino, (C₃-C₈)Cycloalkoxy-[(C₁-C₆)alkyl]-carbonylamino und (C₃-C₈)Cycloalkoxy-[(C₁-C₆)alkoxy]-carbonylamino, Phenylcarbonyl, Phenyl-[(C₁-C₆)alkyl]-carbonyl, Phenyl-[(C₁-C₆)alkoxy]-carbonyl, Phenoxycarbonyl, Phenoxy-[(C₁-C₆)alkyl]-carbonyl, Phenoxy-[(C₁-C₆)alkoxy]-carbonyl, Phenylcarbonyloxy, Phenyl-[(C₁-C₆)alkyl]-carbonyloxy, Phenyl-[(C₁-C₆)alkoxy]-carbonyloxy, Phenoxycarbonyloxy, Phenoxy-[(C₁-C₆)alkyl]-carbonyloxy, Phenoxy-[(C₁-C₆)alkoxy]-carbonyloxy, Phenylcarbonylamino, Phenyl-[(C₁-C₆)alkyl]-carbonylamino, Phenyl-[(C₁-C₆)alkoxy]-carbonylamino, Phenoxycarbonylamino, Phenoxy-[(C₁-C₆)alkyl]-carbonylamino, Phenoxy-[(C₁-C₆)alkoxy]-carbonylamino, wobei jeder der letztgenannten 42 Reste im cyclischen Teil gegebenenfalls mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxyund Nitro substituiert ist, und
(c) Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)_{q}-CH(OR')₂, in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und q eine ganze Zahl von 0 bis 6 bedeuten, und
(d) Reste der Formel R"O-CHR'"CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten, besteht, substituiert ist,
   oder
- R¹: (C₃-C₉)Cycloalkyl, (C₅-C₉)Cycloalkenyl, (C₅-C₉)Cycloalkinyl oder Phenyl, wobei jeder der letztgenannten 4 Reste unsubstituiert oder durch einen oder mehrere Reste aus der Gruppe, die aus den Resten [Untergruppen (a')-(e')]
(a') Halogen, Cyano, Thio, Nitro, Hydroxy, Carboxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio und Reste der Formeln -NR*R**, wobei die Reste R* und R** weiter unten definiert sind,
(b') Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-NR*R**, -C(=O)-O-NR*R**, -O-C(=O)-NR*R**, -N(R*)-C(=O)-R^{C}, -N(R*)-C(=O)-NR*R**, -N(R*)-C(=O)-O-R^{C}, -P(=O)(R^{C})(R^{D}), -P(=O)(OR^{C})(R^{D}), -P(=O)(OR^{C})(OR^{D}) oder -O-P(=O)(OR^{C})(OR^{D}), vorzugsweise einen Rest der Formel -C(=O)-R^{C}, -C(=O)-O-R^{C}, -O-C(=O)-R^{C} und -O-C(=O)-O-R^{C}, insbesondere einen Rest der Formel -C(=O)-O-R^{C}, -O-C(=O)-R^{C} und -O-C(=O)-O-R^{C}, wobei R*, R**, R^{C} und R° wie weiter unten definiert sind,
   und vorzugsweise die Reste (b1') [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl,
   wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist, und vorzugsweise die Reste (b2') (C₃-C₈)Cycloalkylcarbonyl, (C₃-C₈)Cycloalkyl-[(C₁-C₆)alkyl]-carbonyl, (C₃-C₈)Cycloalkyl-[(C₁-C₆)alkoxy]-carbonyl, (C₃-C₈)Cycloalkoxycarbonyl, (C₃-C₈)Cycloalkoxy-[(C₁-C₆)alkyl]-carbonyl, (C₃-C₈)Cycloalkoxy-[(C₁-C₆)alkoxy]-carbonyl, (C₃-C₈)Cycloalkylcarbonyloxy, (C₃-C₈)Cycloalkyl-[(C₁-C₆)alkyl]-carbonyloxy, (C₅-C₈)Cycloalkenyl-[(C₁-C₆)alkyl]-carbonyloxy, (C₅-C₈)Cycloalkinyl-[(C₁-C₆)alkyl]-carbonyloxy, (C₃-C₈)Cycloalkyl-[(C₁-C₆)alkoxy]-carbonyloxy, (C₅-C₈)Cycloalkenyl-[(C₁-C₆)alkoxy]-carbonyloxy, (C₅-C₈)Cycloalkinyl-[(C₁-C₆)alkoxy]-carbonyloxy, (C₃-C₈)Cycloalkoxycarbonyloxy, (C₃-C₈)Cycloalkoxy-[(C₁-C₆)alkyl]-carbonyloxy, (C₃-C₈)Cycloalkoxy-[(C₁-C₆)alkoxy]-carbonyloxy, (C₃-C₈)Cycloalkylcarbonylamino, (C₃-C₈)Cycloalkyl-[(C₁-C₆)alkyl]-carbonylamino, (C₅-C₈)Cycloalkenyl-[(C₁-C₆)alkyl]-carbonylamino, (C₅-C₈)Cycloalkinyl-[(C₁-C₆)alkyl]-carbonylamino, (C₃-C₈)Cycloalkyl-[(C₁-C₆)alkoxy]-carbonylamino, (C₃-C₈)Cycloalkoxycarbonylamino, (C₃-C₈)Cycloalkoxy-[(C₁-C₆)alkyl]-carbonylamino und (C₃-C₈)Cycloalkoxy-[(C₁-C₆)alkoxy]-carbonylamino, Phenylcarbonyl, Phenyl-[(C₁-C₆)alkyl]-carbonyl, Phenyl-[(C₁-C₆)alkoxy]-carbonyl, Phenoxycarbonyl, Phenoxy-[(C₁-C₆)alkyl]-carbonyl, Phenoxy-[(C₁-C₆)alkoxy]-carbonyl, Phenylcarbonyloxy, Phenyl-[(C₁-C₆)alkyl]-carbonyloxy, Phenyl-[(C₁-C₆)alkoxy]-carbonyloxy, Phenoxycarbonyloxy, Phenoxy-[(C₁-C₆)alkyl]-carbonyloxy, Phenoxy-[(C₁-C₆)alkoxy]-carbonyloxy, Phenylcarbonylamino, Phenyl-[(C₁-C₆)alkyl]-carbonylamino, Phenyl-[(C₁-C₆)alkoxy]-carbonylamino, Phenoxycarbonylamino, Phenoxy-[(C₁-C₆)alkyl]-carbonylamino, Phenoxy-[(C₁-C₆)alkoxy]-carbonylamino,
   wobei jeder der letztgenannten 42 Reste im cyclischen Teil gegebenenfalls mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxyund Nitro substituiert ist, und
(c') Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)_{q}-CH(OR')₂, in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und q eine ganze Zahl von 0 bis 6 bedeuten, und
(d') Reste der Formel R"O-CHR'"CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten, und
(e') ein Rest der Formel Het¹, der unsubstituiert oder durch einen oder mehrere gleiche oder verschiedene Reste R^{B} substituiert ist, besteht, substituiert ist,
   oder
- R¹: einen mehrcyclischen Rest auf Basis von (C₃-C₉)Cycloalkyl, (C₅-C₉)Cycloalkenyl, (C₅-C₉)Cycloalkinyl oder Phenyl, wobei der Basisring mit einem carbocyclischen oder heterocyclischen Ring, vorzugsweise einem 5- oder 6-gliedrigen Ring mit 0 oder 1 bis 3 Heteroringatomen aus der Gruppe N, O und S kondensiert ist, vorzugsweise benzokondensiert ist, und wobei der Basisring oder das mehrcyclische System unsubstituiert oder durch einen oder mehrere gleiche oder verschiedene Reste R^{B} substituiert ist, vorzugsweise unsubstitutiert oder durch einen oder mehrere Reste aus der Gruppe Halogen, Cyano, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
oder
- R¹: einen heterocyclischen Rest Het¹, der im Ring oder im mehrcyclischen System unsubstituiert oder durch einen oder mehrere gleiche oder verschiedene Reste R^{B} substituiert ist, vorzugsweise unsubstituiert oder durch einen oder mehrere Reste aus der Gruppe Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
bedeutet,
wobei in den obengenannten und den nachfolgenden Resten
- Het¹: jeweils unabhängig voneinander einen gesättigten, teilweise ungesättigten oder heteroaromatischen monocyclischen Heterocyclylrest mit 3 bis 9 Ringatomen, vorzugsweise mit 5 oder 6 Ringatomen, oder 9- oder 10-gliedrigen bicyclischen Heterocyclus, enthaltend 1, 2, 3 oder 4 Heteroatome ausgewählt aus der Gruppe bestehend aus O, N und S, vorzugsweise einen 5- oder 6-gliedrigen Heterocyclus mit 1 bis 3 Heteroringatomen aus der Gruppe N, O und S, der gegebenenfalls auch mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise gegebenenfalls benzokondensiert ist, bedeutet,
- R*,: R** jeweils unabhängig voneinander (d. h. auch von anderen Gruppen NR*R**) H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste R^{bb} substituiert ist, bedeuten oder
- R* und R**: zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
- R^{A}: Halogen, Cyano, Hydroxy oder (C₁-C₆)Alkoxy bedeutet,
- R^{B}: Halogen, Cyano, Hydroxy, Oxo, Nitro, (C₁-C₆)Alkyl, (C₁-C₄)Haloalkyl, Cyano-(C₁-C₄)alkyl, Hydroxy-(C₁-C₄)alkyl, Nitro-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkyl, (C₁-C₄)Alkoxy-(C₁-C₄)alkoxy, (C₁-C₄)Haloalkoxy-(C₁-C₄)alkyl, (C₁-C₄)Haloalkoxy-(C₁-C₄)alkoxy, (C₁-C₆)Alkylthio, (C₂-C₆)Alkenylthio, (C₂-C₆)Alkinylthio, (C₁-C₆)Alkylsulfinyl, (C₁-C₆)Haloalkylsulfinyl, (C₁-C₆)Alkylsulfonyl, (C₁-C₆)Haloalkylsulfonyl, ein Rest der Formel R^{aa}-C(=O)- oder R^{aa}-C(=O)-(C₁-C₆)alkyl, wobei die R^{aa} weiter unten definiert sind, -NR*R**, wobei R* und R** weiter unten definiert sind, Tri-[(C₁-C₄)alkyl]-silyl, Tri-[(C₁-C₄)alkyl]-silyl-(C₁-C₆)alkyl, (C₃-C₆)Cycloalkyl, (C₃-C₆)Cycloalkoxy, (C₃-C₆)Cycloalkyl-(C₁-C₄)alkyl, (C₃-C₆)Cycloalkyl-(C₁-C₈)alkoxy, Phenyl, Phenyl-(C₁-C₆)alkyl, Phenoxy, Phenoxy-(C₁-C₆)alkyl, Phenylamino, Phenylamino-(C₁-C₆)alkyl oder einen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, wobei jeder der letztgenannten 11 Reste im cyclischen Teil gegebenenfalls durch einen oder mehrere gleiche oder verschiedene Reste R^{bb} substituiert ist, substituiert ist, bedeutet
- R^{C}, R^{D}: jeweils unabhängig voneinander (auch unabhängig von Resten R^{C}, R^{D} in anderen Gruppen)
Wasserstoff, (C₁-C₈)Alkyl, (C₂-C₈)Alkenyl oder (C₂-C₈)Alkinyl bedeutet,
wobei jeder der letztgenannten 3 Reste unsubstituiert oder durch einen oder mehrere Reste aus der Gruppe Halogen, Cyano, Nitro, Hydroxy, (C₁-C₆)Alkoxy, (C₂-C₆)Alkenyloxy, (C₂-C₆)Alkinyloxy, (C₁-C₈)Haloalkoxy, (C₁-C₄)Alkoxy-(C₁-C₄)alkoxy, (C₁-C₈)Alkylthio, (C₁-C₈)Haloalkylthio, (C₁-C₈)Alkylsulfinyl, (C₁-C₈)Haloalkylsulfinyl, (C₁-C₈)Alkylsulfonyl, (C₁-C₈)Haloalkylsulfonyl und Tri-[(C₁-C₄)alkyl]-silyl substituiert ist,
oder
(C₃-C₈)Cycloalkyl, (C₅-C₈)Cycloalkenyl, (C₅-C₈)Cycloalkinyl, Phenyl, (C₃-C₈)Cycloalkyl-(C₁-C₆)alkyl, (C₅-C₈)Cycloalkenyl-(C₁-C₆)alkyl, (C₅-C₈)Cycloalkinyl-(C₁-C₆)alkyl, Phenyl-(C₁-C₆)alkyl, (C₃-C₈)Cycloalkyloxy-(C₁-C₆)alkyl, (C₃-C₈)Cycloalkyl-S(O)ₚ-(C₁-C₆)alkyl, (C₅-C₈)Cycloalkenyloxy-(C₁-C₆)alkyl, (C₅-C₈)Cycloalkinyloxy-(C₁-C₆)alkyl, Phenoxy-(C₁-C₆)alkyl, Phenyl-S(O)ₚ-(C₁-C₆)alkyl, (C₃-C₈)Cycloalkylamino-(C₁-C₆)alkyl, (C₅-C₈)Cycloalkenylamino-(C₁-C₆)alkyl, (C₅-C₈)Cycloalkinylamino-(C₁-C₆)alkyl, Phenylamino-(C₁-C₆)alkyl, Het¹, Het¹-(C₁-C₆)alkyl, Het¹-O-(C₁-C₆)alkyl oder Het¹-S(O)ₚ-(C₁-C₆)alkyl, wobei Het¹ die genannte Bedeutung hat,
wobei jeder der letztgenannten 22 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist und p jeweils unabhängig voneinander 0, 1 oder 2 bedeutet,
bedeuten,
- R^{aa}: jeweils unabhängig voneinander Wasserstoff, OH, (C₁-C₆)Alkyl, (C₁-C₄)Haloalkyl, (C₂-C₆)Alkenyl, (C₂-C₆)Alkinyl, (C₁-C₆)Alkoxy, (C₁-C₆)Alkoxy-(C₁-C₆)alkyl, (C₁-C₆)Alkoxy-(C₁-C₆)alkyloxy, (C₁-C₄)Haloalkoxy, (C₁-C₄)Haloalkoxy-(C₁-C₆)alkyl, (C₁-C₄)Haloalkoxy-(C₁-C₆)alkoxy, (C₃-C₆)Alkenyloxy, (C₃-C₆)Alkenyloxy-(C₁-C₆)alkyl, (C₃-C₆)Alkenyloxy-(C₁-C₆)alkoxy, (C₃-C₆)Alkinyloxy, (C₃-C₆)Alkinyloxy-(C₁-C₆)alkyl, (C₃-C₆)Alkinyloxy-(C₁-C₆)alkoxy, -NR*R*, wobei R* und R** wie oben definiert sind, Tri-[(C₁-C₄)alkyl]-silyl, Tri-[(C₁-C₄)alkyl]-silyl-(C₁-C₆)alkyl, Tri-[(C₁-C₄)alkyl]-silyl-(C₁-C₆)alkoxy, (C₃-C₆)Cycloalkyl, (C₃-C₆)Cycloalkoxy, (C₃-C₆)Cycloalkyl-(C₁-C₈)alkyl, (C₃-C₆)Cycloalkyl-(C₁-C₈)alkoxy, (C₅-C₆)Cycloalkenyl, (C₅-C₆)Cycloalkenyl-(C₁-C₆)alkyl, (C₅-C₆)Cycloalkenyloxy, (C₅-C₆)Cycloalkinyl, (C₅-C₆)Cycloalkinyl-(C₁-C₆)alkyl, (C₅-C₆)Cycloalkinyl-(C₁-C₆)alkoxy, Phenyl, Phenyl-(C₁-C₆)alkyl, Phenyl-(C₁-C₆)alkoxy, Phenoxy, Phenoxy-(C₁-C₆)alkyl, Phenoxy-(C₁-C₆)alkoxy, Phenylthio, Phenyl-S(O)ₚ-(C₁-C₆)alkyl, Phenyl-S(O)ₚ-(C₁-C₆)alkoxy, wobei p jeweils unabhängig voneinander 0, 1 oder 2 bedeutet, Phenylamino, Phenylamino-(C₁-C₆)alkyl, Phenylamino-(C₁-C₆)alkoxy oder einen gegebenenfalls über eine Alkylengruppe oder eine Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 20 Reste im cyclischen Teil gegebenenfalls durch einen oder mehrere gleiche oder verschiedene Reste R^{bb} substituiert ist, bedeutet und
- R^{bb}: jeweils unabhängig voneinander Halogen, (C₁-C₄)Alkyl, (C₁-C₄)Haloalkyl, (C₁-C₄)Alkoxy oder (C₁-C₄)Haloalkoxy bedeutet.

Weiter bevorzugt sind dabei auch Verbindungen (I) oder deren Salze, worin
- R¹: Wasserstoff, (C₁-C₁₈)Alkyl, (C₂-C₁₈)Alkenyl oder (C₂-C₁₈)Alkinyl, vorzugsweise H, (C₁-C₁₂)Alkyl, (C₂-C₁₂)Alkenyl oder (C₂-C₁₂)Alkinyl, insbesondere H, (C₁-C₈)Alkyl, (C₂-C₈)Alkenyl oder (C₂-C₈)Alkinyl, weiter bevorzugt H oder (C₁-C₆)Alkyl, (C₂-C₆)Alkenyl oder (C₂-C₆)Alkinyl, weiter bevorzugt (C₁-C₄)Alkyl,
wobei jeder der letztgenannten 13 Reste, die C-Atome enthalten, unsubstituiert oder durch einen oder mehrere Reste aus der Gruppe, die aus den Resten [Untergruppen (a)-(d)]
(a) Halogen, Cyano, Thio, Nitro, Hydroxy, Carboxy, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio,(C₂-C₆)Alkenylthio, (C₂-C₆)Alkinylthio, (C₁-C₆)Haloalkylthio, (C₂-C₆)Haloalkenylthio, (C₂-C₆)Haloalkinylthio, (C₁-C₆)Alkylsulfinyl, (C₂-C₆)Alkenylsulfinyl, (C₂-C₆)Alkinylsulfinyl, (C₁-C₆)Haloalkylsulfinyl, (C₂-C₆)Haloalkenylsulfinyl, (C₂-C₆)Haloalkinylsulfinyl, (C₁-C₆)Alkylsulfonyl, (C₂-C₆)Alkenylsulfonyl, (C₂-C₆)Alkinylsulfonyl, (C₁-C₆)Haloalkylsulfonyl, (C₂-C₆)Haloalkenylsulfonyl, (C₂-C₆)Haloalkinylsulfonyl, Reste der Formel -NR^{*}R^{**}, wobei R^{*} und R^{**} weiter unten definiert sind, und (C₃-C₆)Cycloalkyl, (C₅-C₆)Cycloalkenyl, (C₅-C₆)Cycloalkinyl, (C₃-C₆)Cycloalkyl-(C₁-C₄)alkoxy, (C₅-C₆)Cycloalkenyl-(C₁-C₄)alkoxy, (C₅-C₆)Cycloalkinyl-(C₁-C₄)alkoxy, (C₃-C₆)Cycloalkoxy, (C₅-C₆)Cycloalkenyloxy, (C₅-C₆)Cycloalkinyloxy, (C₃-C₆)Cycloalkoxy-(C₁-C₄)alkoxy, Phenyl, Phenyl-(C₁-C₆)alkoxy, Phenoxy, Phenoxy-(C₁-C₄)alkoxy, Phenyl-S(O)ₚ-, Phenyl-(C₁-C₆)alkyl-S(O)ₚ-, Phenyloxy-(C₁-C₆)alkyl-S(O)ₚ₋, einen Rest Het¹, Het¹-(C₁-C₆)alkoxy, Het¹-O-, Het¹-O-(C₁-C₄)alkoxy, Het¹-(C₁-C₆)alkoxy, Het¹-S(O)ₚ-, Het¹-O-(C₁-C₄)alkyl-S(O)ₚ-, wobei der heterocyclische Rest Het¹ weiter unten definiert ist,
   wobei jeder der letztgenannten 24 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert und p jeweils unabhängig voneinander 0, 1 oder 2 bedeutet,
   und
   vorzugsweise die Reste (a1) Halogen, Cyano, Nitro, Hydroxy, Carboxy, (C₁-C₆)Alkoxy, (C₁-C₆)Haloalkoxy, (C₁-C₄)Alkoxy-(C₁-C₄)alkoxy, (C₁-C₆)Alkylthio, (C₁-C₆)Haloalkylthio, (C₁-C₆)Alkylsulfonyl, (C₁-C₆)Haloalkylsulfonyl, (C₃-C₆)Cycloalkyl, (C₅-C₆)Cycloalkenyl, (C₅-C₆)Cycloalkinyl, (C₃-C₆)Cycloalkyl-(C₁-C₄)alkoxy, (C₅-C₄)Cycloalkenyl-(C₁-C₄)alkoxy, (C₅-C₄)Cycloalkinyl-(C₁-C₄)alkoxy, (C₃-C₄)Cycloalkoxy, (C₃-C₄)Cycloalkoxy-(C₁-C₄)alkoxy, Phenyl, Phenyl-(C₁-C₄)alkoxy, Phenoxy und Phenoxy-(C₁-C₄)alkoxy, Phenylthio, Phenylsulfinyl, Phenylsulfonyl,
   wobei jeder der Reste (a1) im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
(b) Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-NR*R**, -C(=O)-O-NR*R**, -O-C(=O)-NR*R**, -N(R*)-C(=O)-R^{C}, -N(R*)-C(=O)-NR*R**, -N(R*)-C(=O)-O-R^{C}, -P(=O)(R^{C})(R^{D}), -P(=O)(OR^{C})(R^{D}), -P(=O)(OR^{C})(OR^{D}) oder -O-P(=O)(OR^{C})(OR^{D}), vorzugsweise einen Rest der Formel -C(=O)-R^{C}, -C(=O)-O-R^{C}, -O-C(=O)-R^{C} und -O-C(=O)-O-R^{C}, insbesondere einen Rest der Formel -C(=O)-O-R^{C}, -O-C(=O)-R^{C} und -O-C(=O)-O-R^{C}, wobei R*, R**, R^{C} und R^{D} wie weiter unten definiert sind,
   vorzugsweise die Reste (b1)
   [(C₁-C₆)Alkoxy]-carbonyl, [(C₁-C₆)Alkoxy]-thiocarbonyl, [(C₂-C₆)Alkenyloxy]-carbonyl, [(C₂-C₈)Alkinyloxy]-carbonyl, [(C₁-C₆)Alkylthio]-carbonyl, [(C₂-C₆)Alkenylthio]-carbonyl, [(C₂-C₆)Alkinylthio]-carbonyl, (C₁-C₆)Alkanoyl, [(C₂-C₆)Alkenyl]-carbonyl, [(C₂-C₆)Alkinyl]-carbonyl, [(C₁-C₆)Alkyl]-carbonylamino, [(C₂-C₆)Alkenyl]-carbonylamino, [(C₂-C₆)Alkinyl]-carbonylamino, [(C₁-C₆)Alkoxy]-carbonylamino, [(C₂-C₆)Alkenyloxy]-carbonylamino, [(C₂-C₆)Alkinyloxy]-carbonylamino, [(C₁-C₆)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₆)Alkoxy]-carbonyloxy, [(C₂-C₆)Alkenyloxy]-carbonyloxy und [(C₂-C₆)Alkinyloxy]-carbonyloxy, wobei jeder der letztgenannten 23 Reste unsubstituiert oder durch einen oder mehrere Reste aus der Gruppe Halogen, NO₂, (C₁-C₄)Alkoxy und gegebenenfalls durch Halogen, CN, NO₂, (C₁-C₄)Alkyl, (C₁-C₄)Alkoxy und (C₁-C₄)Alkylthio substituiertes Phenyl substituiert ist, und vorzugsweise die Reste (b2) (C₃-C₆)Cycloalkylcarbonyl, (C₃-C₆)Cycloalkyl-[(C₁-C₄)alkyl]-carbonyl, (C₃-C₆)Cycloalkyl-[(C₁-C₄)alkoxy]-carbonyl, (C₃-C₆)Cycloalkoxycarbonyl, (C₃-C₆)Cycloalkoxy-[(C₁-C₄)alkyl]-carbonyl, (C₃-C₆)Cycloalkoxy-[(C₁-C₄)alkoxy]-carbonyl, (C₃-C₆)Cycloalkylcarbonyloxy, (C₃-C₆)Cycloalkyl-[(C₁-C₄)alkyl]-carbonyloxy, (C₅-C₆)Cycloalkenyl-[(C₁-C₄)alkyl]-carbonyloxy, (C₅-C₆)Cycloalkinyl-[(C₁-C₄)alkyl]-carbonyloxy, (C₃-C₆)Cycloalkyl-[(C₁-C₄)alkoxy]-carbonyloxy, (C₅-C₆)Cycloalkenyl-[(C₁-C₄)alkoxy]-carbonyloxy, (C₅-C₆)Cycloalkinyl-[(C₁-C₄)alkoxy]-carbonyloxy, (C₃-C₆)Cycloalkoxycarbonyloxy, (C₃-C₆)Cycloalkoxy-[(C₁-C₄)alkyl]-carbonyloxy, (C₃-C₆)Cycloalkoxy-[(C₁-C₄)alkoxy]-carbonyloxy, (C₃-C₆)Cycloalkylcarbonylamino, (C₃-C₆)Cycloalkyl-[(C₁-C₄)alkyl]-carbonylamino, (C₅-C₆)Cycloalkenyl-[(C₁-C₄)alkyl]-carbonylamino, (C₅-C₆)Cycloalkinyl-[(C₁-C₄)alkyl]-carbonylamino, (C₃-C₆)Cycloalkyl-[(C₁-C₄)alkoxy]-carbonylamino, (C₃-C₆)Cycloalkoxycarbonylamino, (C₃-C₆)Cycloalkoxy-[(C₁-C₄)alkyl]-carbonylamino und (C₃-C₆)Cycloalkoxy-[(C₁-C₄)alkoxy]-carbonylamino, Phenylcarbonyl, Phenyl-[(C₁-C₄)alkyl]-carbonyl, Phenyl-[(C₁-C₄)alkoxy]-carbonyl, Phenoxycarbonyl, Phenoxy-[(C₁-C₄)alkyl]-carbonyl, Phenoxy-[(C₁-C₄)alkoxy]-carbonyl, Phenylcarbonyloxy, Phenyl-[(C₁-C₄)alkyl]-carbonyloxy, Phenyl-[(C₁-C₄)alkoxy]-carbonyloxy, Phenoxycarbonyloxy, Phenoxy-[(C₁-C₄)alkyl]-carbonyloxy, Phenoxy-[(C₁-C₄)alkoxy]-carbonyloxy, Phenylcarbonylamino, Phenyl-[(C₁-C₄)alkyl]-carbonylamino, Phenyl-[(C₁-C₄)alkoxy]-carbonylamino, Phenoxycarbonylamino, Phenoxy-[(C₁-C₄)alkyl]-carbonylamino, Phenoxy-[(C₁-C₄)alkoxy]-carbonylamino, wobei jeder der letztgenannten 42 Reste im cyclischen Teil gegebenenfalls mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist, und
(c) Reste der Formeln -SiR'₃, -O-SiR'₃, (R')₃Si-(C₁-C₄)alkoxy, -CO-O-NR'₂, -O-N=CR'₂, -N=CR'₂, -O-NR'₂, -CH(OR')₂ und -O-(CH₂)_{q}-CH(OR')₂, in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und q eine ganze Zahl von 0 bis 6 bedeuten, und
(d) Reste der Formel R"O-CHR'"CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten, besteht, substituiert ist,
oder
- R¹: (C₃-C₆)Cycloalkyl, (C₅-C₆)Cycloalkenyl, (C₅-C₆)Cycloalkinyl oder Phenyl, wobei jeder der letztgenannten 4 Reste unsubstituiert oder durch einen oder mehrere Reste aus der Gruppe, die aus den Resten [Untergruppen (a')-(e')]
(a') Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio und Reste der Formeln -NR*R**, wobei die Reste R* und R** weiter unten definiert sind,
(b') Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-NR*R**, -C(=O)-O-NR*R**, -O-C(=O)-NR*R**, -N(R*)-C(=O)-R^{C}, -N(R*)-C(=O)-NR*R**, -N(R*)-C(=O)-O-R^{C}, -P(=O)(R^{C})(R^{D}), -P(=O)(OR^{C})(R^{D}), -P(=O)(OR^{C})(OR^{D}) oder -O-P(=O)(OR^{C})(OR^{D}), vorzugsweise einen Rest der Formel -C(=O)-R^{C}, -C(=O)-O-R^{C}, -O-C(=O)-R^{C} und -O-C(=O)-O-R^{C}, insbesondere einen Rest der Formel -C(=O)-O-R^{C}, -O-C(=O)-R^{C} und -O-C(=O)-O-R^{C}, wobei R*, R**, R^{C} und R^{D} wie weiter unten definiert sind, und vorzugsweise die Reste (b1') [(C₁-C₆)Alkoxy]-carbonyl, [(C₁-C₆)Alkoxy]-thiocarbonyl, [(C₂-C₆)Alkenyloxy]-carbonyl, [(C₂-C₆)Alkinyloxy]-carbonyl, [(C₁-C₆)Alkylthio]-carbonyl, [(C₂-C₆)Alkenylthio]-carbonyl, [(C₂-C₆)Alkinylthio]-carbonyl, (C₁-C₈)Alkanoyl, [(C₂-C₆)Alkenyl]-carbonyl, [(C₂-C₆)Alkinyl]-carbonyl, (C₁-C₄)Alkylimino, (C₁-C₄)Alkoxyimino, [(C₁-C₆)Alkyl]-carbonylamino, [(C₂-C₆)Alkenyl]-carbonylamino, [(C₂-C₆)Alkinyl]-carbonylamino, [(C₁-C₆)Alkoxy]-carbonylamino, [(C₂-C₆)Alkenyloxy]-carbonylamino, [(C₂-C₆)Alkinyloxy]-carbonylamino, [(C₁-C₆)Alkylamino]-carbonylamino, [(C₁-C₄)Alkyl]-carbonyloxy, [(C₂-C₄)Alkenyl]-carbonyloxy, [(C₂-C₄)Alkinyl]-carbonyloxy, [(C₁-C₆)Alkoxy]-carbonyloxy, [(C₂-C₆)Alkenyloxy]-carbonyloxy, [(C₂-C₆)Alkinyloxy]-carbonyloxy, (C₁-C₆)Alkylsulfinyl und (C₁-C₆)Alkylsulfonyl, wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist, und vorzugsweise die Reste (b2') (C₃-C₆)Cycloalkylcarbonyl, (C₃-C₆)Cycloalkyl-[(C₁-C₄)alkyl]-carbonyl, (C₃-C₆)Cycloalkyl-[(C₁-C₄)alkoxy]-carbonyl, (C₃-C₆)Cycloalkoxycarbonyl, (C₃-C₆)Cycloalkoxy-[(C₁-C₄)alkyl]-carbonyl, (C₃-C₆)Cycloalkoxy-[(C₁-C₄)alkoxy]-carbonyl, (C₃-C₆)Cycloalkylcarbonyloxy, (C₃-C₆)Cycloalkyl-[(C₁-C₄)alkyl]-carbonyloxy, (C₅-C₆)Cycloalkenyl-[(C₁-C₄)alkyl]-carbonyloxy, (C₅-C₆)Cycloalkinyl-[(C₁-C₄)alkyl]-carbonyloxy, (C₃-C₆)Cycloalkyl-[(C₁-C₄)alkoxy]-carbonyloxy, (C₅-C₆)Cycloalkenyl-[(C₁-C₄)alkoxy]-carbonyloxy, (C₅-C₆)Cycloalkinyl-[(C₁-C₄)alkoxy]-carbonyloxy, (C₃-C₆)Cycloalkoxycarbonyloxy, (C₃-C₆)Cycloalkoxy-[(C₁-C₄)alkyl]-carbonyloxy, (C₃-C₆)Cycloalkoxy-[(C₁-C₄)alkoxy]-carbonyloxy, (C₃-C₆)Cycloalkylcarbonylamino, (C₃-C₆)Cycloalkyl-[(C₁-C₄)alkyl]-carbonylamino, (C₅-C₆)Cycloalkenyl-[(C₁-C₄)alkyl]-carbonylamino, (C₅-C₆)Cycloalkinyl-[(C₁-C₄)alkyl]-carbonylamino, (C₃-C₆)Cycloalkyl-[(C₁-C₄)alkoxy]-carbonylamino, (C₃-C₆)Cycloalkoxycarbonylamino, (C₃-C₆)Cycloalkoxy-[(C₁-C₄)alkyl]-carbonylamino und (C₃-C₆)Cycloalkoxy-[(C₁-C₄)alkoxy]-carbonylamino, Phenylcarbonyl, Phenyl-[(C₁-C₄)alkyl]-carbonyl, Phenyl-[(C₁-C₄)alkoxy]-carbonyl, Phenoxycarbonyl, Phenoxy-[(C₁-C₄)alkyl]-carbonyl, Phenoxy-[(C₁-C₄)alkoxy]-carbonyl, Phenylcarbonyloxy, Phenyl-[(C₁-C₄)alkyl]-carbonyloxy, Phenyl-[(C₁-C₄)alkoxy]-carbonyloxy, Phenoxycarbonyloxy, Phenoxy-[(C₁-C₄)alkyl]-carbonyloxy, Phenoxy-[(C₁-C₄)alkoxy]-carbonyloxy, Phenylcarbonylamino, Phenyl-[(C₁-C₄)alkyl]-carbonylamino, Phenyl-[(C₁-C₄)alkoxy]-carbonylamino, Phenoxycarbonylamino, Phenoxy-[(C₁-C₄)alkyl]-carbonylamino, Phenoxy-[(C₁-C₄)alkoxy]-carbonylamino, wobei jeder der letztgenannten 42 Reste im cyclischen Teil gegebenenfalls mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise benzokondensiert ist, und im Ring oder im mehrcyclischen System unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist, und
(c') Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)_{q}-CH(OR')₂, in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und q eine ganze Zahl von 0 bis 6 bedeuten, und
(d') Reste der Formel R"O-CHR'"CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten, und
(e') ein Rest der Formel Het¹, der unsubstituiert oder durch einen oder mehrere gleiche oder verschiedene Reste R^{B} substituiert ist, besteht, substituiert ist,
oder
- R¹: einen mehrcyclischen Rest auf Basis von (C₃-C₆)Cycloalkyl, (C₅-C₆)Cycloalkenyl, (C₅-C₆)Cycloalkinyl oder Phenyl, wobei der Basisring mit einem carbocyclischen oder heterocyclischen Ring, vorzugsweise einem 5- oder 6-gliedrigen Ring mit 0 oder 1 bis 3 Heteroringatomen aus der Gruppe N, O und S kondensiert ist, vorzugsweise benzokondensiert ist, und wobei der Basisring oder das mehrcyclische System unsubstituiert oder durch einen oder mehrere gleiche oder verschiedene Reste R^{B} substituiert ist, vorzugsweise unsubstitutiert oder durch einen oder mehrere Reste aus der Gruppe Halogen, Cyano, Nitro, Hydroxy, Carboxy, (C₁-C₄)Alkyl, (C₁-C₄)Haloalkyl, (C₁-C₄)Alkoxy-(C₁-C₄)alkyl, (C₂-C₄)Alkenyl, (C₂-C₄)Haloalkenyl, (C₂-C₄)Alkinyl, (C₂-C₄)Haloalkinyl, (C₁-C₄)Alkoxy, (C₂-C₄)Alkenyloxy, (C₂-C₄)Alkinyloxy, (C₁-C₄)Haloalkoxy, (C₁-C₄)Alkoxy-(C₁-C₄)alkoxy, (C₁-C₄)Alkylthio, (C₂-C₄)Alkenylthio, (C₂-C₄)Alkinylthio, (C₃-C₆)Cycloalkyl, (C₃-C₆)Cycloalkoxy, [(C₁-C₄)Alkoxy]-carbonyl, [(C₁-C₄)Haloalkoxy]-carbonyl und Oxo substituiert ist,
oder
- R¹: einen heterocyclischen Rest Het¹, der im Ring oder im mehrcyclischen System der unsubstituiert oder durch einen oder mehrere gleiche oder verschiedene Reste R^{B} substituiert ist, vorzugsweise unsubstituiert oder durch einen oder mehrere Reste aus der Gruppe Halogen, Cyano, Thio, Nitro, Hydroxy, Carboxy, (C₁-C₄)Alkyl, (C₁-C₄)Haloalkyl, (C₁-C₄)Alkoxy-(C₁-C₄)alkyl, (C₂-C₄)Alkenyl, (C₂-C₄)Haloalkenyl, (C₂-C₄)Alkinyl, (C₂-C₆)Haloalkinyl, (C₁-C₄)Alkoxy, (C₂-C₄)Alkenyloxy, (C₂-C₄)Alkinyloxy, (C₁-C₄)Haloalkoxy, (C₁-C₄)Alkoxy-(C₁-C₄)alkoxy, (C₁-C₄)Alkylthio, (C₂-C₄)Alkenylthio, (C₂-C₄)Alkinylthio, (C₃-C₆)Cycloalkyl, (C₃-C₆)Cycloalkoxy, [(C₁-C₄)Alkoxy]-carbonyl, [(C₁-C₄)Haloalkoxy]-carbonyl und Oxo substituiert ist,
bedeutet,
wobei Het¹, R*, R**, R^{A}, R^{B}, R^{C}, R^{D}, R^{aa} und R^{bb} die oben bereits genannten Bedeutungen haben, vorzugsweise
- Het¹: jeweils unabhängig voneinander einen gesättigten, teilweise ungesättigten oder heteroaromatischen monocyclischen Heterocyclylrest mit 3 bis 9 Ringatomen, vorzugsweise mit 5 oder 6 Ringatomen, oder 9- oder 10-gliedrigen bicyclischen Heterocyclus, enthaltend 1, 2, 3 oder 4 Heteroatome ausgewählt aus der Gruppe bestehend aus O, N und S, vorzugsweise einen 5- oder 6-gliedrigen Heterocyclus mit 1 bis 3 Heteroringatomen aus der Gruppe N, O und S, der gegebenenfalls auch mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise gegebenenfalls benzokondensiert ist, bedeutet,
- R*, R**: jeweils unabhängig voneinander (d. h. auch von anderen Gruppen NR*R**) H, (C₁-C₆)Alkyl, (C₂-C₆)Alkenyl, (C₂-C₆)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste R^{bb} substituiert ist, bedeuten oder vorzugsweise H, (C₁-C₄)Alkyl, Allyl, Propargyl, (C₁-C₄)Alkoxy-(C₁-C₄)alkyl, Formyl, Acetyl, n-Propanoyl, i-Propanoyl, Trifluoracetyl, Trichloracetyl. Methoxycarbonyl, Ethoxycarbonyl, n- oder i-Propoxycarbonyl, n-, i-, sec-, t-Butoxycarbonyl, [(C₁-C₄)Haloalkoxy]-carbonyl, Cyclopropyl, Cyclobutyl, Cyclopentyl, Cyclohexyl, Cyclopropyl-methyl, Phenyl, Benzyl, 1- oder 2-Phenyl-ethyl bedeuten,
- R* und R**: zusammen mit dem N-Atom einen vorzugsweise gesättigten 5- bis 6-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten, vorzugsweise 1-Piperidin-, 1-Piperazin-, 1-Pyrrolidin-, 1-Pyrazolidin-, 1-Piperazolidin- oder 1-Morpholinrest bedeuten,
- R^{A}: Halogen, Cyano, Hydroxy oder (C₁-C₆)Alkoxy bedeutet,
- R^{B}: Halogen, Cyano, Hydroxy, Oxo, Nitro, (C₁-C₄)Alkyl, (C₁-C₄)Haloalkyl, Cyano-(C₁-C₄)alkyl, Hydroxy-(C₁-C₄)alkyl, Nitro-(C₁-C₄)alkyl, (C₂-C₄)Alkenyl, (C₂-C₄)Alkinyl, (C₁-C₄)Alkoxy, (C₂-C₄)Alkenyloxy, (C₂-C₄)Alkinyloxy, (C₁-C₄)Haloalkoxy, (C₁-C₄)Alkoxy-(C₁-C₄)alkyl, (C₁-C₄)Alkoxy-(C₁-C₄)alkoxy, (C₁-C₄)Alkylthio, (C₁-C₄)Alkylsulfonyl, (C₁-C₄)Haloalkylsulfonyl, ein Rest der Formel R^{aa}-C(=O)- oder R^{aa}-C(=O)-(C₁-C₆)alkyl, wobei die R^{aa} weiter unten definiert sind, -NR*R**, wobei R* und R** weiter unten definiert sind, Cyclopropyl, Cyclopropyl-methyl, Phenyl, Benzyl, 1- oder 2-Phenylethyl, Phenoxy, 2-Phenoxy-ethyl oder einen 5- oder 6-gliedrigen monocyclischen oder 9- oder 10-gliedrigen bicyclischen Heterocyclus, enthaltend 1, 2 oder 3 4 Heteroatome ausgewählt aus der Gruppe bestehend aus O, N und S, wobei jeder der letztgenannten 9 Reste im cyclischen Teil gegebenenfalls durch einen oder mehrere gleiche oder verschiedene Reste R^{bb} substituiert ist, substituiert ist, bedeutet
- R^{C}, R^{D}: jeweils unabhängig voneinander (auch unabhängig von Resten R^{C}, R^{D} in anderen Gruppen)
Wasserstoff, (C₁-C₆)Alkyl, (C₂-C₆)Alkenyl oder (C₂-C₆)Alkinyl bedeutet, wobei jeder der letztgenannten 3 Reste unsubstituiert oder durch einen oder mehrere Reste aus der Gruppe Halogen, Cyano, Nitro, Hydroxy, (C₁-C₄)Alkoxy, (C₂-C₄)Alkenyloxy, (C₂-C₄)Alkinyloxy, (C₁-C₄)Haloalkoxy, (C₁-C₄)Alkoxy-(C₁-C₄)alkoxy, (C₁-C₄)Alkylthio, (C₁-C₆)Alkylsulfonyl und (C₁-C₆)Haloalkylsulfonyl substituiert ist,
oder
(C₃-C₆)Cycloalkyl, (C₅-C₆)Cycloalkenyl, (C₅-C₆)Cycloalkinyl, Phenyl, (C₃-C₆)Cycloalkyl-(C₁-C₆)alkyl, Phenyl-(C₁-C₄)alkyl, Phenoxy-(C₁-C₄)alkyl, oder Phenylamino-(C₁-C₆)alkyl, Reste Het¹, Het¹-(C₁-C₆)alkyl, Het¹-O-(C₁-C₆)alkyl, wobei Het¹ die genannte Bedeutung hat,
wobei jeder der letztgenannten 12 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
bedeuten,
- R^{aa}: jeweils unabhängig voneinander Wasserstoff, OH, (C₁-C₆)Alkyl, (C₁-C₄)Haloalkyl, (C₂-C₄)Alkenyl, (C₂-C₄)Alkinyl, (C₁-C₆)Alkoxy, (C₁-C₄)Alkoxy-(C₁-C₄)alkyl, (C₁-C₄)Alkoxy-(C₁-C₄)alkyloxy, (C₁-C₄)Haloalkoxy, (C₁-C₄)Haloalkoxy-(C₁-C₄)alkyl, (C₁-C₄)Haloalkoxy-(C₁-C₄)alkoxy, -NR*R*, wobei R* und R** wie oben definiert sind, (C₃-C₆)Cycloalkyl, (C₃-C₆)Cycloalkoxy, (C₃-C₆)Cycloalkyl-(C₁-C₆)alkyl, (C₃-C₆)Cycloalkyl-(C₁-C₆)alkoxy, Phenyl, Phenyl-(C₁-C₄)alkyl, Phenyl-(C₁-C₄)alkoxy, Phenoxy, Phenoxy-(C₁-C₄)alkyl, Phenoxy-(C₁-C₄)alkoxy, Phenylamino, Phenylamino-(C₁-C₄)alkyl, Phenylamino-(C₁-C₄)alkoxy oder einen gegebenenfalls über eine (C₁-C₄)Alkylengruppe oder eine (C₁-C₄)Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 14 Reste im cyclischen Teil gegebenenfalls durch einen oder mehrere gleiche oder verschiedene Reste R^{bb} substituiert ist, bedeutet und
- R^{bb}: jeweils unabhängig voneinander Halogen, (C₁-C₄)Alkyl, (C₁-C₄)Haloalkyl, (C₁-C₄)Alkoxy oder (C₁-C₄)Haloalkoxy, vorzugsweise Halogen, Methyl, CF₃, CCl₃. Methoxy, Ethoxy, OCH₂F, OCF₂H oder OCF₃ bedeutet.

Weiter bevorzugt sind dabei auch Verbindungen (I) oder deren Salze, worin
- R¹: H, (C₁-C₆)Alkyl, (C₂-C₆)Alkenyl, (C₂-C₆)Alkinyl, wobei jeder der letztgenannten 3 Reste unsubstituiert oder durch einen oder mehrere Reste aus der Gruppe Halogen, (C₁-C₄)Alkoxy, (C₁-C₄)Alkylthio, Alkylsulfinyl, Alkylsulfonyl, (C₃-C₆)Cycloalkyl, das unsubstituiert oder durch einen oder mehrere Reste aus der Gruppe Halogen und (C₁-C₄)Alkyl substituiert ist, und Phenyl, Phenoxy, Phenylthio, Phenylsulfinyl, Phenylsulfonyl, wobei der Phenylring in den letztgenannten 5 Resten jeweils unsubstituiert oder durch einen oder mehrere Reste aus der Gruppe Halogen, (C₁-C₄)Alkyl, (C₁-C₄)Alkoxy und (C₁-C₄)Haloalkyl substituiert ist, substituiert ist,
oder
- R¹: (C₃-C₆)Cycloalkyl, unsubstituiert oder durch einen oder mehrere Reste aus der Gruppe (C₁-C₄)Alkyl, (C₁-C₄)Alkoxy und (C₁-C₄)Haloalkyl substituiert ist,
bedeutet.

Besonders bevorzugt sind dabei auch Verbindungen (I) oder deren Salze, worin
- R¹: H, (C₁-C₄)Alkyl, (C₂-C₄)Alkenyl oder (C₂-C₄)Alkinyl, wobei jeder der letztgenannten 3 Reste unsubstituiert oder durch einen oder mehrere Reste aus der Gruppe Halogen, (C₁-C₄)Alkoxy, (C₁-C₄)Alkylthio, Cyclopropyl, Cyclobutyl, wobei jeder der letztgenannten beiden Reste unsubstituiert oder durch einen oder mehrere Reste aus der Gruppe Halogen und (C₁-C₄)Alkyl substituiert ist, und Phenyl, Phenylthio, Phenylsulfinyl, Phenylsulfonyl, wobei jeder der letztgenannten 4 Reste unsubstituiert oder durch einen oder mehrere Reste aus der Gruppe Halogen, (C₁-C₄)Alkyl, (C₁-C₄)Alkoxy und (C₁-C₄)Haloalkyl substituiert ist, substituiert ist,
bedeutet.

Weiter bevorzugt bedeutet
- R¹: auch einen mehrcyclischen Rest auf Basis von (C₃-C₉)Cycloalkyl, (C₅-C₉)Cycloalkenyl, (C₅-C₉)Cycloalkinyl oder Phenyl, wobei der Basisring mit einem carbocyclischen oder heterocyclischen Ring, vorzugsweise einem 5- oder 6-gliedrigen Ring mit 0 oder 1 bis 3 Heteroringatomen aus der Gruppe N, O und S kondensiert ist, vorzugsweise benzokondensiert ist, und wobei der Basisring oder das mehrcyclische System unsubstituiert oder durch einen oder mehrere Reste aus der Gruppe Halogen, Cyano, Nitro, (C₁-C₆)Alkyl, (C₁-C₆)Haloalkyl, (C₁-C₄)Alkoxy-(C₁-C₄)alkyl, (C₂-C₄)Alkenyl, (C₂-C₄)Alkinyl, (C₁-C₄)Alkoxy, (C₁-C₄)Haloalkoxy, (C₁-C₄)Alkoxy-(C₁-C₄)alkoxy, (C₁-C₄)Alkylthio, (C₃-C₆)Cycloalkyl, (C₃-C₆)Cycloalkoxy, [(C₁-C₄)Alkoxy]-carbonyl und [(C₁-C₄)Haloalkoxy]-carbonyl substituiert ist.

Bevorzugt sind auch Verbindungen (I) oder deren Salze, worin
- R¹: einen gesättigten, teilweise ungesättigten oder heteroaromatischen Heterocyclylrest mit 3 bis 9 Ringatomen, vorzugsweise mit 5 oder 6 Ringatomen, der 1 bis 4 Heteroatome, vorzugsweise 1 bis 3 Heteroringatome aus der Gruppe N, O und S enthält und der unsubstituiert oder durch einen oder mehrere Reste aus der Gruppe Halogen, Cyano, Thio, Nitro, Hydroxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
bedeutet.

Bevorzugt sind auch Verbindungen (I) oder deren Salze, worin
- R¹: einen Rest der Formel SiR^{a}R^{b}R^{c}, -NR^{a}R^{b} oder -N=CR^{c}R^{d}, vorzugsweise der Formel -NR^{a}R^{b} oder -N=CR^{c}R^{d},
wobei in den letztgenannten 5 Formeln jeder der Reste R^{a}, R^{b}, R^{c} und R^{d} unabhängig voneinander Wasserstoff, (C₁-C₄)Alkyl, (C₂-C₄)Alkenyl, (C₂-C₄)Alkinyl, Benzyl, substituiertes Benzyl, Phenyl oder substituiertes Phenyl bedeutet, dabei aber SiH₃ für SiR^{a}R^{b}R^{c} ausgenommen ist, oder R^{a} und R^{b} zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeuten oder R^{c} und R^{d} zusammen mit dem C-Atom einen 3- bis 8-gliedrigen carbocyclischen Rest oder heterocyclischen Rest, welcher 1 bis 3 Heteroringatome aus der Gruppe N, O und S enthalten kann, wobei der carbocyclische oder heterocyclischen Rest unsubstituiert oder durch einen oder mehrere Reste aus der Gruppe (C₁-C₄)Alkyl und (C₁-C₄)Haloalkyl substituiert ist, bedeuten,
bedeutet.

Besonders bevorzugt sind auch Verbindungen (I) oder deren Salze, worin
- R¹: H, Methyl, Ethyl, n-Propyl, i-Propyl, n-Butyl, s-Butyl, i-Butyl, t-Butyl, Allyl, Propargyl (Prop-2-in-1-yl), But-2-in-1-yl, But-3-in-1-yl, 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, 1-(2E)-1-methylbut-2-en-1-yl, (E)-Pent-3-en-2-yl oder (Z)-Pent-3-en-2-yl,
Phenyl, 2-Carboxy-phenyl, 2-Chlorphenyl, 3-Chlorphenyl, 4-Chlorphenyl, 2-Fluorphenyl, 3-Fluorphenyl, 4-Fluorphenyl, 3-Methoxyphenyl, 4-Methoxyphenyl, 2-Methoxyphenyl, 3-Methoxyphenyl, 4-Methoxyphenyl, Benzyl, 2-Fluoro-benzyl, 2-Fluoro-benzyl, 3-Fluoro-benzyl, 4-Fluoro-benzyl, 2,3-Difluoro-benzyl, 2,4-Difluoro-benzyl, 2,5-Difluoro-benzyl, 2,6-Difluoro-benzyl, 3,4-Difluoro-benzyl, 3,5-Difluoro-benzyl, 2-Phenyl-ethyl, 1-Phenyl-ethyl, (4-Chlorphenyl)-methyl [d. h. = CH₂(4-Cl-Ph)], (4-Fluorphenyl)-methyl [d. h. = CH₂(4-F-Ph)], (4-Methoxyphenyl)-methyl [d. h. = CH₂(4-OMe-Ph)], 2-Phenoxy-ethyl, 2-Phenylthio-ethyl, 2-Phenylsulfinyl-ethyl, 2-Phenylsulfonyl-ethyl,
2-Methoxyethyl, 2,2,2-Trifluorethyl, 1,1,1-Trifluorprop-2-yl, 2,2-Difluorethyl, 1,3-Difluorprop-2-yl, 2,3-Dimethoxypropyl, 2,3-Dimethoxyprop-2-yl, 2,2-Dimethoxy-eth-2-yl, 2-(2,2,2-Trifluorethoxy)-ethyl, 2-Fluorethyl, 2-Chlorethyl, 2-Bromethyl, 2-lodethyl, 2,2,3,3,3-Pentafluorpropyl, 1-Hydroxy-prop-2-yl, 2-Hydroxy-prop-2-yl, 2-Hydroxy-prop-1-yl, 3-Hydroxy-propyl, 3-Hydroxy-prop-2-yl,
(2-Methoxyethoxy)-methyl; 2-(2-Methoxyethoxy)-ethyl; (2-Ethoxyethoxy)-methyl; 2-(2-Ethoxyethoxy)-ethyl,
(Acetoxy)-methyl, (Propanoyloxy)-methyl, (2-Methylpropanoyloxy)-methyl, (2,2-Dimethylpropanoyloxy)-methyl, 1-(Acetoxy)-ethyl, 2-(Acetoxy)-ethyl, 2-(Propanoyloxy)-ethyl, 1-(Propanoyloxy)-ethyl, 1-(2-Methylpropanoyloxy)-eth-1-yl, 2-(2-Methylpropanoyloxy)-eth-1-yl, 2-(2,2-Dimethylpropanoyloxy)-ethyl [d. h. 1-(t-Butylcarbonyloxy)-ethyl], 2-(2,2-Dimethylpropanoyloxy)-ethyl; 1-(2,2-Dimethylpropanoyloxy)-2-methylprop-1-yl, 1-(t-Butylcarbonyloxy)-2-methylprop-1-yl,
(Methoxycarbonyl)-methyl, (Ethoxycarbonyl)-methyl, (n-Propoxycarbonyl)-methyl, (i-Propoxycarbonyl)-methyl, (n-Butoxycarbonyl)-methyl, (s-Butoxycarbonyl)-methyl, (i-Butoxycarbonyl)-methyl, (t-Butoxycarbonyl)-methyl, 1-(Methoxycarbonyl)-ethyl, 2-(Methoxycarbonyl)-ethyl, 1-(Ethoxycarbonyl)-ethyl, 2-(Ethoxycarbonyl)-ethyl, 1-(n-Propoxycarbonyl)-ethyl, 2-(n-Propoxycarbonyl)-ethyl, 1-(i-Propoxycarbonyl)-ethyl, 2-(i-Propoxycarbonyl)-ethyl, 1-(n-Butoxycarbonyl)-ethyl, 2-(n-Butoxycarbonyl)-ethyl, 1-(s-Butoxycarbonyl)-ethyl, 2-(s-Butoxycarbonyl)-ethyl, 1-(i-Butoxycarbonyl)-ethyl, 2-(i-Butoxycarbonyl)-ethyl, 1-(t-Butoxycarbonyl)-ethyl, 2-(t-Butoxycarbonyl)-ethyl,
(Methoxycarbonyloxy)-methyl, (Ethoxycarbonyloxy)-methyl, (n-Propoxycarbonyloxy)-methyl, (i-Propoxycarbonyloxy)-methyl, (n-Butoxycarbonyloxy)-methyl, (s-Butoxycarbonyloxy)-methyl, (i-Butoxycarbonyloxy)-methyl, (t-Butoxycarbonyloxy)-methyl, 1-(Methoxycarbonyloxy)-ethyl, 2-(Methoxycarbonyloxy)-ethyl, 1-(Ethoxycarbonyloxy)-ethyl, 2-(Ethoxycarbonyloxy)-ethyl, 1-(n-Propoxycarbonyloxy)-ethyl, 2-(n-Propoxycarbonyloxy)-ethyl, 1-(i-Propoxycarbonyloxy)-ethyl, 2-(i-Propoxycarbonyloxy)-ethyl, 1-(n-Butoxycarbonyloxy)-ethyl, 2-(n-Butoxycarbonyloxy)-ethyl, 1-(s-Butoxycarbonyloxy)-ethyl, 2-(s-Butoxycarbonyloxy)-ethyl, 1-(i-Butoxycarbonyloxy)-ethyl, 2-(i-Butoxycarbonyloxy)-ethyl, 1-(t-Butoxycarbonyloxy)-ethyl, 2-(t-Butoxycarbonyloxy)-ethyl, (Cyclohexoxycarbonyloxy)-methyl, 1-(Cyclohexoxycarbonyloxy)-eth-1-yl, 2-(Cyclohexoxycarbonyloxy)-eth-1-yl,
(Acetyl)-methyl, 1-(Acetyl)-ethyl, 2-(Acetyl)-ethyl, 1-(Acetyl)-propyl, 2-(Acetyl)-propyl, 3-(Acetyl)-propyl, (Propanoyl)-methyl, 1-(Propanoyl)-ethyl, 2-(Propanoyl)-ethyl, 1-(Propanoyl)-propyl, 2-(Propanoyl)-propyl, 3-(Propanoyl)-propyl, 1-(Propanoyl)-2-methyl-propyl,
2-(Ethylidenaminooxy)-ethyl, 2-(Prop-2-ylidenaminooxy)-ethyl, 2-(But-2-ylidenaminooxy)-ethyl, 2-(Pent-3-ylidenaminooxy)-ethyl, (N,N-Dimethylamino)-methyl, 2-(N,N-Dimethylamino)-eth-1-yl, 1-(N,N-Dimethylamino)-eth-1-yl, 2-(N,N-Diethylamino)-eth-1-yl, 1-(N,N-Diethylamino)-eth-1-yl, (N,N-Diethylamino)-methyl, (N,N-Dimethylaminocarbonyl)-methyl, 1-(N,N-Dimethylaminocarbonyl)-ethyl, 2-(N,N-Dimethylaminocarbonyl)-ethyl, (N,N-Diethylaminocarbonyl)-methyl, 1-(N,N-Diethylaminocarbonyl)-ethyl, 2-(N,N-Diethylaminocarbonyl)-ethyl, 1-(Dimethylamino)-prop-2-yl [d. h. 2-(Dimethylamino)-1-methyl-ethyl], 1-(Diethylamino)-prop-2-yl,
Trimethylsilyl-methyl, 1-(Trimethylsilyl)-ethyl, 2-(Trimethylsilyl)-ethyl, Triethylsilyl-methyl, 1-(Triethylsilyl)-ethyl, 2-(Triethylsilyl)-ethyl, Cyclopropyl, Cyclopropylmethyl, 1-Cyclopropyl-ethyl, 2-Cyclopropyl-ethyl, (1-Methyl-cyclopropyl)-methyl, 1-(1-Methyl-cyclopropyl)-ethyl, 2-(1-Methyl-cyclopropyl)-ethyl, (2,2-Dichlorcyclopropyl)-methyl, 1-(2,2-Dichlorcyclopropyl)-ethyl, 2-(2,2-Dichlorcyclopropyl)-ethyl, (2,2-Dimethyl-cyclopropyl)-methyl, 1-(2,2-Dimethyl-cyclopropyl)-ethyl, 2-(2,2-Dimethyl-cyclopropyl)-ethyl, Cyclobutyl-methyl, Cyclopentyl-methyl, Cyclohexyl-methyl oder Pyrid-2-yl, Pyrid-3-yl, Pyrid-4-yl, 2-Chlorpyrid-3-yl, 3-Chlorpyrid-2-yl, Thien-2-yl, Thien-3-yl, 2-Chlorthien-3-yl, 3-Chlorthien-2-yl, 4-Chlorthien-2-yl, (1-Ethyl-5-methyl-1H-pyrazol-4-yl)-methyl, 1-(1-Ethyl-5-methyl-1H-pyrazol-4-yl)-ethyl, 2-(1-Ethyl-5-methyl-1H-pyrazol-4-yl)-ethyl (1-Ethyl-3-methyl-1H-pyrazol-4-yl)-methyl, 1-(1-Ethyl-3-methyl-1H-pyrazol-4-yl)-ethyl, 2-(1-Ethyl-3-methyl-1H-pyrazol-4-yl)-ethyl,
Tetrahydrofuran-2-yl, Tetrahydrofuran-3-yl, Tetrahydrofuran-2-yl-methyl, Tetrahydrofuran-3-yl-methyl, (5-Methyl-2-oxo-1,3-dioxol-4-yl)methyl; Oxetan-3-yl, (Oxetan-3-yl)methyl, (Oxetan-2-yl)methyl, (1,3-Dioxolan-2-yl)-methyl, (1,3-Dioxolan-4-yl)-methyl, 5-Methyl-2-oxo-1,3-dioxolan-4-yl)methyl, (Morpholin-4-yl)-methyl; 1-(Morpholin-4-yl)-ethyl, 2-(Morpholin-4-yl)-ethyl, 2,3-Dihydro-1H-inden-2-yl, Dihydro-1H-inden-3-yl, Dihydro-1H-inden-4-yl, Dihydro-1H-inden-5-yl,
1H-Inden-2-yl, 1H-Inden-3-yl, 1H-Inden-4-yl, 1H-Inden-5-yl, 1H-Inden-6-yl oder 1H-Inden-7-yl
bedeutet.

Ganz besonders bevorzugt sind dabei Verbindungen (I) und deren Salze, worin
- R¹: H, Methyl, Ethyl, n-Propyl, i-Propyl, n-Butyl, s-Butyl, i-Butyl, t-Butyl, Phenyl, Benzyl, CH₂(4-Cl-Ph), d. h. (4-Chlorphenyl)-methyl, CH₂(4-F-Ph), d. h. (4-Fluorphenyl)-methyl, CH₂(4-OMe-Ph), d. h. (4-Methoxyphenyl)-methyl, 2-Fluoro-benzyl, 2-Fluoro-benzyl, 3-Fluoro-benzyl, 4-Fluoro-benzyl, 2,3-Difluoro-benzyl, 2,4-Difluoro-benzyl, 2,5-Difluoro-benzyl, 2,6-Difluoro-benzyl, 3,4-Difluoro-benzyl, 3,5-Difluoro-benzyl, 2-Phenoxy-ethyl, 2-Phenylthio-ethyl, 2-Phenylsulfinyl-ethyl, 2-Phenylsulfonyl-ethyl, 2-Methoxyethyl, Tetrahydrofuran-2-yl-methyl, 2-(Dimethylamino)ethyl, Oxetan-3-yl, (3-Methyloxetan-3-yl)methyl, 2,2,2-Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2-Dichlorcyclopropyl)-methyl, (2,2-Dimethyl-cyclopropyl)-methyl, Allyl, Propargyl (Prop-2-in-1-yl), 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, 1-(2E)-1-methylbut-2-en-1-yl, (E)-Pent-3-en-2-yl oder (Z)-Pent-3-en-2-yl, Cyclobutylmethyl, Cyclopentylmethyl, Cyclohexylmethyl oder 1-Ethyl-5-methyl-1H-pyrazol-4-methyl, d. h. (1-Ethyl-5-methyl-1H-pyrazol-4-yl)-methyl,
bedeutet.

Ganz besonders bevorzugt sind dabei Verbindungen (I) und deren Salze, worin
- R¹: H, Methyl, Ethyl n-Butyl, s-Butyl, i-Butyl, t-Butyl, Allyl und Propargyl,
insbesondere Methyl oder Ethyl bedeutet.

Weiterhin bevorzugt sind Verbindungen (I), worin
- (R²)ₙ: n Substituenten R² bedeutet,
wobei R² (wenn n = 1) oder jeder der Substituenten R² (wenn n größer als 1) unabhängig voneinander Halogen, Cyano, Nitro, (C₁-C₆)Alkyl, (C₁-C₆)Alkoxy, (C₁-C₆)Alkylthio, (C₁-C₆)Alkylsulfinyl, (C₁-C₆)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, Tri-[(C₁-C₄)alkyl]-silyl oder Tri-[(C₁-C₄)alkyl]-silyl-(C₁-C₄)alkyl bedeutet oder wobei jeweils zwei am Ring ortho-ständige Gruppen R² gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht, wobei die Gruppe -Z¹-A*-Z² zusammen mit den an die Gruppe gebundenen C-Atomen des Phenylrings einen ankondensierten 5 oder 6 Ring bilden, und
- (R³)ₘ: m Substituenten R³ bedeutet, wobei R³ (wenn m = 1) oder jeder Substituenten R³ (wenn m größer als 1) unabhängig voneinander Halogen, Cyano, Nitro, (C₁-C₆)Alkyl, (C₁-C₆)Alkoxy, (C₁-C₆)Alkylthio, (C₁-C₆)Alkylsulfinyl, (C₁-C₆)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, NR*R**, Tri-[(C₁-C₄)alkyl]-silyl oder Tri-[(C₁-C₄)alkyl]-silyl-(C₁-C₄)alkyl bedeutet oder wobei jeweils zwei am Ring ortho-ständige Gruppen R³ gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht, wobei die Gruppe -Z³-A**-Z⁴ zusammen mit den an die Gruppe gebundenen C-Atomen des Phenylrings einen ankondensierten 5 oder 6 Ring bilden,
- R*, R**: jeweils unabhängig voneinander oder gemeinsam mit dem N-Atom die genannte Bedeutung haben und
- n, m: jeweils unabhängig voneinander 0, 1, 2, 3, 4 oder 5, vorzugsweise 0, 1, 2 oder 3, insbesondere 0, 1 oder 2 bedeuten, mit der Maßgabe, dass nicht beide gleichzeitig 0 bedeuten.

Bevorzugt sind dabei auch Verbindungen (I) und deren Salze, worin
- (R²)ₙ: n Substituenten R² bedeutet,
wobei, im Falle dass n = 1 ist, der Rest R² oder, im Falle dass n größer als 1 ist, jeder der Substituenten R² unabhängig voneinander
Halogen, Cyano, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Alkenyl, (C₂-C₄)Alkinyl, Tri-[(C₁-C₄)Alkyl]-silyl oder Tri-[(C₁-C₄)Alkyl]-silyl -(C₁-C₄)Alkyl bedeutet,
vorzugsweise Fluor, Chlor, Brom, Iod, Cyano, Nitro, Methyl, Ethyl, Methoxy, Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, insbesondere Halogen wie Fluor oder Chlor bedeutet, und
- n: 0, 1, 2, 3, 4 oder 5, vorzugsweise 0, 1, 2 oder 3, insbesondere 0, 1 oder 2 bedeutet, mit der Maßgabe, dass n und m nicht beide gleichzeitig 0 bedeuten.

Bevorzugt sind dabei auch Verbindungen (I) und deren Salze, worin
- (R³)ₘ: m Substituenten R³ bedeutet,
wobei, im Falle dass m = 1 ist, der Rest R³ oder, im Falle dass m größer als 1 ist, jeder der Substituenten R³ unabhängig voneinander Halogen, Cyano, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₆)Alkenyl, (C₂-C₆)Alkinyl oder Tri-[(C₁-C₄)Alkyl]silyl-Z^{b}-, wobei Z^{b} = für eine kovalente Bindung oder (C₁-C₄)Alkylen steht, bedeutet oder
jeweils zwei am Ring ortho-ständige Gruppen R³ gemeinsam eine Gruppe der Formel -Z³-A**-Z⁴ bedeuten, wobei
A** für eine Alkylengruppe steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für O oder S steht und
- Z⁴: für O oder S steht, wobei die Gruppe -Z³-A**-Z⁴ zusammen mit den an die Gruppe gebundenen C-Atomen des Phenylrings einen ankondensierten 5 oder 6 Ring bilden,
vorzugsweise jeder der Substituenten R³ unabhängig voneinander Halogen, Cyano, Nitro, Methyl, Ethyl, Methoxy, Ethoxy, Methylthio, Ethylthio, (C₁-C₂)Alkylsulfinyl, (C₁-C₂)Alkylsulfonyl, (C₁-C₂)Haloalkyl, (C₁-C₂)Haloalkoxy, (C₁-C₂)Haloalkylthio, (C₁-C₂)Haloalkylsulfinyl, (C₁-C₂)Haloalkylsulfonyl, (C₂-C₄)Alkenyl, (C₂-C₄)Alkinyl, Tri-[(C₁-C₂)Alkyl]-silyl-Z^{b}, wobei Z^{b} = für eine kovalente Bindung oder (C₁-C₂)Alkylen steht, bedeutet,
insbesondere jeder der Substituenten R³ unabhängig voneinander Halogen, wie Fluor, Chlor, Brom, Iod, Cyano, Nitro, Methyl, Methoxy, Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, insbesondere Halogen wie Fluor oder Chlor bedeutet und
- m: 0, 1, 2, 3, 4 oder 5, vorzugsweise 0, 1, 2, 3 oder 4, insbesondere 0, 1, 2 oder 3 bedeutet, mit der Maßgabe, dass n und m in Formel (I) nicht beide gleichzeitig 0 bedeuten.

Weiter bevorzugt sind Verbindungen der Formel (I) oder deren Salze, worin
- n: 0 (= die Zahl Null, d. h. keine Substituenten R² vorhanden, d. h. alle freien Bindungen am Ring sind mit Wasserstoff besetzt) bedeutet oder vorzugsweise
- (R²)ₙ: 2-Brom, 3-Brom, 4-Brom, 2-Chlor, 3-Chlor, 4-Chlor, 2-Fluor, 3-Fluor, 4-Fluor, 2-Cyano, 3-Cyano, 4-Cyano, 2-Methyl, 3-Methyl, 4-Methyl, 2-Ethyl, 3-Ethyl, 4-Ethyl, 2-CF₃, 3-CF₃, 4-CF₃, 2-Methoxy, 3-Methoxy, 4-Methoxy, 2-Ethoxy, 3-Ethoxy, 4-Ethoxy, 2-Methylthio, 3-Methylthio, 4-Methylthio, 2-Methylsulfinyl, 3-Methylsulfinyl, 4-Methylsulfonyl, 2-Methylsulfonyl, 3-Methylsulfonyl, 4-Methylsulfonyl, 2,3-Dimethyl, 2,4-Dimethyl, 2,5-Dimethyl, 2,6-Dimethyl, 3,4-Dimethyl, 3,5-Dimethyl, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor oder 3,4,5-Trichlor oder auch (4-Br-3-F), (3-Br-4-F), (3-Br-5-F), (4-Br-3-Cl), (3-Br-4-Cl), (4-CN-3-F), (3-CN-4-F), (3-CN-5-F), (4-CN-3-Cl) oder (3-CN-4-Cl) bedeutet, wobei die Bezifferung der Reste sich auf die Position des Restes am Phenyl-1-yl-Rest bezieht, in dem das C-Atom, das in 4-Position am Buttersäure-Grundkörper gebunden ist, die 1-Position im Ring hat..

Weiter bevorzugt sind Verbindungen der Formel (I) oder deren Salze, worin
- m: 0 (= die Zahl Null, d. h. keine Substituenten R³ vorhanden, d. h. alle freien Bindungen am Ring sind mit Wasserstoff besetzt) bedeutet oder vorzugsweise
- (R³)ₘ: 2-Brom, 3-Brom, 4-Brom, 2-Chlor, 3-Chlor, 4-Chlor, 2-Fluor, 3-Fluor, 4-Fluor, 2-Cyano, 3-Cyano, 4-Cyano, 2-Methyl, 3-Methyl, 4-Methyl, 2-Ethyl, 3-Ethyl, 4-Ethyl, 2-CF₃, 3-CF₃, 4-CF₃, 2-Methoxy, 3-Methoxy, 4-Methoxy, 2-Ethoxy, 3-Ethoxy, 4-Ethoxy, 2-Methylthio, 3-Methylthio, 4-Methylthio, 2-Methylsulfinyl, 3-Methylsulfinyl, 4-Methylsulfonyl, 2-Methylsulfonyl, 3-Methylsulfonyl, 4-Methylsulfonyl, 2,3-Dimethyl, 2,4-Dimethyl, 2,5-Dimethyl, 2,6-Dimethyl, 3,4-Dimethyl, 3,5-Dimethyl, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor oder 3,4,5-Trichlor oder auch 2-Nitro, 3-Nitro, 4-Nitro, 2,5-Dicyano, 2,6-Dicyano, (4-Br-2-F), (4-Br-3-F), (4-CN-3-F), (4-NO₂-3-F), (4-OMe-3-F), (3-CN-4-F), (3-NO₂-4-F), (3-CN-4-Cl), (3-NO₂-4-Cl) oder (5-CN-2-F) bedeutet, wobei die Bezifferung der Reste sich auf die Position des Restes am Phenyl-1-yl-Rest bezieht, in dem das C-Atom, das in 3-Position am Buttersäure-Grundkörper gebunden ist, die 1-Position im Ring hat.

Weiter bevorzugt sind Verbindungen der Formel (I) oder deren Salze, worin
- (R²)ₙ: 3-Brom, 4-Brom, 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F), (4-Cl-3-F), 3,4,5-Trifluor oder 3,4,5-Trichlor oder auch 3-Cyano, 4-Cyano, (3-Br-4-F), (4-Br-3-F), (3-CN-4-F) oder (4-CN-3-F) bedeutet wobei die Bezifferung der Reste sich auf die Position des Restes am Phenyl-1-yl-Rest bezieht, in dem das C-Atom, das in 4-Position am Buttersäure-Grundkörper gebunden ist, die 1-Position im Ring hat.

Dabei sind insbesondere bevorzugt:
Verbindungen der Formel (I) oder deren Salze, worin (R²)ₙ 3-Chlor bedeutet;
Verbindungen der Formel (I) oder deren Salze, worin (R²)ₙ 4-Chlor bedeutet;
Verbindungen der Formel (I) oder deren Salze, worin (R²)ₙ 3-Fluor bedeutet;
Verbindungen der Formel (I) oder deren Salze, worin (R²)ₙ 4-Fluor bedeutet;
Verbindungen der Formel (I) oder deren Salze, worin (R²)ₙ 3-Cyano bedeutet;
Verbindungen der Formel (I) oder deren Salze, worin (R²)ₙ 3,4-Difluor bedeutet;
Verbindungen der Formel (I) oder deren Salze, worin (R²)ₙ 3,4-Dichlor bedeutet;
Verbindungen der Formel (I) oder deren Salze, worin (R²)ₙ 3,5-Difluor bedeutet;
Verbindungen der Formel (I) oder deren Salze, worin (R²)ₙ 3,5-Dichlor bedeutet;
Verbindungen der Formel (I) oder deren Salze, worin (R²)ₙ (3-CI-4-F) bedeutet;
Verbindungen der Formel (I) oder deren Salze, worin (R²)ₙ (3-CI-5-F) bedeutet;
Verbindungen der Formel (I) oder deren Salze, worin (R²)ₙ (4-CI-3-F) bedeutet.

Weiter bevorzugt sind Verbindungen der Formel (I) oder deren Salze, worin
- (R³)ₘ: 2-Chlor, 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor oder 3,4,5-Trichlor oder auch 2-Cyano, 3-Cyano, 4-Cyano, 2-Nitro, 3-Nitro oder 4-Nitro bedeutet, wobei die Bezifferung der Reste sich auf die Position des Restes am Phenyl-1-yl-Rest bezieht, in dem das C-Atom, das in 3-Position am Buttersäure-Grundkörper gebunden ist, die 1-Position im Ring hat.

Dabei sind insbesondere bevorzugt:
Verbindungen der Formel (I) oder deren Salze, worin (R³)ₘ 3-Chlor bedeutet;
Verbindungen der Formel (I) oder deren Salze, worin (R³)ₘ 4-Chlor bedeutet;
Verbindungen der Formel (I) oder deren Salze, worin (R³)ₘ 2-Fluor bedeutet;
Verbindungen der Formel (I) oder deren Salze, worin (R³)ₘ 3-Fluor bedeutet;
Verbindungen der Formel (I) oder deren Salze, worin (R³)ₘ 4-Fluor bedeutet;
Verbindungen der Formel (I) oder deren Salze, worin (R³)ₘ 2,3-Difluor bedeutet;
Verbindungen der Formel (I) oder deren Salze, worin (R³)ₘ 2,4-Difluor bedeutet;
Verbindungen der Formel (I) oder deren Salze, worin (R³)ₘ 2,5-Difluor bedeutet;
Verbindungen der Formel (I) oder deren Salze, worin (R³)ₘ 2,6-Difluor bedeutet;
Verbindungen der Formel (I) oder deren Salze, worin (R³)ₘ 3,4-Difluor bedeutet;
Verbindungen der Formel (I) oder deren Salze, worin (R³)ₘ 3,5-Difluor bedeutet;
Verbindungen der Formel (I) oder deren Salze, worin (R³)ₘ (3-Cl-2-F) bedeutet;
Verbindungen der Formel (I) oder deren Salze, worin (R³)ₘ (3-Cl-4-F) bedeutet;
Verbindungen der Formel (I) oder deren Salze, worin (R³)ₘ (3-Cl-5-F) bedeutet;
Verbindungen der Formel (I) oder deren Salze, worin (R³)ₘ (3-Cl-6-F) bedeutet;
Verbindungen der Formel (I) oder deren Salze, worin (R³)ₘ (4-Cl-2-F) bedeutet;
Verbindungen der Formel (I) oder deren Salze, worin (R³)ₘ (4-Cl-3-F) bedeutet.

Weiter bevorzugt sind:
Verbindungen der Formel (I) oder deren Salze, worin (R²)ₙ 3-Chlor und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-CI-2-F) oder (4-CI-3-F) bedeuten;
Verbindungen der Formel (I) oder deren Salze, worin (R²)ₙ 4-Chlor und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten;
Verbindungen der Formel (I) oder deren Salze, worin (R²)ₙ 3-Fluor und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten;
Verbindungen der Formel (I) oder deren Salze, worin (R²)ₙ 3-Cyano und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten;
Verbindungen der Formel (I) oder deren Salze, worin (R²)ₙ 4-Fluor und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten;
Verbindungen der Formel (I) oder deren Salze, worin (R²)ₙ 3,4-Difluor und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten;
Verbindungen der Formel (I) oder deren Salze, worin (R²)ₙ 3,4-Dichlor und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten;
Verbindungen der Formel (I) oder deren Salze, worin (R²)ₙ 3,5-Difluor und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten;
Verbindungen der Formel (I) oder deren Salze, worin (R²)ₙ 3,5-Dichlor und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten;
Verbindungen der Formel (I) oder deren Salze, worin (R²)ₙ (3-Cl-4-F) und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten;
Verbindungen der Formel (I) oder deren Salze, worin (R²)ₙ (4-Cl-3-F) und (R³)ₘ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten.

Auch weiter bevorzugt sind:
Verbindungen der Formel (I) oder deren Salze, worin (R³)ₘ 3-Chlor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ 4-Chlor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ 3-Fluor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ 4-Fluor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ 2,3-Difluor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ 2,4-Difluor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ 2,5-Difluor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ 2,6-Difluor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ 3,4-Difluor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ 3,5-Difluor und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ (3-Cl-2-F) und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ (3-Cl-4-F) und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ (3-Cl-5-F) und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ (3-Cl-6-F) und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ (4-Cl-2-F) und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten;
Verbindungen der Formel (I) oder deren Salze, worin (R³)ₘ (4-Cl-3-F) und (R²)ₙ 3-Chlor, 4-Chlor, 3-Fluor, 4-Fluor, 3,4-Difluor, 3,5-Difluor, 3,4-Dichlor, 3,5-Dichlor, (3-Cl-4-F), (3-Cl-5-F) oder (4-Cl-3-F) oder auch 3-Cyano bedeuten.

Weiter bevorzugt sind dabei auch Verbindungen der Formel (I) oder deren Salze, worin
- (R³)ₘ: 3-Chlor, 4-Chlor, 2-Fluor, 3-Fluor, 4-Fluor, 2,3-Difluor, 2,4-Difluor, 2,5-Difluor, 3,4-Difluor, 3,5-Difluor, 3,4-Dichlor, 3,5-Dichlor, (3-Cl-2-F), (3-Cl-4-F), (3-Cl-5-F), (3-Cl-6-F), (4-Cl-2-F) oder (4-Cl-3-F) oder auch 3-Cyano, 4-Cyano, 3-Nitro oder 4-Nitro bedeutet, wobei die Bezifferung der Reste sich auf die Position des Restes am Phenyl-1-yl-Rest bezieht, in dem das C-Atom, das in 3-Position am Buttersäure-Grundkörper gebunden ist, die 1-Position im Ring hat.

Weiter bevorzugt sind:
Verbindungen der Formel (I) oder deren Salze, worin (R²)ₙ 3-Chlor und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten;
Verbindungen der Formel (I) oder deren Salze, worin (R²)ₙ 4-Chlor und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten;
Verbindungen der Formel (I) oder deren Salze, worin (R²)ₙ 3-Fluor und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten;
Verbindungen der Formel (I) oder deren Salze, worin (R²)ₙ 4-Fluor und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten;
Verbindungen der Formel (I) oder deren Salze, worin (R²)ₙ 3,4-Difluor und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten;
Verbindungen der Formel (I) oder deren Salze, worin (R²)ₙ 3,4-Dichlor und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten;
Verbindungen der Formel (I) oder deren Salze, worin (R²)ₙ 3,5-Difluor und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten;
Verbindungen der Formel (I) oder deren Salze, worin (R²)ₙ 3,5-Dichlor und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten;
Verbindungen der Formel (I) oder deren Salze, worin (R²)ₙ (3-Cl-4-F) und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten;
Verbindungen der Formel (I) oder deren Salze, worin (R²)ₙ (4-Cl-3-F) und (R³)ₘ 3-Chlor, 4-Chlor, 2-Fluor, 3-Fluor, 4-Fluor, 2,3-Difluor, 2,4-Difluor, 2,5-Difluor, 3,4-Difluor oder 3,5-Difluor oder auch 3-Cyano, 4-Cyano, 3-Nitro oder 4-Nitro bedeuten.

Weiter bevorzugt sind Verbindungen der Formel (I) oder deren Salze, worin n = 1 bedeutet.
Weiter bevorzugt sind Verbindungen der Formel (I) oder deren Salze, worin n = 1 und R² Halogen, wie Fluor oder Chlor, bedeuten,
Weiter bevorzugt sind Verbindungen der Formel (I) oder deren Salze, worin n = 2 oder 3, insbesondere 2 bedeutet.
Weiter bevorzugt sind Verbindungen der Formel (I) oder deren Salze, worin n = 2 und jedes R² aus der Gruppe Halogen ausgewählt ist und vorzugsweise Fluor oder Chlor, insbesondere Fluor bedeutet.

Generell sind unter den Verbindungen mit den obengenannten Bedeutungen für einzelne Gruppen oder Kombinationen von Gruppen R¹, (R²)ₙ beziehungsweise (R³)ₘ solche bevorzugt, in denen die übrigen Gruppen oder Kombinationen von Gruppen in den Verbindungen gemäß bevorzugt genannten Bedeutungen definiert sind.

Die erfindungsgemäßen Verbindungen der Formel (I) umfassen alle Stereoisomeren, welche aufgrund der Asymmetriezentren oder Doppelbindungen im Molekül, deren Konfiguration in der Formel nicht speziell bezeichnet oder die nicht speziell angegeben sind, auftreten können, und deren Mischung, inklusive der racemischen Verbindungen und der teilweise mit bestimmten Stereoisomeren angereicherten Mischungen.

Die Erfindung umfasst auch alle Tautomeren, wie Keto- und Enol-Tautomeren, und deren Mischungen und Salze, wenn entsprechende funktionelle Gruppen vorhanden sind.

Die erfindungsgemäßen Verbindungen der Formel (I) können alternativ durch verschiedene Verfahren dargestellt werden.

In den nachfolgenden Verfahren werden partiell Lösemittel (gleichbedeutend mit "Lösungsmittel") verwendet. In diesem Zusammenhang bezeichnen "inerte Lösemittel" jeweils Lösemittel, die unter den jeweiligen Reaktionsbedingungen inert sind, jedoch nicht unter beliebigen Reaktionsbedingungen inert sein müssen.

In den nachfolgenden Verfahren können die beschriebenen Reaktionen alternativ auch in einem Mikrowellenofen durchgeführt werden.

Gegenstand der vorliegenden Erfindung sind auch Verfahren zur Herstellung der Verbindungen der allgemeinen Formel (I) und/oder deren Salze. Dazu gehören Verfahren, die analog bekannter Methoden durchgeführt werden.

Zur Herstellung der erfindungsgemäßen Verbindungen (I) können zunächst die entsprechenden Diastereomerengemische in Form ihrer racemischen Gemische verwendet werden. Die Herstellung der Diastereomerengemische der Cyanobutyrate ist im Prinzip bekannt; siehe beispielsweise EP-A 5341, EP-A 266725, EP-A270 830, JP 04/297454, JP 04/297455, JP 05/058979, WO 2011/003776, WO 2011/003775. Analog den in den zitierten Schriften beschriebenen Syntheserouten lassen sich die Verbindungen nach Standardverfahren der organischen Chemie hergestellen.

Beispielsweise werden Diastereomerengemische [Formel (I')] mit einem Gehalt an der herzustellenden Verbindung (I) erhalten, dadurch gekennzeichnet, dass man
(a) Verbindungen der Formel (II) ("Cyanomethylbenzole"/"Phenylacetonitrile") 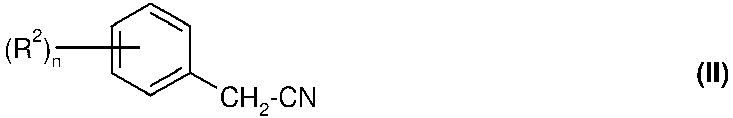 mit Verbindungen der Formel (III) (Zimtsäurederivaten) oder deren Salzen, 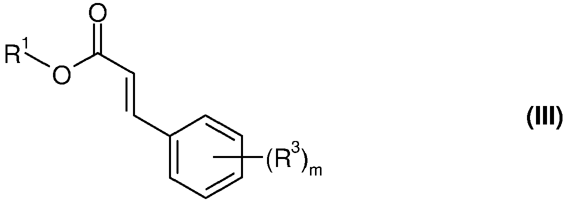 zu Verbindungen der Formel (I') (Diastereomere/racemisch) 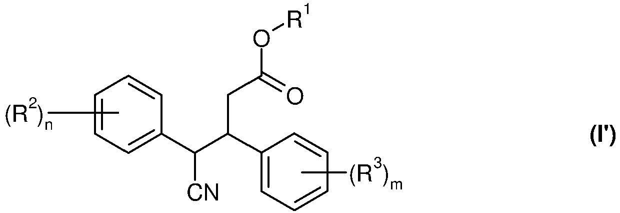 umsetzt, wobei R¹, R², R³, m und n in den Verbindungen (II), (III) und (I') wie in der jeweils herzustellenden Verbindung der Formel (I) definiert sind.

Die für die Herstellung der Verbindungen (I') und (I) benötigten Ausgangsstoffe (II) und (III) sind gemäß der zitierten Literatur bekannt oder können analog der zitierten Literatur hergestellt werden.

Die Umsetzung nach Variante (a) kann beispielsweise nach Methoden und unter Bedingungen, wie sie für Michael-Additionen bekannt sind, durchgeführt werden. Die Umsetzung erfolgt beispielsweise bei Temperaturen von -100°C bis 150°C, vorzugsweise -78°C bis 100°C, in einem organischen oder anorganischen Lösungsmittel, in der Regel in Gegenwart einer Base oder eines Katalysators oder beidem [vgl. J. Chem. Soc. (1945), S. 438].

Geeignete Lösungsmittel sind beispielsweise organische Lösungsmittel wie:
- aliphatische Kohlenwasserstoffe wie Pentan, Hexan, Cyclohexan oder Petrolether;
- aromatische Kohlenwasserstoffe wie Toluol, o-, m- oder p-Xylol,
- halogenierte Kohlenwasserstoffe wie Methylenchlorid, Chloroform oder Chlorbenzol,
- Ether wie Diethylether, Diisopropylether, tert.-Butylmethylether, Dioxan, Anisol und Tetrahydrofuran (THF),
- Nitrile wie Acetonitril oder Propionitril,
- Ketone wie Aceton, Methylethylketon, Diethylketon oder tert.-Butylmethylketon,
- Alkohole wie Methanol, Ethanol, n-Propanol, Isopropanol, n-Butanol und tert.-Butanol, sowie
- Dimethylsulfoxid, Dimethylformamid, Dimethylacetamid, Sulfolan,
- Gemische aus den genannten organischen Lösungsmitteln.

Geeignet sind in Einzelfällen auch anorganische Lösungsmittel wie Wasser oder Gemische organischer Lösungsmittel mit Wasser.

Bevorzugte Lösungsmittel sind THF und Methanol und deren Gemische mit anderen organischen Lösungsmitteln.

Die Umsetzung nach Herstellungsvariante (a) erfolgt vorzugsweise in Gegenwart einer Base, beispielsweise aus der Gruppe anorganischer Verbindungen wie der Alkalimetall- und Erdalkalimetallhydroxide, zum Beispiel (z. B.) Lithiumhydroxid, Natriumhydroxid, Kaliumhydroxid oder Calziumhydroxid, der Alkalimetall- und Erdalkalimetalloxide, zum Beispiel Lithiumoxid, Natriumoxid, Calziumoxid oder Magnesiumoxid, der Alkalimetall- und Erdalkalimetallhydride, z. B. Lithiumhydrid, Natriumhydrid, Kaliumhydrid oder Calziumhydrid, der Alkalimetallamide, zum Beispiel Lithiumamid, Natriumamid oder Kaliumamid, der Alkalimetall- und Erdalkalimetallcarbonate, z. B. Lithiumcarbonat, Kaliumcarbonat oder Calziumcarbonat, der Alkalimetallhydrogencarbonate, z. B. Natriumhydrogencarbonat, oder der metallorganischen Verbindungen wie vorzugsweise der Alkalimetallalkyle, z. B. Methyllithium, Butyllithium oder Phenyllithium, der Alkylmagnesiumhalogenide, z. B. Methylmagnesiumchlorid, oder der Alkalimetall- und Erdalkalimetallalkoholate, z. B. Natriummethanolat, Natriumethanolat, Kaliumethanolat, Kalium- tert.Butanolat oder Dimethoxymagnesium.

Auch können als Basen organische Basen eingesetzt werden, beispielsweise aus der Gruppe der tertiären aliphatischen Amine, z. B. Trimethylamin, Triethylamin, Tributylamin, Di-isopropylethylamin oder N-Methylpiperidin, oder der aromatischen tertiären Amine, z. B. Pyridin oder substituierte Pyridine wie Collidin, Lutidin oder 4-Dimethylaminopyridin, oder der bicyclische Amine wie 7-Methyl-1,5,7-triazabicyclo[4.4.0]- dec-5-en oder 1,8-Diazabicyclo[5.4.0]undec-7en (DBU). Bevorzugte Basen sind beispielsweise Kalium-tert.Butanolat, Lithium-bis(trimethylsilyl)amid oder 7-Methyl-1,5,7-triazabicyclo[4.4.0]- dec-5-en.

Die Menge an Base kann im Allgemeinen breit variiert weren. Beispielsweise kann es sinnvoll sein, die Base in katalytischen Mengen, im Unterschuss, äquimolar oder im Überschuß einzusetzen. Eine vorzugsweise flüssige organische Base kann gegebenenfalls auch als Lösungsmittel verwendet werden.

Geeignete Katalysatoren für die Michael-Addition nach Variante (a) sind saure Katalysatoren, bespielsweise aus der Gruppe der anorganischen Säuren, z. B. Broensted-Säuren, wie Fluorwasserstoffsäure, Salzsäure, Bromwasserstoffsäure, Schwefelsäure oder Perchlorsäure, oder Lewis-Säuren wie Bortrifluorid, Aluminiumtrichlorid, Eisen-III-chlorid, Zinn-IV-chlorid, Titan-IV-chlorid, Scandium-IIItriflat oder Zink-II-chlorid, sowie der organischen Säuren, z. B. Ameisensäure, Essigsäure, Propionsäure, Oxalsäure, Toluolsulfonsäure, Benzolsulfonsäure, Camphersulfonsäure, Zitronensäure oder Trifluoressigsäure.

Die Menge an saurem Katalysator kann im Allgemeinen breit variiert weren. Beispielsweise kann es sinnvoll sein, die Säure in katalytischen Mengen, im Unterschuss, äquimolar oder im Überschuß einzusetzen. Eine vorzugsweise flüssige Säure kann gegebenenfalls auch als Lösungsmittel verwendet werden.

Beispielsweise werden Diastereomerengemische bzw. racemische Diastereomere [gemeinsam hier mit Formel (I') bezeichnet] mit einem Gehalt an der herzustellenden Verbindungen (I) auch durch Umesterung erhalten, dadurch gekennzeichnet, dass man
(b) Verbindungen der Formel (I"), 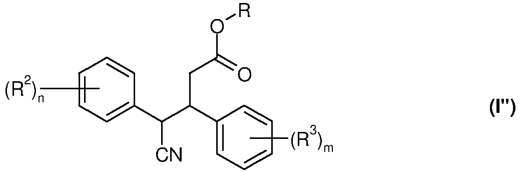 in denen R ein Rest aus der Gruppe der für R¹ möglichen Reste bedeutet aber von dem Rest R¹ in der herzustellenden Verbindung (I') verschieden ist, mit einer Verbindung der Formel R¹-OH, in der R¹ wie in Formel (I) definiert ist, zur Verbindung (I') umsetzt, wobei R², R³, m und n in der Verbindung (I") wie in der jeweils herzustellenden Verbindung der Formel (I) definiert sind.
   Mit Hilfe der Umesterung lassen sich auch stereochemisch angereicherte Verbindungen der oben genannten Formel (I) erhalten, dadurch gekennzeichet, dass man
(c) stereochemisch angereicherte Verbindungen der Formel (I'") 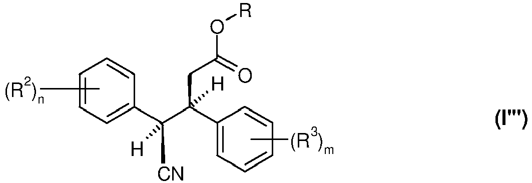 in denen R ein Rest aus der Gruppe der für R¹ möglichen Reste bedeutet aber von dem Rest R¹ in der herzustellenden Verbindung (I) verschieden ist, mit einer Verbindung der Formel R¹-OH, in der R¹ wie in der herzustellenden Verbindung der Formel (I) definiert ist.

Die Umesterungen (b) und (c) können beispielsweise mit einem geeigneten Alkohol R¹-OH in Gegenwart eines Katalysators, gegebenenfalls in Gegenwart eines aprotischen Lösungsmittels erfolgen. Weiterhin sind in der Regel Bedingungen vorteilhaft, mit denen das chemische Gleichgewicht auf die Seite des gewünschten Produkts verschoben wird, beispielsweise mit einem großen Überschuss des Alkohols R¹-OH unter praktisch wasserfreien Bedingungen, z. B. in Gegenwart eines Molekularsiebs.

Die Umsetzungen (Umesterungen) können in der Regel bei Temperaturen von 0°C bis180°C, vorzugsweise 20°C bis 100°C in Gegenwart einer Lewis- bzw. Broenstedt-Säure oder eines Enzyms durchgeführt werden [vgl. J. Org. Chem. 2002, 67, 431].

Geeignete Lösungsmittel sind z. B. folgende organische aprotische Lösungsmittel:
- aliphatische Kohlenwasserstoffe wie Pentan, Hexan, Cyclohexan oder Petrolether;
- aromatische Kohlenwasserstoffe wie Toluol, o-, m- oder p-Xylol,
- halogenierte Kohlenwasserstoffe wie Methylenchlorid (Dichlormethan), Chloroform oder Chlorbenzol,
- Ether wie Diethylether, Diisopropylether, tert.-Butylmethylether, Dioxan, Anisol oder Tetrahydrofuran (THF),
- Nitrile wie Acetonitril oder Propionitril,
- Ketone wie Aceton, Methylethylketon, Diethylketon oder
   tert.-Butylmethylketon,
- Dimethylsulfoxid, Dimethylformamid, Dimethylacetamid oder Sulfolan oder
- Gemische aus den genannten organischen Lösungsmitteln.

Bevorzugtes Lösungsmittel ist der Alkohol R¹-OH, der zugleich als Reaktant für die Umesterung verwendet wird, gegebenenfalls in Kombination mit einem der genannten aprotischen organischen Lösungsmittel.

Alternativ kann man den gewünschten Ester aus einem anderen Ester auch zweistufig durch saures oder basisches Verseifen des anderen Esters zur freien Säure, d. h. zu Verbindungen (I") oder (I"'), worin R jeweils H bedeutet, und nachfolgender Veresterung mit einem Alkohol R¹-OH erhalten.

Die Herstellung von Diastereomerengemischen bzw. racemischen Diastereomeren [gemeinsam hier mit Formel (I') bezeichnet] mit einem Gehalt an der herzustellenden Verbindungen (I) gemäß Variante (d) oder optisch aktiven Verbindungen (I) gemäß Variante (e) ist demnach dadurch gekennzeichnet, dass man eine freie Säure der genannten Formel (I") bzw. Formel (I"'), worin die Reste R jeweils Wasserstoff bedeuten, mit einem Alkohol der Formel R¹-OH nach üblichen Methoden verestert, gegebenenfalls kombiniert mit einer vorherigen Herstellung (d-1) bzw. (e-1) der freien Säure aus einem anderen Ester der Formel (I") bzw. Formel (I"'), worin die Reste R jeweils ungleich Wasserstoff sind.

Die Veresterung aus der freien Säure der Formel (I")/R = H bzw. (I"')/R = H kann beispielsweise analog üblichen Methoden erfolgen, beispielsweise bei Temperaturen von 0°C bis 120°C, vorzugsweise 20°C bis 50°C, gegebenenfalls in Gegenwart eines Katalysators, in einem weitgehend wasserfreien Medium bzw. unter Bedingungen, in denen Wasser inklusive das bei der Veresterung entstehende Wasser gebunden oder anderweitig entfernt wird. Als Katalysator kommen wasserfreie Säuren und Basen, vorzugsweise organische Säuren oder Basen in Frage; siehe Handbücher für chemische Verfahren zur Veresterung von Carbonsäuren; siehe auch z. B. J. Am. Chem. Soc. 2007,129 (43),13321; J. Org. Chem. 1984,49 (22), 4287.

Geeignete Lösungsmittel für die Veresterung sind die oben zu den Verfahrensvarianten (b) und (c) genannten aprotischen organischen Lösungsmittel geeignet, inklusive des Alkohols R¹-OH, der zugleich als Reaktant für die Veresterung verwendet wird, gegebenenfalls in Kombination mit einem der genannten aprotischen organischen Lösungsmittel.

Geeignete Katalysatoren für die Veresterung sind die zur genannten Verfahrensvariante (a) (Michael-Addition) erwähnten Basen oder sauren oder basischen Katalysatoren in wasserfreier Form oder mit möglichst geringem Wasseranteil. Bevorzugte Katalysatoren sind die Basen Lithiumhydroxid, Kaliumcarbonat oder organische Amine wie Pyridine, subsitutierte Pyridine und DBU.

Die der Veresterung gegebenenfalls vorgeschaltete Verseifung [Verfahrensvarianten (b-1) bzw. (c-1)] Verseifung anderer Ester der Formeln (I") bzw. Formel (I"'), jeweils mit R ungleich H, kann analog üblichen Methoden erfolgen, beispielsweise bei Temperaturen von 0°C bis 120°C, vorzugsweise 20°C bis 50°C, gegebenenfalls in Gegenwart eines Katalysators, in einem wasserhaltigen Medium/Lösungsmittel; siehe Handbücher für chemische Verfahren zur Verseifung von Carbonsäureestern; siehe auch z. B. J. Am. Chem. Soc. 2007,129 (43),13321; J. Org. Chem. 1984,49 (22), 4287.

Geeignete Lösungsmittel für die Verseifung [Verfahrensvarianten (b-1) bzw. (c-1)] ist Wasser oder ein wasserhaltiges organisches Lösungsmittel, beispielsweise auf Basis der zur genannten Verfahrensvariante (a) (Michael-Addition) erwähnten organischen Lösungmittel, vorzugsweise Wasser oder polare organische Lösungsmittel mit einem Wassergehalt wie THF.

Geeignete Katalysatoren für die Verseifung sind die zur genannten Verfahrensvariante (a) (Michael-Addition) erwähnten Säuren, Basen oder sauren oder basischen Katalysatoren, jeweils mit einem Wassergehalt. Bevorzugte Katalysatoren sind wässrige Säuren und Basen, insbesondere Basen wie Lithiumhydroxid, Natriumhydroxid, Kaliumcarbonat, Pyridine, substituierte Pyridine und DBU in Gegenwart von organischen Lösungsmitteln.

Die Katalysatoren für die Veresterung oder die Verseifung können im Allgemeinen in katalytischen Mengen eingesetzt werden. Eine Verwendung in größeren Mengen, auch äquimolar oder im molaren Überschuß ist in der Regel auch möglich. Eine Verwendung als Lösungsmittel kommt oftmals auch in Frage.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden.

Sofern die Zwischen- und Endprodukte als Feststoffe erhalten werden, kann die Reinigung auch durch Umkristallisieren oder Digerieren erfolgen. Sofern einzelne Verbindungen (I') bzw. (I) nicht auf den voranstehend beschriebenen Wegen zugänglich sind, können sie durch Derivatisierung anderer Verbindungen (I') bzw. (I) hergestellt werden.

Die Verbindungen der Formel (I'), die in Form der Diastereomerengemische anfallen, enthalten zwei Chiralitätszentren in Position 3 und 4 des Cyanobutyrat-Grundgerüsts und können je nach Substitutionsmuster ein oder mehrere weitere Chiralitätszentren enthalten.

Aufgrund der zwei Chiralitätszentren in Position 3 und 4 existieren 4 Stereoisomere, und zwar zwei Erythro-Enantiomere mit den Konfigurationen (3S,4R) [= Erythro-1] bzw. (3R,4S) [= Erythro-2] und zwei Threo-Enantiomere mit den Konfigurationen (3S,4S) [= Threo-1] bzw. (3R,4R) [= Threo-2]; siehe nachfolgendes Schema:

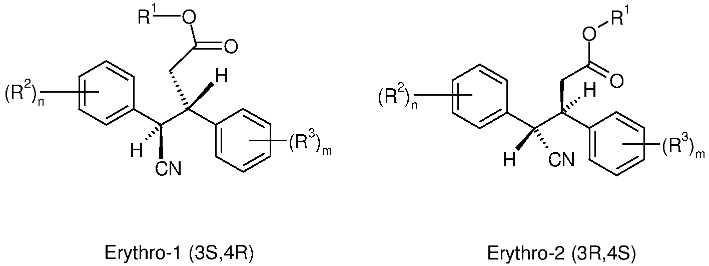

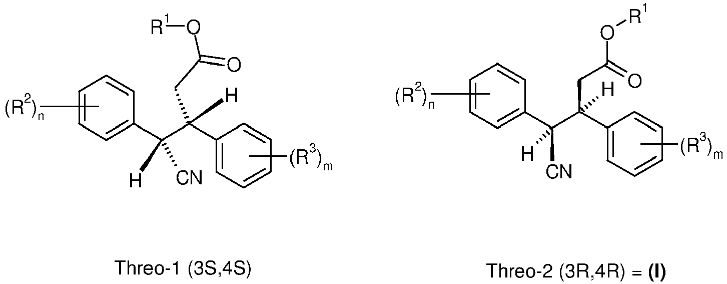

Die erfindungsgemäßen Verbindungen (I) stellen die reinen Enantiomere Threo-2 oder Gemische mit einem Gehalt von Threo-2 und Threo-1 mit einer stereochemische Reinheit von 60 bis 100 % Threo-2, vorzugsweise 70 bis 100 % Threo-2, mehr bevorzugt 80 bis 100 % Threo-2, insbesondere 90 bis 100 % Threo-2, bezogen auf das vorliegende Gemisch der threo-Enantiomeren dar.

Zur Herstellung der erfindungsgemäßen Verbindungen (I) aus den Verbindungen (I') ist es erforderlich das Stereoisomer (Enantiomer) Threo-2 aus dem Gemisch der Stereoisomeren entsprechend anzureichern. Ein zweckmäßiges Verfahren beinhaltet daher zunächst eine Isolierung der Threo-Isomeren Threo-1 und Threo-2 aus dem Diastereomerengemisch (I'), welches noch die Erythro-Isomeren enthält und anschließende Racemattrennung mit Isolierung oder Anreicherung des Enantiomeren Threo-2 aus dem Gemisch mit dem Enantiomeren Threo-1.

Die Isolierung der Threo-Isomeren als racemisches Gemisch kann analog der oben erwähnten üblichen Trenn- und Reinigungsverfahren erfolgen (Diastereomerentrennung).

Für die daran anschließende Herstellung von Verbindungen der Formel (I) kommen Racemattrennungsmethoden in Frage, die dem Fachmann aus analogen Fällen allgemein bekannt sind (vgl. Handbücher der Stereochemie), z. B. im Anschluss an Verfahren zur Trennung von Gemischen in Diastereomere, z. B. durch physikalische Verfahren wie Kristallisation, Chromatographieverfahren, vor allem Säulenchromatographie und Hochdruckflüssigchromatographie, Destillation, gegebenenfalls unter reduziertem Druck, Extraktion und andere Verfahren, können verbleibende Gemische von Enantiomeren in der Regel durch chromatographische Trennung an chiralen Festphasen getrennt werden. Für präparative Mengen oder im industriellen Maßstab kommen Verfahren wie die Kristallisation diastereomerer Salze, die aus den Diastereomerengemischen mit optisch aktiven Säuren und gegebenenfalls bei vorhandenen sauren Gruppen mit optisch aktiven Basen erhalten werden können, in Frage.

Zur Racemattrennung durch Kristallisation diastereomerer Salze kommen als optisch aktive Säure z. B. Camphersulfonsäure, Camphersäure, Bromcamphersulfonsäure, Chinasäure, Weinsäure, Dibenzoylweinsäure und andere analoge Säure in Betracht; als optisch aktive Basen kommen z. B. Chinin, Cinchonin, Chinidin, Brucin, 1-(S)- oder 1-(R)-Phenylethylamin und andere analoge Basen in Frage.

Die Kristallisationen werden dann meist in wässrigen, alkoholischen oder wässrigorganischen Lösungsmittel durchgeführt, wobei das Diastereomer mit der geringeren Löslichkeit gegebenenfalls nach Animpfen zunächst ausfällt. Das eine Enantiomer der Verbindung der Formel (I) wird danach aus dem ausgefällten Salz oder das andere aus dem Kristallisat durch Ansäuern bzw. mit Base freigesetzt.

Gegenstand der Erfindung ist daher auch das Verfahren zur Herstellung der Verbindungen (I), dadurch gekennzeichet, dass man mit Verbindungen (I') eine Racemattrennung durchführt und die Verbindung (I) in einer stereochemischen Reinheit von 60 bis 100 %, vorzugsweise 70 bis 100 %, mehr bevorzugt 80 bis 100 % , insbesondere 90 bis 100 %, bezogen auf das vorliegende Gemisch der threo-Enantiomeren isoliert.

Alternativ zu den genannten Racemattrennungsverfahren eignen sich zur Herstellung der Threo-2-Enantiomeren (I) prinzipiell auch enantioselektive Verfahren ausgehend von stereochemisch reinen Ausgangsstoffen.

Zur Herstellung der Säureadditionssalze der Verbindungen der Formel (I) kommen allgemein folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzol und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat oder Kalium-tert.-butylat, oder Ammoniak, Ethanolamin oder quartäres Ammoniumhydroxid der Formel [NRR'R"R"']⁺ OH⁻.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Eine Kollektion aus Verbindungen der Formel (I), die nach den obengenannten Verfahren synthetisiert werden können, können zusätzlich in parallelisierter Weise hergestellt werden, wobei dies in manueller, teilweise automatisierter oder vollständig automatisierter Weise geschehen kann. Dabei ist es möglich, sowohl die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S. H. DeWitt in "Annual Reports in Combinatorial Chemistry and Molecular Diversity: Automated Synthesis", Band 1, Verlag Escom, 1997, Seite 69 bis 77 beschrieben wird.

Zur parallelisierten Reaktionsdurchführung und Aufarbeitung können eine Reihe von im Handel erhältlichen Geräten verwendet werden wie sie beispielsweise von den Firmen Stem Corporation, Woodrolfe Road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen (I) oder von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA. Die aufgeführten Apparaturen ermöglichen eine modulare Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise von Roboter bedient werden. Derartige Automationssysteme können zum Beispiel von der Firma Zymark Corporation, Zymark Center, Hopkinton, MA 01748, USA bezogen werden.

Neben den beschriebenen Methoden kann die Herstellung von Verbindungen der Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z. B.: Barry A. Bunin in "The Combinatorial Index", Verlag Academic Press, 1998.

Die Verwendung von Festphasen unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß den hier beschriebenen Verfahren liefert Verbindungen der Formel (I) in Form von Substanzkollektionen oder -bibliotheken. Gegenstand der vorliegenden Erfindung sind daher auch Bibliotheken der Verbindungen der Formel (I), die mindestens zwei Verbindungen der Formel (I) enthalten, und deren Vorprodukte.

Die erfindungsgemäßen Verbindungen der Formel (I) (und/oder deren Salze), oben und im Folgenden zusammen als "erfindungsgemäße Verbindungen", "erfindungsgemäße Verbindungen (I)" oder kurz als "Verbindungen (I)" bezeichnet, weisen eine ausgezeichnete herbizide Wirksamkeit gegen ein breites Spektrum wirtschaftlich wichtiger mono und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen), den Boden, in dem oder auf dem die Pflanzen wachsen, (z. B. den Boden von Kulturland oder Nicht-Kulturland) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen und/oder deren Salze in wirtschaftlich bedeutenden transgenen Kulturen von Nutz und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten.

Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung in Getreide wie Weizen, Gerste, Roggen, Hafer, Hirse und Reis im Vor- oder Nachauflauf, insbesondere im Weizen im Nachauflauf. Bevorzugt ist auch die Verwendung in Mais im Vor- oder Nachauflauf, insbesondere im Mais im Vorauflauf.

Bevorzugt ist auch die Verwendung in Soja im Vor- oder Nachauflauf, insbesondere Soja im Nachauflauf.

Gegenstand der Erfindung ist auch das Verfahren (Anwendungsverfahren) zur Bekämpfung von Schadpflanzen oder zur Wachstumsregulierung von Pflanzen, dadurch gekennzeichnet, dass man eine wirksame Menge von einer oder mehreren Verbindungen der Formel (I) oder deren Salzen auf die Pflanzen (Schadpflanzen, ggf. zusammen mit den Nutzpflanzen) Pflanzensamen, den Boden, in dem oder auf dem die Pflanzen wachsen, oder die Anbaufläche appliziert.

Die erfindungsgemäßen Verbindungen (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) und/oder deren Salze enthalten.

Die Verbindungen der Formel (I) und/oder deren Salze können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. ÖI-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I) und/oder dessen Salze. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2006 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequatchlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumicloracpentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacetmethyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosateisopropylammonium, Glyphosate-Kalium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyr-isopropylammonium, Imazaquin, Imazaquin-ammonium, Imazethapyr, Imazethapyr-ammonium, Imazosulfuron, Inabenfide, Indanofan, Indaziflam, Indolessigsäure (IAA), 4-Indol-3-ylbuttersäure (IBA), lodosulfuron, lodosulfuronmethyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamidedihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamone, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuronmethyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

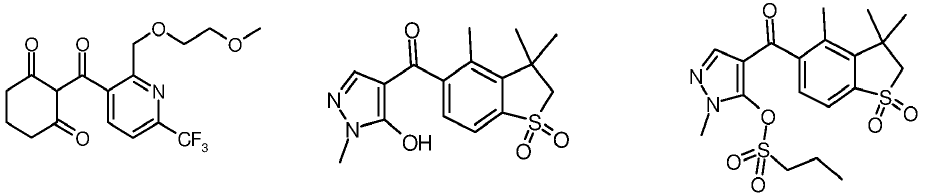

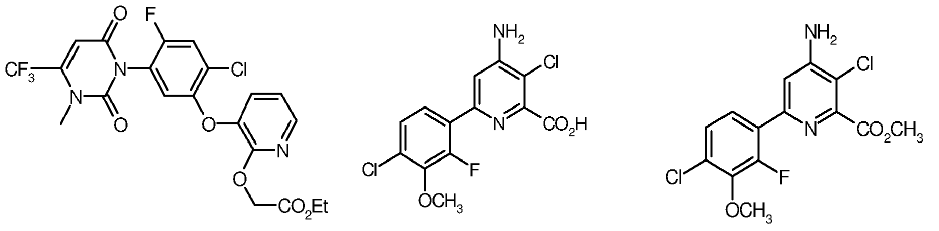

Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) und deren Kombinationen mit weiteren Pestiziden in Frage:
A) Verbindungen der Formel (S-I), 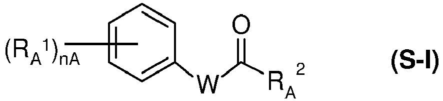 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W_{A}¹) bis (W_{A}⁴),

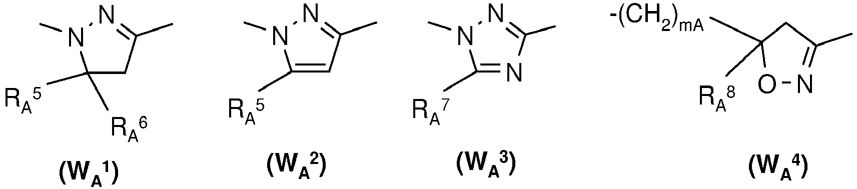

   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-I) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubstituiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäureethylester (S1-1) ("Mefenpyr-diethyl", siehe Pestic. Man.), und verwandte Verbindungen, wie sie in der WO 91/07874 beschrieben sind;
   b) Derivate der Dichlorphenylpyrazolcarbonsäure, vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1 -dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind;
   c) Verbindungen vom Typ der Triazolcarbonsäuren, vorzugsweise Verbindungen wie Fenchlorazol(-ethylester), d.h.
      1-(2,4-Dichlorphenyl)-5-trichlormethyl-(1H)-1,2,4-triazol-3-carbonsäureethylester (S1-6), und verwandte Verbindungen wie sie in EP-A-174 562 und EP-A-346 620 beschrieben sind;
   d) Verbindungen vom Typ der 5-Benzyl- oder 5-Phenyl-2-isoxazolin-3- carbonsäure, oder der 5,5-Diphenyl-2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie in WO 91/08202 beschrieben sind, bzw. der 5,5-Diphenyl-2-isoxazolin-carbonsäureethylester (S1-9) ("Isoxadifen-ethyl") oder -n-propylester (S1-10) oder der 5-(4-Fluorphenyl)-5-phenyl-2-isoxazolin-3-carbonsäureethylester (S1-11), wie sie in der Patentanmeldung WO-A-95/07897 beschrieben sind.
B) Chinolinderivate der Formel (S-II), 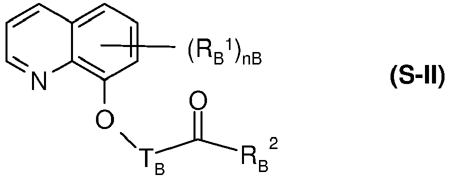 wobei die Symbole und Indizes folgende Bedeutungen haben:
   - R_{B}¹: Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-II) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2), vorzugsweise (5-Chlor-8-chinolinoxy)essigsäure-(1-methylhexyl)-ester (Common name "Cloquintocet-mexyl" (S2-1) (siehe Pestic. Man.), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie deren Hydrate und Salze wie sie in der WO-A-2002/034048 beschrieben sind.
   b) Verbindungen vom Typ der (5-Chlor-8-chinolinoxy)malonsäure, vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
C) Verbindungen der Formel (S-III) 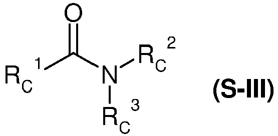 wobei die Symbole und Indizes folgende Bedeutungen haben:
   - R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{C}², R_{C}³: ist gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B.
   "Dichlormid" (siehe Pestic.Man.) (= N,N-Diallyl-2,2-dichloracetamid),
   "R-29148" (= 3-Dichloracetyl-2,2,5-trimethyl-1,3-oxazolidin von der Firma Stauffer),
   "R-28725" (= 3-Dichloracetyl-2,2,-dimethyl-1,3-oxazolidin von der Firma Stauffer),
   "Benoxacor" (siehe Pestic. Man.) (= 4-Dichloracetyl-3,4-dihydro-3-methyl-2H-1,4-benzoxazin),
   "PPG-1292" (= N-Allyl-N-[(1,3-dioxolan-2-yl)methyl]-dichloracetamid von der Firma PPG Industries),
   "DKA-24" (= N-Allyl-N-[(allylaminocarbonyl)methyl]-dichloracetamid von der Firma Sagro-Chem),
   "AD-67" oder "MON 4660" (= 3-Dichloracetyl-1-oxa-3-aza-spiro[4,5]decan von der Firma Nitrokemia bzw. Monsanto),
   "TI-35" (= 1-Dichloracetyl-azepan von der Firma TRI-Chemical RT) "Diclonon" (Dicyclonon) oder "BAS145138" oder "LAB145138" (= 3-Dichloracetyl-2,5,5-trimethyl-1,3-diazabicyclo[4.3.0]nonan von der Firma BASF) und
   "Furilazol" oder "MON 13900" (siehe Pestic. Man.) (= (RS)-3-Dichloracetyl-5-(2-furyl)-2,2-dimethyloxazolidin)
D) N-Acylsulfonamide der Formel (S-IV) und ihre Salze, 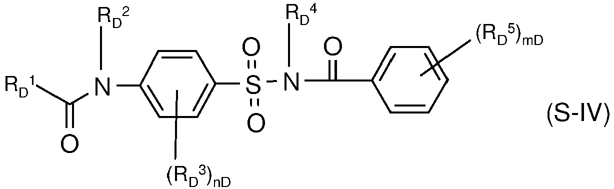 worin
   - R_{D}¹: Wasserstoff, einen Kohlenwasserstoffrest, einen Kohlenwasserstoffoxyrest, einen Kohlenwasserstoffthiorest oder einen Heterocyclylrest, der vorzugsweise über ein C-Atom gebunden ist, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist,
   wobei jeder Kohlenwasserstoffteil vorzugsweise 1 bis 20 C-Atome aufweist und ein C-haltiger Rest R_{D}¹ inklusive Substituenten vorzugsweise 1 bis 30 C-Atome aufweist;
   - R_{D}²: Wasserstoff oder (C₁-C₄)Alkyl, vorzugsweise Wasserstoff, oder
   - R_{D}¹ und R_{D}²: zusammen mit der Gruppe der Formel -CO-N- den Rest eines 3- bis 8-gliedrigen gesättigten oder ungesättigten Rings;
   - R_{D}³: gleich oder verschieden Halogen, Cyano, Nitro, Amino, Hydroxy, Carboxy, Formyl, CONH₂, SO₂NH₂ oder einen Rest der Formel -Z^{b}-R^{b} ;
   - R_{D}⁴: Wasserstoff oder (C₁-C₄)Alkyl, vorzugsweise H;
   - R_{D}⁵: gleich oder verschieden Halogen, Cyano, Nitro, Amino, Hydroxy, Carboxy, CHO, CONH₂, SO₂NH₂ oder einen Rest der Formel -Z^{c}-R^{c} ;
   - R^{a}: einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - R^{b},R^{c}: gleich oder verschieden einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - Z^{a}: eine divalente Gruppe der Formel -O-, -S-, -CO-, -CS-, -CO-O-, -CO-S-, -O-CO-, -S-CO-, -SO-, -SO₂-, -NR*-, -CO-NR*-, -NR*-CO-, -SO₂-NR*- oder -NR*-SO₂-, wobei die rechts angegebene Bindung der jeweiligen divalenten Gruppe die Bindung zum Rest R^{a} ist und wobei die R* in den letztgenannten 5 Resten unabhängig voneinander jeweils H, (C₁-C₄)-Alkyl oder Halo-(C₁-C₄)-alkyl bedeuten;
   - Z^{b},Z^{c}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*-oder -NR*-CO-, wobei die rechts angegebene Bindung der jeweiligen divalenten Gruppe die Bindung zum Rest R^{b} bzw. R^{c} ist und wobei die R* in den letztgenannten 5 Resten unabhängig voneinander jeweils H, (C₁-C₄)-Alkyl oder Halo-(C₁-C₄)-alkyl bedeuten;
   - n_{D}: eine ganze Zahl von 0 bis 4, vorzugsweise 0, 1 oder 2, insbesondere 0 oder 1, und
   - m_{D}: eine ganze Zahl von 0 bis 5, vorzugsweise 0, 1, 2 oder 3, insbesondere 0, 1 oder 2;
   bedeuten;
E) Acylsulfamoylbenzoesäureamide der allgemeinen Formel (S-V), gegebenenfalls auch in Salzform, 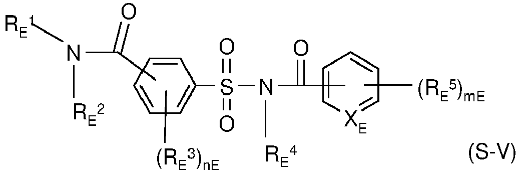 worin
   - X_{E}: CH oder N;
   - R_{E}¹: Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
   - R_{E}²: Wasserstoff, Hydroxy, (C₁-C₆)Alkyl, (C₂-C₆)Alkenyl, (C₂-C₆)Alkinyl, (C₁-C₆)Alkoxy, (C₂-C₆)Alkenyloxy, wobei die fünf letztgenannten Reste gegebenenfalls durch einen oder mehrere, gleiche oder verschiedene Reste aus der Gruppe Halogen, Hydroxy, (C₁-C₄)Alkyl, (C₁-C₄)Alkoxy und (C₁-C₄)Alkylthio substituiert sind, oder
   - R_{E}¹ und R_{E}²: zusammen mit dem sie tragenden Stickstoffatom einen 3- bis 8-gliedrigen gesättigten oder ungesättigten Ring;
   - R_{E}³: Halogen, Cyano, Nitro, Amino, Hydroxy, Carboxy, CHO, CONH₂, SO₂NH₂ oder Z^{b}-R^{b};
   - R_{E}⁴: Wasserstoff, (C₁-C₄)Alkyl, (C₂-C₄)Alkenyl oder (C₂-C₄)Alkinyl;
   - R_{E}⁵: Halogen, Cyano, Nitro, Amino, Hydroxy, Carboxy, Phosphoryl, CHO, CONH₂, SO₂NH₂ oder Z^{c}-R^{c},
   - R^{a}: einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)-alkyl]-amino substituiert sind;
   - R^{b}, R^{c}: gleich oder verschieden einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
   - Z^{a}: eine divalente Einheit aus der Gruppe O, S, CO, CS, C(O)O, C(O)S, SO, SO₂, NR^{d}, C(O)NR^{d} oder SO₂NR^{d};
   - Z^{b}, Z^{c}: gleich oder verschieden eine direkte Bindung oder eine divalente Einheit aus der Gruppe O, S, CO, CS, C(O)O, C(O)S, SO, SO₂, NR^{d}, SO₂NR^{d} oder C(O)NR^{d};
   - R^{d}: Wasserstoff, (C₁-C₄)-Alkyl oder (C₁-C₄)-Haloalkyl;
   - n_{E}: eine ganze Zahl von 0 bis 4, und
   - m_{E}: für den Fall, daß X für CH steht, eine ganze Zahl von 0 bis 5, und für den Fall, daß X für N steht, eine ganze Zahl von 0 bis 4
   bedeuten;
   davon bevorzugt sind Verbindungen (auch in Form ihrer Salze) vom Typ der Acylsulfamoylbenzoesäureamide, z.B. der nachfolgenden Formel (S-VI), die z.B. bekannt sind aus WO 99/16744, 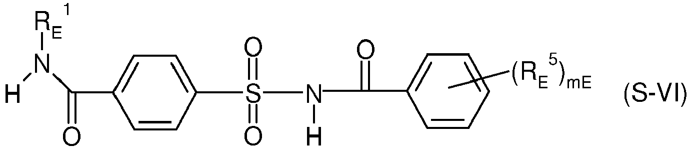
   z.B. solche worin
   R_{E}¹ = Cyclopropyl und R_{E}⁵ = 2-OMe ist ("Cyprosulfamide", S3-1),
   R_{E}¹ = Cyclopropyl und R_{E}⁵ = 5-Cl-2-OMe ist (S3-2),
   R_{E}¹ = Ethyl und R_{E}⁵ = 2-OMe ist (S3-3),
   R_{E}¹ = Isopropyl und R_{E}⁵ = 5-Cl-2-OMe ist (S3-4) und
   R_{E}¹ = Isopropyl und R_{E}⁵ = 2-OMe ist (S3-5);
F) Verbindungen vom Typ der N-Acylsulfamoylphenylharnstoffe der Formel (S-VII), die z.B. bekannt sind aus der EP-A-365484, 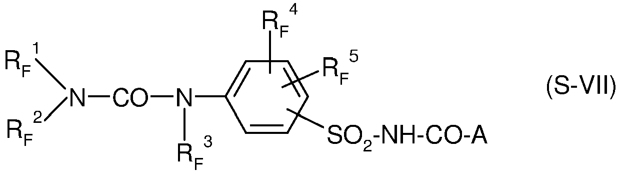 worin
   - A: für einen Rest aus der Gruppe 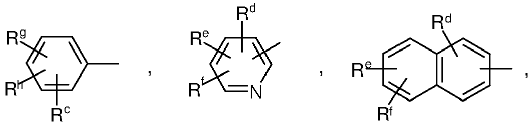 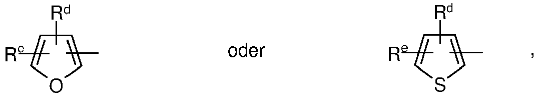
   R_{F}¹ und R_{F}² unabhängig voneinander für Wasserstoff, (C₁-C₈)Alkyl, (C₃-C₈)Cycloalkyl, (C₃-C₆)Alkenyl, (C₃-C₆)Alkinyl, 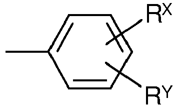 oder durch (C₁-C₄)Alkoxy oder 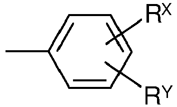 substituiertes (C₁-C₄)Alkoxy, oder
   - R_{F}¹ und R_{F}²: gemeinsam für eine (C₄-C₆)Alkylenbrücke oder eine durch Sauerstoff, Schwefel, SO, SO₂, NH oder -N(C₁-C₄-Alkyl)- unterbrochene (C₄-C₆)Alkylenbrücke,
   - R_{F}³: für Wasserstoff oder (C₁-C₄)Alkyl,
   - R_{F}⁴ und R_{F}⁵: unabhängig voneinander für Wasserstoff, Halogen, Cyan, Nitro, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl, -COOR^{j}, -CONR^{k}R^{m}, -CORⁿ, -SO₂NR^{k}R^{m} oder -OSO₂-C₁-C₄-Alkyl, oder R^{a} und R^{b} gemeinsam für eine (C₃-C₄)Alkylenbrücke, die durch Halogen oder C₁-C₄-Alkyl substituiert sein kann, oder eine (C₃-C₄)Alkenylenbrücke, die durch Halogen oder (C₁-C₄)Alkyl substituiert sein kann, oder eine C₄-Alkadienylenbrücke, die durch Halogen oder (C₁-C₄)Alkyl substituiert sein kann, und
   - R^{g} und R^{h}: unabhängig voneinander für Wasserstoff, Halogen, C₁-C₄-Alkyl, Trifluormethyl, Methoxy, Methylthio oder -COOR^{j} stehen, wobei
   - R^{c}: Wasserstoff, Halogen, (C₁-C₄)Alkyl oder Methoxy,
   - R^{d}: Wasserstoff, Halogen, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, -COOR^{j} oder -CONR^{k}R^{m},
   - R^{e}: Wasserstoff, Halogen, C₁-C₄-Alkyl, -COOR^{j}, Trifluormethyl oder Methoxy, oder R^{d} und R^{e} gemeinsam für eine (C₃-C₄)Alkylenbrücke,
   - R^{f}: Wasserstoff, Halogen oder (C₁-C₄)Alkyl,
   - R^{X} und R^{Y}: unabhängig voneinander Wasserstoff, Halogen, (C₁-C₄)Alkyl, (C₁-C₄)Alkoxy, (C₁-C₄)Alkylthio, -COOR⁴, Trifluormethyl, Nitro oder Cyan,
   - R^{j}, R^{k} und R^{m}: unabhängig voneinander Wasserstoff oder (C₁-C₄)Alkyl,
   - R^{k} und R^{m}: gemeinsam eine (C₄-C₆)Alkylenbrücke oder eine durch Sauerstoff, NH oder -N(C₁-C₄-Alkyl)- unterbrochene C₄-C₆-Alkylenbrücke, und
   - Rⁿ: (C₁-C₄)Alkyl, Phenyl oder durch Halogen, (C₁-C₄)Alkyl, Methoxy, Nitro oder Trifluormethyl substituiertes Phenyl bedeuten,
   davon bevorzugt sind:
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   1-[4-(N-Naphthoylsulfamoyl)phenyl]-3,3-dimethylharnstoff, einschließlich der Stereoisomeren und der in der Landwirtschaft gebräuchlichen Salze,
G) Wirkstoffe aus der Klasse der Hydroxyaromaten und der aromatischaliphatischen Carbonsäurederivate, z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 1,2-Dihydro-2-oxo-6-trifluoromethylpyridin-3-carboxamid, 2-Hydroxyzimtsäure, 2,4-Dichlorzimtsäure, wie sie in der WO 2004084631, WO 2005015994, WO 2006007981, WO 2005016001 beschrieben sind;
H) Wirkstoffe aus der Klasse der 1,2-Dihydrochinoxalin-2-one, z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO 2005112630 beschrieben sind,
I) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (siehe Pestic. Man.) (= Piperidin-1-thiocarbonsäure-S-1-methyl-1-phenylethylester), das als Safener für Reis gegen Schäden des Herbizids Molinate bekannt ist,
   "Daimuron" oder "SK 23" (siehe Pestic. Man.) (= 1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (= 3-(2-Chlorphenylmethyl)-1-(1-methyl-1-phenylethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist, "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
K) Verbindungen der Formel (S-IX), wie sie in der WO-A-1998/38856 beschrieben sind 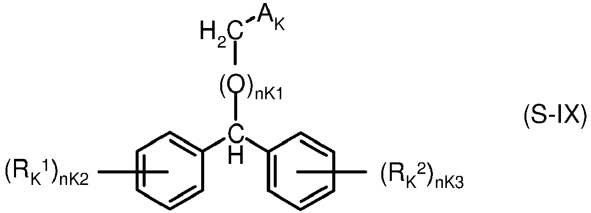 worin die Symbole und Indizes folgende Bedeutungen haben:
   - R_{K}¹, R_{K}²: unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{K}: COOR_{K}³ oder COOR_{K}⁴
   - R_{K}³, R_{K}⁴: unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{K}¹: 0 oder 1 und
   - n_{K}², n_{K}³: unabhängig voneinander 0, 1 oder 2;
   vorzugsweise:
   Methyl-(diphenylmethoxy)acetat (CAS-Regno: 41858-19-9),
L) Verbindungen der Formel (S-X),
   wie sie in der WO A-98/27049 beschrieben sind 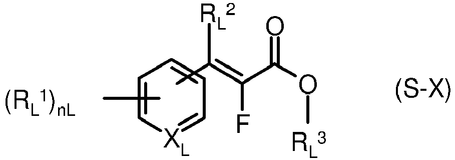 worin die Symbole und Indizes folgende Bedeutungen haben:
   - X_{L}: CH oder N,
   - n_{L}: für den Fall, dass X=N ist, eine ganze Zahl von 0 bis 4 und für den Fall, dass X=CH ist, eine ganze Zahl von 0 bis 5 ,
   - R_{L}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)Alkylsulfonyl, (C₁-C₄)Alkoxycarbonyl, ggf. substituiertes. Phenyl, ggf. substituiertes Phenoxy,
   - R_{L}²: Wasserstoff oder (C₁-C₄)Alkyl
   - R_{L}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze.
M) Wirkstoffe aus der Klasse der 3-(5-Tetrazolylcarbonyl)-2-chinolone, z.B. 1,2-Dihydro-4-hydroxy-1-ethyl-3-(5-tetrazolylcarbonyl)-2-chinolon (CAS-Regno: 219479-18-2), 1,2-Dihydro-4-hydroxy-1-methyl-3-(5-tetrazolylcarbonyl)-2-chinolon (CAS-Regno: 95855-00-8), wie sie in der WO-A-1999000020 beschrieben sind,
N) Verbindungen der Formeln (S-XI) oder (S-XII)
   wie sie in der WO-A-2007023719 und WO-A-2007023764 beschrieben sind 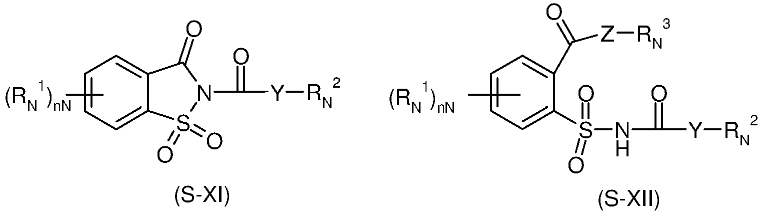 worin
   - R_{N}¹: Halogen, (C₁-C₄)Alkyl, Methoxy, Nitro, Cyano, CF₃, OCF₃
   - Y, Z: unabhängig voneinander O oder S,
   - n_{N}: eine ganze Zahl von 0 bis 4,
   - R_{N}²: (C₁-C₁₆)Alkyl, (C₂-C₆)Alkenyl, (C₃-C₆)Cycloalkyl, Aryl; Benzyl, Halobenzyl,
   - R_{N}³: Wasserstoff, (C₁-C₆)Alkyl bedeuten;
O) eine oder mehreren Verbindungen aus Gruppe:
   1,8-Naphthalsäureanhydrid,
   O,O-Diethyl S-2-ethylthioethyl phosphordithioat (Disulfoton),
   4-Chlorphenyl-methylcarbamat (Mephenate),
   O,O-Diethyl-O-phenylphosphorotioat (Dietholate),
   4-Carboxy-3,4-dihydro-2H-1-benzopyran-4-essigsäure (CL-304415, CAS-Regno: 31541-57-8),
   2-propenyl 1-oxa-4-azaspiro[4.5]decane-4-carbodithioate (MG-838, CAS-Regno: 133993-74-5),
   Methyl-[(3-oxo-1H-2-benzothiopyran-4(3H)-yliden)methoxy]acetate (aus WO-A-98/13361; CAS-Regno: 205121-04-6),
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon-O-1,3-dioxolan-2-ylmethyloxim (Fluxofenim),
   4,6-Dichlor-2-phenylpyrimidin (Fenclorim),
   Benzyl-2-chlor-4-trifluormethyl-1,3-thiazol-5-carboxylat (Flurazole),
   2-Dichlormethyl-2-methyl-1,3-dioxolan (MG-191),
   einschließlich der Stereoisomeren und der in der Landwirtschaft gebräuchlichen Salze.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I) und/oder deren Salze. Sie kann innerhalb weiter Grenzen schwanken. Für die Anwendung als Herbizid zur Bekämpfung von Schadpflanzen liegt sie beispielsweise im Bereich von 0,001 bis 10,0 kg/ha oder mehr Aktivsubstanz, vorzugsweise im Bereich von 0,005 bis 5 kg/ha, insbesondere im Bereich von 0,01 bis 1 kg/ha Aktivsubstanz. Dies gilt sowohl für die Anwendung im Vorauflauf oder im Nachauflauf.

Bei der Anwendung als Pflanzenwachstumsregulator, beispielsweise als Halmverkürzer bei Kulturpflanzen, wie sie oben genannt worden sind, vorzugsweise Getreidepflanzen wie Weizen, Gerste, Roggen, Triticale, Hirse, Reis oder Mais, liegt die Aufwandmenge beispielsweise im Bereich von 0,001 bis 2 kg/ha oder mehr Aktivsubstanz, vorzugsweise im Bereich von 0,005 bis 1 kg/ha, insbesondere im Bereich von 10 bis 500 g/ha Aktivsubstanz, ganz besonders von 20 bis 250 g/ha Aktivsubstanz. (bitte prüfen, ob dies genannt werden soll) Dies gilt sowohl für die Anwendung im Vorauflauf oder im Nachauflauf, wobei die Nachauflaufbehandlung in der Regel bevorzugt ist.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Nachfolgend sind beispielhaft einige Synthesebeispiele von Verbindungen der allgemeinen Formel (I) beschrieben. In den Beispielen beziehen sich Mengenangaben (auch Prozentangaben) auf das Gewicht, sofern nichts anderes speziell angegeben ist.

Die Symbole ">" und "<" bedeuten "größer als" beziehungsweise "keiner als". Das Symbol "≥" bedeutet "größer als oder gleich", das Symbol "≤" bedeutet "kleiner als oder gleich".

Wenn im Rahmen der Beschreibung und der Beispiele Bezeichnungen "R" und "S" für die absolute Konfiguration an einem Chiralitätszentrum der Stereoisomeren der Formel (I) angegeben ist, folgt diese der RS-Nomenklatur nach der Cahn-Ingold-Prelog-Regel, wenn nichts anderes näher definiert ist.

### (A) Synthesebeispiele

### Beispiel A1: (3R,4R)-3-(4-Chlorphenyl)-4-cyan-4-(3-fluorphenyl)-butansäuremethylester (Tabelle 1, Beispiel 1)

### a) Herstellung der diastereomeren 3-(4-Chlorphenyl)-4-cyan-4-(3-fluorphenyl)-butansäuremethylester:

Zu 1,200 g (6,103 mmol) Methyl-3-(4-chlorphenyl)acrylat in 6,0 ml Methanol gab man unter Schutzgas (Ar) 0,824 g (6,103 mmol) 3-(Fluorphenyl)acetonitril und 0,1 ml Natriummethylat-Lösung (30 % in Methanol) und rührte 48 h bei 50°C. Man entfernte das Lösemittel unter reduziertem Druck, nahm den Rückstand in Ethylacetat auf und wusch zweimal mit je 25 ml Wasser. Die vereinigten organischen Phasen wurden über Natriumsulfat getrocknet und das Lösemittel unter reduziertem Druck entfernt. Nach Chromatographie des Rückstands an Kieselgel (Ethylacetat / Heptan = 15:85) erhielt man 0,401 g (18% d. Th.) der diastereomeren Methyl-3-(4-chlorphenyl)-4-cyan-4-(3-fluorphenyl)butanoate (erythro:threo = 60:40, Vergleich der Dubletts im ¹H-NMR in CDCl₃ bei 4,38 und 4,08 ppm).

### b) Herstellung von (3R,4R)-3-(4-Chlorphenyl)-4-cyan-4-(3-fluorphenyl)-butansäuremethylester:

Durch präparative Chromatographie [(80 ml/min n-Heptan/2-Propanol (80:20)] von 100 mg des unter a) erhaltenen Diastereomerengemisches (gelöst in 4,0 ml Methanol) an einer chiralen Festphase [Chiralpak IC, 20 µm, (250 x 50)-mm Säule] erhielt man 16,75 mg der Titelverbindung, die als letztes der vier Stereoisomere (Retentionszeit = 11,3 min) eluiert wurde.

Der spezifische Drehwert [α] betrug -38°.

Zu ¹H-NMR in CDCl₃ und Retentionszeit bei analytischer HPLC siehe Tabelle 1.

Die absolute Konfiguration der Titelverbindung wurde durch Röntgenstrukturanalyse zugeordnet.

### Beispiel A2: (3R,4R)-3-(3-Chlorphenyl)-4-cyan-4-(3,4-difluorphenyl)-butansäuremethylester (Tabelle 1, Beispiel 10)

### a) Herstellung der diastereomeren 3-(3-Chlorphenyl)-4-cyan-4-(3,4-difluorphenyl)-butansäuremethylester:

Zu 1,000 g (5,086 mmol) Methyl-3-(3-chlorphenyl)acrylat in 12,0 ml Methanol gab man unter Schutzgas (Ar) 0,779 g (5,086 mmol) (3,4-Difluorphenyl)-acetonitril und 0,1 ml Natriummethylat-Lösung (30 % in Methanol) und rührte 4 h bei 100°C in einem geschlossenen Gefäß im Mikrowellenofen. Man entfernte das Lösemittel unter reduziertem Druck, nahm den Rückstand in Dichlormethan auf und wusch zweimal mit je 25 ml Wasser. Die vereinigten organischen Phasen wurden über Natriumsulfat getrocknet und das Lösemittel unter reduziertem Druck entfernt. Nach Chromatographie des Rückstands an Kieselgel (Ethylacetat / Heptan = 20:80) erhielt man 0,738 g (37% d. Th.) der diastereomeren Methyl-3-(3-chlorphenyl)-4-cyan-4-(3,4-difluorphenyl)butanoate (erythro:threo = 43:57, Vergleich der Dubletts im ¹H-NMR in CDCl₃ bei 4,40 und 4,08 ppm).

### b) Herstellung von (3R,4R)-3-(3-Chlorphenyl)-4-cyan-4-(3,4-difluorphenyl)-butansäuremethylester:

Durch präparative Chromatographie [(80 ml/min n-Heptan/2-Propanol (90:10)] von 120 mg des unter a) erhaltenen Diastereomerngemisches (gelöst in 4,0 ml Methanol) an einer chiralen Festphase [Chiralpak IC, 20 µm, (250 x 50)-mm Säule] erhielt man 16,40 mg der Titelverbindung [chemische Reinheit > 95% (NMR), Isomerenreinheit 97% (chirale HPLC)], die als letztes der vier Stereoisomere (Retentionszeit = 19,1 min) eluiert wurde. Spezifischer Drehwert [α]: -43°. ¹H-NMR in CDCl₃ und Retentionszeit bei analytischer HPLC: siehe Tabelle 1.

### Beispiel A3: (3R,4R)-3-(3-Chlorphenyl)-4-cyan-4-(3,4-difluorphenyl)butansäure (Tabelle 1, Beispiel 107)

### a) Herstellung der diastereomeren 3-(3-Chlorphenyl)-4-cyan-4-(3,4-difluorphenyl)-butansäuren:

Zu 0,525 g (1,500 mmol) (3R,4R)-3-(3-Chlorphenyl)-4-cyan-4-(3,4-difluorphenyl)-butansäuremethylester in 8,0 ml Methanol gab man unter Schutzgas (Ar) 0,120 g (3,000 mmol) 2 molare wässrige Natronlauge und rührte 3 h bei 25°C. Man entfernte das Methanol unter reduziertem Druck. Der Rückstand wurde mit konzentrierter Salzsäure angesäuert (pH = 3) und dreimal mit jeweils 15 ml Dichlormethan extrahiert. Die vereinigten organischen Phasen wurden über Natriumsulfat getrocknet und das Lösemittel unter reduziertem Druck entfernt. Man erhielt 0,502 g (99.7 % d. Th.) von diastereomeren 3-(3-Chlorphenyl)-4-cyan-4-(3,4-difluorphenyl)-butansäuren (erythro:threo = 58:42, Vergleich der Dubletts im ¹H-NMR in CDCl₃ bei 4,35 und 4,05 ppm).

### b) (3R,4R)-3-(3-Chlorphenyl)-4-cyan-4-(3,4-difluorphenyl)butansäure:

Durch präparative Chromatographie [(80 ml/min n-Heptan/2-Propanol (80:20), Zugabe von 0,05% Trifluoressigsäure in die Heptanphase] des unter a) erhaltenen Diastereomerengemisches an einer chiralen Festphase [Chiralpak IC, 20 µm, (250 x 50)-mm Säule] erhielt man 107 mg der Titelverbindung [chemische Reinheit > 95% (NMR), Isomerenreinheit 96% (chirale HPLC)], die als letztes der beiden entstandenen Stereoisomere (Retentionszeit = 19,1 min) eluiert wurde. Spezifischer Drehwert [a]: -40°. ¹H-NMR in CDCl₃ und Retentionszeit bei analytischer HPLC siehe Tabelle 1.

### Beispiel A4: (3R,4R)-3-(3-Chlorphenyl)-4-cyan-4-(3,4-difluorphenyl)-butansäure-methylester (Tabelle 1, Beispiel 109)

Zu 0,100 g (0,286 mmol) (3R,4R)-3-(3-chlorphenyl)-4-cyan-4-(3,4-difluorphenyl)-butansäuremethylester in 3,0 ml (40,136 mmol) n-Propanol gab man unter Schutzgas (Ar) einen Tropfen konzentrierte Schwefelsäure und rührte 5 h bei 90°C. Man entfernte das Lösemittel im Vakuum, nahm den Rückstand in Dichlormethan auf und wusch zweimal mit je 15 ml gesättigter wässriger NatriumhydrogencarbonatLösung. Die vereinigten organischen Phasen wurden über Natriumsulfat getrocknet und das Lösemittel im Vakuum entfernt. Nach Chromatographie des Rückstands an Kieselgel (Gradient: Start bei Ethylacetat / Heptan = 5:95 innerhalb von 20 Minuten auf Ethylacetat / Heptan = 20:80) erhielt man 0,082 g (76% d. Th.) der Titelverbindung [chemische Reinheit > 95% (NMR), Isomerenreinheit 96% (chirale HPLC)]. Spezifischer Drehwert [α]: - 38°. ¹H-NMR in CDCl₃ und Retentionszeit bei analytischer HPLC siehe Tabelle 1.

Die in der nachfolgenden Tabelle 1 beschriebenen Verbindungen mit der absoluten Konfiguration (3R, 4R) erhält man gemäß oder analog zu den oben beschriebenen Beispielen.

In der Tabelle 1 bedeuten:
- Bsp.. =: Beispiel Nummer
- H =: Wasserstoff(-atom)
- Me =: Methyl
- Et =: Ethyl
- n-Pr =: n-Propyl
- i-Pr =: Isopropyl
- n-Bu =: n-Butyl
- i-Bu =: Isobutyl
- Rtz =: Retentionszeit
- F, Cl, Br, I =: Fluor, Chlor, Brom bzw. Jod entsprechend den üblichen chemischen Atomsymbolen
- MeO oder OMe =: Methoxy
- CN =: Cyano
- NO₂ =: Nitro

Die Position eines Substituenten am Phenylring, beispielsweise in Position 2, ist dem Symbol oder der Abkürzung des Restes vorangestellt, z.B.
- 2-Cl =: 2-Chlor
- 2-Me =: 2-Methyl

Nummerierungen der Substituentenpositionen bei di- oder trisubstituiertem Substitutionsmuster sind analog vorangestellt, z. B.
- 3,5-Me₂ =: 3,5-Dimethyl (z. B. als Substitution am Phenylring)
- 2,3-Cl₂ =: 2,3-Dichlor (z. B. als Substitution am Phenylring)
- 3,4-F₂ =: 3,4-Difluor (z. B. als Substitution am Phenylring)

Andere Abkürzungen sind analog der oben angegebenen Beispiele zu verstehen.
- "(R²)ₙ = "H" =: unsubstituierter Cyclus (n = 0)
- "(R³)ₘ = "H" =: unsubstituierter Cyclus (m = 0)

Im Übrigen gelten die üblichen chemischen Symbole und Formeln, wie z. B. CH₂ für Methylen oder CF₃ für Trifluormethyl oder OH für Hydroxyl. Zusammengesetzte Bedeutungen sind entsprechend aus den genannten Abkürzungen zusammengesetzt definiert.

Die in der Tabelle 1 angegebenen Retentionszeiten ("Rtz") wurden durch analytische HPLC der Verbindungen (I) an einer chiralen Festphase unter den folgenden experimentellen Bedingungen erhalten:
Die Verbindungen der Formel (I) wurden in einer Konzentration von 1 mg/mL in analysenreinem Dichlormethan gelöst und direkt der HPLC zugeführt.
HPLC Anlage: Waters AllianceHT 2795
Säule: Chiralpak IC, 250 x 4,6mm, 5µm DAIC 83325
Säulen-Temperatur: 25 Grad Celcius
Detektor: PDA 996, Messwellenlänge: 210 nm
Auswertung: Waters Empower 2 Software, SP C

### Bedingungen (Bdg.) a bis d:

a: Eluent: n-Heptan/2-Propanol (70/30), Chromasolv, Fluss: 1,0mL/min
b: Eluent: n-Heptan/2-Propanol (75/25), Chromasolv, Fluss: 1,0mL/min
c: Eluent: n-Heptan/2-Propanol (80/20), Chromasolv, Fluss: 1,0mL/min
d: Eluent: n-Heptan/2-Propanol (90/10), Chromasolv, Fluss: 0,6mL/min
e: Eluent: n-Heptan/2-Propanol (60/40), Chromasolv, Fluss: 1,0mL/min

Die chromatographisch gereinigten Verbindungen (I) haben eine stereochemische Reinheit von über 95%

**Tabelle 1: Verbindungen der Formel (I)**

| | | | | |
|---|---|---|---|---|
| 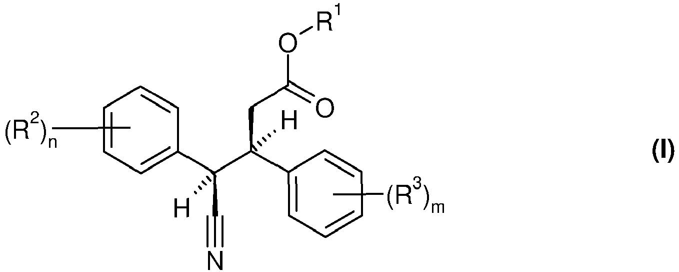 | | | | |

| Bsp. | R¹ | (R²)ₙ | (R³)ₘ | Rtz [min] / Bdg. |
|---|---|---|---|---|
| 1 | Me | 3-F | 4-Cl | 6,4 / a |
| 2 | Me | 3-F | 3-F | 8,3 / c |
| 3 | Me | 3,4-F₂ | 4-Cl | 8,5 / c |
| 4 | Me | 3-F | 4-Br | 7,2 / b |
| 5 | Me | 3-Cl | 3-F | 21,1 / d |
| 6 | Me | 3,4-F₂ | 3-F | 24,3 / d |
| 7 | Me | H | 3-F | 24,5 / d |
| 8 | Me | 4-F | 3-F | 25,9 / d |
| 9 | Me | 3-F | 3-Cl | 20,1 / d |
| 10 | Me | 3,4-F₂ | 3-Cl | 23,1 / d |
| 11 | Me | 3-Br | 3-F | 20,7 / d |
| 12 | Me | 3,5-F₂ | 3-F | 16,9 / d |
| 13 | Me | 3-F | 2-F | 19,2 / d |
| 14 | Me | 3,4,5-F₃ | 4-Cl | 19,4 / d |
| 15 | Me | 4-F | 3-Cl | 23,1 / d |
| 16 | Me | 3,4-F₂ | 3-F, 4-Cl | 21,6 / d |
| 17 | Me | 3,4-F₂ | 2-F | 19,9 / d |
| 18 | Me | 3-F | 2,3-F₂ | 19,5 / d |
| 19 | Me | 4-F | 3,5-F₂ | 20,1 / d |
| 20 | Me | 3-F | 4-F | 20,6 / d |
| 21 | Me | 3,4-F₂ | 3-Cl, 5-F | 20,3 / d |
| 22 | Me | 3-F | 3-Cl, 5-F | 17,8 / d |
| 23 | Me | 3-F | 2,5-F₂ | 18,7 / d |
| 24 | Me | 3,4-F₂ | 2,3-F₂ | 21,5 / d |
| 25 | Me | 3,4-F₂ | 2,5-F₂ | 20,3 / d |
| 26 | Me | 3-Cl | 3-Cl, 5-F | 16,7 / d |
| 27 | Me | 3-F, 4-Cl | 3-Cl | 21,2 / d |
| 28 | Me | 3,4-F₂ | 3-NO₂ | 29,7 / c |
| 29 | Me | 3,4-F₂ | H | 24,8 / d |
| 30 | Me | 3-Cl, 4-F | 3-F | 23,4 / d |
| 31 | Et | 3,4-F₂ | 4-OMe | 29.6 / d |
| 32 | H | 3-F | 4-Cl | |
| 33 | Et | 3-F | 4-Cl | |
| 34 | n-Pr | 3-F | 4-Cl | |
| 35 | i-Pr | 3-F | 4-Cl | |
| 36 | n-Bu | 3-F | 4-Cl | |
| 37 | i-Bu | 3-F | 4-Cl | |
| 38 | 2,2-Difluorethyl | 3-F | 4-Cl | |
| 39 | 2,2,2-Trifluorethyl | 3-F | 4-Cl | |
| 40 | 2-Fluorbenzyl | 3-F | 4-Cl | |
| 41 | 2,5-Difluorbenzyl | 3-F | 4-Cl | |
| 42 | 2,5-Dichlorbenzyl | 3-F | 4-Cl | |
| 43 | 3,5-Dichlorbenzyl | 3-F | 4-Cl | |
| 44 | 2,4-Difluorbenzyl | 3-F | 4-Cl | |
| 45 | 2,6-Difluorbenzyl | 3-F | 4-Cl | |
| 46 | 2,3-Difluorbenzyl | 3-F | 4-Cl | |
| 47 | Ethinyl | 3-F | 4-Cl | |
| 48 | Prop-1-in-1-yl | 3-F | 4-Cl | |
| 49 | Methoxymethyl | 3-F | 4-Cl | |
| 50 | 2-F-Phenyl | 3-F | 4-Cl | |
| 51 | 3-F-Phenyl | 3-F | 4-Cl | |
| 52 | 4-F-Phenyl | 3-F | 4-Cl | |
| 53 | Oxetan-3-yl | 3-F | 4-Cl | 18,4 / d |
| 54 | 2-(Phenylsulfanyl)ethyl | 3-F | 4-Cl | 23,9 / d |
| 55 | 2-(Phenylsulfinyl)ethyl | 3-F | 4-Cl | |
| 56 | 2-(Phenylsulfonyl)ethyl | 3-F | 4-Cl | |
| 57 | H | 3-F | 3-F | |
| 58 | Et | 3-F | 3-F | |
| 59 | n-Pr | 3-F | 3-F | |
| 60 | i-Pr | 3-F | 3-F | |
| 61 | n-Bu | 3-F | 3-F | |
| 62 | i-Bu | 3-F | 3-F | |
| 63 | 2,2-Difluorethyl | 3-F | 3-F | |
| 64 | 2,2,2-Trifluorethyl | 3-F | 3-F | |
| 65 | 2-Fluorbenzyl | 3-F | 3-F | |
| 66 | 2,5-Difluorbenzyl | 3-F | 3-F | |
| 67 | 2,5-Dichlorbenzyl | 3-F | 3-F | |
| 68 | 3,5-Dichlorbenzyl | 3-F | 3-F | |
| 69 | 2,4-Difluorbenzyl | 3-F | 3-F | |
| 70 | 2,6-Difluorbenzyl | 3-F | 3-F | |
| 71 | 2,3-Difluorbenzyl | 3-F | 3-F | |
| 72 | Ethinyl | 3-F | 3-F | |
| 73 | Prop-1-in-1-yl | 3-F | 3-F | |
| 74 | Methoxymethyl | 3-F | 3-F | |
| 75 | 2-F-Phenyl | 3-F | 3-F | |
| 76 | 3-F-Phenyl | 3-F | 3-F | |
| 77 | 4-F-Phenyl | 3-F | 3-F | |
| 78 | Oxetan-3-yl | 3-F | 3-F | |
| 79 | 2-(Phenylsulfanyl)ethyl | 3-F | 3-F | |
| 80 | 2-(Phenylsulfinyl)ethyl | 3-F | 3-F | |
| 81 | 2-(Phenylsulfonyl)ethyl | 3-F | 3-F | |
| 82 | H | 3,4-F₂ | 3-F | |
| 83 | Et | 3,4-F₂ | 3-F | 20,7 / d |
| 84 | n-Pr | 3,4-F₂ | 3-F | 18,4 / d |
| 85 | i-Pr | 3,4-F₂ | 3-F | 16,9 / d |
| 86 | n-Bu | 3,4-F₂ | 3-F | 18,4 / d |
| 87 | i-Bu | 3,4-F₂ | 3-F | 16,2 / d |
| 88 | 2,2-Difluorethyl | 3,4-F₂ | 3-F | |
| 89 | 2,2,2-Trifluorethyl | 3,4-F₂ | 3-F | |
| 90 | 2-Fluorbenzyl | 3,4-F₂ | 3-F | |
| 91 | 2,5-Difluorbenzyl | 3,4-F₂ | 3-F | |
| 92 | 2,5-Dichlorbenzyl | 3,4-F₂ | 3-F | |
| 93 | 3,5-Dichlorbenzyl | 3,4-F₂ | 3-F | |
| 94 | 2,4-Difluorbenzyl | 3,4-F₂ | 3-F | |
| 95 | 2,6-Difluorbenzyl | 3,4-F₂ | 3-F | |
| 96 | 2,3-Difluorbenzyl | 3,4-F₂ | 3-F | |
| 97 | Ethinyl | 3,4-F₂ | 3-F | |
| 98 | Prop-1-in-1-yl | 3,4-F₂ | 3-F | |
| 99 | Methoxymethyl | 3,4-F₂ | 3-F | |
| 100 | 2-F-Phenyl | 3,4-F₂ | 3-F | |
| 101 | 3-F-Phenyl | 3,4-F₂ | 3-F | |
| 102 | 4-F-Phenyl | 3,4-F₂ | 3-F | |
| 103 | Oxetan-3-yl | 3,4-F₂ | 3-F | |
| 104 | 2-(Phenylsulfanyl)ethyl | 3,4-F₂ | 3-F | |
| 105 | 2-(Phenylsulfinyl)ethyl | 3,4-F₂ | 3-F | |
| 106 | 2-(Phenylsulfonyl)ethyl | 3,4-F₂ | 3-F | |
| 107 | H | 3,4-F₂ | 3-Cl | 8,7 / c |
| 108 | Et | 3,4-F₂ | 3-Cl | 19,7 / d |
| 109 | n-Pr | 3,4-F₂ | 3-Cl | 17,6 / d |
| 110 | i-Pr | 3,4-F₂ | 3-Cl | |
| 111 | n-Bu | 3,4-F₂ | 3-Cl | 17,1 / d |
| 112 | i-Bu | 3,4-F₂ | 3-Cl | 15,6 / d |
| 113 | 2,2-Difluorethyl | 3,4-F₂ | 3-Cl | |
| 114 | 2,2,2-Trifluorethyl | 3,4-F₂ | 3-Cl | |
| 115 | 2-Fluorbenzyl | 3,4-F₂ | 3-Cl | |
| 116 | 2,5-Difluorbenzyl | 3,4-F₂ | 3-Cl | |
| 117 | 2,5-Dichlorbenzyl | 3,4-F₂ | 3-Cl | |
| 118 | 3,5-Dichlorbenzyl | 3,4-F₂ | 3-Cl | |
| 119 | 2,4-Difluorbenzyl | 3,4-F₂ | 3-Cl | |
| 120 | 2,6-Difluorbenzyl | 3,4-F₂ | 3-Cl | |
| 121 | 2,3-Difluorbenzyl | 3,4-F₂ | 3-Cl | |
| 122 | Ethinyl | 3,4-F₂ | 3-Cl | |
| 123 | Prop-1-in-1-yl | 3,4-F₂ | 3-Cl | |
| 124 | Methoxymethyl | 3,4-F₂ | 3-Cl | |
| 125 | 2-F-Phenyl | 3,4-F₂ | 3-Cl | |
| 126 | 3-F-Phenyl | 3,4-F₂ | 3-Cl | |
| 127 | 4-F-Phenyl | 3,4-F₂ | 3-Cl | |
| 128 | Oxetan-3-yl | 3,4-F₂ | 3-Cl | |
| 129 | 2-(Phenylsulfanyl)ethyl | 3,4-F₂ | 3-Cl | |
| 130 | 2-(Phenylsulfinyl)ethyl | 3,4-F₂ | 3-Cl | |
| 131 | 2-(Phenylsulfonyl)ethyl | 3,4-F₂ | 3-Cl | |
| 132 | H | 3-F | 3-F | |
| 133 | H | 3,4-F₂ | 4-Cl | |
| 134 | H | 3-F | 4-Br | |
| 135 | H | 3-Cl | 3-F | |
| 136 | H | H | 3-F | |
| 137 | H | 4-F | 3-F | |
| 138 | H | 3-F | 3-Cl | |
| 139 | H | 3-Br | 3-F | |
| 140 | H | 2,5-F₂ | 3-F | |
| 141 | H | 3-F | 2-F | |
| 142 | H | 3,4,5-F₃ | 4-Cl | |
| 143 | H | 4-F | 3-Cl | |
| 144 | H | 3,4-F₂ | 3-F, 4-Cl | |
| 145 | H | 3,4-F₂ | 2-F | |
| 146 | H | 3-F | 2,3-F₂ | |
| 147 | H | 4-F | 3,5-F₂ | |
| 148 | H | 3-F | 4-F | |
| 149 | H | 3,4-F₂ | 3-Cl, 5-F | |
| 150 | H | 3-F | 3-Cl, 5-F | |
| 151 | H | 3-F | 2,5-F₂ | |
| 152 | H | 3,4-F₂ | 2,3-F₂ | |
| 153 | H | 3,4-F₂ | 2,5-F₂ | |
| 154 | H | 3-Cl | 3-Cl, 5-F | |
| 155 | H | 3-F, 4-Cl | 3-Cl | |
| 156 | H | 3,4-F₂ | 3-NO₂ | |
| 157 | H | 3,4-F₂ | H | |
| 158 | H | 3-Cl, 4-F | 3-F | |
| 159 | H | 3,4-F₂ | 4-OMe | |
| 160 | Me | 3-F | H | |
| 161 | Me | 3-F | 2,4--F₂ | |
| 162 | Me | 3-F | 2,6-F₂ | 19,5 / d |
| 163 | Me | 3-F | 3,4-F₂ | |
| 164 | Me | 3-F | 3,5-F₂ | |
| 165 | Me | 3-F | 2-Cl | |
| 166 | Me | 3-F | 2,3-Cl₂ | |
| 167 | Me | 3-F | 2,4-Cl₂ | |
| 168 | Me | 3-F | 2,5-Cl₂ | |
| 169 | Me | 3-F | 2,6-Cl₂ | |
| 170 | Me | 3-F | 3,4-Cl₂ | |
| 171 | Me | 3-F | 3,5-Cl₂ | |
| 172 | Me | 3,4-F₂ | 4-F | |
| 173 | Me | 3,4-F₂ | 2,4-F₂ | |
| 174 | Me | 3,4-F₂ | 2,6-F₂ | 19,3 / d |
| 175 | Me | 3,4-F₂ | 3,4-F₂ | |
| 176 | Me | 3,4-F₂ | 3,5-F₂ | |
| 177 | Me | 3,4-F₂ | 2-Cl | |
| 178 | Me | 3,4-F₂ | 2,3-Cl₂ | |
| 179 | Me | 3,4-F₂ | 2,4-Cl₂ | |
| 180 | Me | 3,4-F₂ | 2,5-Cl₂ | |
| 181 | Me | 3,4-F₂ | 2,6-Cl₂ | |
| 182 | Me | 3,4-F₂ | 3,4-Cl₂ | |
| 183 | Me | 3,4-F₂ | 3,5-Cl₂ | |
| 184 | Me | 3-Cl | H | |
| 185 | Me | 3-Cl | 2-F | |
| 186 | Me | 3-Cl | 4-F | |
| 187 | Me | 3-Cl | 2,3-F₂ | |
| 188 | Me | 3-Cl | 2,4-F₂ | |
| 189 | Me | 3-Cl | 2,5-F₂ | |
| 190 | Me | 3-Cl | 2,6-F₂ | |
| 191 | Me | 3-Cl | 3,4-F₂ | |
| 192 | Me | 3-Cl | 3,5-F₂ | |
| 193 | Me | 3-Cl | 2-Cl | |
| 194 | Me | 3-Cl | 3-Cl | |
| 195 | Me | 3-Cl | 4-Cl | |
| 196 | Me | 3-Cl | 2,3-Cl₂ | |
| 197 | Me | 3-Cl | 2,4-Cl₂ | |
| 198 | Me | 3-Cl | 2,5-Cl₂ | |
| 199 | Me | 3-Cl | 2,6-Cl₂ | |
| 200 | Me | 3-Cl | 3,4-Cl₂ | |
| 201 | Me | 3-Cl | 3,5-Cl₂ | |
| 202 | Me | 3,4-Cl₂ | H | |
| 203 | Me | 3,4-Cl₂ | 2-F | |
| 204 | Me | 3,4-Cl₂ | 3-F | |
| 205 | Me | 3,4-Cl₂ | 4-F | |
| 206 | Me | 3,4-Cl₂ | 2,3-F₂ | |
| 207 | Me | 3,4-Cl₂ | 2,4-F₂ | |
| 208 | Me | 3,4-Cl₂ | 2,5-F₂ | 19,8 / d |
| 209 | Me | 3,4-Cl₂ | 2,6-F₂ | |
| 210 | Me | 3,4-Cl₂ | 3,4-F₂ | |
| 211 | Me | 3,4-Cl₂ | 3,5-F₂ | |
| 212 | Me | 3,4-Cl₂ | 2-Cl | |
| 213 | Me | 3,4-Cl₂ | 3-Cl | |
| 214 | Me | 3,4-Cl₂ | 4-Cl | |
| 215 | Me | 3,4-Cl₂ | 2,3-Cl₂ | |
| 216 | Me | 3,4-Cl₂ | 2,4-Cl₂ | |
| 217 | Me | 3,4-Cl₂ | 2,5-Cl₂ | |
| 218 | Me | 3,4-Cl₂ | 2,6-Cl₂ | |
| 219 | Me | 3,4-Cl₂ | 3,4-Cl₂ | |
| 220 | Me | 3,4-Cl₂ | 3,5-Cl₂ | |
| 221 | Me | 3-Cl, 4-F | H | |
| 222 | Me | 3-Cl, 4-F | 2-F | |
| 223 | Me | 3-Cl, 4-F | 4-F | |
| 224 | Me | 3-Cl, 4-F | 2,3-F₂ | |
| 225 | Me | 3-Cl, 4-F | 2,4-F₂ | |
| 226 | Me | 3-Cl, 4-F | 2,5-F₂ | |
| 227 | Me | 3-Cl, 4-F | 2,6-F₂ | 19,2 / d |
| 228 | Me | 3-Cl, 4-F | 3,4-F₂ | |
| 229 | Me | 3-Cl, 4-F | 3,5-F₂ | |
| 230 | Me | 3-Cl, 4-F | 2-Cl | |
| 231 | Me | 3-Cl, 4-F | 3-Cl | |
| 232 | Me | 3-Cl, 4-F | 4-Cl | |
| 233 | Me | 3-Cl, 4-F | 2,3-Cl₂ | |
| 234 | Me | 3-Cl, 4-F | 2,4-Cl₂ | |
| 235 | Me | 3-Cl, 4-F | 2,5-Cl₂ | |
| 236 | Me | 3-Cl, 4-F | 2,6-Cl₂ | |
| 237 | Me | 3-Cl, 4-F | 3,4-Cl₂ | |
| 238 | Me | 3-Cl, 4-F | 3,5-Cl₂ | |
| 239 | Me | 3-Cl, 4-F | H | |
| 240 | Me | 3-F, 4-Cl | 2-F | |
| 241 | Me | 3-F, 4-Cl | 3-F | |
| 242 | Me | 3-F, 4-Cl | 4-F | |
| 243 | Me | 3-F, 4-Cl | 2,3-F₂ | |
| 244 | Me | 3-F, 4-Cl | 2,4-F₂ | |
| 245 | Me | 3-F, 4-Cl | 2,5-F₂ | |
| 246 | Me | 3-F, 4-Cl | 2,6-F₂ | |
| 247 | Me | 3-F, 4-Cl | 3,4-F₂ | |
| 248 | Me | 3-F, 4-Cl | 3,5-F₂ | |
| 249 | Me | 3-F, 4-Cl | 2-Cl | |
| 250 | Me | 3-F, 4-Cl | 4-Cl | |
| 251 | Me | 3-F, 4-Cl | 2,3-Cl₂ | |
| 252 | Me | 3-F, 4-Cl | 2,4-Cl₂ | |
| 253 | Me | 3-F, 4-Cl | 2,5-Cl₂ | |
| 254 | Me | 3-F, 4-Cl | 2,6-Cl₂ | |
| 255 | Me | 3-F, 4-Cl | 3,4-Cl₂ | |
| 256 | Me | 3-F, 4-Cl | 3,5-Cl₂ | |
| 257 | H | 3-F | 2,6-F₂ | |
| 258 | Et | 3-F | 2,6-F₂ | |
| 259 | n-Pr | 3-F | 2,6-F₂ | |
| 260 | i-Pr | 3-F | 2,6-F₂ | |
| 261 | n-Bu | 3-F | 2,6-F₂ | |
| 262 | i-Bu | 3-F | 2,6-F₂ | |
| 263 | 2,2-Difluorethyl | 3-F | 2,6-F₂ | |
| 264 | 2,2,2-Trifluorethyl | 3-F | 2,6-F₂ | |
| 265 | 2-Fluorbenzyl | 3-F | 2,6-F₂ | |
| 266 | 2,5-Difluorbenzyl | 3-F | 2,6-F₂ | |
| 267 | 2,5-Dichlorbenzyl | 3-F | 2,6-F₂ | |
| 268 | 3,5-Dichlorbenzyl | 3-F | 2,6-F₂ | |
| 269 | 2,4-Difluorbenzyl | 3-F | 2,6-F₂ | |
| 270 | 2,6-Difluorbenzyl | 3-F | 2,6-F₂ | |
| 271 | 2,3-Difluorbenzyl | 3-F | 2,6-F₂ | |
| 272 | Ethinyl | 3-F | 2,6-F₂ | |
| 273 | Prop-1-in-1-yl | 3-F | 2,6-F₂ | |
| 274 | Methoxymethyl | 3-F | 2,6-F₂ | |
| 275 | 2-F-Phenyl | 3-F | 2,6-F₂ | |
| 276 | 3-F-Phenyl | 3-F | 2,6-F₂ | |
| 277 | 4-F-Phenyl | 3-F | 2,6-F₂ | |
| 278 | Oxetan-3-yl | 3-F | 2,6-F₂ | |
| 279 | 2-(Phenylsulfanyl)ethyl | 3-F | 2,6-F₂ | |
| 280 | 2-(Phenylsulfinyl)ethyl | 3-F | 2,6-F₂ | |
| 281 | 2-(Phenylsulfonyl)ethyl | 3-F | 2,6-F₂ | |
| 282 | H | 3,4-F₂ | 2,6-F₂ | |
| 283 | Et | 3,4-F₂ | 2,6-F₂ | 19,1 / d |
| 284 | n-Pr | 3,4-F₂ | 2,6-F₂ | |
| 285 | i-Pr | 3,4-F₂ | 2,6-F₂ | |
| 286 | n-Bu | 3,4-F₂ | 2,6-F₂ | |
| 287 | i-Bu | 3,4-F₂ | 2,6-F₂ | |
| 288 | 2,2-Difluorethyl | 3,4-F₂ | 2,6-F₂ | |
| 289 | 2,2,2-Trifluorethyl | 3,4-F₂ | 2,6-F₂ | |
| 290 | 2-Fluorbenzyl | 3,4-F₂ | 2,6-F₂ | |
| 291 | 2,5-Difluorbenzyl | 3,4-F₂ | 2,6-F₂ | |
| 292 | 2,5-Dichlorbenzyl | 3,4-F₂ | 2,6-F₂ | |
| 293 | 3,5-Dichlorbenzyl | 3,4-F₂ | 2,6-F₂ | |
| 294 | 2,4-Difluorbenzyl | 3,4-F₂ | 2,6-F₂ | |
| 295 | 2,6-Difluorbenzyl | 3,4-F₂ | 2,6-F₂ | |
| 296 | 2,3-Difluorbenzyl | 3,4-F₂ | 2,6-F₂ | |
| 297 | Ethinyl | 3,4-F₂ | 2,6-F₂ | |
| 298 | Prop-1-in-1-yl | 3,4-F₂ | 2,6-F₂ | |
| 299 | Methoxymethyl | 3,4-F₂ | 2,6-F₂ | |
| 300 | 2-F-Phenyl | 3,4-F₂ | 2,6-F₂ | |
| 301 | 3-F-Phenyl | 3,4-F₂ | 2,6-F₂ | |
| 302 | 4-F-Phenyl | 3,4-F₂ | 2,6-F₂ | |
| 303 | Oxetan-3-yl | 3,4-F₂ | 2,6-F₂ | |
| 304 | 2-(Phenylsulfanyl)ethyl | 3,4-F₂ | 2,6-F₂ | |
| 305 | 2-(Phenylsulfinyl)ethyl | 3,4-F₂ | 2,6-F₂ | |
| 306 | 2-(Phenylsulfonyl)ethyl | 3,4-F₂ | 2,6-F₂ | |
| 307 | Me | H | H | |
| 308 | Me | H | 2-F | |
| 309 | Me | H | 4-F | |
| 310 | Me | H | 2,3-F₂ | |
| 311 | Me | H | 2,4-F₂ | |
| 312 | Me | H | 2,5-F₂ | |
| 313 | Me | H | 2,6-F₂ | |
| 314 | Me | H | 3,4-F₂ | |
| 315 | Me | H | 3,5-F₂ | |
| 316 | Me | H | 2-Cl | |
| 317 | Me | H | 3-Cl | |
| 318 | Me | H | 4-Cl | |
| 319 | Me | H | 2,3-Cl₂ | |
| 320 | Me | H | 2,4-Cl₂ | |
| 321 | Me | H | 2,5-Cl₂ | |
| 322 | Me | H | 2,6-Cl₂ | |
| 323 | Me | H | 3,4-Cl₂ | |
| 324 | Me | H | 3,5-Cl₂ | |
| 325 | Me | 3-CN | 3-F | |
| 326 | Me | 3-CN | 3-Cl | 33,88 / a |
| 327 | Me | 3-CN | 3-CN | |
| 328 | Me | 3-CN | 4-F | |
| 329 | Me | 3-CN | 4-Cl | 32,97 / a |
| 330 | Me | 3-CN | 2,5-F₂ | 18,64 / e |
| 331 | Me | 3-CN | 2,6-F₂ | |
| 332 | H | 3-CN | 3-F | |
| 333 | H | 3-CN | 3-Cl | |
| 334 | H | 3-CN | 3-CN | |
| 335 | H | 3-CN | 4-F | |
| 336 | H | 3-CN | 4-Cl | |
| 337 | H | 3-CN | 2,5-F₂ | |
| 338 | H | 3-CN | 2,6-F₂ | |
| 339 | Me | 3-CN, 4-F | 3-F | 31,94 / a |
| 340 | Me | 3-CN, 4-F | 3-Cl | 30,59 / a |
| 341 | Me | 3-CN, 4-F | 3-CN | 37,23 / e |
| 342 | Me | 3-CN, 4-F | 4-F | 12,64 / e |
| 343 | Me | 3-CN, 4-F | 4-Cl | 20,15 / e |
| 344 | Me | 3-CN, 4-F | 2,5-F₂ | 16,64 / e |
| 345 | Me | 3-CN, 4-F | 2,6-F₂ | 17,95 / a |
| 346 | H | 3-CN, 4-F | 3-F | |
| 347 | H | 3-CN, 4-F | 3-Cl | |
| 348 | H | 3-CN, 4-F | 3-CN | |
| 349 | H | 3-CN, 4-F | 4-F | |
| 350 | H | 3-CN, 4-F | 4-Cl | |
| 351 | H | 3-CN, 4-F | 2,5-F₂ | |
| 352 | H | 3-CN, 4-F | 2,6-F₂ | |
| 353 | Me | 3-Br, 4-F | 3-F | |
| 354 | Me | 3-Br, 4-F | 3-Cl | |
| 355 | Me | 3-Br, 4-F | 3-CN | |
| 356 | Me | 3-Br, 4-F | 4-F | |
| 357 | Me | 3-Br, 4-F | 4-Cl | |
| 358 | Me | 3-Br, 4-F | 2,5-F₂ | 20,01 / d |
| 359 | Me | 3-Br, 4-F | 2,6-F₂ | 19,73 / d |
| 360 | H | 3-Br, 4-F | 3-F | |
| 361 | H | 3-Br, 4-F | 3-Cl | |
| 362 | H | 3-Br, 4-F | 3-CN | |
| 363 | H | 3-Br, 4-F | 4-F | |
| 364 | H | 3-Br, 4-F | 4-Cl | |
| 365 | H | 3-Br, 4-F | 2,5-F₂ | |
| 366 | H | 3-Br, 4-F | 2,6-F₂ | |
| 367 | Me | 3-F, 4-CN | 3-F | |
| 368 | Me | 3-F, 4-CN | 3-Cl | |
| 369 | Me | 3-F, 4-CN | 3-CN | |
| 370 | Me | 3-F, 4-CN | 4-F | |
| 371 | Me | 3-F, 4-CN | 4-Cl | |
| 372 | Me | 3-F, 4-CN | 2,5-F₂ | |
| 373 | Me | 3-F, 4-CN | 2,6-F₂ | |
| 374 | H | 3-F, 4-CN | 3-F | |
| 375 | H | 3-F, 4-CN | 3-Cl | |
| 376 | H | 3-F, 4-CN | 3-CN | |
| 377 | H | 3-F, 4-CN | 4-F | |
| 378 | H | 3-F, 4-CN | 4-Cl | |
| 379 | H | 3-F, 4-CN | 2,5-F₂ | |
| 380 | H | 3-F, 4-CN | 2,6-F₂ | |
| 381 | Me | 3-F | 3-CN | |
| 382 | Me | 3,4-F₂ | 3-CN | 14,71 / e |
| 383 | H | 3-F | 3-CN | |
| 384 | H | 3,4-F₂ | 3-CN | |
| 385 | Me | 3-F | 4-NO₂ | |
| 386 | Me | 3,4-F₂ | 4-NO₂ | |
| 387 | H | 3-F | 4-NO₂ | |
| 388 | H | 3,4-F₂ | 4-NO₂ | |
| 389 | Me | 3-F | 3-F, 4-OMe | |
| 390 | Me | 3,4-F₂ | 3-F, 4-OMe | 43,06 / a |
| 391 | H | 3-F | 3-F, 4-OMe | |
| 392 | H | 3,4-F₂ | 3-F, 4-OMe | |
| 393 | Me | 3-F | 3-NO₂ | |
| 394 | H | 3-F | 3-NO₂ | |
| 395 | Me | 3-CN, 4-F | 3,5-F₂ | 26,67 / a |
| 396 | H | 3-CN, 4-F | 3,5-F₂ | |
| 397 | Me | 3,5-F₂ | 3-Cl | |
| 398 | Me | 3,5-F₂ | 3-CN | |
| 399 | Me | 3,5-F₂ | 4-F | |
| 400 | Me | 3,5-F₂ | 4-Cl | |
| 401 | Me | 3,5-F₂ | 2,5-F₂ | |
| 402 | Me | 3,5-F₂ | 2,6-F₂ | |
| 403 | H | 3,5-F₂ | 3-F | |
| 404 | H | 3,5-F₂ | 3-Cl | |
| 405 | H | 3,5-F₂ | 3-CN | |
| 406 | H | 3,5-F₂ | 4-F | |
| 407 | H | 3,5-F₂ | 4-Cl | |
| 408 | H | 3,5-F₂ | 2,5-F₂ | |
| 409 | H | 3,5-F₂ | 2,6-F₂ | |
| 410 | Me | 2,5-F₂ | 3-F | |
| 411 | Me | 2,5-F₂ | 3-Cl | |
| 412 | Me | 2,5-F₂ | 3-CN | |
| 413 | Me | 2,5-F₂ | 4-F | |
| 414 | Me | 2,5-F₂ | 4-Cl | |
| 415 | Me | 2,5-F₂ | 2,5-F₂ | |
| 416 | Me | 2,5-F₂ | 2,6-F₂ | |
| 417 | H | 2,5-F₂ | 3-Cl | |
| 418 | H | 2,5-F₂ | 3-CN | |
| 419 | H | 2,5-F₂ | 4-F | |
| 420 | H | 2,5-F₂ | 4-Cl | |
| 421 | H | 2,5-F₂ | 2,5-F₂ | |
| 422 | H | 2,5-F₂ | 2,6-F₂ | |

Weiter physikalische Daten zu Tabelle 1:
¹H-NMR-Daten (CDCl₃) - Chemische Verschiebung ausgewählter charakteristischer Signale in ppm:

| | |
|---|---|
| Bsp. 1: | 2,89 (m, 2H), 3,58 (s, 3H), 3,64 (m, 1H), 4,08 (d, 1H), 7,09 (d, 2H), 7,28 (d, 2H) |
| Bsp. 2: | 2,90 (m, 2H), 3,58 (s, 3H), 3,66 (m, 1H), 4,09 (d, 1H), 6,88 (m, 1H), 6,90 (m, 1H) |
| Bsp. 3: | 2,89 (m, 2H), 3,59 (s, 3H), 3,61 (m, 1H), 4,06 (d, 1H), 7,05 (d, 2H), 7,29 (d, 2H) |
| Bsp. 4: | 2,89 (m, 2H), 3,58 (s, 3H), 3,63 (m, 1H), 4,09 (d, 1H), 7,03 (d, 2H), 7,43 (d, 2H) |
| Bsp. 5: | 2,89 (m, 2H), 3,59 (s, 3H), 3,65 (m, 1H), 4,08 (d, 1H), 7,20 (s, 1H) |
| Bsp. 6: | 2,90 (d, 2H), 3,60 (s, 3H), 3,62 (q, 1H), 4,08 (d, 1H), 7,11 (dd, 1H) |
| Bsp. 7: | 2,89 (m, 2H), 3,58 (s, 3H), 3,68 (m, 1H), 4,10 (d, 1H), 6,87 (m, 1H), 6,97 (m, 2H) |
| Bsp. 8: | 2,90 (d, 2H), 3,59 (s, 3H), 3,63 (q, 1H), 4,08 (d, 1H), 7,02 (t, 2H) |
| Bsp. 9: | 2,90 (m, 2H), 3,58 (s, 3H), 3,62 (m, 1H), 4,10 (d, 1H), 7,13 (s, 1H) |
| Bsp. 10: | 2,90 (d, 2H), 3,60 (s, 3H), 3,61 (q, 1H), 4,08 (d, 1H), 6,89 (m, 1H) |
| Bsp. 11: | 2,89 (m, 2H), 3,58 (s, 3H), 3,65 (m, 1H), 4,07 (d, 1H), 7,11 (d, 1H), 7,20 (t, 1H), 7,47 (d, 1H) |
| Bsp. 12: | 2,90 (m, 2H), 3,59 (s, 3H), 3,65 (q, 1H), 4,10 (d, 1H), 6,95 (d, 1H) |
| Bsp. 13: | 2,90 (dd, 1H), 3,03 (dd, 1H), 3,55 (s, 3H), 3,96 (m, 1H), 4,21 (d, 1H), 7,19 (t, 1H) |
| Bsp. 14: | 2,89 (d, 2H), 3,60 (s, 3H), 3,60 (q, 1H), 4,04 (d, 1H), 6,81 (t, 1H), 7,07 (d, 2H), 7,29 (d, 2H) |
| Bsp. 15: | 2,89 (d, 2H), 3,59 (s, 3H), 3,61 (q, 1H), 4,08 (d, 1H), 7,01 (m, 3H), 7,11 (m, 3H) |
| Bsp. 16: | 2,87 (d, 2H), 3,60 (s, 3H), 3,61 (q, 1H), 4,05 (d, 1H), 7,03 (m, 1H), 7,14 (dd, 1H), 7,34 (t, 1H) |
| Bsp. 17: | 2,95 (dd, 1H), 3,02 (dd, 1H), 3,57 (s, 3H), 3,91 (m, 1H), 4,17 (d, 1H), 7,08 (m, 6H) |
| Bsp. 18: | 2,90 (dd, 1H), 3,02 (dd, 1H), 3,56 (s, 3H), 3,99 (m, 1H), 4,19 (d, 1H), 7,05 (m, 6H), 7,31 (m, 1H) |
| Bsp. 19: | 2,88 (m, 2H), 3,60 (s, 3H), 3,61 (m, 1H), 4,07 (d, 1H), 7,04 (m, 2H), 7,16 (m, 2H) |
| Bsp. 20: | DMSO-d6: 2,91 (m, 2H), 3,44 (s, 3H), 3,75 (dd, 1H), 4,65 (d, 1H), 7,08 (m, 5H), 7,24 (m, 2H), 7,34 (m, 1H) |
| Bsp. 21: | 2,88 (d, 2H), 3,59 (q, 1H), 3,61 (s, 3H), 4,08 (d, 1H), 6,29 (d, 1H), 7,15 (dd, 1H) |
| Bsp. 22: | 2,88 (m, 2H), 3,59 (s, 3H), 3,62 (m, 1H), 4,08 (d, 1H), 6,78 (m, 1H), 7,34 (m, 1H) |
| Bsp. 23: | 2,90 (dd, 1H), 3,00 (dd, 1H), 3,57 (s, 3H), 3,93 (m, 1H), 4,19 (d, 1H), 7,09 (d, 1H), 7,32 (dd, 1H) |
| Bsp. 24: | 2,94 (dd, 1H), 3,01 (dd, 1H), 3,57 (s, 3H), 3,96 (m, 1H), 4,15 (d, 1H), 6,92 (t, 1H) |
| Bsp. 25: | 2,91 (dd, 1H), 2,98 (dd, 1H), 3,58 (s, 3H), 3,88 (m, 1H), 4,15 (d, 1H), 6,87 (m, 1H), 7,11 (m, 2H) |
| Bsp. 26: | 2,84 (dd, 1H), 2,89 (dd, 1H), 3,59 (s, 3H), 3,60 (m, 1H), 4,06 (d, 1H), 6,96 (s, 1H)), 7,21 (s, 1H) |
| Bsp. 27: | 2.88 (m, 2H), 3.58 (s, 3H), 3.61 (dd, 1H), 4.09 (d, 1H), 6.90 (m, 1H), 7.01 (m, 2H), 7.12 (s, 1H), 7.25 (m, 2H), 7.36 (m, 1H) |
| Bsp. 28: | 2.96 (d, 2H), 3.59 (s, 3H), 3.77 (dd, 1H), 4.13 (d, 1H), 6.90 (m, 1H), 7.03 (m, 1H), 7.13 (s, 1H), 7.51 (m, 2H), 8.01 (m, 1H), 8.16 (m, 1H) |
| Bsp. 29: | 2.91 (d, 2H), 3.57 (s, 3H), 3.63 (dd, 1H), 4.07 (d, 1H), 6.87 (m, 1H), 6.96 (m, 1H), 7.15 (m, 3H), 7.28 (m, 3H) |
| Bsp. 30: | 2,89 (d, 2H), 3,58 (s, 3H), 3,62 (q, 1H), 4,06 (d, 1H), 7,09 (t, 1H) |
| Bsp. 31: | 1.13 (t, 3H), 2.85 (d, 2H), 3.57 (dd, 1H), 3.78 (s, 3H), 4.00 (q, 2H), 4.02 (d, 1H), 6.80 (d, 1H), 6.85 (m, 2H), 6.95 (m, 1H), 6.98 (d, 2H), 7.09 (m, 1H) |
| Bsp. 53: | 2,96 (m, 2H), 3,62 (q, 1H), 4,05 (d, 1H), 4,41 (m, 2H), 4,78 (q, 2H), 5,29 (quint, 1H), 7,10 (d, 2H), 7,30 (d, 2H) |
| Bsp. 54: | 2,85 (m, 2H), 2,97 (t, 2H), 3,60 (q, 1H), 4,08 (d, 1H), 4,11 (m, 2H), 7,09 (d, 2H), 7,28 (d, 2H) |
| Bsp. 55: | 2,85 (m, 2H), 2,94 (m, 2H), 3,62 (m, 1H), 4,07 (m, 1H), 4,36 (m, 2H) |
| Bsp. 83: | 1,13 (t, 3H), 2,89 (d, 2H), 3,62 (q, 1H), 4,03 (m, 3H), 6,83 (d, 1H) |
| Bsp. 84: | 0,83 (t, 3H), 1,51 (sext, 2H), 2,89 (d, 2H), 3,61 (q, 1H), 3,93 (t, 2H), 4,06 (d, 1H), 6,84 (d, 1H) |
| Bsp. 85: | 1,06 (d, 3H), 1,10 (d, 3H), 2,85 (m, 2H), 3,60 (q, 1H), 4,04 (d, 1H), 4,87 (m, 1H), 6,82 (d, 1H) |
| Bsp. 86: | 0,86 (t, 3H), 1,24 (sext, 2H), 1,47 (quint, 2H), 2,88 (d, 2H), 3,61 (q, 1H), 3,97 (t, 2H), 4,06 (d, 1H), 6,83 (d, 1H) |
| Bsp. 87: | 0,82 (d, 6H), 1,79 (sept, 1H), 2,89 (m, 2H), 3,61 (q, 1H), 3,74 (d, 2H), 4,06 (d, 1H), 6,84 (d, 1H) |
| Bsp. 107: | 2,88 (d, 2H), 3,56 (q, 1H), 4,05 (d, 1H), 6,89 (m, 1H) |
| Bsp. 108: | 1,13 (t, 3H), 2,87 (d, 2H), 3,59 (m, 1H), 4,03 (m, 3H), 6,88 (m, 1H) |
| Bsp. 109: | 0,83 (t, 3H), 1,54 (sext, 2H), 2,88 (d, 2H), 3,58 (q, 1H), 3,93 (t, 2H), 4,06 (d, 1H), 6,88 (m, 1H) |
| Bsp. 111: | 0,86 (t, 3H), 1,25 (sext, 2H), 1,45 (quint, 2H), 2,87 (d, 2H), 3,59 (q, 1H), 3,97 (t, 2H), 4,06 (d, 1H), 6,89 (m, 1H) |
| Bsp. 112: | 0,82 (d, 6H), 1,80 (sept, 1H), 2,88 (m, 2H), 3,59 (q, 1H), 3,75 (d, 2H), 4,03 (d, 1H), 6,88 (m, 1H) |
| Bsp. 162: | 3,12 (m, 1H), 3,21 (m, 1H), 3,61 (s, 3H), 4,16 (m, 2H), 6,75 (t, 2H), 6,91 (m, 2H), 6,98 (d, 1H), 7,14 (m, 1H), 7,21 (m, 1H) |
| Bsp. 174: | 3,11 (m, 1H), 3,19 (m, 1H), 3,61 (s, 3H), 4,13 (m, 2H), 6,77 (t, 2H), 6,91 (m, 1H), 7,02 (m, 2H), 7,16 (m, 1H) |
| Bsp. 208: | 2,91 (dd, 1H), 2,95 (dd, 1H), 3,58 (s, 3H), 3,89 (m, 1H), 4,15 (d, 1H), 6,88 (m, 1H), 6,96 (m, 1H), 7,02 (m, 1H), 7,11 (dd, 1H), 7,37 (d, 1H), 7,42 (d, 1H) |
| Bsp. 227: | 3,12 (m, 1H), 3,18 (m, 1H), 3,61 (s, 3H), 4,12 (m, 2H), 6,77 (t, 2H), 7,00 (t, 1H), 7,05 (m, 1H), 7,16 (m, 1H), 7,22 (m, 1H) |
| Bsp. 283 | 1,13 (t, 3H), 3,09 (m, 1H), 3,20 (m, 1H), 4,08 (m, 2H), 4,13 (m, 2H), 6,77 (t, 2H), 6,91 (m, 1H), 7,02 (m, 2H), 7,16 (m, 1H) |
| Bsp. 326: | 2,90 (m, 2H), 3,59 (s, 3H), 3,64 (q, 1H), 4,14 (d, 1H), 7,00 (m, 1H), 7,06 (s, 1H), 7,25 (m, 2H), 7,44 (m, 3H), 7,63 (m, 1H) |
| Bsp. 329: | 2,89 (m, 2H), 3,58 (s, 3H), 3,64 (q, 1H), 4,13 (d, 1H), 7,03 (d, 2H), 7,26 (d, 2H), 7,37 (m, 1H), 7,45 (m, 2H), 7,63 (m, 1H) |
| Bsp. 330: | 2,98 (m, 2H), 3,58 (s, 3H), 3,92 (m, 1H), 4,21 (d, 1H), 6,83 (m, 1H), 7,00 (m, 2H), 7,51 (m, 3H), 7,62 (m, 1H) |
| Bsp. 339: | 2,92 (d, 2H), 3,61 (s, 3H), 3,62 (m, 1H), 4,13 (d, 1H), 6,79 (m, 1H), 6,87 (m, 1H), 6,99 (m, 1H), 7,19 (m, 1H), 7,28 (m, 1H), 7,37 (m, 2H) |
| Bsp. 340: | 2,90 (d, 2H), 3,61 (s, 3H), 3,61 (q, 1H), 4,14 (d, 1H), 6,97 (m, 1H), 7,06 (m, 1H), 7,23 (m, 3H), 7,48 (m, 2H) |
| Bsp. 341: | 2,92 (m, 2H), 3,58 (s, 3H), 3,89 (q, 1H), 4,17 (d, 1H), 7,21 (m, 1H), 7,48 (m, 6H) |
| Bsp. 342: | 2,89 (d, 2H), 3,60 (s, 3H), 3,63 (m, 1H), 4,12 (d, 1H), 7,02 (m, 4H), 7,19 (m, 1H), 7,32 (m, 2H) |
| Bsp. 343: | 2,89 (d, 2H), 3,61 (s, 3H), 3,61 (m, 1H), 4,13 (d, 1H), 7,01 (m, 2H), 7,18 (m, 1H), 7,38 (m, 4H) |
| Bsp. 344: | 2,97 (m, 2H), 3,60 (s, 3H), 3,88 (m, 1H), 4,21 (d, 1H), 6,83 (m, 1H), 7,01 (m, 2H), 7,21 (m, 1H), 7,49 (m, 2H) |
| Bsp. 345: | 3,17 (m, 2H), 3,62 (s, 3H), 4,14 (m, 1H), 4,19 (d, 1H), 6,79 (m, 2H), 7,17 (m, 2H), 7,33 (m, 1H), 7,49 (m, 1H) |
| Bsp. 358: | 2,94 (d, 2H), 3,61 (s, 3H), 3,62 (m, 1H), 4,15 (d, 1H), 6,87 (m, 1H), 6,98 (m, 2H), 7,08 (m, 1H), 7,19 (m, 1H), 7,47 (m, 1H) |
| Bsp. 359: | 3,17 (m, 2H), 3,61 (s, 3H), 4,13 (m, 2H), 6,78 (t, 2H), 6,99 (t, 1H), 7,16 (m, 2H), 7,46 (m, 1H) |
| Bsp. 382: | 2,92 (d, 2H), 3,59 (s, 3H), 3,68 (q, 1H), 4,09 (d, 1H), 6,89 (m, 1H), 7,01 (m, 1H), 7,13 (m, 1H), 7,42 (m, 3H), 7,59 (m, 1H) |
| Bsp. 390: | 2,85 (m, 2H), 3,53 (q, 1H), 3,59 (s, 3H), 3,86 (s, 3H), 4,03 (d, 1H), 6,83 (m, 4H), 6,99 (m, 1H), 7,11 (m, 1H) |
| Bsp. 395: | 2,89 (d, 2H), 3,60 (q, 1H), 3,62 (s, 3H), 4,14 (d, 1H), 6,65 (m, 2H), 6,75 (m, 1H), 7,21 (m, 1H), 7,42 (m, 2H) |

### B) Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| 75 Gewichtsteile | einer Verbindung der Formel (I), |
|---|---|
| 10 | " ligninsulfonsaures Calcium, |
| 5 | " Natriumlaurylsulfat, |
| 3 | " Polyvinylalkohol und |
| 7 | " Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| | |
|---|---|
| 25 Gewichtsteile einer Verbindung der Formel (I), | |
| 5 " | 2,2'-dinaphthylmethan-6,6'-disulfonsaures Natrium |
| 2 " | oleoylmethyltaurinsaures Natrium, |
| 1 Gewichtsteil | Polyvinylalkohol, |
| 17 Gewichtsteile | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### (C) Biologische Beispiele

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten erfindungsgemäßen Verbindungen (I) werden dann als wäßrige Suspension bzw. Emulsion mit einer Wasseraufwandmenge von umgerechnet 600 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen.

Erfindungsgemäße Verbindungen (I), wie beispielsweise die Verbindungen Nr. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 53, 54, 55, 83, 84, 85, 86, 87, 107, 108, 109, 111, 112, 162, 174, 208, 227, 283, 326, 329, 330, 339, 340, 343, 344, 358, 359 und 390 aus Tabelle 1, weisen eine gute herbizide Wirksamkeit gegen mehrere Schadpflanzen bei einer Aufwandmenge von 320 g oder weniger Aktivsubstanz pro Hektar im Vorauflauf auf. Beispielsweise haben diese Verbindungen eine sehr gute Wirkung (90-100%) gegen Schadpflanzen wie Alopecurus myosuroides, Echinochloa crus galli und Setaria viridis im Vorauflaufverfahren bei einer Aufwandmenge von 0,32 kg Aktivsubstanz pro Hektar.

Außerdem weisen beispielsweise die Verbindungen Nr. 1, 2, 3, 4, 5, 6, 8, 10, 12, 13, 16, 17, 18, 20, 21, 22, 23, 24, 25, 26, 27, 28, 29, 31, 53, 54, 83, 84, 85, 86, 87, 108, 109, 162, 174, 283, 326, 329, 330, 339, 340, 343, 344, 345, 358 und 359 sehr gute herbizide Wirkung (90-100%) gegen Schadpflanzen wie Lolium multiflorum und Veronica persica im Vorauflaufverfahren bei einer Aufwandmenge von 0,32 kg Aktivsubstanz pro Hektar auf.

Außerdem weisen beispielsweise die Verbindungen Nr. 2, 3, 4, 5, 6, 8, 9, 10, 11, 12, 13, 15, 17, 18, 20, 21, 22, 23, 24, 25, 26, 29, 31, 53, 54, 83, 84, 85, 86, 87, 108, 109, 111, 162, 174, 208, 227, 283, 326, 329, 330, 339, 340, 343, 344, 345, 358, 359 und 390 sehr gute herbizide Wirkung (90-100%) gegen Schadpflanzen wie Amaranthus retroflexus im Vorauflaufverfahren bei einer Aufwandmenge von 0,32 kg Aktivsubstanz pro Hektar auf.

Außerdem weisen beispielsweise die Verbindungen Nr. 2, 3, 5, 6, 8, 13, 17, 18, 20, 21, 22, 23, 24, 25, 28, 83, 84, 85, 87, 108, 162, 174, 227, 283, 326, 329, 330, 340, 343, 344, 345, 358 und 359 sehr gute herbizide Wirkung (90 bis 100%) gegen Schadpflanzen wie Avena fatua im Vorauflaufverfahren bei einer Aufwandmenge von 0,32 kg Aktivsubstanz pro Hektar auf.

Außerdem weisen beispielsweise die Verbindungen Nr. 1, 2, 3, 5, 6, 8, 10, 13, 16, 17, 19, 20, 21, 22, 23, 24, 25, 26, 29, 30, 31, 54, 83, 84, 85, 87, 109, 111, 162, 174, 208, 227, 283, 330, 340, 343, 344, 345, 358, 359 und 390 sehr gute herbizide Wirkung (90 bis 100%) gegen Schadpflanzen wie Polygonum convolvulus im Vorauflaufverfahren bei einer Aufwandmenge von 0,32 kg Aktivsubstanz pro Hektar auf.

Außerdem weisen beispielsweise die Verbindungen Nr. 1, 2, 3, 5, 8, 10, 13, 17, 19, 20, 21, 22, 24, 25, 26, 27, 29, 31, 53, 54, 83, 84, 85, 87, 108, 109, 111, 162, 174, 283, 330, 340, 343, 344, 345, 358 und 359 sehr gute herbizide Wirkung (90 bis 100%) gegen Schadpflanzen wie Stellaria media im Vorauflaufverfahren bei einer Aufwandmenge von 0,32 kg Aktivsubstanz pro Hektar auf.

In der Regel besitzen die genannten Wirkstoffe und andere Wirkstoffe aus der Tabelle 1 noch ein wesentlich breiteres Wirkstoffspektrum.

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt, wobei die in Form von benetzbaren Pulvern (WP) formulierten erfindungsgemäßen Verbindungen (I) als wäßrige Suspension bzw. Emulsion mit einer Wasseraufwandmenge von umgerechnet 600 l/ha unter Zusatz von 0,2% Netzmittel auf die grünen Pflanzenteile gesprüht werden. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen.

Wie die Ergebnisse zeigen, weisen erfindungsgemäße Verbindungen (I), wie beispielsweise die Verbindungen Nr. 1, 2, 3, 5, 6, 7, 8, 9, 10, 13, 15, 16, 17, 18, 19, 20, 21, 22, 23, 24, 25, 26, 29, 30, 83, 84, 87, 108, 109, 111, 112, 162, 174, 208, 283, 326, 329, 358, 359, 330, 339, 340, 343, 344, 345, 359 und 390 aus Tabelle 1, eine gute herbizide Wirksamkeit (90-100%) gegen Schadpflanzen wie Echinochloa crus galli im Nachauflaufverfahren bei einer Aufwandmenge von 0,32 kg Aktivsubstanz pro Hektar auf.

Aufßerdem weisen beispielsweise die Verbindungen Nr. 1, 2, 5, 6, 7, 10, 13, 16, 18, 23, 24, 25, 26, 29, 30, 53, 54, 174, 283, 329, 339, 340 und 345 aus Tabelle 1 eine gute herbizide Wirksamkeit (90-100%) gegen Schadpflanzen wie Lolium multiflorum im Nachauflaufverfahren bei einer Aufwandmenge von 0,32 kg Aktivsubstanz pro Hektar auf.

Aufßerdem weisen beispielsweise die Verbindungen Nr. 5, 6, 7, 8, 9, 10, 18, 19, 20, 21, 22, 25, 29, 53, 54, 55, 108, 109, 111, 208, 329, 339, 340, 343, 344, 345 und 359 aus Tabelle 1 eine gute herbizide Wirksamkeit (90-100%) gegen Schadpflanzen wie Setaria viridis im Nachauflaufverfahren bei einer Aufwandmenge von 0,32 kg Aktivsubstanz pro Hektar auf.

Aufßerdem weisen beispielsweise die Verbindungen Nr. 1, 3, 6, 8, 9, 10, 15, 18, 23, 24, 25, 26, 27, 28, 29, 30, 162, 174, 339, 340, 341, 344, 345 und 359 aus Tabelle 1 eine gute herbizide Wirksamkeit (90 bis 100%) gegen Schadpflanzen wie Alopecurus myosuroides im Nachauflaufverfahren bei einer Aufwandmenge von 0,32 kg Aktivsubstanz pro Hektar auf.

Aufßerdem weisen beispielsweise die Verbindungen Nr. 1, 5, 6, 7, 8, 10, 15, 18, 21, 23 25, 26, 27, 29, 339, 340, 344, 345 und 358 aus Tabelle 1 eine gute herbizide Wirksamkeit (90 bis 100%) gegen Schadpflanzen wie Veronica persica im Nachauflaufverfahren bei einer Aufwandmenge von 0,32 kg Aktivsubstanz pro Hektar auf.

In der Regel besitzen die genannten Wirkstoffe und andere Wirkstoffe aus der Tabelle 1 noch ein wesentlich breiteres Wirkstoffspektrum.

### 3. Herbizide Wirkung und Kulturpflanzenverträglichkeit

3.1 In weiteren Versuchen im Gewächshaus werden Samen von Kulturpflanzen in Töpfen in sandigem Lehmboden ausgelegt, mit Erde abgedeckt und unter guten Wachstumsbedingungen angezogen und analog den Schadpflanzen in Abschnitt 1 im Vorauflaufverfahren behandelt und ausgewertet. Die Ergebnisse zeigen, dass die erfindungsgemäßen Verbindungen zweikeimblättrige Kulturen wie z.B. Soja, Baumwolle, Raps, Zuckerrüben und Kartoffeln im Vorauflaufverfahren selbst bei hohen Wirkstoffdosierungen ungeschädigt lassen. Einige Substanzen schonen darüber hinaus aus Gramineen-Kulturen wie z.B. Gerste, Weizen, Roggen, Sorghum-Hirsen, Mais oder Reis.
   Beispielsweise zeigen die Verbindungen Nr. 1, 2, 3, 4, 7, 9, 11, 13, 19, 20 und 23 aus Tabelle 1 eine Selektivität in Mais (kein Schaden oder weniger als 30% Schaden) im Vorauflaufverfahren bei 320 g/ha. Im Hinblick auf die in Abschnitt 1 genannte herbizide Wirkung bei 320 g/ha eignen sich die Verbindungen zur Bekämpfung von unerwünschtem Pflanzenwuchs in Maiskulturen im Vorauflauf.
   Beispielsweise zeigen die Verbindungen Nr. 1, 15 und 27 aus Tabelle 1 eine Selektivität in Weizen (kein Schaden oder weniger als 30% Schaden) im Vorauflaufverfahren bei 320 g/ha. Im Hinblick auf die in Abschnitt 1 genannte herbizide Wirkung bei 320 g/ha eignen sich die Verbindungen zur Bekämpfung von unerwünschtem Pflanzenwuchs in Getreidekulturen im Vorauflauf.
   Beispielsweise zeigen die Verbindungen Nr. 19, 29, 30 und 55 aus Tabelle 1 eine Selektivität in Raps (kein Schaden oder weniger als 30% Schaden) im Vorauflaufverfahren bei 320 g/ha. Im Hinblick auf die in Abschnitt 1 genannte herbizide Wirkung bei 320 g/ha eignen sich die Verbindungen zur Bekämpfung von unerwünschtem Pflanzenwuchs in Rapskulturen im Vorauflauf.
3.2 In weiteren Versuchen im Gewächshaus werden Samen von Kulturpflanzen und Samen von Schadpflanzen in Töpfen in sandigem Lehmboden ausgelegt, mit Erde abgedeckt und unter guten Wachstumsbedingungen angezogen. Die in Form von benetzbaren Pulvern (WP) formulierten erfindungsgemäßen Verbindungen (I) werden dann als wäßrige Suspension bzw. Emulsion mit einer Wasseraufwandmenge von umgerechnet 300 l/ha unter Zusatz von 0,2% Netzmittel auf die Oberfläche der Abdeckerde appliziert. Nach ca. 4 Wochen Standzeit der Versuchspflanzen im Gewächshaus unter optimalen Wachstumsbedingungen wird die Wirkung der Präparate visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen.
   Die Ergebnisse zeigen, dass die Verbindungen (I) effektiv zur Kontrolle von Schadpflanzen in zweikeimblättrige Kulturen wie z.B. Soja, Baumwolle, Raps, Zuckerrüben und Kartoffeln und auch in Gramineen-Kulturen wie z.B. Gerste, Weizen, Roggen, Sorghum-Hirsen, Mais oder Reis im Vorauflaufverfahren eingesetzt werden können.

Beispielsweise zeigen die Verbindungen Nr. 1, 4, 6, 8 und 10 aus Tabelle 1 eine Selektivität in Weizen (kein Schaden oder weniger als 10% Schaden) im Vorauflaufverfahren bei 80 g/ha und zugleich eine gute bis sehr gute herbizide Wirksamkeit (75 bis 100%) gegen Schadpflanzen wie Alopecurus myosuroides. Lolium multiflorum und Amaranthus retroflexus

Beispielsweise zeigen die Verbindungen Nr. 1, 6, 7, 8, 10, 11 und 111 aus Tabelle 1 eine Selektivität in Weizen (kein Schaden oder weniger als 10% Schaden) im Vorauflaufverfahren bei 80 g/ha und zugleich eine gute bis sehr gute herbizide Wirksamkeit (75 bis 100%) gegen Schadpflanzen wie Echinochloa crus-galli, Setaria viridis und Veronica persica.

Darüberhinaus zeigen die Verbindungen Nr. 1, 4, 6, 14, 15 und 111 aus Tabelle 1 eine Selektivität in Weizen (kein Schaden oder weniger als 10% Schaden) im Vorauflaufverfahren bei 80 g/ha und zugleich eine gute bis sehr gute herbizide Wirksamkeit (75 bis 100%) gegen Schadpflanzen wie Echinochloa crus-galli, Setaria viridis und Viola tricolor.

Beispielsweise zeigen die Verbindungen Nr. 1, 7, 11, 13, 19, 29, 30, 55, 85 und 87 aus Tabelle 1 eine Selektivität in Raps (kein Schaden oder weniger als 10% Schaden) im Vorauflaufverfahren bei 80 g/ha und zugleich eine gute bis sehr gute herbizide Wirksamkeit (75 bis 100%) gegen Schadpflanzen wie Echinochloa crus-galli, Setaria viridis und Veronica persica.

Beispielsweise zeigen die Verbindungen Nr. 1, 7, 11, 13, 85 und 87 aus Tabelle 1 eine Selektivität in Raps (kein Schaden oder weniger als 10% Schaden) im Vorauflaufverfahren bei 80 g/ha und zugleich eine gute bis sehr gute herbizide Wirksamkeit (75 bis 100%) gegen Schadpflanzen wie Alopecurus myosuroides.

Beispielsweise zeigen die Verbindungen Nr. 1, 2, 3, 4, 5, 9, 11, 13, 14, 23, 27, 29 und 162 aus Tabelle 1 eine Selektivität in Mais (kein Schaden oder weniger als 10% Schaden) im Vorauflaufverfahren bei 80 g/ha und zugleich eine gute bis sehr gute herbizide Wirksamkeit (75 bis 100%) gegen Schadpflanzen wie Alopecurus myosuroides.

Beispielsweise zeigen die Verbindungen Nr. 1, 2, 3, 4, 5, 9, 11, 13, 14, 19, 23, 27, 29, 30, 31, 111, 162, 227 und 339 aus Tabelle 1 eine Selektivität in Mais (kein Schaden oder weniger als 10% Schaden) im Vorauflaufverfahren bei 80 g/ha und zugleich eine gute bis sehr gute herbizide Wirksamkeit (75 bis 100%) gegen Schadpflanzen wie Echinochloa crus-galli und Setaria viridis.

Weiterhin zeigen die Verbindungen Nr. 1, 2, 23 und 29 aus Tabelle 1 eine Selektivität in Mais (kein Schaden oder weniger als 10% Schaden) im Vorauflaufverfahren bei 80 g/ha und zugleich eine gute bis sehr gute herbizide Wirksamkeit (75 bis 100%) gegen Schadpflanzen wie Lolium multiflorum, Amaranthus retroflexus und Veronica persica.

Beispielsweise zeigen die Verbindungen Nr. 1, 2, 3, 5, 7, 9, 11, 12, 13, 18, 19, 20, 22, 23 und 174 aus Tabelle 1 eine Selektivität in Mais (0-20% Schaden) im Vorauflaufverfahren bei 50-62 g/ha. Bei diesen Aufwandmengen weisen die Verbindungen eine gute bis sehr gute herbizide Wirksamkeit (75 bis 100%) gegen Schadpflanzen wie Brachiaria platyphylla, Echinochloa crus-galli, Eleusine indica, Setaria faberi, Setaria viridis und Sorghum halepense im Vorauflaufverfahren auf. Die Verbindungen eignen sich daher zur Bekämpfung von unerwünschtem Pflanzenwuchs in Maiskulturen im Vorauflauf.

Beispielsweise zeigen die Verbindungen Nr. 1, 2, 3, 5, 7, 11, 12, 13 und 20 aus Tabelle 1 eine Selektivität auch in Soja (0-20% Schaden) im Vorauflaufverfahren bei 50-62 g/ha. Bei diesen Aufwandmengen weisen die Verbindungen eine gute bis sehr gute herbizide Wirksamkeit (75 bis 100%) gegen Schadpflanzen wie Brachiaria platyphylla, Echinochloa crus-galli, Eleusine indica, Setaria faberi, Setearia viridis und Sorghum halepense im Vorauflaufverfahren auf. Die Verbindungen eignen sich daher zur Bekämpfung von unerwünschtem Pflanzenwuchs in Soja im Vorauflauf.

### Vergleichsversuch (Vorauflaufverfahren):

Die Verbindung von Beispiel Nr. 1 aus Tabelle 1 (ein Threo-2-Enantiomer) weist im im Vorauflaufverfahren gemäß dem ersten Absatz von Abschnitt 3.2 bei einer Aufwandmenge von 80 g/ha eine Wirkung von 80 bis 100% bei Schadpflanzen wie Alopecurus myosuroides, Avena fatua, Echinochloa crus-galli, Lolium multiflorum, Setaria virides, Amaranthus retroflexus, Polygonum convolvulus, Veronica persica und Viola tricolor bei zugleich sehr guter Selektivität in Weizen (0% Schaden) auf. Dagegen zeigt das der Verbindung Nr. 1 entsprechende erythro-threo-Gemisch (erythro:threo im Gewichtsverhältnis 64:36) bei gleicher Aufwandmenge von 80 g/ha kaum Herbizidwirkung bei den genannten Schadpflanzen auf, d. h. 30 bis 50% Wirkung bei Schadpflanzen wie Echinochloa crus-galli, Setaria virides und Viola tricolor sowie 0% Wirkung bei den übrigen genannten Schadpflanzen.

Das Testbeispiel zeigt, dass das erfindungsgemäße Threo-2-Isomer nicht nur stärker wirksam ist als das entsprechende Diastereomerengemisch, sondern entgegen Hinweisen aus EP-0005341 überraschenderweise auch zur selektiven Unkrautbekämpfung eingesetzt werden kann.
3.3 In weiteren Versuchen im Gewächshaus werden Samen von Kulturpflanzen und Samen von Schadpflanzen in Töpfen in sandigem Lehmboden ausgelegt, mit Erde abgedeckt und unter guten Wachstumsbedingungen angezogen. Danach werden die Versuchspflanzen im 2-3-Blattstadium behandelt, wobei die in Form von benetzbaren Pulvern (WP) formulierten erfindungsgemäßen Verbindungen (I) als wäßrige Suspension bzw. Emulsion mit einer Wasseraufwandmenge von umgerechnet 300 l/ha unter Zusatz von 0,2% Netzmittel auf die grünen Pflanzenteile gesprüht werden. 4 Wochen nach der Behandlung und Standzeit der Versuchspflanzen im Gewächshaus unter optimalen Wachstumsbedingungen wird die Wirkung der Präparate visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen.
   Die Ergebnisse zeigen, dass die Verbindungen (I) effektiv zur Kontrolle von Schadpflanzen in zweikeimblättrige Kulturen wie z.B. Soja, Baumwolle, Raps, Zuckerrüben und Kartoffeln und auch in Gramineen-Kulturen wie z.B. Gerste, Weizen, Roggen, Sorghum-Hirsen, Mais oder Reis im Nachauflaufverfahren eingesetzt werden können.

Beispielsweise zeigen die Verbindungen Nr. 1, 13 und 20 aus Tabelle 1 eine Selektivität in Mais (0-30% Schaden) im Nachauflaufverfahren bei einer Aufwandmenge von 100 g/ha. Bei diesen Aufwandmengen weisen die Verbindungen eine gute bis sehr gute herbizide Wirksamkeit (70 bis 95 %) gegen Schadpflanzen wie Echinochloa crus-galli, Setaria faberi, Setaria viridis, Sorghum halepense, Euphorbia heterophylla, Solanum nigrum und Polygonum convolvulus im Nachauflaufverfahren auf. Die Verbindungen eignen sich daher zur Bekämpfung von unerwünschtem Pflanzenwuchs in Maiskulturen im Nachauflauf.

Darüberhinaus zeigen die Verbindungen Nr. 1, 2, 3, 5, 6, 8, 11, 12, 13, 18, 19, 20 und 23 aus Tabelle 1 eine Selektivität in Soja (0-30% Schaden) im Nachauflaufverfahren bei einer Aufwandmenge von 100 g/ha. Bei diesen Aufwandmengen weisen die Verbindungen eine gute bis sehr gute herbizide Wirksamkeit (70 bis 99 %) gegen Schadpflanzen wie Echinochloa crus-galli, Setaria spp., Sorghum halepense, Solanum nigrum und Polygonum convolvulus im Nachauflaufverfahren auf. Die Verbindungen eignen sich daher zur Bekämpfung von unerwünschtem Pflanzenwuchs in Sojakulturen im Nachauflauf.

Darüber hinaus zeigen die Verbindungen Nr. 1 und 20 aus Tabelle 1 eine Selektivität in Mais (kein Schaden oder weniger als 10% Schaden) im Nachauflaufverfahren bei 80 g/ha und zugleich eine gute bis sehr gute herbizide Wirksamkeit (75 bis 100%) gegen Schadpflanzen wie Avena fatua, Echinochloa crus-galli und Veronica persica.

Auch zeigen die Verbindungen Nr. 1, 4, 19, 21, 26, 27, 55, 85, 86, 87, 111, 112 und 208 aus Tabelle 1 eine Selektivität in Reis (kein Schaden oder weniger als 20% Schaden) im Nachauflaufverfahren bei 80 g/ha und zugleich eine gute bis sehr gute herbizide Wirksamkeit (75 bis 100%) gegen Schadpflanzen wie Echinochloa crus-galli.

### Vergleichsversuch (Nachauflaufverfahren):

Die Verbindung von Beispiel Nr. 86 aus Tabelle 1 (ein Threo-2-Enantiomer) weist im im Nachauflaufverfahren gemäß dem ersten Absatz von Abschnitt 3.3 bei einer Aufwandmenge von 80 g/ha eine gute herbizide Wirkung von 70 bis 90% bei Schadpflanzen wie Avena fatua (90%), Echinochloa crus-galli (80%), Setaria virides (80%), Polygonum convolvulus (80%), Veronica persica (70%) und Viola tricolor (80%) bei zugleich guter Selektivität in Weizen (bis 10% Schaden), Reis (bis 10% Schaden) und Raps (0% Schaden) auf.

Dagegen zeigt das der Verbindung Nr. 86 entsprechende erythro-threo-Gemisch (erythro:threo im Gewichtsverhältnis 58:42) bei gleicher Aufwandmenge von 80 g/ha eine insgesamt schlechtere Herbizidwirkung bei den genannten Schadpflanzen auf, d. h. Avena fatua (40%), Echinochloa crus-galli (80%), Setaria virides (80%), Polygonum convolvulus (60%), Veronica persica (0%) und Viola tricolor (10%) bei zugleich überraschend verschlechterter Selektivität in Weizen (80% Schaden) und Raps (20% Schaden) auf.

Das Testbeispiel zeigt, dass das erfindungsgemäße Threo-2-Isomer nicht nur stärker wirksam ist als das entsprechende Diastereomerengemisch, sondern entgegen Hinweisen aus EP-0005341 überraschenderweise auch besser zur selektiven Unkrautbekämpfung eingesetzt werden kann.

## Patentansprüche

1. Verbindungen der Formel (I) oder deren Salze, jeweils in der optisch aktiven (3R,4R)-threo-Form, 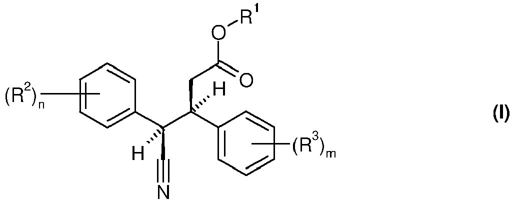 worin
R¹ Wasserstoff oder einen hydrolysierbaren Rest bedeutet,
(R²)ₙ n Substituenten R² bedeutet,
wobei R² (wenn n = 1) oder jeder der Substituenten R² (wenn n größer als 1) unabhängig voneinander Halogen, Cyano, Nitro, (C₁-C₈)Alkyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, Tri-[(C₁-C₄)alkyl]-silyl oder Tri-[(C₁-C₄)alkyl]-silyl-(C₁-C₄)alkyl bedeutet
oder wobei jeweils zwei am Ring ortho-ständige Gruppen R² gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z² zusammen mit den an die Gruppe gebundenen C-Atomen des Phenylrings einen ankondensierten 5 oder 6 Ring bilden, und
(R³)ₘ m Substituenten R³ bedeutet,
wobei R³ (wenn m = 1) oder jeder der Substituenten R³ (wenn m größer als 1) unabhängig voneinander Halogen, Cyano, Nitro, (C₁-C₈)Alkyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, NR*R**, wobei R* und R** weiter unten definiert sind, Tri-[(C₁-C₄)alkyl]-silyl oder Tri-[(C₁-C₄)alkyl]-silyl-(C₁-C₄)alkyl bedeutet
oder wobei jeweils zwei am Ring ortho-ständige Gruppen R³ gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden, und
n, m jeweils unabhängig voneinander 0, 1, 2, 3, 4 oder 5, vorzugsweise 0, 1, 2, oder 3 bedeuten, mit der Maßgabe, dass nicht beide gleichzeitig 0 bedeuten,
wobei die stereochemische Konfiguration am C-Atom in Position 3 des Buttersäurederivats eine stereochemische Reinheit von 60 bis 100 % (*R*), vorzugsweise 70 bis 100 % (*R*), mehr bevorzugt 80 bis 100 % (*R*), insbesondere 90 bis 100 % (*R*), bezogen auf das vorliegende Gemisch der threo-Enantiomeren aufweist und
die stereochemische Konfiguration am C-Atom in Position 4 des Buttersäurederivats eine stereochemische Reinheit von 60 bis 100 % (*R*), vorzugsweise 70 bis 100 % (*R*), mehr bevorzugt 80 bis 100 % (*R*), insbesondere 90 bis 100 % (*R*), bezogen auf das vorliegende Gemisch der threo-Enantiomeren aufweist.

2. Verbindungen oder deren Salze gemäß Anspruch 1, **dadurch gekennzeichnet, dass**
R¹ Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C-Atome, vorzugsweise 1 bis 24 C-Atome, insbesondere 1 bis 20 C-Atome aufweist, bedeutet oder
R¹ einen Rest der Formel SiR^{a}R^{b}R^{c}, -NR^{a}R^{b} oder -N=CR^{c}R^{d} bedeutet, wobei in den letztgenannten 3 Formeln jeder der Reste R^{a}, R^{b}, R^{c} und R^{d} unabhängig voneinander Wasserstoff oder einen gegebenenfalls substituierten Kohlenwasserstoffrest bedeutet, dabei aber SiH₃ für SiR^{a}R^{b}R^{c} ausgenommen ist, oder R^{a} und R^{b} zusammen mit dem N-Atom einen 3- bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C-Atom einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten,
wobei jeder der Reste R^{a}, R^{b}, R^{c} und R^{d} inklusive Substituenten bis 30 C-Atome, vorzugsweise bis 24 C-Atome, insbesondere bis 20 C-Atome aufweist, oder
R¹ einen Rest der Formel -C(=O)-R^{e} oder -P(=O)(R^{f})₂ bedeutet, wobei R^{e} und die R^{f} unabhängig voneinander jeweils Wasserstoff, OH, (C₁-C₈)Alkyl, (C₁-C₄)Haloalkyl, (C₂-C₈)Alkenyl, (C₂-C₈)Alkinyl, (C₁-C₆)Alkoxy, (C₁-C₆)Alkoxy-(C₁-C₈)alkyl, (C₁-C₄)Haloalkoxy, (C₁-C₄)Haloalkoxy-(C₁-C₈)alkyl, (C₃-C₈)Alkenyloxy, (C₃-C₈)Alkenyloxy-(C₁-C₈)alkyl, (C₃-C₈)Alkinyloxy, (C₃-C₈)Alkinyloxy-(C₁-C₈)alkyl, -NR*R**, wobei R* und R** weiter unten definiert sind, Tri-[(C₁-C₄)alkyl]-silyl, Tri-[(C₁-C₄)alkyl]-silyl-(C₁-C₈)alkyl, (C₃-C₆)Cycloalkyl, (C₃-C₆)Cycloalkyl-(C₁-C₈)alkyl, (C₅-C₆)Cycloalkenyl, (C₅-C₆)Cycloalkenyl-(C₁-C₈)alkyl, (C₅-C₆)Cycloalkinyl, (C₅-C₆)Cycloalkinyl-(C₁-C₈)alkyl, Phenyl, Phenyl-(C₁-C₈)alkyl, Phenoxy, Phenoxy-(C₁-C₈)alkyl, Phenylamino, Phenylamino-(C₁-C₈)alkyl, einen Rest Het¹, Het¹-(C₁-C₆)alkyl oder Het¹-O-(C₁-C₆)alkyl, wobei der heterocyclische Rest Het¹ weiter unten definiert ist, bedeuten,
wobei jeder der letztgenannten 15 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist,
wobei in den obengenannten und den nachfolgenden Resten
Het¹ jeweils unabhängig voneinander einen gesättigten, teilweise ungesättigten oder heteroaromatischen monocyclischen Heterocyclylrest mit 3 bis 9 Ringatomen oder einen 9- oder 10-gliedrigen bicyclischen Heterocyclus, jeweils enthaltend 1, 2, 3 oder 4 Heteroatome, ausgewählt aus der Gruppe bestehend aus O, N und S, bedeutet,
R*, R** jeweils unabhängig voneinander (unabhängig auch von anderen Resten NR*R**) H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste R^{bb} substituiert ist, bedeuten oder
R* und R** zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
R^{A} Halogen, Cyano, Hydroxy oder (C₁-C₆)Alkoxy bedeutet,
R^{B} Halogen, Cyano, Hydroxy, Oxo, Nitro, (C₁-C₈)Alkyl, (C₁-C₆)Haloalkyl, Cyano-(C₁-C₆)alkyl, Hydroxy-(C₁-C₆)alkyl, Nitro-(C₁-C₆)alkyl, (C₂-C₈)Alkenyl, (C₂-C₈)Haloalkenyl, (C₂-C₈)Alkinyl, (C₂-C₈)Haloalkinyl, (C₁-C₈)Alkoxy, (C₂-C₈)Alkenyloxy, (C₂-C₈)Alkinyloxy, (C₁-C₈)Haloalkoxy, (C₁-C₆)Alkoxy-(C₁-C₆)alkyl, (C₁-C₆)Alkoxy-(C₁-C₄)alkoxy, (C₁-C₆)Haloalkoxy-(C₁-C₄)alkyl, (C₁-C₆)Haloalkoxy-(C₁-C₄)alkoxy, (C₁-C₈)Alkylthio, (C₂-C₆)Alkenylthio, (C₂-C₆)Alkinylthio, (C₁-C₈)Alkylsulfinyl, (C₁-C₆)Haloalkylsulfinyl, (C₁-C₈)Alkylsulfonyl, (C₁-C₆)Haloalkylsulfonyl, einen Rest der Formel R^{aa}-C(=O)-, R^{aa}-C(=O-(C₁-C₆)alkyl, wobei die R^{aa} weiter unten definiert sind, -NR*R**, wobei R* und R** weiter unten definiert sind, Tri-[(C₁-C₄)alkyl]-silyl, Tri-[(C₁-C₄)alkyl]-silyl-(C₁-C₆)alkyl, (C₃-C₈)Cycloalkyl, (C₃-C₈)Cycloalkoxy, (C₃-C₈)Cycloalkyl-(C₁-C₈)alkyl, (C₃-C₈)Cycloalkyl-(C₁-C₈)alkoxy, Phenyl, Phenyl-(C₁-C₈)alkyl, Phenoxy, Phenoxy-(C₁-C₈)alkyl, Phenylamino, Phenylamino-(C₁-C₈)alkyl oder einen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, wobei jeder der letztgenannten 11 Reste im cyclischen Teil gegebenenfalls durch einen oder mehrere gleiche oder verschiedene Reste R^{bb} substituiert ist, bedeutet,
R^{aa} jeweils unabhängig voneinander Wasserstoff, OH, (C₁-C₈)Alkyl, (C₁-C₆)Haloalkyl, (C₂-C₈)Alkenyl, (C₂-C₈)Alkinyl, (C₁-C₈)Alkoxy, (C₁-C₆)Alkoxy-(C₁-C₆)alkyl, (C₁-C₆)Alkoxy-(C₁-C₆)alkyloxy, (C₁-C₆)Haloalkoxy, (C₁-C₆)Haloalkoxy-(C₁-C₆)alkyl, (C₁-C₆)Haloalkoxy-(C₁-C₆)alkoxy, (C₃-C₈)Alkenyloxy, (C₃-C₈)Alkenyloxy-(C₁-C₆)alkyl, (C₃-C₈)Alkenyloxy-(C₁-C₆)alkoxy, (C₃-C₈)Alkinyloxy, (C₃-C₈)Alkinyloxy-(C₁-C₆)alkyl, (C₃-C₈)Alkinyloxy-(C₁-C₆)alkoxy, -NR*R*, wobei R* und R** wie oben definiert sind, Tri-[(C₁-C₄)alkyl]-silyl, Tri-[(C₁-C₄)alkyl]-silyl-(C₁-C₆)alkyl, Tri-[(C₁-C₄)alkyl]-silyl-(C₁-C₆)alkoxy, (C₃-C₆)Cycloalkyl, (C₃-C₈)Cycloalkoxy, (C₃-C₈)Cycloalkyl-(C₁-C₈)alkyl, (C₃-C₈)Cycloalkyl-(C₁-C₈)alkoxy, (C₅-C₈)Cycloalkenyl, (C₅-C₈)Cycloalkenyl-(C₁-C₆)alkyl, (C₅-C₈)Cycloalkenyloxy, (C₅-C₈)Cycloalkinyl, (C₅-C₈)Cycloalkinyl-(C₁-C₆)alkyl, (C₅-C₈)Cycloalkinyl-(C₁-C₆)alkoxy, Phenyl, Phenyl-(C₁-C₈)alkyl, Phenyl-(C₁-C₈)alkoxy, Phenoxy, Phenoxy-(C₁-C₈)alkyl, Phenoxy-(C₁-C₈)alkoxy, Phenylamino, Phenylamino-(C₁-C₈)alkyl, Phenylamino-(C₁-C₈)alkoxy oder einen gegebenenfalls über eine Alkylengruppe oder eine Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 20 Reste im cyclischen Teil gegebenenfalls durch einen oder mehrere gleiche oder verschiedene Reste R^{bb} substituiert ist, bedeutet und
R^{bb} jeweils unabhängig voneinander Halogen, (C₁-C₄)Alkyl, (C₁-C₄)Haloalkyl, (C₁-C₄)Alkoxy oder (C₁-C₄)Haloalkoxy oder im Fall gesättigter oder teilungesättigter cyclischer Basisgruppen auch Oxo bedeutet.

3. Verbindungen oder deren Salze gemäß Anspruch 1 oder 2, **dadurch gekennzeichnet, dass**
R¹ Wasserstoff, (C₁-C₁₈)Alkyl, (C₂-C₁₈)Alkenyl oder (C₂-C₁₈)Alkinyl, wobei jeder der letztgenannten 3 Reste unsubstituiert oder durch einen oder mehrere Reste aus der Gruppe, die aus den Resten [Untergruppen (a)-(d)]
(a) Halogen, Cyano, Thio, Nitro, Hydroxy, Carboxy, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, (C₁-C₈)Haloalkylthio, (C₂-C₈)Haloalkenylthio, (C₂-C₈)Haloalkinylthio, (C₁-C₈)Alkylsulfinyl, (C₂-C₈)Alkenylsulfinyl, (C₂-C₈)Alkinylsulfinyl, (C₁-C₈)Haloalkylsulfinyl, (C₂-C₈)Haloalkenylsulfinyl, (C₂-C₈)Haloalkinylsulfinyl, (C₁-C₈)Alkylsulfonyl, (C₂-C₈)Alkenylsulfonyl, (C₂-C₈)Alkinylsulfonyl, (C₁-C₈)Haloalkylsulfonyl, (C₂-C₈)Haloalkenylsulfonyl, (C₂-C₈)Haloalkinylsulfonyl, Reste der Formel -NR*R**, wobei R* und R** weiter unten definiert sind, und
(C₃-C₈)Cycloalkyl, (C₅-C₈)Cycloalkenyl, (C₅-C₈)Cycloalkinyl, (C₃-C₈)Cycloalkyl-(C₁-C₆)alkoxy, (C₃-C₈)Cycloalkyl-(C₁-C₆)alkyl-S(O)ₚ-, (C₅-C₈)Cycloalkenyl-(C₁-C₆)alkoxy, (C₅-C₈)Cycloalkenyl-(C₁-C₆)alkyl-S(O)ₚ-, (C₅-C₈)Cycloalkinyl-(C₁-C₆)alkoxy, (C₅-C₈)Cycloalkinyl-(C₁-C₆)alkyl-S(O)ₚ-, (C₃-C₈)Cycloalkoxy, (C₃-C₆)Cycloalkyl-S(O)ₚ-, (C₅-C₈)Cycloalkenyloxy, (C₆-C₆)Cycloalkenyl-S(O)ₚ-, (C₅-C₈)Cycloalkinyloxy, (C₆-C₆)Cycloalkinyl-S(O)ₚ-, (C₃-C₈)Cycloalkoxy-(C₁-C₆)alkoxy, (C₃-C₈)Cycloalkoxy-(C₁-C₆)alkyl-S(O)ₚ-, Phenyl, Phenyl-(C₁-C₆)alkoxy, Phenoxy, Phenyl-S(O)ₚ-, Phenyl-(C₁-C₆)alkyl-S(O)ₚ-, Phenoxy-(C₁-C₆)alkoxy, Phenoxy-(C₁-C₆)alkyl-S(O)ₚ-, einen Rest Het¹, Het¹-S(O)ₚ-, Het¹-(C₁-C₆)alkoxy, Het¹-O-, Het¹-O-(C₁-C₆)alkoxy, wobei der heterocyclische Rest Het¹ weiter unten definiert ist,
wobei und jeder der letztgenannten 29 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist und p jeweils unabhängig voneinander 0, 1 oder 2 bedeutet,
(b) Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-NR*R**, -C(=O)-O-NR*R**, -O-C(=O)-NR*R**, -N(R*)-C(=O)-R^{C}, -N(R*)-C(=O)-NR*R**, -N(R*)-C(=O)-O-R^{C}, -P(=O)(R^{C})(R^{D}), -P(=O)(OR^{C})(R^{D}), -P(=O)(OR^{C})(OR^{D}) oder -O-P(=O)(OR^{C})(OR^{D}), wobei R*, R**, R^{C} und R^{D} wie weiter unten definiert sind,
(c) Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)_{q}-CH(OR')₂, in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und q eine ganze Zahl von 0 bis 6 bedeuten, und
(d) Reste der Formel R"O-CHR'"CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten,
besteht, substituiert ist,
oder
R¹ (C₃-C₉)Cycloalkyl, (C₅-C₉)Cycloalkenyl, (C₅-C₉)Cycloalkinyl oder Phenyl, wobei jeder der letztgenannten 4 Reste unsubstituiert oder durch einen oder mehrere Reste aus der Gruppe, die aus den Resten [Untergruppen (a')-(e')]
(a') Halogen, Cyano, Thio, Nitro, Hydroxy, Carboxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio und Reste der Formeln -NR*R**, wobei die Reste R* und R** weiter unten definiert sind,
(b') Reste der Formeln -C(=O)-R^{C}, -C(=O)-O-R^{C}, -O-C(=O)-R^{C}, -O-C(=O)-O-R^{C}, -C(=O)-S-R^{C}, -C(=S)-S-R^{C}, -C(=S)-S-R^{C}, -C(=O)-NR*R**, -C(=O)-O-NR*R**, -O-C(=O)-NR*R**, -N(R*)-C(=O)-R^{C}, -N(R*)-C(=O)-NR*R**, -N(R*)-C(=O)-O-R^{C}, -P(=O)(R^{C})(R^{D}), -P(=O)(OR^{C})(R^{D}), -P(=O)(OR^{C})(OR^{D}) oder -O-P(=O)(OR^{C})(OR^{D}), wobei R*, R**, R^{C} und R^{D} wie weiter unten definiert sind,
(c') Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)_{q}-CH(OR')₂, in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und q eine ganze Zahl von 0 bis 6 bedeuten, und
(d') Reste der Formel R"O-CHR'''CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten, und
(e') ein Rest der Formel Het¹, der unsubstituiert oder durch einen oder mehrere gleiche oder verschiedene Reste R^{B} substituiert ist,
besteht, substituiert ist,
oder
R¹ einen mehrcyclischen Rest auf Basis von (C₃-C₉)Cycloalkyl, (C₅-C₉)Cycloalkenyl, (C₅-C₉)Cycloalkinyl oder Phenyl, wobei der Basisring mit einem carbocyclischen oder heterocyclischen Ring, vorzugsweise einem 5- oder 6-gliedrigen Ring mit 0 oder 1 bis 3 Heteroringatomen aus der Gruppe N, O und S kondensiert ist, vorzugsweise benzokondensiert ist, und wobei der Basisring oder das mehrcyclische System unsubstituiert oder durch einen oder mehrere gleiche oder verschiedene Reste R^{B} substituiert ist, vorzugsweise unsubstitutiert oder durch einen oder mehrere Reste aus der Gruppe Halogen, Cyano, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
oder
R¹ einen heterocyclischen Rest Het¹, der im Ring oder im mehrcyclischen System unsubstituiert oder durch einen oder mehrere gleiche oder verschiedene Reste R^{B} substituiert ist, vorzugsweise unsubstituiert oder durch einen oder mehrere Reste aus der Gruppe Halogen, Cyano, Thio, Nitro, Hydroxy, Carboxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy,(C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
bedeutet,
wobei in den obengenannten und den nachfolgenden Resten
Het¹ jeweils unabhängig voneinander einen gesättigten, teilweise ungesättigten oder heteroaromatischen monocyclischen Heterocyclylrest mit 3 bis 9 Ringatomen, vorzugsweise mit 5 oder 6 Ringatomen, oder 9- oder 10-gliedrigen bicyclischen Heterocyclus, enthaltend 1, 2, 3 oder 4 Heteroatome ausgewählt aus der Gruppe bestehend aus O, N und S, vorzugsweise einen 5- oder 6-gliedrigen Heterocyclus mit 1 bis 3 Heteroringatomen aus der Gruppe N, O und S, der gegebenenfalls auch mit einem carbocyclischen oder heterocyclischen Ring, vorzugsweise einem carbocyclischen Ring mit 3 bis 6 C-Atomen oder einem heterocyclischen Ring mit 5 oder 6 Ringatomen und 1 bis 3 Heteroringatomen aus der Gruppe N, O und S kondensiert ist, vorzugsweise gegebenenfalls benzokondensiert ist, bedeutet,
R*, R** jeweils unabhängig voneinander (d. h. auch von anderen Gruppen NR*R**) H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus gegebenenfalls durch einen oder mehrere gleiche oder verschiedene Reste R^{bb} substituiert ist, bedeuten oder
R* und R** zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
R^{A} Halogen, Cyano, Hydroxy oder (C₁-C₆)Alkoxy bedeutet,
R^{B} Halogen, Cyano, Hydroxy, Oxo, Nitro, (C₁-C₆)Alkyl, (C₁-C₄)Haloalkyl, Cyano-(C₁-C₄)alkyl, Hydroxy-(C₁-C₄)alkyl, Nitro-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkyl, (C₁-C₄)Alkoxy-(C₁-C₄)alkoxy, (C₁-C₄)Haloalkoxy-(C₁-C₄)alkyl, (C₁-C₄)Haloalkoxy-(C₁-C₄)alkoxy, (C₁-C₆)Alkylthio, (C₂-C₆)Alkenylthio, (C₂-C₆)Alkinylthio, (C₁-C₆)Alkylsulfinyl , (C₁-C₆)Haloalkylsulfinyl, (C₁-C₆)Alkylsulfonyl, (C₁-C₆)Haloalkylsulfonyl, ein Rest der Formel R^{aa}-C(=O)- oder R^{aa}-C(=O)-(C₁-C₆)alkyl, wobei die R^{aa} weiter unten definiert sind, -NR*R**, wobei R* und R** weiter unten definiert sind, Tri-[(C₁-C₄)alkyl]-silyl, Tri-[(C₁-C₄)alkyl]-silyl-(C₁-C₆)alkyl, (C₃-C₆)Cycloalkyl, (C₃-C₆)Cycloalkoxy, (C₃-C₆)Cycloalkyl-(C₁-C₄)alkyl, (C₃-C₆)Cycloalkyl-(C₁-C₈)alkoxy, Phenyl, Phenyl-(C₁-C₆)alkyl, Phenoxy, Phenoxy-(C₁-C₆)alkyl, Phenylamino, Phenylamino-(C₁-C₆)alkyl oder einen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, wobei jeder der letztgenannten 11 Reste im cyclischen Teil gegebenenfalls durch einen oder mehrere gleiche oder verschiedene Reste R^{bb} substituiert ist, substituiert ist, bedeutet,
R^{C}, R^{D} jeweils unabhängig voneinander (auch unabhängig von Resten R^{C}, R^{D} in anderen Gruppen)
Wasserstoff, (C₁-C₈)Alkyl, (C₂-C₈)Alkenyl oder (C₂-C₈)Alkinyl bedeutet, wobei jeder der letztgenannten 3 Reste unsubstituiert oder durch einen oder mehrere Reste aus der Gruppe Halogen, Cyano, Nitro, Hydroxy, (C₁-C₆)Alkoxy,(C₂-C₆)Alkenyloxy, (C₂-C₆)Alkinyloxy, (C₁-C₈)Haloalkoxy, (C₁-C₄)Alkoxy-(C₁-C₄)alkoxy, (C₁-C₈)Alkylthio, (C₁-C₈)Haloalkylthio, (C₁-C₈)Alkylsulfinyl, (C₁-C₈)Haloalkylsulfinyl, (C₁-C₈)Alkylsulfonyl, (C₁-C₈)Haloalkylsulfonyl und Tri-[(C₁-C₄)alkyl]-silyl substituiert ist,
oder
(C₃-C₈)Cycloalkyl, (C₅-C₈)Cycloalkenyl, (C₅-C₈)Cycloalkinyl, Phenyl, (C₃-C₈)Cycloalkyl-(C₁-C₆)alkyl, (C₅-C₈)Cycloalkenyl-(C₁-C₆)alkyl, (C₅-C₈)Cycloalkinyl-(C₁-C₆)alkyl, Phenyl-(C₁-C₆)alkyl, (C₃-C₈)Cycloalkyloxy-(C₁-C₆)alkyl, (C₃-C₈)Cycloalkyl-S(O)ₚ-(C₁-C₆)alkyl, (C₅-C₈)Cycloalkenyloxy-(C₁-C₆)alkyl, (C₅-C₈)Cycloalkinyloxy-(C₁-C₆)alkyl, Phenoxy-(C₁-C₆)alkyl, Phenyl-S(O)ₚ-(C₁-C₆)alkyl, (C₃-C₈)Cycloalkylamino-(C₁-C₆)alkyl, (C₅-C₈)Cycloalkenylamino-(C₁-C₆)alkyl, (C₅-C₈)Cycloalkinylamino-(C₁-C₆)alkyl, Phenylamino-(C₁-C₆)alkyl, Het¹, Het¹-(C₁-C₆)alkyl, Het¹-O-(C₁-C₆)alkyl oder Het¹-S(O)ₚ-(C₁-C₆)alkyl, wobei Het¹ die genannte Bedeutung hat,
wobei jeder der letztgenannten 22 Reste im acyclischen Teil unsubstituiert oder durch einen oder mehrere gleiche oder verschiedene Reste R^{A} substituiert ist und im cyclischen Teil unsubstituiert oder durch einen oder mehrere gleiche oder verschiedene Reste R^{B} substituiert ist und p jeweils unabhängig voneinander 0, 1 oder 2 bedeutet,
bedeuten,
R^{aa} jeweils unabhängig voneinander Wasserstoff, OH, (C₁-C₆)Alkyl, (C₁-C₄)Haloalkyl, (C₂-C₆)Alkenyl, (C₂-C₆)Alkinyl, (C₁-C₆)Alkoxy, (C₁-C₆)Alkoxy-(C₁-C₆)alkyl, (C₁-C₆)Alkoxy-(C₁-C₆)alkyloxy, (C₁-C₄)Haloalkoxy, (C₁-C₄)Haloalkoxy-(C₁-C₆)alkyl, (C₁-C₄)Haloalkoxy-(C₁-C₆)alkoxy, (C₃-C₆)Alkenyloxy, (C₃-C₆)Alkenyloxy-(C₁-C₆)alkyl, (C₃-C₆)Alkenyloxy-(C₁-C₆)alkoxy, (C₃-C₆)Alkinyloxy, (C₃-C₆)Alkinyloxy-(C₁-C₆)alkyl, (C₃-C₆)Alkinyloxy-(C₁-C₆)alkoxy, -NR*R*, wobei R* und R** wie oben definiert sind, Tri-[(C₁-C₄)alkyl]-silyl, Tri-[(C₁-C₄)alkyl]-silyl-(C₁-C₆)alkyl, Tri-[(C₁-C₄)alkyl]-silyl-(C₁-C₆)alkoxy, (C₃-C₆)Cycloalkyl, (C₃-C₆)Cycloalkoxy, (C₃-C₆)Cydoalkyl-(C₁-C₈)alkyl, (C₃-C₆)Cycloalkyl-(C₁-C₈)alkoxy, (C₅-C₆)Cycloalkenyl, (C₅-C₆)Cycloalkenyl-(C₁-C₆)alkyl, (C₅-C₆)Cycloalkenyloxy,(C₅-C₆)Cycloalkinyl, (C₅-C₆)Cycloalkinyl-(C₁-C₆)alkyl, (C₅-C₆)Cycloalkinyl-(C₁-C₆)alkoxy, Phenyl, Phenyl-(C₁-C₆)alkyl, Phenyl-(C₁-C₆)alkoxy, Phenoxy, Phenoxy-(C₁-C₆)alkyl, Phenoxy-(C₁-C₆)alkoxy, Phenylthio, Phenyl-S(O)ₚ-(C₁-C₆)alkyl, Phenyl-S(O)ₚ-(C₁-C₆)alkoxy, wobei p jeweils unabhängig voneinander 0, 1 oder 2 bedeutet, Phenylamino, Phenylamino-(C₁-C₆)alkyl, Phenylamino-(C₁-C₆)alkoxy oder einen gegebenenfalls über eine Alkylengruppe oder eine Alkoxygruppe gebundenen 5- oder 6-gliedrigen monocyclischen oder 9- oder 10-gliedrigen bicyclischen Heterocyclus, enthaltend 1, 2, 3 oder 4 Heteroatome ausgewählt aus der Gruppe bestehend aus O, N und S, bedeutet, wobei jeder der letztgenannten 20 Reste im cyclischen Teil gegebenenfalls durch einen oder mehrere gleiche oder verschiedene Reste R^{bb} substituiert ist, bedeutet und
R^{bb} jeweils unabhängig voneinander Halogen, (C₁-C₄)Alkyl, (C₁-C₄)Haloalkyl, (C₁-C₄)Alkoxy oder (C₁-C₄)Haloalkoxy bedeutet.

4. Verbindungen oder deren Salze gemäß einem der Ansprüche 1 bis 3, **dadurch gekennzeichnet, dass**
(R²)ₙ n Substituenten R² bedeutet,
wobei R² (wenn n = 1) oder jeder derSubstituenten R² (wenn n größer als 1) unabhängig voneinander Halogen, Cyano, Nitro, (C₁-C₆)Alkyl, (C₁-C₆)Alkoxy, (C₁-C₆)Alkylthio, (C₁-C₆)Alkylsulfinyl, (C₁-C₆)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, Tri-[(C₁-C₄)alkyl]-silyl oder Tri-[(C₁-C₄)alkyl]-silyl-(C₁-C₄)alkyl bedeutet
oder wobei jeweils zwei am Ring ortho-ständige Gruppen R² gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl,(C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z² zusammen mit den an die Gruppe gebundenen C-Atomen des Phenylrings einen ankondensierten 5 oder 6 Ring bilden, und
(R³)ₘ m Substituenten R³ bedeutet,
wobei R³ (wenn m = 1) oder jeder der Substituenten R³ (wenn m größer als 1) unabhängig voneinander Halogen, Cyano, Nitro, (C₁-C₆)Alkyl, (C₁-C₆)Alkoxy, (C₁-C₆)Alkylthio, (C₁-C₆)Alkylsulfinyl, (C₁-C₆)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, NR*R**, Tri-[(C₁-C₄)alkyl]-silyl oder Tri-[(C₁-C₄)alkyl]-silyl-(C₁-C₄)alkyl bedeutet oder wobei jeweils zwei am Ring ortho-ständige Gruppen R³ gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl,(C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden,
R*, R** jeweils unabhängig voneinander oder gemeinsam mit dem N-Atom die genannte Bedeutung haben und
n, m jeweils unabhängig voneinander 0, 1, 2, 3, 4 oder 5, vorzugsweise 0, 1, 2 oder 3, insbesondere 0, 1 oder 2 bedeuten, mit der Maßgabe, dass nicht beide gleichzeitig 0 bedeuten,

5. Verbindungen oder deren Salze gemäß einem der Ansprüche 1 bis 4, **dadurch gekennzeichnet, dass**
(R²)ₙ n Substituenten R² bedeutet, wobei, im Falle dass n = 1 ist, der Substituent R² oder, im Falle dass n größer als 1 ist, jeder der Substituenten R² unabhängig voneinander Halogen, Cyano, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Alkenyl, (C₂-C₄)Alkinyl, Tri-[(C₁-C₄)Alkyl]-silyl oder Tri-[(C₁-C₄)Alkyl]-silyl -(C₁-C₄)Alkyl bedeutet und
n 0, 1, 2, 3, 4 oder 5, vorzugsweise 0, 1, 2 oder 3, insbesondere 0, 1 oder 2 bedeutet, mit der Maßgabe, dass n und m nicht beide gleichzeitig 0 bedeuten.

6. Verbindungen oder deren Salz gemäß einem der Ansprüche 1 bis 5, **dadurch gekennzeichnet, dass**
(R³)ₘ m Substituenten R³ bedeutet, wobei, im Falle dass m = 1 ist, der Substituent R³ oder, im Falle dass m größer als 1 ist, jeder der Substituenten R³ unabhängig voneinander Halogen, Cyano, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₆)Alkenyl, (C₂-C₆)Alkinyl oder Tri-[(C₁-C₄)Alkyl]silyl-Z^{b}-, wobei Z^{b} = für eine kovalente Bindung oder (C₁-C₄)Alkylen steht, bedeutet oder
jeweils zwei am Ring ortho-ständige Gruppen R³ gemeinsam eine Gruppe der Formel -Z³-A**-Z⁴ bedeuten, wobei
A** für eine Alkylengruppe steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für O oder S steht und
Z⁴ für O oder S steht, wobei die Gruppe -Z³-A**-Z⁴ zusammen mit den an die Gruppe gebundenen C-Atomen des Phenylrings einen ankondensierten 5 oder 6 Ring bilden,
m 0, 1, 2, 3, 4 oder 5, vorzugsweise 0, 1, 2, 3 oder 4, insbesondere 0, 1, 2 oder 3 bedeutet, mit der Maßgabe, dass n und m in Formel (I) nicht beide gleichzeitig 0 bedeuten.

7. Verbindungen oder deren Salze gemäß einem der Ansprüche 1 bis 6, **dadurch gekennzeichnet, dass**
(R²)ₙ n Substituenten R² bedeutet, wobei, im Falle dass n = 1 ist, der Substituent R² oder, im Falle dass n größer als 1 ist, jeder der Substituenten R² unabhängig voneinander Fluor, Chlor, Brom, Iod, Cyano, Nitro, Methyl, Ethyl, Methoxy, Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, und
(R³)ₘ m Substituenten R³ bedeutet, wobei, im Falle dass m = 1 ist, der Substituent Rest R³ oder, im Falle dass m größer als 1 ist, jeder der Substituenten R³ unabhängig voneinander Halogen, Cyano, Nitro, Methyl, Methoxy, Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, und
m 0, 1, 2, 3, 4 oder 5, vorzugsweise 0, 1, 2, 3 oder 4, insbesondere 0, 1, 2 oder 3 bedeutet und
n 0, 1, 2, 3, 4 oder 5, vorzugsweise 0, 1, 2 oder 3, insbesondere 0, 1 oder 2 bedeutet,
mit der Maßgabe, dass n und m nicht beide gleichzeitig 0 bedeuten.

8. Verbindungen oder deren Salze gemäß einem der Ansprüche 1 bis 7, **dadurch gekennzeichnet, dass**
(R²)ₙ 2-Brom, 3-Brom, 4-Brom, 2-Chlor, 3-Chlor, 4-Chlor, 2-Fluor, 3-Fluor, 4-Fluor, 2-Cyano, 3-Cyano, 4-Cyano, 2-Methyl, 3-Methyl, 4-Methyl, 2-Ethyl, 3-Ethyl, 4-Ethyl, 2-CF₃, 3-CF₃, 4-CF₃, 2-Methoxy, 3-Methoxy, 4-Methoxy, 2-Ethoxy, 3-Ethoxy, 4-Ethoxy, 2-Methylthio, 3-Methylthio, 4-Methylthio, 2-Methylsulfinyl, 3-Methylsulfinyl, 4-Methylsulfonyl, 2-Methylsulfonyl, 3-Methylsulfonyl, 4-Methylsulfonyl, 2,3-Dimethyl, 2,4-Dimethyl, 2,5-Dimethyl, 2,6-Dimethyl, 3,4-Dimethyl, 3,5-Dimethyl, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), (4-Br-3-F), (3-Br-4-F), (3-Br-5-F), (4-Br-3-Cl), (3-Br-4-Cl), (4-CN-3-F), (3-CN-4-F), (3-CN-5-F), (4-CN-3-Cl), (3-CN-4-Cl), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor oder 3,4,5-Trichlor und
(R³)ₘ 2-Brom, 3-Brom, 4-Brom, 2-Chlor, 3-Chlor, 4-Chlor, 2-Fluor, 3-Fluor, 4-Fluor, 2-Cyano, 3-Cyano, 4-Cyano, 2-Methyl, 3-Methyl, 4-Methyl, 2-Ethyl, 3-Ethyl, 4-Ethyl, 2-CF₃, 3-CF₃, 4-CF₃, 2-Methoxy, 3-Methoxy, 4-Methoxy, 2-Ethoxy, 3-Ethoxy, 4-Ethoxy, 2-Methylthio, 3-Methylthio, 4-Methylthio, 2-Methylsulfinyl, 3-Methylsulfinyl, 4-Methylsulfonyl, 2-Methylsulfonyl, 3-Methylsulfonyl, 4-Methylsulfonyl, 2-Nitro, 3-Nitro, 4-Nitro, 2,3-Dimethyl, 2,4-Dimethyl, 2,5-Dimethyl, 2,6-Dimethyl, 3,4-Dimethyl, 3,5-Dimethyl, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, 2,5-Dicyano, 2,6-Dicyano, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), (4-Br-2-F), (4-Br-3-F), (4-CN-3-F), (4-NO₂-3-F), (4-OMe-3-F), (3-CN-4-F), (3-NO₂-4-F), (3-CN-4-Cl), (3-NO₂-4-Cl), (5-CN-2-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor oder 3,4,5-Trichlor
bedeuten.

9. Verfahren zur Herstellung einer Verbindung der Formel (I), wie sie gemäß einem der Ansprüche 1 bis 8 definiert ist, oder deren Salz, **dadurch gekennzeichnet, dass** man
(a) Verbindungen der Formel (II) 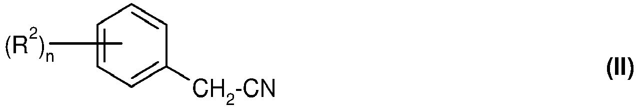 mit Verbindungen der Formel (III) oder deren Salzen, 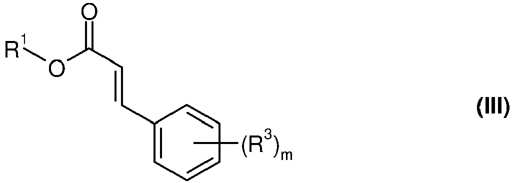 zu Verbindungen der Formel (I') 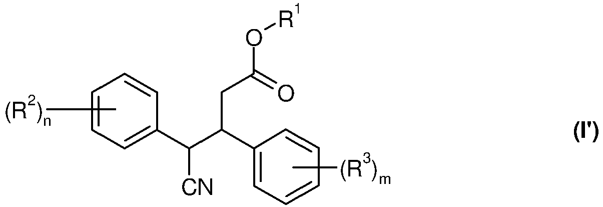 umsetzt, wobei R¹, R², R³, m und n in den Verbindungen (II), (III) und (I') wie in der jeweils herzustellenden Verbindung der Formel (I) definiert sind, und das (3R, 4R)-threo-Isomere aus dem Isomerengemisch (I') analog allgemeinen Trennverfahren isoliert
oder
(b) Verbindungen der Formel (I"), 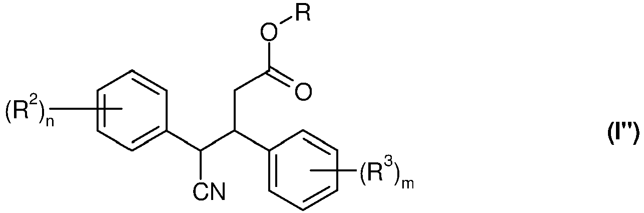 in denen R ein Rest aus der Gruppe der für R¹ möglichen Reste bedeutet aber von dem Rest R¹ in der herzustellenden Verbindung (I') verschieden ist, mit einer Verbindung der Formel R¹-OH, in der R¹ wie in der herzustellenden Verbindungen der Formel (I) definiert ist, zur Verbindung (I') umsetzt, wobei R², R³, m und n in der Verbindung (I") wie in der jeweils herzustellenden Verbindung der Formel (I) definiert sind, und das (3R, 4R)-threo-Isomere aus dem Isomerengemisch (I') analog allgemeinen Trennverfahren isoliert
oder
(c) stereochemisch angereicherte Verbindungen der (3R, 4R)-threo-Isomere der Formel (I''') 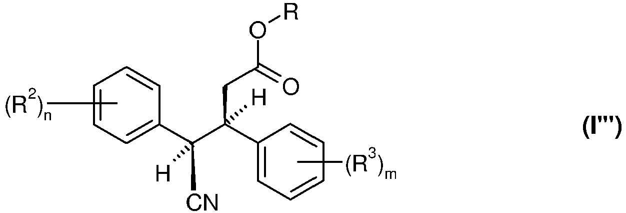 in denen R ein Rest aus der Gruppe der für R¹ möglichen Reste bedeutet aber von dem Rest R¹ in der herzustellenden Verbindung (I) verschieden ist, mit einer Verbindung der Formel R¹-OH, in der R¹ wie in der herzustellenden Verbindung der Formel (I) definiert ist, zur Verbindung (I) umsetzt, wobei R², R³, m und n in der Verbindung (I''') wie in der herzustellenden Verbindung der Formel (I) definiert sind.

10. Herbizides oder pflanzenwachstumsregulierendes Mittel, **dadurch gekennzeichnet, dass** es eine oder mehrere Verbindungen der Formel (I) oder deren Salze, wie sie nach einem der Ansprüche 1 bis 8 definiert sind, und im Pflanzenschutz übliche Formulierungshilfsmittel enthält.

11. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salzen, wie sie nach einem der Ansprüche 1 bis 7 definiert sind, auf die Pflanzen, Pflanzensamen, den Boden, in dem oder auf dem die Pflanzen wachsen, oder die Anbaufläche appliziert.

12. Verfahren nach Anspruch 11, **dadurch gekennzeichnet, dass** die Verbindungen der Formel (I) oder deren Salze zur Bekämpfung von Schadpflanzen oder zur Wachstumsregulierung in Kulturen von Nutz- oder Zierpflanzen eingesetzt werden.

13. Verfahren nach Anspruch 12, **dadurch gekennzeichnet, dass** die Kulturpflanzen transgene Kulturpflanzen sind.

14. Verwendung der Verbindungen der Formel (I) oder deren Salze gemäß einem Ansprüche 1 bis 8 als Herbizide oder Pflanzenwachstumsregulatoren.

15. Verwendung gemäß Anspruch 14, **dadurch gekennzeichnet, dass** die Verbindungen in Kulturen von Nutz oder Zierpflanzen eingesetzt werden.

## Claims

1. Compounds of the formula (I) or salts thereof, in each case in the optically active (3R,4R)-threo form, 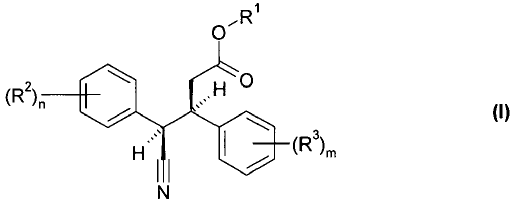 in which
R¹ is hydrogen or a hydrolyzable radical,
(R²)ₙ is n substituents R²,
where R² (if n = 1) or each of the substituents R² (if n is greater than 1) independently of the others is halogen, cyano, nitro, (C₁-C₈)-alkyl, (C₁-C₈)-alkoxy, (C₁-C₈)-alkylthio, (C₁-C₈)-alkylsulfinyl, (C₁-C₈)-alkylsulfonyl, (C₁-C₆)-haloalkyl, (C₁-C₆)-haloalkoxy, (C₁-C₆)-haloalkylthio, (C₁-C₆)-haloalkylsulfinyl, (C₁-C₆)-haloalkylsulfonyl, (C₂-C₆)-alkenyl, (C₂-C₆)-alkynyl, tri-[(C₁-C₄)-alkyl]-silyl or tri-[(C₁-C₄)-alkyl]-silyl- (C₁-C₄)-alkyl
or where in each case two groups R² located ortho at the ring together are a group of the formula-Z¹-A*-Z² in which
A* is an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z¹ is a direct bond, O or S and
Z² is a direct bond, O or S,
where the group -Z¹-A*-Z² together with the carbon atoms, attached to the group, of the phenyl ring form a fused-on 5- or 6-membered ring, and
(R³)ₘ is m substituents R³,
where R³ (if m = 1) or each of the substituents R³ (if m is greater than 1) independently of the others is halogen, cyano, nitro, (C₁-C₈)-alkyl, (C₁-C₈)-alkoxy, (C₁-C₈)-alkylthio, (C₁-C₈)-alkylsulfinyl, (C₁-C₈)-alkylsulfonyl, (C₁-C₆)-haloalkyl, (C₁-C₆)-haloalkoxy, (C₁-C₆)-haloalkylthio, (C₁-C₆)-haloalkylsulfinyl, (C₁-C₆)-haloalkylsulfonyl, (C₂-C₆) -alkenyl, (C₂-C₆)-alkynyl, NR*R**, where R* and R** are defined below, tri-[(C₁-C₄)-alkyl]-silyl or tri-[(C₁-C₄)-alkyl]-silyl-(C₁-C₄)-alkyl
or where in each case two groups R³ located ortho at the ring together are a group of the formula-Z³-A**-Z⁴ in which
A** is an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z³ is a direct bond, O or S and
Z⁴ is a direct bond, O or S,
where the group -Z³-A**-Z⁴ together with the carbon atoms, attached to the group, of the phenyl ring form a fused-on 5- or 6-membered ring, and
n, m are each independently of one another 0, 1, 2, 3, 4 or 5, preferably 0, 1, 2 or 3, with the proviso that the two are not simultaneously 0,
where the stereochemical configuration at the carbon atom in position 3 of the butyric acid derivative has a stereochemical purity of 60 to 100% (*R*)*,* preferably 70 to 100% (R), more preferably 80 to 100% (R), in particular 90 to 100% (*R*), based on the mixture of threo enantiomers present, and
the stereochemical configuration at the carbon atom in position 4 of the butyric acid derivative has a stereochemical purity of 60 to 100% (*R*)*,* preferably 70 to 100% (*R*), more preferably 80 to 100% (*R*), in particular 90 to 100% (*R*), based on the mixture of threo enantiomers present.

2. Compounds or salts thereof according to Claim 1, **characterized in that**
R¹ is hydrogen or an optionally substituted hydrocarbon radical or an optionally substituted heterocyclyl radical, where each of the two lastmentioned carbon-containing radicals including substituents has 1 to 30 carbon atoms, preferably 1 to 24 carbon atoms, in particular 1 to 20 carbon atoms, or
R¹ is a radical of the formula SiR^{a}R^{b}R^{c}, -NR^{a}R^{b} or-N=CR^{c}R^{d}, where in the 3 lastmentioned formulae each of the radicals R^{a}, R^{b}, R^{c} and R^{d} independently of the others is hydrogen or an optionally substituted hydrocarbon radical, where, however, SiH₃ for SiR^{a}R^{b}R^{c} is excluded, or R^{a} and R^{b} together with the nitrogen atom are a 3- to 9-membered heterocycle which, in addition to the nitrogen atom, may contain one or two further ring heteroatoms from the group consisting of N, O and S and which is unsubstituted or substituted, or R^{c} and R^{d} together with the carbon atom are a 3- to 9-membered carbocyclic radical or a heterocyclic radical which may contain 1 to 3 ring heteroatoms from the group consisting of N, O and S, where the carbocyclic or heterocyclic radical is unsubstituted or substituted,
where each of the radicals R^{a}, R^{b}, R^{c} and R^{d} including substituents has up to 30 carbon atoms, preferably up to 24 carbon atoms, in particular up to 20 carbon atoms, or
R¹ is a radical of the formula -C(=O)-R^{e} or-P(=O)(R^{f})₂, where R^{e} and the R^{f} independently of one another are each hydrogen, OH, (C₁-C₈)-alkyl, (C₁-C₄)-haloalkyl, (C₂-C₈)-alkenyl, (C₂-C₈)-alkynyl, (C₁-C₆) -alkoxy, (C₁-C₆)-alkoxy-(C₁-C₈)-alkyl, (C₁-C₄)-haloalkoxy, (C₁-C₄)-haloalkoxy- (C₁-C₈)-alkyl, (C₃-C₈)-alkenyloxy, (C₃-C₈)-alkenyloxy-(C₁-C₈)-alkyl, (C₃-C₈)-alkynyloxy, (C₃-C₈)-alkynyloxy- (C₁-C₈)-alkyl, -NR*R**, where R* and R** are defined below, tri-[(C₁-C₄)-alkyl]-silyl, tri-[(C₁-C₄)-alkyl]-silyl-(C₁-C₈)-alkyl, (C₃-C₆)-cycloalkyl, (C₃-C₆)-cycloalkyl- (C₁-C₈)-alkyl, (C₅-C₆)-cycloalkenyl, (C₅-C₆)-cycloalkenyl-(C₁-C₈)-alkyl, (C₅-C₆)-cycloalkynyl, (C₅-C₆)-cycloalkynyl-(C₁-C₈)-alkyl, phenyl, phenyl-(C₁-C₈)-alkyl, phenoxy, phenoxy-(C₁-C₈)-alkyl, phenylamino, phenylamino-(C₁-C₈)-alkyl, a radical Het¹, Het¹-(C₁-C₆)-alkyl or Het¹-O-(C₁-C₆)-alkyl, where the heterocyclic radical Het¹ is defined below,
where each of the 15 lastmentioned radicals is unsubstituted in the acyclic moiety or substituted by one or more identical or different radicals R^{A} and is unsubstituted in the cyclic moiety or substituted by one or more identical or different radicals R^{B},
where in the radicals mentioned above and in the radicals below
Het¹ in each case independently of the others is a saturated, partially unsaturated or heteroaromatic monocyclic heterocyclyl radical having 3 to 9 ring atoms or a 9- or 10-membered bicyclic heterocycle, each containing 1, 2, 3 or 4 heteroatoms selected from the group consisting of O, N and S,
R*, R** are each independently of one another (and also independently of other radicals NR*R**) H, (C₁-C₈)-alkyl, (C₂-C₈)-alkenyl, (C₂-C₈)-alkynyl, (C₁-C₄)-alkoxy-(C₁-C₄)-alkyl, (C₁-C₆)-alkanoyl, [(C₁-C₄)-haloalkyl]-carbonyl, [(C₁-C₄)-alkoxy]-carbonyl, (C₁-C₄)-haloalkoxy]-carbonyl, (C₃-C₆)-cycloalkyl, (C₃-C₆)-cycloalkyl-(C₁-C₄)-alkyl, phenyl, phenyl-(C₁-C₄)-alkyl, where each of the 4 lastmentioned radicals is optionally substituted in the cycle by one or more identical or different radicals R^{bb} or
R* and R** together with the nitrogen atom are a 3- to 8-membered heterocycle which, in addition to the nitrogen atom, may contain one or two further ring heteroatoms from the group consisting of N, O and S and which may be unsubstituted or substituted by one or more radicals from the group consisting of (C₁-C₄)-alkyl, (C₁-C₄)-haloalkyl and oxo,
R^{A} is halogen, cyano, hydroxyl or (C₁-C₆)-alkoxy,
R^{B} is halogen, cyano, hydroxyl, oxo, nitro, (C₁-C₈)-alkyl, (C₁-C₆)-haloalkyl, cyano- (C₁-C₆)-alkyl, hydroxy- (C₁-C₆)-alkyl, nitro-(C₁-C₆)-alkyl, (C₂-C₈)-alkenyl, (C₂-C₈)-haloalkenyl, (C₂-C₈)-alkynyl, (C₂-C₈) -haloalkynyl, (C₁-C₈)-alkoxy, (C₂-C₈)-alkenyloxy, (C₂-C₈)-alkynyloxy, (C₁-C₈)-haloalkoxy, (C₁-C₆)-alkoxy-(C₁-C₆)-alkyl, (C₁-C₆)-alkoxy-(C₁-C₄)-alkoxy, (C₁-C₆)-haloalkoxy-(C₁-C₄)-alkyl, (C₁-C₆)-haloalkoxy- (C₁-C₄)-alkoxy, (C₁-C₈)-alkylthio, (C₂-C₆)-alkenylthio, (C₂-C₆)-alkynylthio, (C₁-C₈)-alkylsulfinyl , (C₁-C₆)-haloalkylsulfinyl, (C₁-C₈)-alkylsulfonyl, (C₁-C₆)-haloalkylsulfonyl, a radical of the formula R^{aa}-C(=O)-, R^{aa}-C(=O)-(C₁-C₆)-alkyl, where the R^{aa} are defined below, -NR*R**, where R* and R** are defined below, tri-(C₁-C₄)-alkyl]-silyl, tri-[(C₁-C₄)-alkyl]-silyl-(C₁-C₆)-alkyl, (C₃-C₈)-cycloalkyl, (C₃-C₈)-cycloalkoxy, (C₃-C₈)-cycloalkyl- (C₁-C₈)-alkyl, (C₃-C₈)-cycloalkyl- (C₁-C₈)-alkoxy, phenyl, phenyl-(C₁-C₈)-alkyl, phenoxy, phenoxy-(C₁-C₈)-alkyl, phenylamino, phenylamino-(C₁-C₈)-alkyl or a 5- or 6-membered monocyclic or 9- or 10-membered bicyclic heterocycle which contains 1, 2, 3 or 4 heteroatoms selected from the group consisting of O, N and S, where each of the 11 lastmentioned radicals is optionally substituted in the cyclic moiety by one or more identical or different radicals R^{bb},
R^{aa} in each case independently of the others is hydrogen, OH, (C₁-C₈)-alkyl, (C₁-C₆)-haloalkyl, (C₂-C₈)-alkenyl, (C₂-C₈)-alkynyl, (C₁-C₈)-alkoxy, (C₁-C₆)-alkoxy-(C₁-C₆)-alkyl, (C₁-C₆)-alkoxy-(C₁-C₆)-alkyloxy, (C₁-C₆)-haloalkoxy, (C₁-C₆)-haloalkoxy-(C₁-C₆)-alkyl, (C₁-C₆)-haloalkoxy- (C₁-C₆)-alkoxy, (C₃-C₈)-alkenyloxy, (C₃-C₈)-alkenyloxy-(C₁-C₆)-alkyl, (C₃-C₈)-alkenyloxy-(C₁-C₆)-alkoxy, (C₃-C₈)-alkynyloxy, (C₃-C₈)-alkynyloxy- (C₁-C₆) -alkyl, (C₃-C₈)-alkynyloxy-(C₁-C₆)-alkoxy, -NR*R*, where R* and R** are as defined above, tri-[(C₁-C₄)-alkyl]-silyl, tri-[(C₁-C₄)-alkyl]-silyl-(C₁-C₆)-alkyl, tri-(C₁-C₄)-alkyl]-silyl-(C₁-C₆)-alkoxy, (C₃-C₈)-cycloalkyl, (C₃-C₈)-cycloalkoxy, (C₃-C₈)-cycloalkyl-(C₁-C₈)-alkyl, (C₃-C₈)-cycloalkyl-(C₁-C₈)-alkoxy, (C₅-C₈)-cycloalkenyl, (C₅-C₈)-cycloalkenyl- (C₁-C₆)-alkyl, (C₅-C₈)-cycloalkenyloxy, (C₅-C₈)-cycloalkynyl, (C₅-C₈)-cycloalkynyl-(C₁-C₆)-alkyl, (C₅-C₈)-cycloalkynyl-(C₁-C₆)-alkoxy, phenyl, phenyl-(C₁-C₈)-alkyl, phenyl-(C₁-C₈)-alkoxy, phenoxy, phenoxy-(C₁-C₈)-alkyl, phenoxy-(C₁-C₈)-alkoxy, phenylamino, phenylamino-(C₁-C₈)-alkyl, phenylamino-(C₁-C₈)-alkoxy or a 5- or 6-membered monocyclic or 9- or 10-membered bicyclic heterocycle which is optionally attached via an alkylene group or alkoxy group and contains 1, 2, 3 or 4 heteroatoms selected from the group consisting of O, N and S, where each of the 20 lastmentioned radicals is optionally substituted in the cyclic moiety by one or more identical or different radicals R^{bb}, and
R^{bb} in each case independently of the others is halogen, (C₁-C₄)-alkyl, (C₁-C₄)-haloalkyl, (C₁-C₄)-alkoxy or (C₁-C₄)-haloalkoxy or in the case of saturated or partially unsaturated cyclic base groups is also oxo.

3. Compounds or salts thereof according to Claim 1 or 2, **characterized in that**
R¹ is hydrogen, (C₁-C₁₈)-alkyl, (C₂-C₁₈)-alkenyl or (C₂-C₁₈)-alkynyl, where each of the 3 lastmentioned radicals is unsubstituted or substituted by one or more radicals from the group consisting of the radicals [subgroups (a)-(d)]
(a) halogen, cyano, thio, nitro, hydroxyl, carboxyl, (C₁-C₈)-alkoxy, (C₂-C₈)-alkenyloxy, (C₂-C₈)-alkynyloxy, (C₁-C₈)-haloalkoxy, (C₁-C₄)-alkoxy-(C₁-C₄)-alkoxy, (C₁-C₈)-alkylthio, (C₂-C₈)-alkenylthio, (C₂-C₈)-alkynylthio, (C₁-C₈)-haloalkylthio, (C₂-C₈)-haloalkenylthio, (C₂-C₈)-haloalkynylthio, (C₁-C₈)-alkylsulfinyl, (C₂-C₈)-alkenylsulfinyl, (C₂-C₈)-alkynylsulfinyl, (C₁-C₈)-haloalkylsulfinyl, (C₂-C₈)-haloalkenylsulfinyl, (C₂-C₈)-haloalkynylsulfinyl, (C₁-C₈)-alkylsulfonyl, (C₂-C₈)-alkenylsulfonyl, (C₂-C₈)-alkynylsulfonyl, (C₁-C₈)-haloalkylsulfonyl, (C₂-C₈)-haloalkenylsulfonyl, (C₂-C₈)-haloalkynylsulfonyl, radicals of the formula -NR*R**, where R* and R** are defined below, and
(C₃-C₈)-cycloalkyl, (C₅-C₈)-cycloalkenyl, (C₅-C₈)-cycloalkynyl, (C₃-C₈)-cycloalkyl-(C₁-C₆)-alkoxy, (C₃-C₈)-cycloalkyl-(C₁-C₆)-alkyl-S(O)ₚ-, (C₅-C₈)-cycloalkenyl-(C₁-C₆)-alkoxy, (C₅-C₈)-cycloalkenyl-(C₁-C₆)-alkyl-S (O)ₚ-, (C₅-C₈)-cycloalkynyl-(C₁-C₆)-alkoxy, (C₅-C₈)-cycloalkynyl-(C₁-C₆) -alkyl-S(O)ₚ-, (C₃-C₈)-cycloalkoxy, (C₃-C₈)-cycloalkyl-S(O)ₚ-, (C₅-C₈)-cycloalkenyloxy, (C₅-C₈)-cycloalkenyl-S(O)ₚ-, (C₅-C₈)-cycloalkynyloxy, (C₅-C₈)-cycloalkynyl-S(O)ₚ-, (C₃-C₈)-cycloalkoxy- (C₁-C₆)-alkoxy, (C₃-C₈)-cycloalkoxy-(C₁-C₆)-alkyl-S(O)ₚ-, phenyl, phenyl- (C₁-C₆)-alkoxy, phenoxy, phenyl-S(O)ₚ-, phenyl- (C₁-C₆)-alkyl-S(O)ₚ-, phenoxy-(C₁-C₆)-alkoxy, phenoxy-(C₁-C₆)-alkyl-S(O)ₚ-, a radical Het¹, Het¹-S(O)ₚ-, Het¹-(C₁-C₆) -alkoxy, Het¹-O-, Het¹-O-(C₁-C₆)-alkoxy, where the heterocyclic radical Het¹ is defined below,
where each of the 29 lastmentioned radicals is unsubstituted in the acyclic moiety or substituted by one or more identical or different radicals R^{A} and is unsubstituted in the cyclic moiety or substituted by one or more identical or different radicals R^{B} and p is in each case independently of the others 0, 1 or 2,
(b) radicals of the formulae -C(=O)-R^{C}, -C(=O)-O-R^{C}, -O-C(=O)-R^{C}, -O-C(=O)-O-R^{C}, -C(=O)-S-R^{C},-C(=S)-S-R^{C}, -C(=S)-S-R^{C}, -C(=O)-NR*R**,-C(=O)-O-NR*R**, -O-C(=O)-NR*R**, -N(R*)-C(=O)-R^{C}, -N(R*)-C(=O)-NR*R**, -N(R*)-C(=O)-O-R^{C}, (=O)(R^{C})(R^{D}), -P(=O)(OR^{C})(R^{D}), P(=O)(OR^{C})(OR^{D}) or -O-P(=O)(OR^{C})(OR^{D}),
where R*, R**, R^{C} and R^{D} are as defined below,
(c) radicals of the formulae -SiR'₃, -O-SiR'₃, (R')₃Si-(C₁-C₆)-alkoxy, -CO-O-NR'₂, -O-N=CR'₂, -N=CR'₂, -O-NR'₂, -CH(OR')₂ and -O-(CH₂)_{q}-CH(OR')₂,
in which each of the radicals R' independently of the others is H, (C₁-C₄)-alkyl or phenyl, which is unsubstituted or substituted by one or more radicals from the group consisting of halogen, (C₁-C₄)-alkyl, (C₁-C₄)-alkoxy, (C₁-C₄)-haloalkyl, (C₁-C₄)-haloalkoxy and nitro or at two adjacent positions by a (C₂-C₆)-alkylene bridge, and q is an integer from 0 to 6, and
(d) radicals of the formula R"O-CHR'''CH(OR")-(C₁-C₆)-alkoxy,
in which each of the radicals R" independently of the others is H or (C₁-C₄)-alkyl or together the radicals are a (C₁-C₆)-alkylene group and R''' is H or (C₁-C₄)-alkyl,
or
R¹ is (C₃-C₉)-cycloalkyl, (C₅-C₉)-cycloalkenyl, (C₅-C₉)-cycloalkynyl or phenyl, where each of the 4 lastmentioned radicals is unsubstituted or substituted by one or more radicals from the group consisting of the radicals [subgroups (a')-(e')]
(a') halogen, cyano, thio, nitro, hydroxyl, carboxyl, (C₁-C₈)-alkyl, (C₁-C₈)-haloalkyl, (C₁-C₄)-alkoxy- (C₁-C₄)-alkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₂-C₈)-alkenyloxy, (C₂-C₈)-alkynyloxy, (C₁-C₈)-haloalkoxy, (C₁-C₄)-alkoxy- (C₁-C₄)-alkoxy, (C₁-C₈)-alkylthio, (C₂-C₈)-alkenylthio, (C₂-C₈)-alkynylthio and radicals of the formula-NR*R**, where the radicals R* and R** are defined below,
(b') radicals of the formulae -C(=O)-R^{C}, -C(=O)-O-R^{C}, -O-C(=O)-R^{C}, -O-C(=O)-O-R^{C}, -C(=O)-S-R^{C},-C(=S)-S-R^{C}, -C(=S)-S-R^{C}, -C(=O)-NR*R**,-C(=O)-O-NR*R**, -O-C(=O)-NR*R**, -N(R*)-C(=O)-R^{C}, -N(R*)-C(=O)-NR*R**, -N(R*)-C(=O)-O-R^{C}, -P(=O)(R^{C})(R^{D}), -P(=O)(OR^{C})(R^{D}),-P(=O)(OR^{C})(OR^{D}) or -O-P(=O) (OR^{C}) (OR^{D}), where R*, R**, R^{C} and R^{D} are as defined below,
(c') radicals of the formulae -SiR'₃, -O-SiR'₃, (R')₃Si-(C₁-C₆)-alkoxy, -CO-O-NR'₂, -O-N=CR'₂, -N=CR'₂, -O-NR'₂, -CH(OR')₂ and -O-(CH₂)_{q}-CH(OR')₂,
in which each of the radicals R' independently of the others is H, (C₁-C₄)-alkyl or phenyl, which is unsubstituted or substituted by one or more radicals from the group consisting of halogen, (C₁-C₄)-alkyl, (C₁-C₄)-alkoxy, (C₁-C₄)-haloalkyl, (C₁-C₄)-haloalkoxy and nitro or at two adjacent positions by a (C₂-C₆)-alkylene bridge, and q is an integer from 0 to 6, and
(d') radicals of the formula R"O-CHR'''CH(OR")-(C₁-C₆)-alkoxy,
in which each of the radicals R" independently of the others is H or (C₁-C₄)-alkyl or together the radicals are a (C₁-C₆)-alkylene group and R''' is H or (C₁-C₄)-alkyl, and
(e') a radical of the formula Het¹ which is unsubstituted or substituted by one or more identical or different radicals R^{B},
or
R¹ is a polycyclic radical based on (C₃-C₉)-cycloalkyl, (C₅-C₉)-cycloalkenyl, (C₅-C₉)-cycloalkynyl or phenyl, where the base ring is fused with a carbocyclic or heterocyclic ring, preferably a 5- or 6-membered ring having 0 or 1 to 3 ring heteroatoms from the group consisting of N, O and S, preferably benzo-fused, and where the base ring or the polycyclic system is unsubstituted or substituted by one or more identical or different radicals R^{B}, preferably unsubstituted or substituted by one or more radicals from the group consisting of halogen, cyano, nitro, hydroxyl, carboxyl, (C₁-C₆)-alkyl, (C₁-C₆)-haloalkyl, (C₁-C₄)-alkoxy-(C₁-C₄)-alkyl, (C₂-C₆) -alkenyl, (C₂-C₆) -haloalkenyl, (C₂-C₆) -alkynyl, (C₂-C₆) -haloalkynyl, (C₁-C₆) -alkoxy, (C₂-C₆)-alkenyloxy, (C₂-C₆)-alkynyloxy, (C₁-C₆)-haloalkoxy, (C₁-C₄)-alkoxy-(C₁-C₄)-alkoxy, (C₁-C₆) -alkylthio, (C₂-C₆) -alkenylthio, (C₂-C₆) -alkynylthio, (C₃-C₆)-cycloalkyl, (C₃-C₆)-cycloalkoxy, [(C₁-C₈)-alkoxy]-carbonyl, [(C₁-C₆)-haloalkoxy]-carbonyl and oxo,
or
R¹ is a heterocyclic radical Het¹ which is unsubstituted in the ring or in the polycyclic system or substituted by one or more identical or different radicals R^{B}, preferably unsubstituted or substituted by one or more radicals from the group consisting of halogen, cyano, thio, nitro, hydroxyl, carboxyl, (C₁-C₆)-alkyl, (C₁-C₆)-haloalkyl, (C₁-C₄)-alkoxy-(C₁-C₄)-alkyl, (C₂-C₆)-alkenyl, (C₂-C₆) -haloalkenyl, (C₂-C₆)-alkynyl, (C₂-C₆)-haloalkynyl, (C₁-C₆) -alkoxy, (C₂-C₆) -alkenyloxy, (C₂-C₆) -alkynyloxy, (C₁-C₆)-haloalkoxy, (C₁-C₄)-alkoxy- (C₁-C₄)-alkoxy, (C₁-C₆) -alkylthio, (C₂-C₆)-alkenylthio, (C₂-C₆) -alkynylthio, (C₃-C₆)-cycloalkyl, (C₃-C₆)-cycloalkoxy, [(C₁-C₈)-alkoxy]-carbonyl, [(C₁-C₆)-haloalkoxy]-carbonyl and oxo,
where in the radicals mentioned above and in the radicals below
Het¹ in each case independently of the others is a saturated, partially unsaturated or heteroaromatic monocyclic heterocyclyl radical having 3 to 9 ring atoms, preferably having 5 or 6 ring atoms, or a 9- or 10-membered bicyclic heterocycle which contains 1, 2, 3 or 4 heteroatoms selected from the group consisting of O, N and S, preferably a 5- or 6-membered heterocycle having 1 to 3 ring heteroatoms from the group consisting of N, O and S which is optionally also fused to a carbocyclic or heterocyclic ring, preferably a carbocyclic ring having 3 to 6 carbon atoms or a heterocyclic ring having 5 or 6 ring atoms and 1 to 3 ring heteroatoms from the group consisting of N, O and S, preferably optionally benzo-fused,
R*, R** are each independently of one another (i.e. also of other groups NR*R**) H, (C₁-C₈ -alkyl, (C₂-C₈)-alkenyl, (C₂-C₈)-alkynyl, (C₁-C₄)-alkoxy-(C₁-C₄)-alkyl, (C₁-C₆) -alkanoyl, [(C₁-C₄)-haloalkyl]-carbonyl, [(C₁-C₄)-alkoxy]-carbonyl, [(C₁-C₄)-haloalkoxy] -carbonyl, (C₃-C₆)-cycloalkyl, (C₃-C₆)-cycloalkyl-(C₁-C₄)-alkyl, phenyl, phenyl-(C₁-C₄)-alkyl, where each of the 4 lastmentioned radicals in the cycle is optionally substituted by one or more identical or different radicals R^{bb}, or
R* and R** together with the nitrogen atom are a 3- to 8-membered heterocycle which, in addition to the nitrogen atom, may contain one or two further ring heteroatoms from the group consisting of N, O and S and which may be unsubstituted or substituted by one or more radicals from the group consisting of (C₁-C₄)-alkyl, (C₁-C₄)-haloalkyl and oxo,
R^{A} is halogen, cyano, hydroxyl or (C₁-C₆)-alkoxy,
R^{B} is halogen, cyano, hydroxyl, oxo, nitro, (C₁-C₆)-alkyl, (C₁-C₄)-haloalkyl, cyano-(C₁-C₄)-alkyl, hydroxy-(C₁-C₄)-alkyl, nitro-(C₁-C₄)-alkyl, (C₂-C₆)-alkenyl, (C₂-C₆) -haloalkenyl, (C₂-C₆) -alkynyl, (C₂-C₆)-haloalkynyl, (C₁-C₆)-alkoxy, (C₂-C₆) -alkenyloxy, (C₂-C₆) -alkynyloxy, (C₁-C₆)-haloalkoxy, (C₁-C₄)-alkoxy-(C₁-C₄)-alkyl, (C₁-C₄)-alkoxy-(C₁-C₄)-alkoxy, (C₁-C₄)-haloalkoxy-(C₁-C₄)-alkyl, (C₁-C₄)-haloalkoxy-(C₁-C₄)-alkoxy, (C₁-C₆)-alkylthio, (C₂-C₆)-alkenylthio, (C₂-C₆)-alkynylthio, (C₁-C₆)-alkylsulfinyl, (C₁-C₆)-haloalkylsulfinyl, (C₁-C₆)-alkylsulfonyl, (C₁-C₆)-haloalkylsulfonyl, a radical of the formula R^{aa}-C(=O)- or R^{aa}-C(=O)-(C₁-C₆)-alkyl, where the R^{aa} are defined below, -NR*R**, where R* and R** are defined below, tri-[(C₁-C₄)-alkyl]-silyl, tri-[(C₁-C₄)-alkyl]-silyl-(C₁-C₆)-alkyl, (C₃-C₆)-cycloalkyl, (C₃-C₆)-cycloalkoxy, (C₃-C₆)-cycloalkyl-(C₁-C₄)-alkyl, (C₃-C₆)-cycloalkyl-(C₁-C₈)-alkoxy, phenyl, phenyl-(C₁-C₆)-alkyl, phenoxy, phenoxy-(C₁-C₆)-alkyl, phenylamino, phenylamino-(C₁-C₆)-alkyl or a 5- or 6-membered monocyclic or 9- or 10-membered bicyclic heterocycle which contains 1, 2, 3 or 4 heteroatoms selected from the group consisting of O, N and S, where each of the 11 lastmentioned radicals is optionally substituted in the cyclic moiety by one or more identical or different radicals R^{bb},
R^{C}, R^{D} are each independently of one another (also independently of radicals R^{C}, R^{D} in other groups) hydrogen, (C₁-C₈)-alkyl, (C₂-C₈)-alkenyl or (C₂-C₈)-alkynyl,
where each of the 3 lastmentioned radicals is unsubstituted or substituted by one or more radicals from the group consisting of halogen, cyano, nitro, hydroxyl, (C₁-C₆)-alkoxy, (C₂-C₆) -alkenyloxy, (C₂-C₆) -alkynyloxy, (C₁-C₈)-haloalkoxy, (C₁-C₄)-alkoxy-(C₁-C₄)-alkoxy, (C₁-C₈)-alkylthio, (C₁-C₈)-haloalkylthio, (C₁-C₈)-alkylsulfinyl, (C₁-C₈)-haloalkylsulfinyl, (C₁-C₈)-alkylsulfonyl, (C₁-C₈)-haloalkylsulfonyl and tri-[(C₁-C₄)-alkyl]-silyl,
or
(C₃-C₈)-cycloalkyl, (C₅-C₈)-cycloalkenyl, (C₅-C₈)-cycloalkynyl, phenyl, (C₃-C₈)-cycloalkyl-(C₁-C₆)-alkyl, (C₅-C₈)-cycloalkenyl-(C₁-C₆)-alkyl, (C₅-C₈)-cycloalkynyl-(C₁-C₆)-alkyl, phenyl-(C₁-C₆)-alkyl, (C₃-C₈)-cycloalkyloxy-(C₁-C₆)-alkyl, (C₃-C₈)-cycloalkyl-S(O)ₚ-(C₁-C₆)-alkyl, (C₅-C₈)-cycloalkenyloxy- (C₁-C₆) -alkyl, (C₅-C₈)-cycloalkynyloxy-(C₁-C₆)-alkyl, phenoxy-(C₁-C₆)-alkyl, phenyl-S(O)ₚ-(C₁-C₆)-alkyl, (C₃-C₈)-cycloalkylamino-(C₁-C₆)-alkyl, (C₅-C₈)-cycloalkenylamino-(C₁-C₆)-alkyl, (C₅-C₈)-cycloalkynylamino-(C₁-C₆)-alkyl, phenylamino-(C₁-C₆)-alkyl, Het¹, Het¹-(C₁-C₆)-alkyl, Het¹-O-(C₁-C₆)-alkyl or Het¹-S(O)ₚ-(C₁-C₆)-alkyl, where Het¹ has the meaning mentioned,
where each of the 22 lastmentioned radicals is unsubstituted in the acyclic moiety or substituted by one or more identical or different radicals R^{A} and is unsubstituted in the cyclic moiety or substituted by one or more identical or different radicals R^{B} and p is in each case independently of the others 0, 1 or 2,
R^{aa} in each case independently of the others is hydrogen, OH, (C₁-C₆)-alkyl, (C₁-C₄)-haloalkyl, (C₂-C₆)-alkenyl, (C₂-C₆)-alkynyl, (C₁-C₆)-alkoxy, (C₁-C₆)-alkoxy-(C₁-C₆)-alkyl, (C₁-C₆)-alkoxy-(C₁-C₆)-alkyloxy, (C₁-C₄)-haloalkoxy, (C₁-C₄)-haloalkoxy-(C₁-C₆)-alkyl, (C₁-C₄)-haloalkoxy-(C₁-C₆)-alkoxy, (C₃-C₆)-alkenyloxy, (C₃-C₆)-alkenyloxy-(C₁-C₆)-alkyl, (C₃-C₆)-alkenyloxy-(C₁-C₆)-alkoxy, (C₃-C₆)-alkynyloxy, (C₃-C₆)-alkynyloxy-(C₁-C₆)-alkyl, (C₃-C₆)-alkynyloxy-(C₁-C₆)-alkoxy, -NR*R*, where R* and R** are as defined above, tri-[(C₁-C₄)-alkyl]-silyl, tri-[(C₁-C₄)-alkyl]-silyl-(C₁-C₆)-alkyl, tri-[(C₁-C₄)-alkyl]-silyl-(C₁-C₆)-alkoxy, (C₃-C₆)-cycloalkyl, (C₃-C₆)-cycloalkoxy, (C₃-C₆)-cycloalkyl-(C₁-C₈)-alkyl, (C₃-C₆)-cycloalkyl-(C₁-C₈)-alkoxy, (C₅-C₆)-cycloalkenyl, (C₅-C₆)-cycloalkenyl-(C₁-C₆)-alkyl, (C₅-C₆)-cycloalkenyloxy, (C₅-C₆)-cycloalkynyl, (C₅-C₆)-cycloalkynyl-(C₁-C₆)-alkyl, (C₅-C₆)-cycloalkynyl-(C₁-C₆)-alkoxy, phenyl, phenyl-(C₁-C₆)-alkyl, phenyl- (C₁-C₆)-alkoxy, phenoxy, phenoxy- (C₁-C₆) -alkyl, phenoxy-(C₁-C₆)-alkoxy, phenylthio, phenyl-S(O)ₚ-(C₁-C₆)-alkyl, phenyl-S(O)ₚ-(C₁-C₆)-alkoxy, where p in each case independently of the others is 0, 1 or 2, phenylamino, phenylamino-(C₁-C₆)-alkyl, phenylamino-(C₁-C₆)-alkoxy or a 5- or 6-membered monocyclic or 9- or 10-membered bicyclic heterocycle which is optionally attached via an alkylene group or alkoxy group and contains 1, 2, 3 or 4 heteroatoms selected from the group consisting of O, N and S, where each of the 20 lastmentioned radicals is optionally substituted in the cyclic moiety by one or more identical or different radicals R^{bb}, and
R^{bb} in each case independently of the others is halogen, (C₁-C₄)-alkyl, (C₁-C₄)-haloalkyl, (C₁-C₄)-alkoxy or (C₁-C₄)-haloalkoxy.

4. Compounds or salts thereof according to any of Claims 1 to 3, **characterized in that**
(R²)ₙ is n substituents R², where R² (if n = 1) or each of the substituents R² (if n is greater than 1) independently of the others is halogen, cyano, nitro, (C₁-C₆)-alkyl, (C₁-C₆)-alkoxy, (C₁-C₆)-alkylthio, (C₁-C₆)-alkylsulfinyl, (C₁-C₆)-alkylsulfonyl, (C₁-C₆)-haloalkyl, (C₁-C₆)-haloalkoxy, (C₁-C₆)-haloalkylthio, (C₁-C₆)-haloalkylsulfinyl, (C₁-C₆)-haloalkylsulfonyl, (C₂-C₆)-alkenyl, (C₂-C₆)-alkynyl, tri-[(C₁-C₄)-alkyl]-silyl or tri-[(C₁-C₄)-alkyl]-silyl-(C₁-C₄)-alkyl
or where in each case two groups R² located ortho at the ring together are a group of the formula-Z¹-A*-Z² in which
A* is an alkylene group having 1 to 4 carbon atoms which is optionally substituted by one or more radicals from the group consisting of halogen, (C₁-C₄)-alkyl, (C₁-C₄) -haloalkyl, (C₁-C₄)-alkoxy and (C₁-C₄)-haloalkoxy,
Z¹ is a direct bond, O or S and
Z² is a direct bond, O or S,
where the group -Z¹-A*-Z² together with the carbon atoms, attached to the group, of the phenyl ring form a fused-on 5- or 6-membered ring, and
(R³)ₘ is m substituents R³,
where R³ (if m = 1) or each of the substituents R³ (if m is greater than 1) independently of the others is halogen, cyano, nitro, (C₁-C₆)-alkyl, (C₁-C₆)-alkoxy, (C₁-C₆)-alkylthio, (C₁-C₆)-alkylsulfinyl, (C₁-C₆)-alkylsulfonyl, (C₁-C₆)-haloalkyl, (C₁-C₆)-haloalkoxy, (C₁-C₆)-haloalkylthio, (C₁-C₆)-haloalkylsulfinyl, (C₁-C₆)-haloalkylsulfonyl, (C₂-C₆)-alkenyl, (C₂-C₆)-alkynyl, NR*R**, tri-[(C₁-C₄)-alkyl]-silyl or tri-[(C₁-C₄)-alkyl]-silyl-(C₁-C₄)-alkyl
or where in each case two groups R³ located ortho at the ring together are a group of the formula-Z³-A**-Z⁴ in which
A** is an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z³ is a direct bond, O or S and
Z⁴ is a direct bond, O or S, where the group -Z³-A**-Z⁴ together with the carbon atoms, attached to the group, of the phenyl ring form a fused-on 5- or 6-membered ring,
R*, R** each independently of one another or together with the nitrogen atom have the meaning mentioned and
n, m are each independently of one another 0, 1, 2, 3, 4 or 5, preferably 0, 1, 2 or 3, in particular 0, 1 or 2, with the proviso that the two are not simultaneously 0.

5. Compounds or salts thereof according to any of Claims 1 to 4, **characterized in that**
(R²)ₙ is n substituents R², where, in the case that n = 1, the substituent R² or, in the case that n is greater than 1, each of the substituents R² independently of the others is halogen, cyano, nitro, (C₁-C₄)-alkyl, (C₁-C₄)-alkoxy, (C₁-C₄)-alkylthio, (C₁-C₄)-alkylsulfinyl, (C₁-C₄)-alkylsulfonyl, (C₁-C₄)-haloalkyl, (C₁-C₄)-haloalkoxy, (C₁-C₄)-haloalkylthio, (C₁-C₄)-haloalkylsulfinyl, (C₁-C₄)-haloalkylsulfonyl, (C₂-C₄)-alkenyl, (C₂-C₄)-alkynyl, tri-[(C₁-C₄)-alkyl]-silyl or tri-[(C₁-C₄)-alkyl]-silyl-(C₁-C₄)-alkyl and
n is 0, 1, 2, 3, 4 or 5, preferably 0, 1, 2 or 3, in particular 0, 1 or 2, with the proviso that n and m are not both simultaneously 0.

6. Compounds or salts thereof according to any of Claims 1 to 5, **characterized in that**
(R³)ₘ is m substituents R³, where, in the case that m = 1, the substituent R³ or, in the case that m is greater than 1, each of the substituents R³ independently of the others is halogen, cyano, nitro, (C₁-C₄)-alkyl, (C₁-C₄)-alkoxy, (C₁-C₄)-alkylthio, (C₁-C₄)-alkylsulfinyl, (C₁-C₄)-alkylsulfonyl, (C₁-C₄)-haloalkyl, (C₁-C₄)-haloalkoxy, (C₁-C₄)-haloalkylthio, (C₁-C₄)-haloalkylsulfinyl, (C₁-C₄)-haloalkylsulfonyl, (C₂-C₆)-alkenyl, (C₂-C₆) -alkynyl or tri-[(C₁-C₄)-alkyl]silyl-Z^{b}-, where Z^{b} = a covalent bond or (C₁-C₄)-alkylene, or
in each case two groups R³ located ortho at the ring together are a group of the formula -Z³-A**-Z⁴,
where
A** is an alkylene group which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z³ is O or S and
Z⁴ is O or S, where the group -Z³-A**-Z⁴ together with the carbon atoms, attached to the group, of the phenyl ring form a fused-on 5- or 6-membered ring,
m is 0, 1, 2, 3, 4 or 5, preferably 0, 1, 2, 3 or 4, in particular 0, 1, 2 or 3, with the proviso that n and m in formula (I) are not both simultaneously 0.

7. Compounds or salts thereof according to any of Claims 1 to 6, **characterized in that**
(R²)ₙ is n substituents R²,
where, in the case that n = 1, the substituent R² or, in the case that n is greater than 1, each of the substituents R² independently of the other is fluorine, chlorine, bromine, iodine, cyano, nitro, methyl, ethyl, methoxy, methylthio, methylsulfinyl, methylsulfonyl, trifluoromethyl, trifluoromethoxy, trifluoroalkylthio, trifluoromethylsulfinyl or trifluoromethylsulfonyl, and
(R³)ₘ is m substituents R³, where, in the case that m = 1, the substituent R³ or, in the case that m is greater than 1, each of the substituents R³ independently of the other is halogen, cyano, nitro, methyl, methoxy, methylthio, methylsulfinyl, methylsulfonyl, trifluoromethyl, trifluoromethoxy, trifluoroalkylthio, trifluoromethylsulfinyl or trifluoromethylsulfonyl, and
m is 0, 1, 2, 3, 4 or 5, preferably 0, 1, 2, 3 or 4, in particular 0, 1, 2 or 3 and
n is 0, 1, 2, 3, 4 or 5, preferably 0, 1, 2 or 3, in particular 0, 1 or 2,
with the proviso, that n and m are not simultaneously 0.

8. Compounds or salts thereof according to any of Claims 1 to 7, **characterized in that**
(R²)ₙ is 2-bromo, 3-bromo, 4-bromo, 2-chloro, 3-chloro, 4-chloro, 2-fluoro, 3-fluoro, 4-fluoro, 2-cyano, 3-cyano, 4-cyano, 2-methyl, 3-methyl, 4-methyl, 2-ethyl, 3-ethyl, 4-ethyl, 2-CF₃, 3-CF₃, 4-CF₃, 2-methoxy, 3-methoxy, 4-methoxy, 2-ethoxy, 3-ethoxy, 4-ethoxy, 2-methylthio, 3-methylthio, 4-methylthio, 2-methylsulfinyl, 3-methylsulfinyl, 4-methylsulfonyl, 2-methylsulfonyl, 3-methylsulfonyl, 4-methylsulfonyl, 2,3-dimethyl, 2,4-dimethyl, 2,5-dimethyl, 2,6-dimethyl, 3,4-dimethyl, 3,5-dimethyl, 2,3-difluoro, 2,4-difluoro, 2,5-difluoro, 2,6-difluoro, 3,4-difluoro, 3,5-difluoro, 2,3-dichloro, 2,4-dichloro, 2,5-dichloro, 2,6-dichloro, 3,4-dichloro, 3,5-dichloro, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), (4-Br-3-F), (3-Br-4-F), (3-Br-5-F), (4-Br-3-Cl), (3-Br-4-Cl), (4-CN-3-F), (3-CN-4-F), (3-CN-5-F), (4-CN-3-Cl), (3-CN-4-Cl), 2,3,4-trifluoro, 2,3,5-trifluoro, 2,3,6-trifluoro, 2,4,6-trifluoro, 3,4,5-trifluoro, 2,3,4-trichloro, 2,3,5-trichloro, 2,3,6-trichloro, 2,4,6-trichloro or 3,4,5-trichloro and
(R³)ₘ is 2-bromo, 3-bromo, 4-bromo, 2-chloro, 3-chloro, 4-chloro, 2-fluoro, 3-fluoro, 4-fluoro, 2-cyano, 3-cyano, 4-cyano, 2-methyl, 3-methyl, 4-methyl, 2-ethyl, 3-ethyl, 4-ethyl, 2-CF₃, 3-CF₃, 4-CF₃, 2-methoxy, 3-methoxy, 4-methoxy, 2-ethoxy, 3-ethoxy, 4-ethoxy, 2-methylthio, 3-methylthio, 4-methylthio, 2-methylsulfinyl, 3-methylsulfinyl, 4-methylsulfonyl, 2-methylsulfonyl, 3-methylsulfonyl, 4-methylsulfonyl, 2-nitro, 3-nitro, 4-nitro, 2,3-dimethyl, 2,4-dimethyl, 2,5-dimethyl, 2,6-dimethyl, 3,4-dimethyl, 3,5-dimethyl, 2,3-difluoro, 2,4-difluoro, 2,5-difluoro, 2,6-difluoro, 3,4-difluoro, 3,5-difluoro, 2,3-dichloro, 2,4-dichloro, 2,5-dichloro, 2,6-dichloro, 3,4-dichloro, 3,5-dichloro, 2,5-dicyano, 2,6-dicyano, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), (4-Br-2-F), (4-Br-3-F), (4-CN-3-F), (4-NO₂-3-F), (4-OMe-3-F), (3-CN-4-F), (3-NO₂-4-F), (3-CN-4-Cl), (3-NO₂-4-Cl), (5-CN-2-F), 2,3,4-trifluoro, 2,3,5-trifluoro, 2,3,6-trifluoro, 2,4,6-trifluoro, 3,4,5-trifluoro, 2,3,4-trichloro, 2,3,5-trichloro, 2,3,6-trichloro, 2,4,6-trichloro or 3,4,5-trichloro.

9. Process for preparing a compound of the formula (I) as defined in any of Claims 1 to 8 or a salt thereof, **characterized in that**
(a) compounds of the formula (II) 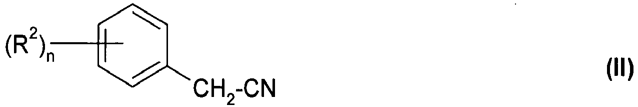 are reacted with compounds of the formula (III) or salts thereof 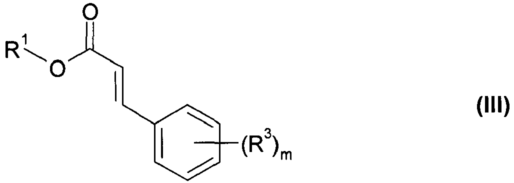 to give compounds of the formula (I') 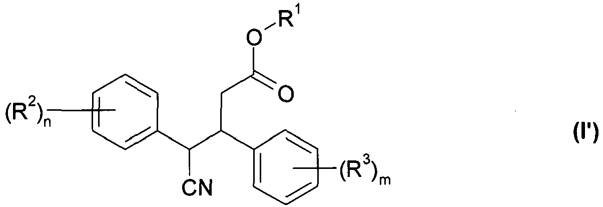 where R¹, R², R³, m and n in the compounds (II), (III) and (I') are as defined in the respective compound of the formula (I) to be prepared, and the (3R,4R)-threo isomer is isolated from the isomer mixture (I') analogously to general separation methods
or
(b) compounds of the formula (I") 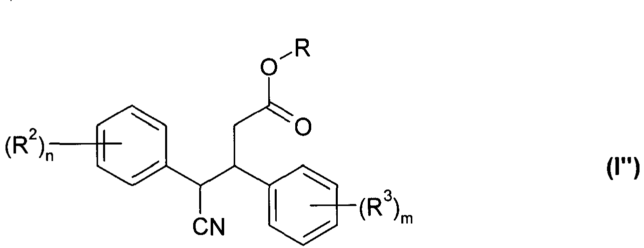 in which R is a radical from the group of the radicals possible for R¹, but different from the radical R¹ in the compound (I') to be prepared, is reacted with a compound of the formula R¹-OH in which R¹ is defined as in the compound of the formula (I) to be prepared, to give compound (I'), where R², R³, m and n in the compound (I") are as defined in the compound of the formula (I) to be prepared in each case, and the (3R,4R)-threo isomer is isolated from the isomer mixture (I') analogously to general separation methods
or
(c) stereochemically enriched compounds of the (3R,4R)-threo isomers of the formula (I"') 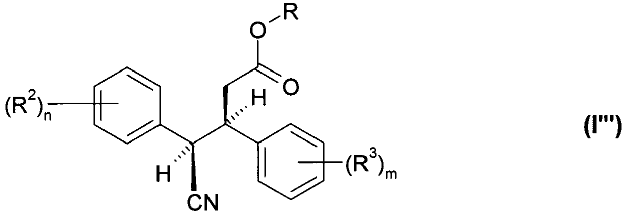 in which R is a radical from the group of the radicals possible for R¹, but different from the radical R¹ in the compound (I) to be prepared, is reacted with a compound of the formula R¹-OH in which R¹ is defined as in the compound of the formula (I) to be prepared, to give compound (I), where R², R³, m and n in the compound (I'") are as defined in the compound of the formula (I) to be prepared.

10. Herbicidal or plant growth-regulating composition, **characterized in that** it comprises one or more compounds of the formula (I) or salts thereof as defined in any of Claims 1 to 8 and formulation auxiliaries customary in crop protection.

11. Method for controlling harmful plants or for regulating the growth of plants, **characterized in that** it comprises applying an effective amount of one or more compounds of the formula (I) or salts thereof as defined in any of Claims 1 to 7 onto the plants, plant seeds, the soil in which or on which the plants grow or the area under cultivation.

12. Method according to Claim 11, **characterized in that** the compounds of the formula (I) or salts thereof are employed for controlling harmful plants or for regulating the growth in crops of useful plants or ornamental plants.

13. Method according to Claim 12, **characterized in that** the crop plants are transgenic crop plants.

14. Use of the compounds of the formula (I) or salts thereof according to any of Claims 1 to 8 as herbicides or plant growth regulators.

15. Use according to Claim 14, **characterized in that** the compounds are applied in crops of useful or ornamental plants.

## Revendications

1. Composés de formule (I) ou leurs sels, chacun sous la forme (3R,4R)-thréo optiquement active, 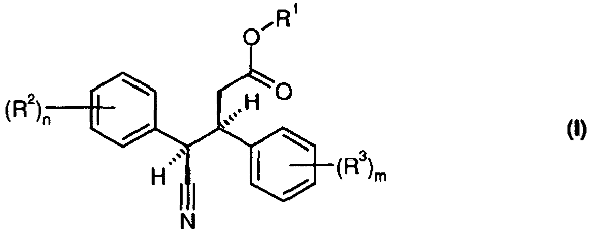 dans laquelle
R¹ signifie hydrogène ou un radical hydrolysable,
(R²)ₙ signifie n substituants R²,
R² (lorsque n = 1) ou chacun des substituants R² (lorsque n est supérieur à 1) signifiant indépendamment les uns des autres halogène, cyano, nitro, alkyle en (C₁-C₈), alcoxy en (C₁-C₈), alkylthio en (C₁-C₈), alkylsulfinyle en (C₁-C₈), alkylsulfonyle en (C₁-C₈), haloalkyle en (C₁-C₆), haloalcoxy en (C₁-C₆), haloalkylthio en (C₁-C₆), haloalkylsulfinyle en (C₁-C₆), haloalkylsulfonyle en (C₁-C₆), alcényle en (C₂-C₆), alcynyle en (C₂-C₆), tri-[alkyle en (C₁-C₄)]-silyle ou tri-[alkyle en (C₁-C₄)]-silyl-alkyle en (C₁-C₄),
ou deux groupes R² en position ortho sur le cycle signifiant ensemble un groupe de formule -Z¹-A*-Z², dans laquelle
A* représente un groupe alkylène de 1 à 4 atomes C, qui est éventuellement substitué par un ou plusieurs radicaux du groupe constitué par halogène, alkyle en (C₁-C₄), haloalkyle en (C₁-C₄), alcoxy en (C₁-C₄) et haloalcoxy en (C₁-C₄),
Z¹ représente une liaison directe, O ou S, et
Z² représente une liaison directe, O ou S,
le groupe -Z¹-A*-Z² formant ensemble avec les atomes C du cycle phényle reliés au groupe un cycle à 5 ou 6 condensé, et
(R³)ₘ signifie m substituants R³,
R³ (lorsque m = 1) ou chacun des substituants R³ (lorsque m est supérieur à 1) signifiant indépendamment les uns des autres halogène, cyano, nitro, alkyle en (C₁-C₈), alcoxy en (C₁-C₈), alkylthio en (C₁-C₈), alkylsulfinyle en (C₁-C₈), alkylsulfonyle en (C₁-C₈), haloalkyle en (C₁-C₆), haloalcoxy en (C₁-C₆), haloalkylthio en (C₁-C₆), haloalkylsulfinyle en (C₁-C₆), haloalkylsulfonyle en (C₁-C₆), alcényle en (C₂-C₆), alcynyle en (C₂-C₆), NR*R**, R* et R** étant définis ci-après, tri-[alkyle en (C₁-C₄)]-silyle ou tri-[alkyle en (C₁-C₄)]-silyl-alkyle en (C₁-C₄),
ou deux groupes R³ en position ortho sur le cycle signifiant ensemble un groupe de formule -Z³-A**-Z⁴, dans laquelle
A** représente un groupe alkylène de 1 à 4 atomes C, qui est éventuellement substitué par un ou plusieurs radicaux du groupe constitué par halogène, alkyle en (C₁-C₄), haloalkyle en (C₁-C₄), alcoxy en (C₁-C₄) et haloalcoxy en (C₁-C₄),
Z³ représente une liaison directe, O ou S, et
Z⁴ représente une liaison directe, O ou S,
le groupe -Z³-A**-Z⁴ formant ensemble avec les atomes C du cycle phényle reliés au groupe un cycle à 5 ou 6 condensé, et
n, m signifiant chacun indépendamment l'un de l'autre 0, 1, 2, 3, 4 ou 5, de préférence 0, 1, 2 ou 3, à condition que les deux ne signifient pas simultanément 0,
la configuration stéréochimique sur l'atome C en position 3 du dérivé d'acide butyrique présentant une pureté stéréochimique de 60 à 100 % (*R*), de préférence de 70 à 100 % (*R*), de manière davantage préférée de 80 à 100 % (*R*)*,* notamment de 90 à 100 % (*R*)*,* par rapport au mélange présent des énantiomères thréo, et
la configuration stéréochimique sur l'atome C en position 4 du dérivé d'acide butyrique présentant une pureté stéréochimique de 60 à 100 % (*R*)*,* de préférence de 70 à 100 % (*R*)*,* de manière davantage préférée de 80 à 100 % (*R*)*,* notamment de 90 à 100 % (*R*)*,* par rapport au mélange présent des énantiomères thréo.

2. Composés ou leurs sels selon la revendication 1, **caractérisés en ce que**
R¹ signifie hydrogène ou un radical hydrocarboné éventuellement substitué ou un radical hétérocyclyle éventuellement substitué, chacun des deux derniers radicaux carbonés cités y compris leurs substituants comprenant 1 à 30 atomes C, de préférence 1 à 24 atomes C, notamment 1 à 20 atomes C, ou
R¹ signifie un radical de formule SiR^{a}R^{b}R^{c}, -NR^{a}R^{b} ou -N=CR^{c}R^{d},
dans les 3 dernières formules citées, chacun des radicaux R^{a}, R^{b}, R^{c} et R^{d} signifiant indépendamment les uns des autres l'hydrogène ou un radical hydrocarboné éventuellement substitué, SiH₃ étant toutefois exclu pour SiR^{a}R^{b}R^{c}, ou R^{a} et R^{b} signifiant ensemble avec l'atome N un hétérocycle de 3 à 9 chaînons, qui peut contenir en plus de l'atome N un ou deux hétéroatomes de cycle supplémentaires du groupe constitué par N, O et S, et qui est non substitué ou substitué, ou R^{c} et R^{d} signifiant ensemble avec l'atome C un radical carbocyclique de 3 à 9 chaînons ou un radical hétérocyclique, qui peut contenir 1 à 3 hétéroatomes de cycle du groupe constitué par N, O et S, le radical carbocyclique ou hétérocyclique étant non substitué ou substitué,
chacun des radicaux R^{a}, R^{b}, R^{c} et R^{d} y compris leurs substituants comprenant jusqu'à 30 atomes C, de préférence jusqu'à 24 atomes C, notamment jusqu'à 20 atomes C, ou
R¹ signifie un radical de formule -C(=O)-R^{e} ou -P(=O)(R^{f})₂, R^{e} et les R^{f} signifiant chacun indépendamment les uns des autres hydrogène, OH, alkyle en (C₁-C₈), haloalkyle en (C₁-C₄), alcényle en (C₂-C₈), alcynyle en (C₂-C₈), alcoxy en (C₁-C₆), alcoxy en (C₁-C₆)-alkyle en (C₁-C₈), haloalcoxy en (C₁-C₄), haloalcoxy en (C₁-C₄)-alkyle en (Ci-Cs), alcényloxy en (C₃-C₈), alcényloxy en (C₃-C₈)-alkyle en (C₁-C₈), alcynyloxy en (C₃-C₈), alcynyloxy en (C₃-C₆)-alkyle en (C₁-C₈),-NR*R**, R* et R** étant définis ci-après, tri-[alkyle en (C₁-C₄)]-silyle, tri-[alkyle en (C₁-C₄)]-silyl-alkyle en (C₁-C₈), cycloalkyle en (C₃-C₆), cycloalkyle en (C₃-C₆)-alkyle en (C₁-C₈), cycloalcényle en (C₅-C₆), cycloalcényle en (C₅-C₆)-alkyle en (C₁-C₈), cycloalcynyle en (C₅-C₆), cycloalcynyle en (C₅-C₆)-alkyle en (C₁-C₈), phényle, phényl-alkyle en (C₁-C₈), phénoxy, phénoxy-alkyle en (C₁-C₈), phénylamino, phénylamino-alkyle en (C₁-C₈), un radical Het¹, Het¹-alkyle en (C₁-C₆) ou Het¹-O-alkyle en (C₁-C₆), le radical hétérocyclique Het¹ étant défini ci-après,
chacun des 15 derniers radicaux cités étant non substitués dans la partie acyclique ou étant substitués par un ou plusieurs radicaux R^{A} identiques ou différents, et étant non substitués dans la partie cyclique ou étant substitués par un ou plusieurs radicaux R^{B} identiques ou différents,
dans les radicaux susmentionnés et suivants,
les Het¹ signifiant chacun indépendamment les uns des autres un radical hétérocyclyle monocyclique saturé, partiellement insaturé ou hétéroaromatique de 3 à 9 atomes de cycle ou un hétérocycle bicyclique à 9 ou 10 chaînons, chacun contenant 1, 2, 3 ou 4 hétéroatomes, choisis dans le groupe constitué par O, N et S,
R*, R** signifiant chacun indépendamment l'un de l'autre (également indépendamment d'autres radicaux NR*R**) H, alkyle en (C₁-C₈), alcényle en (C₂-C₈), alcynyle en (C₂-C₈), alcoxy en (C₁-C₄)-alkyle en (C₁-C₄), alcanoyle en (C₁-C₆), [haloalkyle en (C₁-C₄)]-carbonyle, [alcoxy en (C₁-C₄)]-carbonyle, [haloalcoxy en (C₁-C₄)]-carbonyle, cycloalkyle en (C₃-C₆), cycloalkyle en (C₃-C₆)-alkyle en (C₁-C₄), phényle, phényl-alkyle en (C₁-C₄), chacun des 4 derniers radicaux cités étant éventuellement substitués dans le cycle par un ou plusieurs radicaux R^{bb} identiques ou différents, ou
R* et R** signifiant ensemble avec l'atome N un hétérocycle de 3 à 8 chaînons, qui peut contenir en plus de l'atome N un ou deux hétéroatomes de cycle supplémentaires du groupe constitué par N, O et S, et qui est non substitué ou substitué par un ou plusieurs radicaux du groupe constitué par alkyle en (C₁-C₄), haloalkyle en (C₁-C₄) et oxo,
R^{A} signifiant halogène, cyano, hydroxy ou alcoxy en (C₁-C₆),
R^{B} signifiant halogène, cyano, hydroxy, oxo, nitro, alkyle en (C₁-C₈), haloalkyle en (C₁-C₆), cyano-alkyle en (C₁-C₆), hydroxy-alkyle en (C₁-C₆), nitro-alkyle en (C₁-C₆), alcényle en (C₂-C₈), haloalcényle en (C₂-C₈), alcynyle en (C₂-C₈), haloalcynyle en (C₂-C₈), alcoxy en (C₁-C₈), alcényloxy en (C₂-C₈), alcynyloxy en (C₂-C₈), haloalcoxy en (C₁-C₈), alcoxy en (C₁-C₆) -alkyle en (C₁-C₆), alcoxy en (C₁-C₆)-alcoxy en (C₁-C₄), haloalcoxy en (C₁-C₆)-alkyle en (C₁-C₄), haloalcoxy en (C₁-C₆)-alcoxy en (C₁-C₄), alkylthio en (C₁-C₈), alcénylthio en (C₂-C₆), alcynylthio en (C₂-C₆), alkylsulfinyle en (C₁-C₈), haloalkylsulfinyle en (C₁-C₆), alkylsulfonyle en (C₁-C₈), haloalkylsulfonyle en (C₁-C₆), un radical de formule R^{aa}-C(=O)-, R^{aa}-C(=O)-alkyle en (C₁-C₆), les R^{aa} étant définis ci-après, -NR*R**, R* et R** étant définis ci-après, tri-[alkyle en (C₁-C₄)]-silyle, tri-[alkyle en (C₁-C₄)]-silyl-alkyle en (C₁-C₆), cycloalkyle en (C₃-C₈), cycloalcoxy en (C₃-C₈), cycloalkyle en (C₃-C₈)-alkyle en (C₁-C₈), cycloalkyle en (C₃-C₈)-alcoxy en (C₁-C₈), phényle, phényl-alkyle en (C₁-C₈), phénoxy, phénoxy-alkyle en (C₁-C₈), phénylamino, phénylamino-alkyle en (C₁-C₈) ou un hétérocycle monocyclique à 5 ou 6 chaînons ou bicyclique à 9 ou 10 chaînons, contenant 1, 2, 3 ou 4 hétéroatomes choisis dans le groupe constitué par O, N et S, chacun des 11 derniers radicaux cités étant éventuellement substitués dans la partie cyclique par un ou plusieurs radicaux R^{bb} identiques ou différents,
les R^{aa} signifiant chacun indépendamment les uns des autres hydrogène, OH, alkyle en (C₁-C₈), haloalkyle en (C₁-C₆), alcényle en (C₂-C₈), alcynyle en (C₂-C₈), alcoxy en (C₁-C₈), alcoxy en (C₁-C₆)-alkyle en (C₁-C₆), alcoxy en (C₁-C₆)-alkyloxy en (C₁-C₆), haloalcoxy en (C₁-C₆), haloalcoxy en (C₁-C₆) -alkyle en (C₁-C₆), haloalcoxy en (C₁-C₆) -alcoxy en (C₁-C₆), alcényloxy en (C₃-C₈), alcényloxy en (C₃-C₈)-alkyle en (C₁-C₆), alcényloxy en (C₃-C₈)-alcoxy en (C₁-C₆), alcynyloxy en (C₃-C₈), alcynyloxy en (C₃-C₈)-alkyle en (C₁-C₆), alcynyloxy en (C₃-C₈)-alcoxy en (C₁-C₆), -NR*R**, R* et R** étant tels que définis précédemment, tri-[alkyle en (C₁-C₄)]-silyle, tri-[alkyle en (C₁-C₄)]-silyl-alkyle en (C₁-C₆), tri-[alkyle en (C₁-C₄)]-silyl-alcoxy en (C₁-C₆), cycloalkyle en (C₃-C₈), cycloalcoxy en (C₃-C₈), cycloalkyle en (C₃-C₈)-alkyle en (C₁-C₈), cycloalkyle en (C₃-C₈)-alcoxy en (C₁-C₈), cycloalcényle en (C₅-C₈), cycloalcényle en (C₅-C₈)-alkyle en (C₁-C₆), cycloalcényloxy en (C₅-C₈), cycloalcynyle en (C₅-C₈), cycloalcynyle en (C₅-C₈)-alkyle en (C₁-C₆), cycloalcynyle en (C₅-C₈)-alcoxy en (C₁-C₆), phényle, phényl-alkyle en (C₁-C₈), phényl-alcoxy en (C₁-C₈), phénoxy, phénoxy-alkyle en (C₁-C₈), phénoxy-alcoxy en (C₁-C₈), phénylamino, phénylamino-alkyle en (C₁-C₈), phénylamino-alcoxy en (C₁-C₈) ou un hétérocycle monocyclique à 5 ou 6 chaînons ou bicyclique à 9 ou 10 chaînons éventuellement relié par un groupe alkylène ou un groupe alcoxy, contenant 1, 2, 3 ou 4 hétéroatomes choisis dans le groupe constitué par O, N et S, chacun des 20 derniers radicaux cités étant éventuellement substitués dans la partie cyclique par un ou plusieurs radicaux R^{bb} identiques ou différents, et
les R^{bb} signifiant chacun indépendamment les uns des autres halogène, alkyle en (C₁-C₄), haloalkyle en (C₁-C₄), alcoxy en (C₁-C₄) ou haloalcoxy en (C₁-C₄), ou dans le cas de groupes de base cycliques saturés ou partiellement insaturés, également oxo.

3. Composés ou leurs sels selon la revendication 1 ou 2, **caractérisés en ce que**
R¹ signifie hydrogène, alkyle en (C₁-C₁₈), alcényle en (C₂-C₁₈) ou alcynyle en (C₂-C₁₈), chacun des 3 derniers radicaux cités étant non substitués ou substitués par un ou plusieurs radicaux du groupe constitué par les radicaux [sous-groupes (a) à (d)]
(a) halogène, cyano, thio, nitro, hydroxy, carboxy, alcoxy en (C₁-C₈), alcényloxy en (C₂-C₈), alcynyloxy en (C₂-C₈), haloalcoxy en (C₁-C₈), alcoxy en (C₁-C₄) -alcoxy en (C₁-C₄), alkylthio en (C₁-C₈), alcénylthio en (C₂-C₈), alcynylthio en (C₂-C₈), haloalkylthio en (C₁-C₈), haloalcénylthio en (C₂-C₈), haloalcynylthio en (C₂-C₈), alkylsulfinyle en (C₁-C₈), alcénylsulfinyle en (C₂-C₈), alcynylsulfinyle en (C₂-C₈), haloalkylsulfinyle en (C₁-C₈), haloalcénylsulfinyle en (C₂-C₈), haloalcynyl-sulfinyle en (C₂-C₈), alkylsulfonyle en (C₁-C₈), alcénylsulfonyle en (C₂-C₈), alcynylsulfonyle en (C₂-C₈), haloalkylsulfonyle en (C₁-C₈), haloalcénylsulfonyle en (C₂-C₈), haloalcynylsulfonyle en (C₂-C₈), les radicaux de formule -NR*R**, R* et R** étant définis ci-après, et cycloalkyle en (C₃-C₈), cycloalcényle en (C₅-C₈), cycloalcynyle en (C₅-C₈), cycloalkyle en (C₃-C₈)-alcoxy en (C₁-C₆), cycloalkyle en (C₃-C₈)-alkyle en (C₁-C₆)-S(O)ₚ-, cycloalcényle en (C₅-C₈)-alcoxy en (C₁-C₆), cycloalcényle en (C₅-C₈) -alkyle en (C₁-C₆)-S(O)ₚ-, cycloalcynyle en (C₅-C₈)-alcoxy en (C₁-C₆), cycloalcynyle en (C₅-C₈)-alkyle en (C₁-C₆)-S(O)ₚ-, cycloalcoxy en (C₃-C₈), cycloalkyle en (C₃-C₈)-S(O)ₚ-, cycloalcényloxy en (C₅-C₈), cycloalcényle en (C₅-C₈)-S(O)ₚ-, cycloalcynyloxy en (C₅-C₈), cycloalcynyle en (C₅-C₈)-S(O)ₚ-, cycloalcoxy en (C₃-C₈)-alcoxy en (C₁-C₆), cycloalcoxy en (C₃-C₈)-alkyle en (C₁-C₆)-S(O)ₚ-, phényle, phényl-alcoxy en (C₁-C₆), phénoxy, phényl-S(O)ₚ-, phényl-alkyle en (C₁-C₆)-S(O)ₚ-, phénoxy-alcoxy en (C₁-C₆), phénoxy-alkyle en (C₁-C₆)-S(O)ₚ-, un radical Het¹, Het¹-S(O)ₚ-, Het¹-alcoxy en (C₁-C₆), Het¹-O-, Het¹-O-alcoxy en (C₁-C₆), le radical hétérocyclique Het¹ étant défini ci-après,
chacun des 29 derniers radicaux cités étant non substitués dans la partie acyclique ou substitués par un ou plusieurs radicaux R^{A} identiques ou différents, et non substitués dans la partie cyclique ou substitués par un ou plusieurs radicaux R^{B} identiques ou différents, et les p signifiant chacun indépendamment les uns des autres 0, 1 ou 2,
(b) les radicaux des formules -C(=O)-R^{c}, -C(=O)-O-R^{c}, -O-C(=O)-R^{c}, -O-C(=O)-O-R^{c}, -C(=O)-S-R^{c}, -C(=S)-S-R^{c}, -C(=S)-S-R^{c}, -C(=O)-NR*R**, -C(=O)-O-NR*R**, -O-C(=O)-NR*R**, -N(R*)-C(=O)-R^{c}, -N(R*)-C(=O)-NR*R**, -N(R*)-C(=O)-O-R^{c}, -P(=O)(R^{c})(R^{D}), -P(=O)(OR^{c})(R^{d}),-P(=O)(OR^{c})(OR^{d}) ou -O-P(=O)(OR^{c})(OR^{d}),
R*, R**, R^{C} et R^{D} étant tels que définis ci-après,
(c) les radicaux des formules -SiR'₃, -O-SiR'₃, (R')₃Si-alcoxy en (C₁-C₆), -CO-O-NR'₂, -O-N=CR'₂,-N=CR'₂, -O-NR₂ -CH(OR')₂ et -O-(CH₂)_{q}-CH(OR')₂, dans lesquelles chacun des radicaux R' signifient indépendamment les uns des autres H, alkyle en (C₁-C₄) ou phényle, qui est non substitué ou substitué par un ou plusieurs radicaux du groupe constitué par halogène, alkyle en (C₁-C₄), alcoxy en (C₁-C₄), haloalkyle en (C₁-C₄), haloalcoxy en (C₁-C₄) et nitro, ou substitué sur deux positions voisines par un pont alkylène en (C₂-C₆), et q signifie un nombre entier de 0 à 6, et
(d) les radicaux de formule R"O-CHR"'CH(OR")-alcoxy en (C₁-C₆), dans laquelle chacun des radicaux R" signifient indépendamment les uns des autres H ou alkyle en (C₁-C₄) ou signifient ensemble un groupe alkylène en (C₁-C₆), et R"' signifie H ou alkyle en (C₁-C₄),
ou
R¹ signifie cycloalkyle en (C₃-C₉), cycloalcényle en (C₅-C₉), cycloalcynyle en (C₅-C₉) ou phényle, chacun des 4 derniers radicaux cités étant non substitués ou substitués par un ou plusieurs radicaux du groupe constitué par les radicaux [sous-groupes (a') à (e')]
(a') halogène, cyano, thio, nitro, hydroxy, carboxy, alkyle en (C₁-C₈), haloalkyle en (C₁-C₈), alcoxy en (C₁-C₄)-alkyle en (C₁-C₄), alcényle en (C₂-C₈), haloalcényle en (C₂-C₈), alcynyle en (C₂-C₈), haloalcynyle en (C₂-C₈), alcoxy en (C₁-C₈), alcényloxy en (C₂-C₈), alcynyloxy en (C₂-C₈), haloalcoxy en (C₁-C₈), alcoxy en (C₁-C₄)-alcoxy en (C₁-C₄), alkylthio en (C₁-C₈), alcénylthio en (C₂-C₈), alcynylthio en (C₂-C₈) et les radicaux de formule -NR*R**, les radicaux R* et R** étant définis ci-après,
(b') les radicaux des formules -C(=O)-R^{c}, -C(=O)-O-R^{c}, -O-C(=O)-R^{c}, -O-C(=O)-O-R^{c}, -C(=O)-S-R^{c}, -C(=S)-S-R^{c}, -C(=S)-S-R^{c}, -C(=O)-NR*R**, -C(=O)-O-NR*R**, -O-C(=O)-NR*R**, -N(R*)-C(=O)-R^{c}, -N(R*)-C(=O)-NR*R**, -N(R*)-C(=O)-O-R^{c}, -P(=O)(R^{c})(R^{D}), -P(=O)(OR^{c})(R^{d}),-P(=O)(OR^{c})(OR^{d}) ou -O-P(=O)(OR^{c})(OR^{d}),
dans lesquelles R*, R**, R^{C} et R^{D} sont tels que définis ci-après,
(c') les radicaux des formules -SiR'₃, -O-SiR'₃, (R')₃Si-alcoxy en (C₁-C₆), -CO-O-NR'₂, -O-N=CR'₂,-N=CR'₂, -O-NR₂, -CH(OR')₂ et -O-(CH₂)_{q}-CH(OR')₂, dans lesquelles chacun des radicaux R' signifient indépendamment les uns des autres H, alkyle en (C₁-C₄) ou phényle, qui est non substitué ou substitué par un ou plusieurs radicaux du groupe constitué par halogène, alkyle en (C₁-C₄), alcoxy en (C₁-C₄), haloalkyle en (C₁-C₄), haloalcoxy en (C₁-C₄) et nitro, ou substitué sur deux positions voisines par un pont alkylène en (C₂-C₆), et q signifie un nombre entier de 0 à 6, et
(d') les radicaux de formule R"O-CHR"'CH(OR")-alcoxy en (C₁-C₆), dans laquelle chacun des radicaux R" signifient indépendamment les uns des autres H ou alkyle en (C₁-C₄) ou signifient ensemble un groupe alkylène en (C₁-C₆), et R"' signifie H ou alkyle en (C₁-C₄), et
(e') un radical de formule Het¹, qui est non substitué ou substitué par un ou plusieurs radicaux R^{B} identiques ou différents,
ou
R¹ signifie un radical polycyclique à base de cycloalkyle en (C₃-C₉) , cycloalcényle en (C₅-C₉), cycloalcynyle en (C₅-C₉) ou phényle, le cycle de base étant condensé avec un cycle carbocyclique ou hétérocyclique, de préférence un cycle à 5 ou 6 chaînons contenant 0 ou 1 à 3 hétéroatomes de cycle du groupe constitué par N, O et S, de préférence benzocondensé, et le cycle de base ou le système polycyclique étant non substitué ou substitué par un ou plusieurs radicaux R^{B} identiques ou différents, de préférence non substitué ou substitué par un ou plusieurs radicaux du groupe constitué par halogène, cyano, nitro, hydroxy, carboxy, alkyle en (C₁-C₆), haloalkyle en (C₁-C₆), alcoxy en (C₁-C₄) -alkyle en (C₁-C₄), alcényle en (C₂-C₆) , haloalcényle en (C₂-C₆), alcynyle en (C₂-C₆), haloalcynyle en (C₂-C₆), alcoxy en (C₁-C₆), alcényloxy en (C₂-C₆), alcynyloxy en (C₂-C₆), haloalcoxy en (C₁-C₆), alcoxy en (C₁-C₄) -alcoxy en (C₁-C₄), alkylthio en (C₁-C₆), alcénylthio en (C₂-C₆), alcynylthio en (C₂-C₆), cycloalkyle en (C₃-C₆), cycloalcoxy en (C₃-C₆), [alcoxy en (C₁-C₈)]-carbonyle, [haloalcoxy en (C₁-C₆)]-carbonyle et oxo,
ou
R¹ signifie un radical hétérocyclique Het¹, qui est non substitué dans le cycle ou dans le système polycyclique ou substitué par un ou plusieurs radicaux R^{B} identiques ou différents, de préférence non substitué ou substitué par un ou plusieurs radicaux du groupe constitué par halogène, cyano, thio, nitro, hydroxy, carboxy, alkyle en (C₁-C₆), haloalkyle en (C₁-C₆), alcoxy en (C₁-C₄)-alkyle en (C₁-C₄), alcényle en (C₂-C₆), haloalcényle en (C₂-C₆), alcynyle en (C₂-C₆), haloalcynyle en (C₂-C₆), alcoxy en (C₁-C₆), alcényloxy en (C₂-C₆), alcynyloxy en (C₂-C₆), haloalcoxy en (C₁-C₆), alcoxy en (C₁-C₄)-alcoxy en (C₁-C₄), alkylthio en (C₁-C₆), alcénylthio en (C₂-C₆), alcynylthio en (C₂-C₆), cycloalkyle en (C₃-C₆), cycloalcoxy en (C₃-C₆), [alcoxy en (C₁-C₈)]-carbonyle, [haloalcoxy en (C₁-C₆)]-carbonyle et oxo,
dans les radicaux susmentionnés et suivants,
les Het¹ signifiant chacun indépendamment les uns des autres un radical hétérocyclyle monocyclique saturé, partiellement insaturé ou hétéroaromatique contenant 3 à 9 atomes de cycle, de préférence contenant 5 ou 6 atomes de cycle, ou un hétérocycle bicyclique à 9 ou 10 chaînons, contenant 1, 2, 3 ou 4 hétéroatomes choisis dans le groupe constitué par O, N et S, de préférence un hétérocycle à 5 ou 6 chaînons contenant 1 à 3 hétéroatomes de cycle du groupe constitué par N, O et S, qui est éventuellement également condensé avec un cycle carbocyclique ou hétérocyclique, de préférence un cycle carbocyclique contenant 3 à 6 atomes C ou un cycle hétérocyclique contenant 5 ou 6 atomes de cycle, et 1 à 3 hétéroatomes de cycle du groupe constitué par N, O et S, de préférence éventuellement benzocondensé,
R*, R** signifiant chacun indépendamment l'un de l'autre (c.-à-d. également d'autres groupes NR*R**) H, alkyle en (C₁-C₆), alcényle en (C₂-C₈), alcynyle en (C₂-C₃), alcoxy en (C₁-C₄)-alkyle en (C₁-C₄), alcanoyle en (C₁-C₆), [haloalkyle en (C₁-C₄)]-carbonyle, [alcoxy en (C₁-C₄)] -carbonyle, [haloalcoxy en (C₁-C₄)]-carbonyle, cycloalkyle en (C₃-C₆), cycloalkyle en (C₃-C₆)-alkyle en (C₁-C₄), phényle, phényl-alkyle en (C₁-C₄), chacun des 4 derniers radicaux cités étant éventuellement substitués dans le cycle par un ou plusieurs radicaux R^{bb} identiques ou différents, ou
R* et R** signifiant ensemble avec l'atome N un hétérocycle de 3 à 8 chaînons, qui peut contenir en plus de l'atome N un ou deux hétéroatomes de cycle supplémentaires du groupe constitué par N, O et S, et qui est non substitué ou substitué par un ou plusieurs radicaux du groupe constitué par alkyle en (C₁-C₄), haloalkyle en (C₁-C₄) et oxo,
R^{A} signifiant halogène, cyano, hydroxy ou alcoxy en (C₁-C₆),
R^{B} signifiant halogène, cyano, hydroxy, oxo, nitro, alkyle en (C₁-C₆), haloalkyle en (C₁-C₄), cyano-alkyle en (C₁-C₄), hydroxy-alkyle en (C₁-C₄), nitro-alkyle en (C₁-C₄), alcényle en (C₂-C₆), haloalcényle en (C₂-C₆), alcynyle en (C₂-C₆), haloalcynyle en (C₂-C₆), alcoxy en (C₁-C₆), alcényloxy en (C₂-C₆), alcynyloxy en (C₂-C₆), haloalcoxy en (C₁-C₆), alcoxy en (C₁-C₄) -alkyle en (C₁-C₄), alcoxy en (C₁-C₄)-alcoxy en (C₁-C₄), haloalcoxy en (C₁-C₄) -alkyle en (C₁-C₄), haloalcoxy en (C₁-C₄) -alcoxy en (C₁-C₄), alkylthio en (C₁-C₆), alcénylthio en (C₂-C₆), alcynylthio en (C₂-C₆), alkylsulfinyle en (C₁-C₆), haloalkylsulfinyle en (C₁-C₆), alkylsulfonyle en (C₁-C₆), haloalkylsulfonyle en (C₁-C₆), un radical de formule R^{aa}-C(=O)- ou R^{aa}-C(=O)-alkyle en (C₁-C₆), les R^{aa} étant définis ci-après, -NR*R**, R* et R** étant définis ci-après, tri-[alkyle en (C₁-C₄)]-silyle, tri-[alkyle en (C₁-C₄)]-silyl-alkyle en (C₁-C₆), cycloalkyle en (C₃-C₆), cycloalcoxy en (C₃-C₆), cycloalkyle en (C₃-C₆)-alkyle en (C₁-C₄), cycloalkyle en (C₃-C₆)-alcoxy en (C₁-C₈), phényle, phényl-alkyle en (C₁-C₆), phénoxy, phénoxy-alkyle en (C₁-C₆), phénylamino, phénylamino-alkyle en (C₁-C₆) ou un hétérocycle monocyclique à 5 ou 6 chaînons ou bicyclique à 9 ou 10 chaînons, contenant 1, 2, 3 ou 4 hétéroatomes choisis dans le groupe constitué par O, N et S, chacun des 11 derniers radicaux cités étant éventuellement substitués dans la partie cyclique par un ou plusieurs radicaux R^{bb} identiques ou différents,
R^{C}, R^{D} signifiant chacun indépendamment l'un de l'autre (également indépendamment de radicaux R^{C}, R^{D} dans d'autres groupes) hydrogène, alkyle en (C₁-C₈), alcényle en (C₂-C₈) ou alcynyle en (C₂-C₈), chacun des 3 derniers radicaux cités étant non substitués ou substitués par un ou plusieurs radicaux du groupe constitué par halogène, cyano, nitro, hydroxy, alcoxy en (C₁-C₆), alcényloxy en (C₂-C₆), alcynyloxy en (C₂-C₆), haloalcoxy en (C₁-C₈), alcoxy en (C₁-C₄)-alcoxy en (C₁-C₄), alkylthio en (C₁-C₈), haloalkylthio en (C₁-C₈), alkylsulfinyle en (C₁-C₈), haloalkylsulfinyle en (C₁-C₈), alkylsulfonyle en (C₁-C₈), haloalkylsulfonyle en (C₁-C₆) et tri-[alkyle en (C₁-C₄)]-silyle,
ou
cycloalkyle en (C₃-C₈), cycloalcényle en (C₅-C₈), cycloalcynyle en (C₅-C₈), phényle, cycloalkyle en (C₃-C₈) -alkyle en (C₁-C₆), cycloalcényle en (C₅-C₈)-alkyle en (C₁-C₆), cycloalcynyle en (C₅-C₈)-alkyle en (C₁-C₆), phényl-alkyle en (C₁-C₆), cycloalkyloxy en (C₃-C₈)-alkyle en (C₁-C₆), cycloalkyle en (C₃-C₈) -S(O)ₚ-alkyle en (C₁-C₆), cycloalcényloxy en (C₅-C₈)-alkyle en (C₁-C₆), cycloalcynyloxy en (C₅-C₈)-alkyle en (C₁-C₆), phénoxy-alkyle en (C₁-C₆), phényl-S(O)ₚ-alkyle en (C₁-C₆), cycloalkylamino en (C₃-C₈)-alkyle en (C₁-C₆), cycloalcénylamino en (C₅-C₈)-alkyle en (C₁-C₆), cycloalcynylamino en (C₅-C₈)-alkyle en (C₁-C₆), phénylamino-alkyle en (C₁-C₆), Het¹, Het¹-alkyle en (C₁-C₆), Het¹-O-alkyle en (C₁-C₆)ou Het¹-S(O)ₚ-alkyle en (C₁-C₆), Het¹ ayant la signification indiquée,
chacun des 22 derniers radicaux cités étant non substitués dans la partie acyclique ou substitués par un ou plusieurs radicaux R^{A} identiques ou différents, et non substitués dans la partie cyclique ou substitués par un ou plusieurs radicaux R^{B} identiques ou différents, et les p signifiant chacun indépendamment les uns des autres 0, 1 ou 2,
les R^{aa} signifiant chacun indépendamment les uns des autres hydrogène, OH, alkyle en (C₁-C₆), haloalkyle en (C₁-C₄), alcényle en (C₂-C₆), alcynyle en (C₂-C₆), alcoxy en (C₁-C₆), alcoxy en (C₁-C₆)-alkyle en (C₁-C₆), alcoxy en (C₁-C₆)-alkyloxy en (C₁-C₆), haloalcoxy en (C₁-C₄), haloalcoxy en (C₁-C₄)-alkyle en (C₁-C₆), haloalcoxy en (C₁-C₄)-alcoxy en (C₁-C₆), alcényloxy en (C₃-C₆), alcényloxy en (C₃-C₆)-alkyle en (C₁-C₆), alcényloxy en (C₃-C₆)-alcoxy en (C₁-C₆), alcynyloxy en (C₃-C₆), alcynyloxy en (C₃-C₆)-alkyle en (C₁-C₆), alcynyloxy en (C₃-C₆)-alcoxy en (C₁-C₆), -NR*R**, R* et R** étant tels que définis précédemment, tri-[alkyle en (C₁-C₄)]-silyle, tri-[alkyle en (C₁-C₄)]-silyl-alkyle en (C₁-C₆), tri-[alkyle en (C₁-C₄)]-silyl-alcoxy en (C₁-C₆), cycloalkyle en (C₃-C₆), cycloalcoxy en (C₃-C₆), cycloalkyle en (C₃-C₆)-alkyle en (C₁-C₆), cycloalkyle en (C₃-C₆)-alcoxy en (C₁-C₈), cycloalcényle en (C₃-C₆), cycloalcényle en (C₅-C₆)-alkyle en (C₁-C₆), cycloalcényloxy en (C₅-C₆), cycloalcynyle en (C₅-C₆), cycloalcynyle en (C₃-C₆)-alkyle en (C₁-C₆), cycloalcynyle en (C₅-C₆)-alcoxy en (C₁-C₆), phényle, phényl-alkyle en (C₁-C₆), phényl-alcoxy en (C₁-C₆), phénoxy, phénoxy-alkyle en (C₁-C₆), phénoxy-alcoxy en (C₁-C₆), phénylthio, phényl-S(O)ₚ-alkyle en (C₁-C₆), phényl-S(O)ₚ-alcoxy en (C₁-C₆), les p signifiant chacun indépendamment les uns des autres 0, 1 ou 2, phénylamino, phénylamino-alkyle en (C₁-C₆), phénylamino-alcoxy en (C₁-C₆)ou un hétérocycle monocyclique à 5 ou 6 chaînons ou bicyclique à 9 ou 10 chaînons éventuellement relié par un groupe alkylène ou un groupe alcoxy, contenant 1, 2, 3 ou 4 hétéroatomes choisis dans le groupe constitué par O, N et S, chacun des 20 derniers radicaux cités étant éventuellement substitués dans la partie cyclique par un ou plusieurs radicaux R^{bb} identiques ou différents, et
les R^{bb} signifiant chacun indépendamment les uns des autres halogène, alkyle en (C₁-C₄), haloalkyle en (C₁-C₄), alcoxy en (C₁-C₄) ou haloalcoxy en (C₁-C₄) .

4. Composés ou leurs sels selon l'une quelconque des revendications 1 à 3, **caractérisés en ce que**
(R²)ₙ signifie n substituants R²,
R² (lorsque n = 1) ou chacun des substituants R² (lorsque n est supérieur à 1) signifiant indépendamment les uns des autres halogène, cyano, nitro, alkyle en (C₁-C₆), alcoxy en (C₁-C₆), alkylthio en (C₁-C₆), alkylsulfinyle en (C₁-C₆), alkylsulfonyle en (C₁-C₆), haloalkyle en (C₁-C₆), haloalcoxy en (C₁-C₆), haloalkylthio en (C₁-C₆), haloalkylsulfinyle en (C₁-C₆), haloalkylsulfonyle en (C₁-C₆), alcényle en (C₂-C₆), alcynyle en (C₂-C₆), tri-[alkyle en (C₁-C₄)]-silyle ou tri-[alkyle en (C₁-C₄)]-silyl-alkyle en (C₁-C₄),
ou deux groupes R² en position ortho sur le cycle signifiant ensemble un groupe de formule -Z¹-A*-Z², dans laquelle
A* représente un groupe alkylène de 1 à 4 atomes C, qui est éventuellement substitué par un ou plusieurs radicaux du groupe constitué par halogène, alkyle en (C₁-C₄), haloalkyle en (C₁-C₄), alcoxy en (C₁-C₄) et haloalcoxy en (C₁-C₄),
Z¹ représente une liaison directe, O ou S, et
Z² représente une liaison directe, O ou S,
le groupe -Z¹-A*-Z² formant ensemble avec les atomes C du cycle phényle reliés au groupe un cycle à 5 ou 6 condensé, et
(R³)ₘ signifie m substituants R³,
R³ (lorsque m = 1) ou chacun des substituants R³ (lorsque m est supérieur à 1) signifiant indépendamment les uns des autres halogène, cyano, nitro, alkyle en (C₁-C₆), alcoxy en (C₁-C₆), alkylthio en (C₁-C₆), alkylsulfinyle en (C₁-C₆), alkylsulfonyle en (C₁-C₆), haloalkyle en (C₁-C₆), haloalcoxy en (C₁-C₆), haloalkylthio en (C₁-C₆), haloalkylsulfinyle en (C₁-C₆), haloalkylsulfonyle en (C₁-C₆), alcényle en (C₂-C₆), alcynyle en (C₂-C₆), NR*R**, tri-[alkyle en (C₁-C₄)]-silyle ou tri- [alkyle en (C₁-C₄)]-silyl-alkyle en (C₁-C₄),
ou deux groupes R³ en position ortho sur le cycle signifiant ensemble un groupe de formule -Z³-A**-Z⁴, dans laquelle
A** représente un groupe alkylène de 1 à 4 atomes C, qui est éventuellement substitué par un ou plusieurs radicaux du groupe constitué par halogène, alkyle en (C₁-C₄), haloalkyle en (C₁-C₄), alcoxy en (C₁-C₄) et haloalcoxy en (C₁-C₄),
Z³ représente une liaison directe, O ou S, et
Z⁴ représente une liaison directe, O ou S,
le groupe -Z³-A**-Z⁴ formant ensemble avec les atomes C du cycle phényle reliés au groupe un cycle à 5 ou 6 condensé,
R*, R** ayant chacun indépendamment l'un de l'autre ou ensemble avec l'atome N la signification indiquée, et
n, m signifiant chacun indépendamment l'un de l'autre 0, 1, 2, 3, 4 ou 5, de préférence 0, 1, 2 ou 3, notamment 0, 1 ou 2, à condition que les deux ne signifient pas simultanément 0.

5. Composés ou leurs sels selon l'une quelconque des revendications 1 à 4, **caractérisés en ce que**
(R²)ₙ signifie n substituants R²,
dans le cas où n = 1, le substituant R² ou, dans le cas où n est supérieur à 1, chacun des substituants R² signifiant indépendamment les uns des autres halogène, cyano, nitro, alkyle en (C₁-C₄), alcoxy en (C₁-C₄), alkylthio en (C₁-C₄), alkylsulfinyle en (C₁-C₄), alkylsulfonyle en (C₁-C₄), haloalkyle en (C₁-C₄), haloalcoxy en (C₁-C₄), haloalkylthio en (C₁-C₄), haloalkylsulfinyle en (C₁-C₄), haloalkylsulfonyle en (C₁-C₄), alcényle en (C₂-C₄), alcynyle en (C₂-C₄), tri-[alkyle en (C₁-C₄)]-silyle ou tri-[alkyle en (C₁-C₄)]-silyl-alkyle en (C₁-C₄), et
n signifiant 0, 1, 2, 3, 4 ou 5, de préférence 0, 1, 2 ou 3, notamment 0, 1 ou 2, à condition que n et m ne signifient pas tous les deux simultanément 0.

6. Composés ou leurs sels selon l'une quelconque des revendications 1 à 5, **caractérisés en ce que**
(R³)ₘ signifie m substituants R³,
dans le cas où m = 1, le substituant R³ ou, dans le cas où m est supérieur à 1, chacun des substituants R³ signifiant indépendamment les uns des autres halogène, cyano, nitro, alkyle en (C₁-C₄), alcoxy en (C₁-C₄), alkylthio en (C₁-C₄), alkylsulfinyle en (C₁-C₄), alkylsulfonyle en (C₁-C₄), haloalkyle en (C₁-C₄), haloalcoxy en (C₁-C₄), haloalkylthio en (C₁-C₄), haloalkylsulfinyle en (C₁-C₄), haloalkylsulfonyle en (C₁-C₄), alcényle en (C₂-C₆), alcynyle en (C₂-C₆) ou tri-[alkyle en (C₁-C₄)]-silyle-Z^{b}-, avec Z^{b} = une liaison covalente ou alkylène en (C₁-C₄), ou
deux groupes R³ en position ortho sur le cycle signifiant ensemble un groupe de formule -Z³-A**-Z⁴, dans laquelle
A** représente un groupe alkylène qui est éventuellement substitué par un ou plusieurs radicaux du groupe constitué par halogène, alkyle en (C₁-C₄), haloalkyle en (C₁-C₄), alcoxy en (C₁-C₄) et haloalcoxy en (C₁-C₄),
Z³ représente O ou S, et
Z⁴ représente O ou S, le groupe -Z³-A**-Z⁴ formant ensemble avec les atomes C du cycle phényle reliés au groupe un cycle à 5 ou 6 condensé, et
m signifiant 0, 1, 2, 3, 4 ou 5, de préférence 0, 1, 2, 3 ou 4, notamment 0, 1, 2 ou 3, à condition que n et m dans la formule (I) ne signifient pas tous les deux simultanément 0.

7. Composés ou leurs sels selon l'une quelconque des revendications 1 à 6, **caractérisés en ce que**
(R²)ₙ signifie n substituants R²,
dans le cas où n = 1, le substituant R² ou, dans le cas où n est supérieur à 1, chacun des substituants R² signifiant indépendamment les uns des autres fluor, chlore, brome, iode, cyano, nitro, méthyle, éthyle, méthoxy, méthylthio, méthylsulfinyle, méthylsulfonyle, trifluorométhyle, trifluorométhoxy, trifluoroalkylthio, trifluorométhylsulfinyle ou trifluorométhylsulfonyle, et
(R³)ₘ signifie m substituants R³,
dans le cas où m = 1, le substituant R³ ou, dans le cas où m est supérieur à 1, chacun des substituants R³ signifiant indépendamment les uns des autres halogène, cyano, nitro, méthyle, méthoxy, méthylthio, méthylsulfinyle, méthylsulfonyle, trifluorométhyle, trifluorométhoxy, trifluoroalkylthio, trifluorométhylsulfinyle ou trifluorométhylsulfonyle, et
m signifiant 0, 1, 2, 3, 4 ou 5, de préférence 0, 1, 2, 3 ou 4, notamment 0, 1, 2 ou 3, et
n signifiant 0, 1, 2, 3, 4 ou 5, de préférence 0, 1, 2 ou 3, notamment 0, 1 ou 2,
à condition que n et m ne signifient pas tous les deux simultanément 0.

8. Composés ou leurs sels selon l'une quelconque des revendications 1 à 7, **caractérisés en ce que**
(R²)ₙ signifie 2-bromo, 3-bromo, 4-bromo, 2-chloro, 3-chloro, 4-chloro, 2-fluoro, 3-fluoro, 4-fluoro, 2-cyano, 3-cyano, 4-cyano, 2-méthyle, 3-méthyle, 4-méthyle, 2-éthyle, 3-éthyle, 4-éthyle, 2-CF₃, 3-CF₃, 4-CF₃, 2-méthoxy, 3-méthoxy, 4-méthoxy, 2-éthoxy, 3-éthoxy, 4-éthoxy, 2-méthylthio, 3-méthylthio, 4-méthylthio, 2-méthylsulfinyle, 3-méthylsulfinyle, 4-méthylsulfonyle, 2-méthylsulfonyle, 3-méthylsulfonyle, 4-méthylsulfonyle, 2,3-diméthyle, 2,4-diméthyle, 2,5-diméthyle, 2,6-diméthyle, 3,4-diméthyle, 3,5-diméthyle, 2,3-difluoro, 2,4-difluoro, 2,5-difluoro, 2,6-difluoro, 3,4-difluoro, 3,5-difluoro, 2,3-dichloro, 2,4-dichloro, 2,5-dichloro, 2,6-dichloro, 3,4-dichloro, 3,5-dichloro, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), (4-Br-3-F), (3-Br-4-F), (3-Br-5-F), (4-Br-3-Cl), (3-Br-4-Cl), (4-CN-3-F), (3-CN-4-F), (3-CN-5-F), (4-CN-3-Cl), (3-CN-4-Cl), 2,3,4-trifluoro, 2,3,5-trifluoro, 2,3,6-trifluoro, 2,4,6-trifluoro, 3,4,5-trifluoro, 2,3,4-trichloro, 2,3,5-trichloro, 2,3,6-trichloro, 2,4,6-trichloro ou 3,4,5-trichloro et
(R³)ₘ signifie 2-bromo, 3-bromo, 4-bromo, 2-chloro, 3-chloro, 4-chloro, 2-fluoro, 3-fluoro, 4-fluoro, 2-cyano, 3-cyano, 4-cyano, 2-méthyle, 3-méthyle, 4-méthyle, 2-éthyle, 3-éthyle, 4-éthyle, 2-CF₃, 3-CF₃, 4-CF₃, 2-méthoxy, 3-méthoxy, 4-méthoxy, 2-éthoxy, 3-éthoxy, 4-éthoxy, 2-méthylthio, 3-méthylthio, 4-méthylthio, 2-méthylsulfinyle, 3-méthylsulfinyle, 4-méthylsulfonyle, 2-méthylsulfonyle, 3-méthylsulfonyle, 4-méthylsulfonyle, 2-nitro, 3-nitro, 4-nitro, 2,3-diméthyle, 2,4-diméthyle, 2,5-diméthyle, 2,6-diméthyle, 3,4-diméthyle, 3,5-diméthyle, 2,3-difluoro, 2,4-difluoro, 2,5-difluoro, 2,6-difluoro, 3,4-difluoro, 3,5-difluoro, 2,3-dichloro, 2,4-dichloro, 2,5-dichloro, 2,6-dichloro, 3,4-dichloro, 3,5-dichloro, 2,5-dicyano, 2,6-dicyano, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), (4-Br-2-F), (4-Br-3-F), (4-CN-3-F), (4-NO₂-3-F), (4-OMe-3-F), (3-CN-4-F), (3-NO₂-4-F), (3-CN-4-Cl), (3-NO₂-4-Cl), (5-CN-2-F), 2,3,4-trifluoro, 2,3,5-trifluoro, 2,3,6-trifluoro, 2,4,6-trifluoro, 3,4,5-trifluoro, 2,3,4-trichloro, 2,3,5-trichloro, 2,3,6-trichloro, 2,4,6-trichloro ou 3,4,5-trichloro.

9. Procédé de fabrication d'un composé de formule (I) tel que défini dans l'une quelconque des revendications 1 à 8, ou son sel, **caractérisé en ce que**
(a) des composés de formule (II) 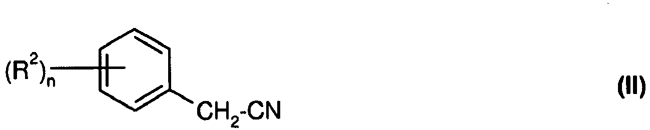 sont mis en réaction avec des composés de formule (III) ou leurs sels 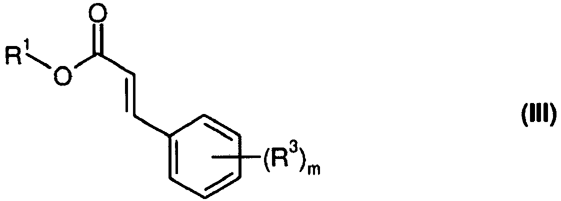 pour former des composés de formule (I') 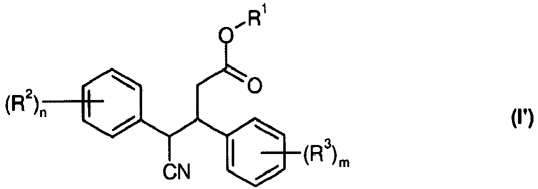 R¹, R², R³, m et n dans les composés (II), (III) et (I') étant tels que définis dans le composé de formule (I) à fabriquer respectif, et l'isomère (3R,4R)-thréo est isolé à partir du mélange d'isomères (I') d'une manière analogue à des procédés de séparation généraux,
ou
(b) des composés de formule (I") 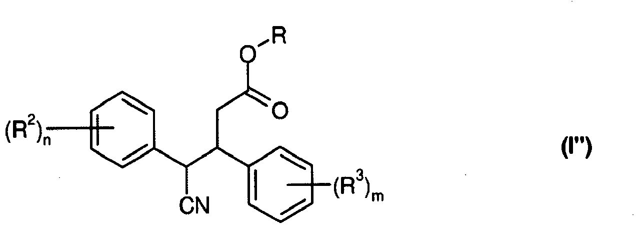 dans laquelle R signifie un radical du groupe des radicaux possibles pour R¹, mais différent du radical R¹ dans le composé (I') à fabriquer, sont mis en réaction avec un composé de formule R¹-OH, dans laquelle R¹ est tel que défini dans les composés de formule (I) à fabriquer, pour former le composé (I'), R², R³, m et n dans le composé (I") étant tels que définis dans le composé de formule (I) à fabriquer respectif, et l'isomère (3R,4R)-thréo est isolé à partir du mélange d'isomères (I') d'une manière analogue à des procédés de séparation généraux,
ou
(c) des composés enrichis stéréochimiquement de l'isomère (3R,4R)-thréo de formule (I"') 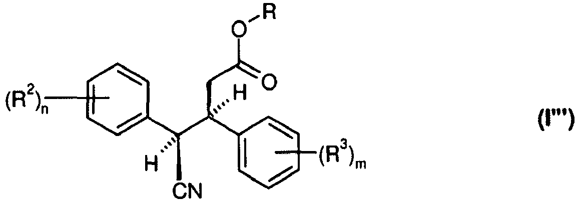 dans laquelle R signifie un radical du groupe des radicaux possibles pour R¹, mais différent du radical R¹ dans le composé (I) à fabriquer, sont mis en réaction avec un composé de formule R¹-OH, dans laquelle R¹ est tel que défini dans le composé de formule (I) à fabriquer, pour former le composé (I), R², R³, m et n dans le composé (I''') étant tels que définis dans le composé de formule (I) à fabriquer.

10. Agent herbicide ou régulant la croissance des plantes, **caractérisé en ce qu'**il contient un ou plusieurs composés de formule (I) ou leurs sels, tels que définis dans l'une quelconque des revendications 1 à 8, et des adjuvants de formulation usuels dans la protection des plantes.

11. Procédé de lutte contre des plantes nuisibles ou de régulation de la croissance de plantes, **caractérisé en ce qu'**une quantité efficace d'un ou de plusieurs composés de formule (I) ou leurs sels, tels que définis dans l'une quelconque des revendications 1 à 7, est appliquée sur les plantes, les graines des plantes, le sol dans lequel ou sur lequel les plantes poussent ou sur la surface cultivée.

12. Procédé selon la revendication 11, **caractérisé en ce que** les composés de formule (I) ou leurs sels sont utilisés pour la lutte contre des plantes nuisibles ou pour la régulation de la croissance dans des cultures de plantes utiles ou ornementales.

13. Procédé selon la revendication 12, **caractérisé en ce que** les plantes cultivées sont des plantes cultivées transgéniques.

14. Utilisation des composés de formule (I) ou leurs sels selon l'une quelconque des revendications 1 à 8 en tant qu'herbicides ou régulateurs de la croissance de plantes.

15. Utilisation selon la revendication 14, **caractérisée en ce que** les composés sont utilisés dans des cultures de plantes utiles ou ornementales.
